(19) Europäisches Patentamt
European Patent Office
Office européen des brevets

(11) EP 1 047 684 B1

(12) **EUROPÄISCHE PATENTSCHRIFT**

(45) Veröffentlichungstag und Bekanntmachung des Hinweises auf die Patenterteilung:
**02.10.2002 Patentblatt 2002/40**

(21) Anmeldenummer: **99904767.3**

(22) Anmeldetag: **12.01.1999**

(51) Int Cl.[7]: **C07D 307/88**, A61K 31/365, C07D 405/10, C07D 409/06, C07D 407/10, C07D 401/10, C07D 405/12, C07D 417/12, C07D 407/12, C07D 405/06, C07D 409/12, C07D 413/12, C07D 417/10, C07D 413/10, C07D 413/14

(86) Internationale Anmeldenummer:
**PCT/EP99/00132**

(87) Internationale Veröffentlichungsnummer:
**WO 99/036416 (22.07.1999 Gazette 1999/29)**

(54) **SUBSTITUIERTE ALPHA,BETA-ANELLIERTE BUTYROLACTONE**
SUBSTITUTED ALPHA,BETA-ANELLATED BUTYROLACTONES
ALPHA,BETA-BUTYROLACTONES ANNELEES SUBSTITUEES

(84) Benannte Vertragsstaaten:
**DE ES FR GB IT**

(30) Priorität: **17.01.1998 DE 19801646**

(43) Veröffentlichungstag der Anmeldung:
**02.11.2000 Patentblatt 2000/44**

(73) Patentinhaber: **BAYER AG**
**51368 Leverkusen (DE)**

(72) Erfinder:
- **STOLLE, Andreas**
  **D-42115 Wuppertal (DE)**
- **ANTONICEK, Horst-Peter**
  **D-51467 Bergisch Gladbach (DE)**
- **LENSKY, Stephen**
  **D-51515 Kürten (DE)**
- **VOERSTE, Arnd**
  **D-50670 Köln (DE)**
- **MÜLLER, Thomas**
  **D-53225 Bonn (DE)**
- **BAUMGARTEN, Jörg**
  **D-42115 Wuppertal (DE)**
- **VON DEM BRUCH, Karsten**
  **D-51375 Leverkusen (DE)**
- **MÜLLER, Gerhard**
  **D-51377 Leverkusen (DE)**
- **STROPP, Udo**
  **D-42781 Haan (DE)**
- **HORVATH, Ervin**
  **D-51373 Leverkusen (DE)**
- **DE VRY, Jean-Marie-Viktor**
  **D-51503 Rösrath (DE)**
- **SCHREIBER, Rudy**
  **D-50935 Köln (DE)**

(56) Entgegenhaltungen:
**EP-A- 0 774 461** **WO-A-96/07405**
**WO-A-96/15099**

- **T. HUDLICKY ET AL.: "Intramolecular Cyclopentene Annulation. 3. Synthesis and Carbon-13 Nuclear Magnetic Resonance Spectroscopy of Bicyclic Cyclopentene Lactones as Potential Perhydroazulene and/or Monoterpene Synthons" JOURNAL OF ORGANIC CHEMISTRY, Bd. 48, Nr. 20, 1983, Seiten 3422-3428, XP002105150 EASTON US**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft α,β-anellierte Butyrolactone, Verfahren zu ihrer Herstellung und ihre Verwendung als Arzneimittel

Die Aminosäure L-Glutamat ist der wichtigste erregende Neurotransmitter im Gehirn. Glutamatrezeptoren lassen sich in zwei große Klassen einteilen: 1. ionotrope Rezeptoren, die Ionenkanäle direkt steuern und 2. metabotrope Rezeptoren (mGluRs).

Metabotrope Glutamatrezeptoren sind eine heterogene Klasse von G-Proteingekoppelten Rezeptoren. Sie modulieren prä- bzw. postsynaptisch die Ausschüttung von Glutamat bzw. die Empfindlichkeit der Zelle gegenüber Glutamat. Die Effekte werden über unterschiedliche Second-Messenger-Kaskaden hervorgerufen. Diese Antwort beeinflußt wiederum die ionotropen Glutamatrezeptoren.

Derzeit kennt man 8 verschiedene Subtypen der metabotropen Glutamatrezeptoren, die sich durch Second-Messenger Kaskade, Pharmakologie und Lokalisation im Hirn unterscheiden (zur Übersicht: Arm. Rev. Pharmacol. Toxicol. 1997, 37, 205).

In J. Org. Chem. 1983, 48, 3422-3428 wird die Synthese bestimmter Tetrahydro-1H-cyclopenta*[c]*furan-1-one beschrieben.

Die vorliegende Erfindung betrifft α,β-anellierte Butyrolactone der allgemeinen Formel (I)

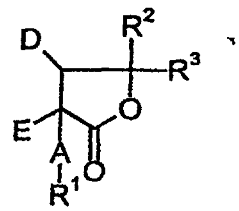

(I)

in welcher

A für Reste der Formeln $-CH_2-$, -CO-, $-CR^4(OH)-$ oder $-(CH_2)_a-CHR^5-$ steht, worin

- a eine Zahl 0, 1, 2, 3 oder 4 bedeutet,
- $R^4$ Wasserstoff oder $(C_1-C_6)$-Alkyl bedeutet

und

- $R^5$ Phenyl bedeutet,

oder

für $(C_2-C_8)$-Alkandiyl, $(C_2-C_6)$-Alkendiyl oder $(C_2-C_6)$-Alkindiyl steht,

$R^1$ für Wasserstoff, $(C_3-C_6)$-Cycloalkyl oder für einen 5- bis 6-gliedrigen Heterocyclus steht, der bis zu 3 Heteroatome aus der Reihe S, O, N und/oder einen Rest der Formel $-NR^6$ enthalten kann, worin

- $R^6$ Wasserstoff oder Methyl bedeutet,

oder

für einen 5- bis 6-gliedrigen, benzokondensierten Heterocyclus steht, der bis zu 2 Heteroatome aus der Reihe S, O, N und/oder einen Rest der Formel $-NR^7$ enthalten kann, und der sowohl über den Phenylring als auch über den Heterocyclus gebunden sein kann, worin

- $R^7$ die oben angegebene Bedeutung von $R^6$ hat und mit dieser gleich oder verschieden ist,

oder

für Reste der Formeln

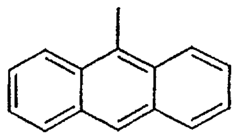
,
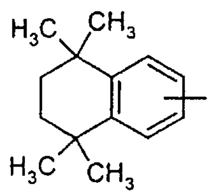
,
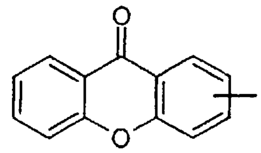
,

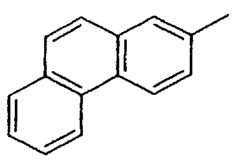
,
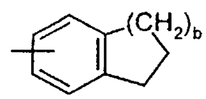
oder
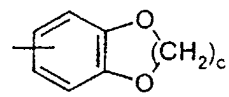

steht,

worin

b und c gleich oder verschieden sind und eine Zahl 1 oder 2 bedeuten,

oder

für $(C_6\text{-}C_{10})$-Aryl steht,

wobei alle oben aufgeführten Ringsysteme gegebenenfalls ein- bis mehrfach, gleich oder verschieden durch Substituenten, ausgewählt aus der Gruppe Halogen, Cyano, Nitro, Trifluormethyl, Hydroxy, $(C_1\text{-}C_6)$-Alkoxy, $(C_1\text{-}C_6)$-Alkyl-carbonyloxy und $(C_3\text{-}C_6)$-Cycloalkyl, Phenyl, Phenoxy, Benzyloxy und einen 5- bis 6-gliedrigen aromatischen Heterocyclus mit bis zu 3 Heteroatomen aus der Reihe S, N und/oder O substituiert sind, die ihrerseits bis zu dreifach gleich oder verschieden durch Cyano oder Halogen substituiert sein können,

und/oder durch $(C_1\text{-}C_6)$-Alkyl und $(C_2\text{-}C_6)$-Alkylen substituiert sind, die ihrerseits durch Halogen, $(C_6\text{-}C_{10})$-Aryl oder durch Reste der Formel $-SR^8$, $-OR^9$ oder $-NR^{10}R^{11}$ oder

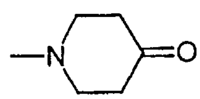

substituiert sein können,

worin

$R^8$ $(C_1\text{-}C_6)$-Alkyl oder Phenyl bedeutet,

$R^9$ Wasserstoff oder $(C_1\text{-}C_6)$-Alkyl bedeutet,

und

$R^{10}$ und $R^{11}$ gleich oder verschieden sind und Wasserstoff, Phenyl oder $(C_1\text{-}C_6)$-Alkyl bedeuten, das gegebenenfalls durch Phenyl substituiert ist, das seinerseits ein- bis mehrfach, gleich oder verschieden durch Halogen, Nitro, Hydroxy oder $(C_1\text{-}C_6)$-Alkoxy substituiert sein kann,

oder

$R^{10}$ und $R^{11}$ gemeinsam mit dem Stickstoffatom einen Rest der Formel

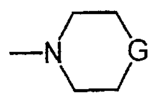

bilden,
worin

G ein Sauerstoffatom, eine $-CH_2$-Gruppe oder einen Rest der Formel $-NR^{12}$- bedeutet,
worin

$R^{12}$ Wasserstoff, Phenyl, Benzyl, ($C_1$-$C_6$)-Alkyl, ($C_1$-$C_6$)-Alkoxy-carbonyl oder einen 5- bis 6-gliedrigen aromatischen Heterocyclus mit bis zu 3 Heteroatomen aus der Reihe S, N und/oder O bedeutet,

und/oder durch Gruppen der Formeln $-CO_2-R^{13}$, $-NR^{14}R^{15}$, $-NR^{16}CO-R^{17}$, $-NR^{18}CO_2-R^{19}$ und $-CO-NR^{20}R^{21}$ substituiert sind,
worin

$R^{13}$ Wasserstoff bedeutet, oder ($C_1$-$C_9$)-Alkyl oder ($C_2$-$C_6$)-Alkenyl bedeutet, die ihrerseits durch Reste der Formeln

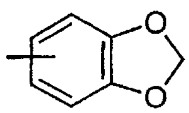 , 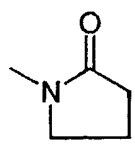 ,

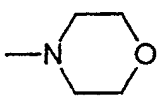 , 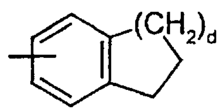

oder
($C_6$-$C_{10}$)-Aryl oder durch einen 5- bis 7-gliedrigen aromatischen Heterocyclus mit bis zu 3 Heteroatomen aus der Reihe S, N und/oder O substituiert sein können,
worin

d eine Zahl 1 oder 2 bedeutet,

oder
($C_6$-$C_{10}$)-Aryl bedeutet, das gegebenenfalls durch Phenyl substituiert ist, das seinerseits durch Cyano oder Halogen substituiert sein kann,

$R^{14}$ und $R^{15}$ gleich oder verschieden sind und Wasserstoff, ($C_3$-$C_6$)-Cycloalkyl, Phenyl oder ($C_1$-$C_6$)-Alkyl bedeuten, das gegebenenfalls durch ($C_3$-$C_6$)-Cycloalkyl oder Phenyl substituiert ist, das seinerseits ein- bis mehrfach, gleich oder verschieden durch Halogen, Hydroxy oder ($C_1$-$C_6$)-Alkoxy substituiert sein kann,

$R^{16}$ Wasserstoff oder ($C_1$-$C_6$)-Alkyl bedeutet,

$R^{17}$ Wasserstoff, Adamantyl, ($C_3$-$C_8$)-Cycloalkyl, ($C_2$-$C_6$)-Alkenyl oder ($C_1$-$C_{12}$)-Alkyl bedeutet, das gegebenenfalls
durch Adamantyl, ($C_3$-$C_6$)-Cycloalkyl, ($C_6$-$C_{10}$)-Aryl, Phenoxy oder einen 5- bis 6-gliedrigen aromatischen Heterocyclus mit bis zu 4 Heteroatomen aus der Reihe S, N und/oder O substituiert ist, wobei Aryl und der Heterocyclus ihrerseits ein- bis mehrfach, gleich oder verschieden durch ($C_1$-$C_6$)-Alkyl, ($C_1$-$C_6$)-Alkoxy, Hydroxy, Nitro oder Halogen substituiert sein können,

und/oder Alkyl gegebenenfalls durch einen Rest der Formel

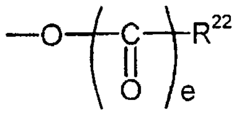

substituiert ist,
worin

e eine Zahl 0 oder 1 bedeutet und

$R^{22}$ $(C_1-C_6)$-Alkyl oder $(C_6-C_{10})$-Aryl bedeutet, das gegebenenfalls ein- bis mehrfach, gleich oder verschieden durch Halogen, Nitro, Hydroxy oder $(C_1-C_6)$-Alkoxy substituiert ist,

oder

$(C_6-C_{10})$-Aryl oder einen 5- bis 6-gliedrigen aromatischen Heterocyclus mit bis zu 3 Heteroatomen aus der Reihe S, N und/oder O bedeutet, die ihrerseits gegebenenfalls ein- bis mehrfach, gleich oder verschieden durch $(C_1-C_6)$ -Alkoxy, $(C,-C_6)$-Alkyl, Hydroxy, Nitro oder Halogen substituiert sein können,

oder

einen Rest der Formel

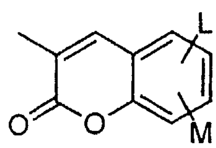

oder -$NR^{23}R^{24}$ bedeutet,
worin

L und M gleich oder verschieden sind und Wasserstoff oder Halogen bedeuten,

$R^{23}$ und $R^{24}$ die oben angegebene Bedeutung von $R^{10}$ und $R^{11}$ haben und mit dieser gleich oder verschieden sind,

$R^{18}$ die oben angegebene Bedeutung von $R^{16}$ hat und mit dieser gleich oder verschieden ist,

$R^{19}$ $(C_3-C_8)$-Cycloalkyl bedeutet, oder
$(C_1-C_8)$-Alkyl oder $(C_2-C_8)$-Alkenyl bedeutet, die ihrerseits gegebenenfalls durch Substituenten, ausgewählt aus der Gruppe Halogen, Phenyl, Hydroxy, Morpholinyl, $(C_3-C_8)$-Cycloalkyl und durch eine Gruppe der Formel -$SiR^{25}R^{26}R^{27}$ substituiert sind,
worin

$R^{25}$, $R^{26}$ und $R^{27}$ gleich oder verschieden sind und $(C_1-C_6)$-Alkyl bedeuten,

$R^{20}$ und $R^{21}$ gleich oder verschieden sind und Wasserstoff, Adamantyl, $(C_3-C_8)$-Cycloalkyl, Phenyl, Phenoxy-substituiertes Phenyl oder einen 5-bis 6-gliedrigen, aromatischen Heterocyclus mit bis zu 3 Heteratomen aus der Reihe S, N und/oder O bedeuten, oder
$(C_2-C_8)$-Alkenyl, $(C_1-C_{12})$-Alkyl oder $(C_2-C_6)$-Alkinyl bedeuten, die gegebenenfalls durch Hydroxy, $(C_3-C_6)$-Cycloalkyl, $(C_1-C_6)$-Alkoxy, Halogen, Trifluormethyl, Phenyl oder durch einen 5- bis 6-gliedrigen aromatischen Heterocyclus mit bis zu 3 Heteroatomen aus der Reihe S, N und/oder O substituiert sind, wobei die Ringsysteme gegebenenfalls bis zu 2-fach gleich oder verschieden durch $(C_1-C_6)$-Alkoxy, $(C_1-C_6)$-Alkoxycarbonyl, Halogen, Phenoxy, Hydroxy oder $(C_1-C_6)$-Alkyl substiutiert sind,
und/oder das unter $R^{20}/R^{21}$ aufgeführte Alkyl gegebenenfalls durch Reste der Formeln -$SCF_3$

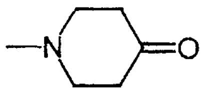 , 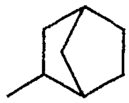 ,

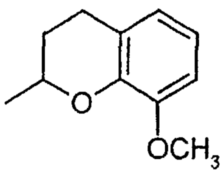

oder $-NR^{28}R^{29}$ substituiert ist, worin

$R^{28}$ und $R^{29}$ gleich oder verschieden sind und Wasserstoff oder ($C_1$-$C_6$)-Alkyl bedeuten,

oder

einen Rest der Formel

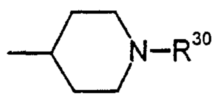

bedeutet,
worin

$R^{30}$ die oben angegebene Bedeutung von $R^{12}$ hat und mit dieser gleich oder verschieden ist,

oder

$R^{20}$ und $R^{21}$ gemeinsam mit dem Stickstoffatom einen Rest der Formel

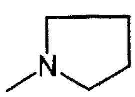 oder 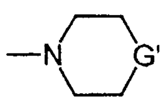

bilden,
worin

G' die oben angegebene Bedeutung von G hat und mit dieser gleich oder verschieden ist,

$R^2$ und $R^3$ gleich oder verschieden sind und für Wasserstoff oder ($C_1$-$C_6$)-Alkyl stehen,

und
D und E gemeinsam Reste der Formeln

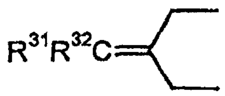

,

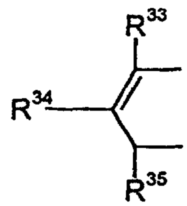

oder

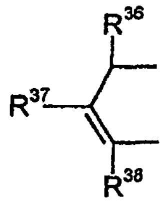

bilden,

worin

$R^{31}$, $R^{32}$, $R^{33}$, $R^{34}$, $R^{35}$, $R^{36}$, $R^{37}$ und $R^{38}$ gleich oder verschieden sind und Wasserstoff, Phenyl oder $(C_1-C_6)$ -Alkyl bedeuten,

und deren pharmazeutisch verträgliche Salze, mit der Ausnahme von (3aS*, 6aR*)-6a-Methyl-3,3a,6,6a-tetrahydro-1H-cyclopenta*[c]*furan-1-on.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweilige Mischungen. Die Racemformen lassen sich ebenso wie die Diastereomeren in bekannter Weise in die stereoisomer einheitlichen Bestandteile trennen.

Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methylpiperidin.

$(C_3-C_8)$-Cycloalkyl und $(C_3-C_6)$-Cycloalkyl stehen im Rahmen der Erfindung für Cyclopropyl, Cyclopentyl, Cyclobutyl, Cyclohexyl, Cycloheptyl oder Cyclooctyl. Bevorzugt seien genannt: Cyclopropyl, Cyclopentyl, Cyclohexyl oder Cycloheptyl.

$(C_6-C_{10})$-Aryl steht im allgemeinen für einen aromatischen Rest mit 6 bis 10 Kohlenstoffatomen. Bevorzugte Arylreste sind Phenyl und Naphthyl.

$(C_1-C_{12})$-Alkyl, $(C_1-C_9)$-Alkyl, $(C_1-C_8)$-Alkyl und $(C_1-C_6)$-Alkyl stehen im Rahmen der Erfindung für einen geradkettigen oder verzweigten Alkylrest mit 1 bis 12, 1 bis 9, 1 bis 8 bzw. 1 bis 6 Kohlenstoffatomen. Bevorzugt ist ein geradkettiger oder verzweigter Alkylrest mit 1 bis 6 Kohlenstoffatomen. Beispielsweise seien genannt: Methyl, Ethyl, n-Propyl, Isopropyl, t-Butyl, n-Pentyl und n-Hexyl.

$(C_2-C_8)$-Alkandiyl steht im Rahmen der Erfindung für einen geradkettigen oder verzweigten Alkandiylrest mit 2 bis 8 Kohlenstoffatomen. Bevorzugt ist ein geradkettiger oder verzweigter Alkandiylrest mit 2 bis 6 Kohlenstoffatomen, besonders bevorzugt mit 2 bis 4 Kohlenstoffatomen. Beispielsweise seien genannt Ethylen, Propylen, Propan-1,2-diyl, Propan-2,2-diyl, Butan-1,3-diyl, Butan-2,4-diyl, Pentan-2,4-diyl, 2-Methyl-pentan-2,4-diyl.

$(C_2-C_6)$-Alkendiyl steht im Rahmen der Erfindung für einen geradkettigen oder verzweigten Alkendiylrest mit 2 bis 6 Kohlenstoffatomen, bevorzugt mit 2 bis 4 Kohlenstoffatomen, besonders bevorzugt mit 3 Kohlenstoffatomen. Beispielsweise seien genannt Ethen-1,2-diyl, Ethen-1,1-diyl, Propen-1,1-diyl, Propen-1,2-diyl, Propen-1,3-diyl, Propen-3,3-diyl, Propen-2,3-diyl, But-2-en-1,4-diyl, Pent-2-en-1,4-diyl, Hex-2-en-1,4-diyl.

$(C_2-C_6)$-Alkindiyl steht im Rahmen der Erfindung für einen geradkettigen oder verzweigten Alkindiylrest mit 2 bis 6 Kohlenstoffatomen, bevorzugt mit 2 bis 4 Kohlenstoffatomen, besonders bevorzugt mit 2 bis 3 Kohlenstoffatomen. Beispielsweise seien genannt Ethin-1,2-diyl, Propin-1,3-diyl, But-2-in-1,4-diyl, Pent-2-in-1,4-diyl, Hex-2-in-1,4-di-

yl.

$(C_1-C_6)$-Alkoxy steht im Rahmen der Erfindung für einen geradkettigen oder verzweigten Alkoxyrest mit 1 bis 6 Kohlenstoffatomen. Bevorzugt ist ein geradkettiger oder verzweigter Alkoxyrest mit 1 bis 4 Kohlenstoffatomen. Beispielsweise seien genannt: Methoxy, Ethoxy, n-Propoxy, Isopropoxy, t-Butoxy, n-Pentoxy und n-Hexoxy.

$(C_1-C_6)$-Alkoxycarbonyl steht im Rahmen der Erfindung für einen geradkettigen oder verzweigten Alkoxycarbonylrest mit 1 bis 6 Kohlenstoffatomen. Bevorzugt ist ein geradkettiger oder verzweigter Alkoxycarbonylrest mit 1 bis 4 Kohlenstoffatomen. Beispielsweise seien genannt: Methoxycarbonyl, Ethoxycarbonyl, n-Propoxycarbonyl, Isopropoxycarbonyl und t-Butoxycarbonyl.

$(C_2-C_8)$-Alkenyl und $(C_2-C_6)$-Alkenyl stehen im Rahmen der Erfindung für einen geradkettigen oder verzweigten Alkenylrest mit 2 bis 8 Kohlenstoffatomen bzw. 2 bis 6 Kohlenstoffatomen. Bevorzugt ist ein geradkettiger oder verzweigter Alkenylrest mit 2 bis 4 Kohlenstoffatomen. Beispielsweise seien genannt: Vinyl, Allyl, Isopropenyl und n-But-2-en-1-yl.

$(C_2-C_6)$-Alkinyl steht im Rahmen der Erfindung für einen geradkettigen oder verzweigten Alkinylrest mit 2 bis 6 Kohlenstoffatomen. Bevorzugt ist ein geradkettiger oder verzweigter Alkinylrest mit 2 bis 4 Kohlenstoffatomen. Beispielsweise seien genannt: Ethinyl, n-Prop-2-in-1-yl und n-But-2-in-1-yl.

Ein 5- bis 6-gliedriger Heterocyclus steht im Rahmen der Erfindung im allgemeinen für einen 5- bis 6-gliedrigen, gegebenenfalls auch aromatischen Heterocyclus der bis zu 3 Heteroatome aus der Reihe S, O und/oder N oder einen Rest der Formel -NH oder $-NCH_3$ enthalten kann. Beispielsweise seien genannt: Pyridyl, Pyrimidyl, Pyridazinyl, Thienyl, Furyl, Pyrrolyl, Thiazolyl, Oxazolyl, Imidazolyl, Piperidinyl oder Morpholinyl. Bevorzugt sind Pyridyl, Pyrimidyl, Pyridazinyl, Furyl und Thiazolyl.

Ein 5- bis 6-gliedriger, benzokondensierter Heterocyclus steht im Rahmen der Erfindung im allgemeinen für einen 5- bis 6-gliedrigen, vorzugsweise 5-gliedrigen Heterocyclus mit bis zu 2 Heteroatomen aus der Reihe S, O, N und/oder einem Rest der Formel -NH, dessen Ringkohlenstoffatome die Anknüpfungsstellen für den Benzolring darstellen. Beispielsweise seien genannt: Indolyl, Benzimidazolyl, Benzothiophenyl, Benzofuranyl, Benzoxazolyl, Chinolyl, Chinoxalinyl oder Chinazolyl. Bevorzugt sind Benzimidazolyl, Chinolyl, Chinoxalinyl, Chinazolyl, Benzothiophenyl und Benzofuranyl.

Bevorzugt sind erfindungsgemäßeVerbindungen der allgemeinen Formel (I),
in welcher

A für Reste der Formeln $-CH_2-$, -CO-, $-CR^4(OH)-$ oder $-(CH_2)_a-CHR^5-$ steht,
worin

a eine Zahl 0, 1, 2 oder 3 bedeutet,

$R^4$ Wasserstoff oder $(C_1-C_4)$-Alkyl bedeutet

und

$R^5$ Phenyl bedeutet,

oder
für $(C_2-C_6)$-Alkandiyl, $(C_2-C_4)$-Alkendiyl oder $(C_2-C_4)$-Alkindiyl steht,

$R^1$ für Wasserstoff, Cyclopropyl, Cyclopentyl oder Cyclohexyl steht, oder für Benzofuranyl, Benzothiophenyl, Benzimidazolyl, Thienyl, Furyl, Chinazolyl, Chinoxalinyl oder Chinolyl steht,

oder
für Reste der Formeln

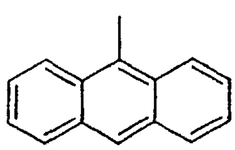 , 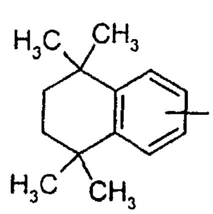 , 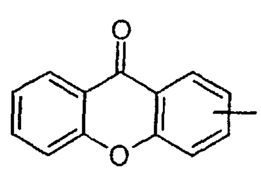 ,

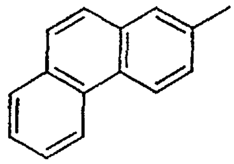 , 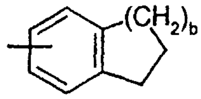 oder 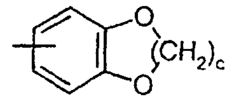

steht,

worin

b und c gleich oder verschieden sind und eine Zahl 1 oder 2 bedeuten,

oder

für Phenyl oder Naphthyl steht,

wobei alle oben aufgeführten Ringsysteme gegebenenfalls ein- bis mehrfach, gleich oder verschieden durch Substituenten, ausgewählt aus der Gruppe Fluor, Chlor, Brom, Jod, Cyano, Nitro, Trifluormethyl, Hydroxy oder ($C_1$-$C_5$)-Alkoxy, ($C_1$-$C_5$)-Alkyl-carbonyloxy, Cyclopropyl, Cyclopentyl, Cyclohexyl, Phenyl, Phenoxy, Pyridyl, Pyrimidyl, Pyridazinyl, Thienyl, Furyl oder Benzyloxy substituiert sind, die ihrerseits bis zu dreifach gleich oder verschieden durch Cyano, Fluor, Chlor, Brom oder Jod substituiert sein können,

und/oder durch ($C_1$-$C_5$)-Alkyl und ($C_2$-$C_4$)-Alkenyl substituiert sind, die ihrerseits durch Fluor, Chlor, Brom, Jod, Phenyl, Naphthyl oder durch Reste der Formel -$SR^8$, -$OR^9$ oder -$NR^{10}R^{11}$ oder

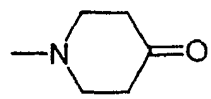

substituiert sein können,

worin

$R^8$ ($C_1$-$C_4$)-Alkyl oder Phenyl bedeutet,

$R^9$ Wasserstoff oder ($C_1$-$C_4$)-Alkyl bedeutet,

und

$R^{10}$ und $R^{11}$ gleich oder verschieden sind und Wasserstoff, Phenyl oder ($C_1$-$C_4$)-Alkyl bedeuten, das gegebenenfalls durch Phenyl substituiert ist, das seinerseits ein- bis mehrfach, gleich oder verschieden durch Fluor, Chlor, Brom, Nitro, Hydroxy oder ($C_1$-$C_4$)-Alkoxy substituiert sein kann,

oder

$R^{10}$ und $R^{11}$ gemeinsam mit dem Stickstoffatom einen Rest der Formel

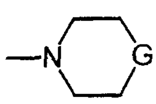

bilden,

worin

G ein Sauerstoffatom, eine -$CH_2$-Gruppe oder einen Rest der Formel -$NR^{12}$- bedeutet,

worin

$R^{12}$ Wasserstoff, Phenyl, Benzyl, ($C_1$-$C_4$)-Alkyl, ($C_1$-$C_4$)-Alkoxycarbonyl, Pyridyl, Pyrimidyl, Pyridazinyl oder Furyl bedeutet,

und/oder durch Gruppen der Formeln $-CO_2-R^{13}$, $-NR^{14}R^{15}$, $-NR^{16}CO-R^{17}$, $-NR^{18}CO_2-R^{19}$ und $-CO-NR^{20}R^{21}$ substituiert sind,
worin

$R^{13}$ Wasserstoff bedeutet, oder
($C_1$-$C_8$)-Alkyl oder ($C_2$-$C_5$)-Alkenyl bedeutet, die ihrerseits durch Reste der Formeln

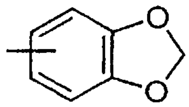 , 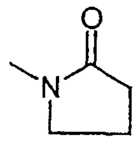 ,

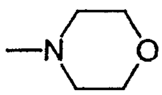 , 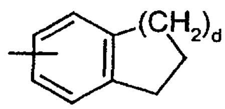

oder
Phenyl, Naphthyl, Pyridyl, Thienyl oder Furyl substituiert sein können,
worin

d eine Zahl 1 oder 2 bedeutet,

oder
Phenyl oder Naphthyl bedeutet, die gegebenenfalls durch Phenyl substituiert sind, das seinerseits durch Cyano, Fluor, Chlor oder Brom substituiert sein kann,

$R^{14}$ und $R^{15}$ gleich oder verschieden sind und Wasserstoff, Cyclopropyl, Cyclopentyl, Cyclohexyl, Phenyl oder ($C_1$-$C_5$)-Alkyl bedeuten, das gegebenenfalls durch Cyclopropyl, Cyclopentyl, Cyclohexyl oder Phenyl substituiert ist, das seinerseits ein- bis mehrfach, gleich oder verschieden durch Fluor, Chlor, Brom, Hydroxy oder ($C_1$-$C_4$)-Alkoxy substituiert sein kann,

$R^{16}$ Wasserstoff oder ($C_1$-$C_3$)-Alkyl bedeutet,

$R^{17}$ Wasserstoff, Adamantyl, Cyclopropyl, Cyclopentyl oder Cyclohexyl bedeutet, oder
($C_2$-$C_4$)-Alkenyl oder ($C_1$-$C_{10}$)-Alkyl bedeutet, das gegebenenfalls durch Adamantyl, Cyclopropyl, Cyclopentyl, Cyclohexyl, Phenyl, Phenoxy-naphthyl, Pyridyl, Thienyl, Tetrazolyl oder Furyl substituiert ist, wobei die Ringsysteme ihrerseits ein- bis mehrfach, gleich oder verschieden durch ($C_1$-$C_4$)-Alkyl, ($C_1$-$C_4$)-Alkoxy, Hydroxy, Nitro, Fluor, Chlor oder Brom substituiert sein können,
und/oder Alkyl gegebenenfalls durch einen Rest der Formel

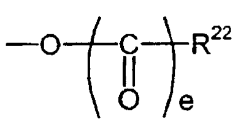

substituiert ist,
worin

e eine Zahl 0 oder 1 bedeutet und

$R^{22}$ ($C_1$-$C_4$)-Alkyl, Phenyl oder Naphthyl bedeutet, die gegebenenfalls ein- bis mehrfach, gleich oder

verschieden durch Fluor, Chlor, Brom, Nitro, Hydroxy oder $(C_1-C_4)$-Alkoxy substituiert sind,

oder

Phenyl, Naphthyl, Thienyl, Furyl oder Pyridyl bedeutet, die ihrerseits gegebenenfalls ein- bis mehrfach, gleich oder verschieden durch $(C_1-C_4)$-Alkoxy, $(C_1-C_4)$-Alkyl, Hydroxy, Nitro, Fluor, Chlor oder Brom substituiert sein können,

oder

einen Rest der Formel

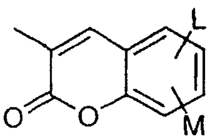

oder -$NR^{23}R^{24}$ bedeutet,

worin

L und M gleich oder verschieden sind und Wasserstoff, Fluor, Chlor oder Brom bedeuten,

$R^{23}$ und $R^{24}$ die oben angegebene Bedeutung von $R^{10}$ und $R^{11}$ haben und mit dieser gleich oder verschieden sind,

$R^{18}$ die oben angegebene Bedeutung von $R^{16}$ hat und mit dieser gleich oder verschieden ist,

$R^{19}$ Cyclopropyl, Cyclopentyl oder Cyclohexyl bedeutet, oder $(C_1-C_7)$-Alkyl oder $(C_2-C_6)$-Alkenyl bedeutet, die ihrerseits gegebenenfalls durch Substituenten, ausgewählt aus der Gruppe Fluor, Chlor, Brom, Phenyl, Hydroxy, Morpholinyl, Cyclopropyl, Cyclopentyl, Cyclohexyl und durch eine Gruppe der Formel -$SiR^{25}R^{26}R^{27}$ substituiert sind,

worin

$R^{25}$, $R^{26}$ und $R^{27}$ gleich oder verschieden sind und $(C_1-C_4)$-Alkyl bedeuten,

$R^{20}$ und $R^{21}$ gleich oder verschieden sind und Wasserstoff, Adamantyl, Cyclopropyl, Cyclopentyl, Cyclohexyl, Phenyl, Phenoxy-substituiertes Phenyl, Pyridyl, Furyl, Thienyl, Thiazolyl oder Pyrryl bedeuten,

oder

$(C_2-C_6)$-Alkenyl, $(C_1-C_{10})$-Alkyl oder $(C_3-C_6)$-Alkinyl bedeuten, die gegebenenfalls durch Hydroxy, Cyclopropyl, Cyclopentyl, Cyclohexyl, $(C_1-C_5)$-Alkoxy, $(C_1-C_6)$-Alkoxycarbonyl, Fluor, Chlor, Brom, Hydroxy, Trifluormethyl, Phenyl, Pyridyl, Furyl, Thienyl oder Pyrryl substituiert sind, wobei die Ringsysteme gegebenenfalls bis zu 2-fach gleich oder verschieden durch $(C_1-C_4)$-Alkoxy, Fluor, Chlor, Brom, Phenoxy, Hydroxy oder $(C_1-C_4)$-Alkyl substituiert sind,

und/oder das unter $R^{20}/R^{21}$ aufgeführte Alkyl gegebenenfalls durch Reste der Formeln

-$SCF_3$,

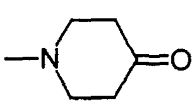 , 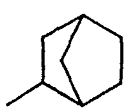 ,

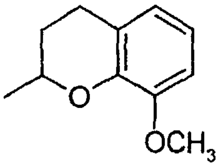

oder -$NR^{28}R^{29}$ substituiert ist,

worin

$R^{28}$ und $R^{29}$ gleich oder verschieden sind und Wasserstoff oder ($C_1$-$C_4$)-Alkyl bedeuten,

oder
einen Rest der Formel

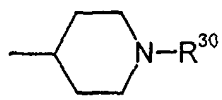

bedeutet,
worin

$R^{30}$ die oben angegebene Bedeutung von $R^{12}$ hat und mit dieser gleich oder verschieden ist,

oder

$R^{20}$ und $R^{21}$ gemeinsam mit dem Stickstoffatom einen Rest der Formel

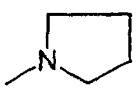

oder

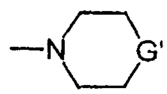

bilden,
worin

G' die oben angegebene Bedeutung von G hat und mit dieser gleich oder verschieden ist,

$R^2$ und $R^3$ gleich oder verschieden sind und für Wasserstoff oder ($C_1$-$C_3$)-Alkyl stehen,

und
D und E gemeinsam Reste der Formeln

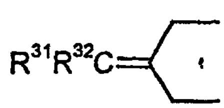

,

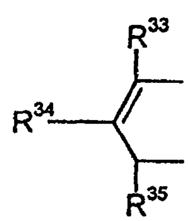

oder

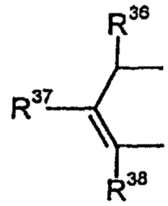

bilden

worin

$R^{31}$, $R^{32}$, $R^{33}$, $R^{34}$, $R^{35}$, $R^{36}$, $R^{37}$ und $R^{38}$ gleich oder verschieden sind und Wasserstoff, Phenyl oder $(C_1-C_3)$-Alkyl bedeuten,

und deren pharmazeutisch verträgliche Salze,

mit der Ausnahme von (3aS*, 6aR*)-6a-Methyl-3,3a,6,6a-tetrahydro-1H-cyclopenta*[c]*furan-1-on.

Besonders bevorzugt sind erfindungsgemäße Verbindungen der allgemeinen Formel (I), in welcher

A für Reste der Formeln $-CH_2-$, -CO-, $-CR^4(OH)-$ oder $-(CH_2)_a-CHR^5-$ steht,
worin

- a eine Zahl 0, 1, 2 oder 3 bedeutet,
- $R^4$ Wasserstoff oder $(C_1-C_3)$-Alkyl bedeutet und
- $R^5$ Phenyl bedeutet,

oder

für $(C_2-C_4)$-Alkandiyl, Propendiyl oder $(C_2-C_3)$-Alkindiyl steht,

$R^1$ für Wasserstoff, Cyclopropyl oder Cyclohexyl steht, oder
für Benzofüranyl, Benzothiophenyl, Benzimidazolyl, Thienyl, Chinazolyl oder Chinoxalinyl steht,

oder

für Reste der Formeln

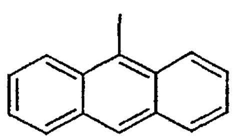
,
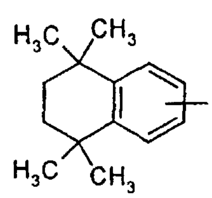
,
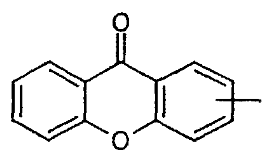
,

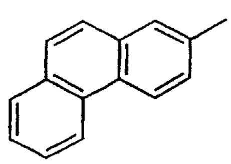
,
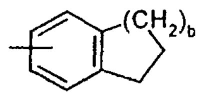
oder
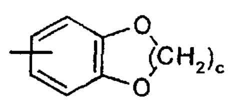

steht,

worin

b und c gleich oder verschieden sind und eine Zahl 1 oder 2 bedeuten,

oder

für Phenyl oder Naphthyl steht,

wobei alle oben aufgeführten Ringsysteme gegebenenfalls ein- bis mehrfach, gleich oder verschieden durch Substituenten, ausgewählt aus der Gruppe Fluor, Chlor, Brom, Jod, Cyano, Nitro, Trifluormethyl oder $(C_1-C_4)$-Alkoxy, $(C_1-C_4)$-Alkyl-carbonyloxy, Cyclohexyl, Phenyl, Phenoxy, Pyridyl, Pyrimidyl, Pyridazinyl oder Benzyloxy substituiert sind, die ihrerseits bis zu dreifach, gleich oder verschieden durch Cyano, Fluor, Chlor, Brom, oder Jod substituiert sein können,

und/oder durch $(C_1-C_4)$-Alkyl und $(C_2-C_3)$-Alkenyl substituiert sind, die ihrerseits durch Chlor, Brom, Jod oder Phenyl oder durch Reste der Formel $-OR^9$ oder $-NR^{10}R^{11}$ oder

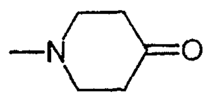

substituiert sein können,
worin

$R^9$ Wasserstoff oder $(C_1-C_3)$-Alkyl bedeutet,

und

$R^{10}$ und $R^{11}$ gleich oder verschieden sind und Wasserstoff, Phenyl oder $(C_1-C_3)$-Alkyl bedeuten, das gegebenenfalls durch Phenyl substituiert ist, das seinerseits durch Chlor, Brom, Hydroxy oder $(C_1-C_3)$-Alkoxy substituiert sein kann,

oder

$R^{10}$ und $R^{11}$ gemeinsam mit dem Stickstoffatom einen Rest der Formel

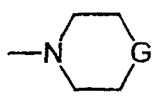

bilden,
worin

G ein Sauerstoffatom oder einen Rest der Formel $-NR^{12}$ bedeutet,

worin

$R^{12}$ Wasserstoff, Phenyl, Benzyl, $(C_1-C_3)$-Alkyl, $(C_1-C_3)$-Alkoxycarbonyl, Pyridyl, Pyrimidyl, Pyridazinyl oder Furyl bedeutet,

und/oder durch Gruppen der Formeln $-CO_2-R^{13}$, $-NR^{14}R^{15}$, $-NR^{16}CO-R^{17}$, $-NR^{18}CO_2-R^{19}$ und $-CO-NR^{20}R^{21}$ substituiert sind,
worin

$R^{13}$ Wasserstoff bedeutet, oder
$(C_1-C_6)$-Alkyl oder Allyl bedeutet, die ihrerseits durch Reste der Formeln

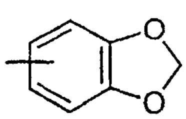
,
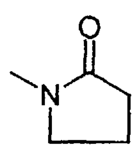
,

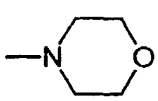
,
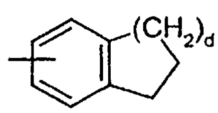

oder
Phenyl, Naphthyl oder Pyridyl substituiert sein können,

worin

d eine Zahl 1 oder 2 bedeutet,

oder

Phenyl bedeutet, das gegebenenfalls durch Phenyl substituiert ist, das seinerseits durch Cyano, Chlor oder Brom substituiert sein kann,

$R^{14}$ und $R^{15}$ gleich oder verschieden sind und Wasserstoff, Cyclohexyl, Phenyl oder $(C_1-C_4)$-Alkyl bedeuten, das gegebenenfalls durch Cyclopropyl, Cyclohexyl oder Phenyl substituiert ist, das seinerseits ein- bis mehrfach, gleich oder verschieden durch Chlor oder $(C_1-C_3)$-Alkoxy substituiert sein kann,

$R^{16}$ Wasserstoff, Methyl oder Ethyl bedeutet,

$R^{17}$ Wasserstoff, Adamantyl, Cyclopentyl oder Cyclohexyl bedeutet, oder $(C_2-C_3)$-Alkenyl oder $(C_1-C_8)$-Alkyl bedeutet, das gegebenenfalls durch Adamantyl, Cyclopropyl, Cyclopentyl, Cyclohexyl, Phenyl, Phenoxy, Thienyl oder Furyl substituiert ist, wobei die Ringsysteme ihrerseits ein- bis mehrfach, gleich oder verschieden durch $(C_1-C_3)$-Alkyl, $(C_1-C_3)$-Alkoxy, Hydroxy, Nitro, Fluor, Chlor oder Brom substituiert sein können,
und/oder Alkyl gegebenenfalls durch einen Rest der Formel

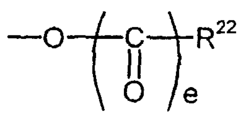

substituiert ist,
worin

e eine Zahl 0 oder 1 ist und

$R^{22}$ $(C_1-C_3)$-Alkyl, Phenyl oder Naphthyl bedeutet, die gegebenenfalls ein- bis mehrfach, gleich oder verschieden durch Fluor, Chlor, Brom, Nitro, Hydroxy oder $(C_1-C_3)$-Alkoxy substituiert sind,

oder

Phenyl, Naphthyl, Thienyl oder Furyl bedeutet, die ihrerseits gegebenenfalls ein- bis mehrfach, gleich oder verschieden durch $(C_1-C_3)$-Alkoxy, $(C_1-C_3)$-Alkyl, Nitro, Fluor, Chlor oder Brom substituiert sein können,
oder

einen Rest der Formel

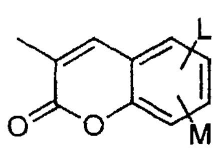

oder -$NR^{23}R^{24}$ bedeutet,
worin

L und M gleich oder verschieden sind und Wasserstoff, Fluor oder Chlor bedeuten,
$R^{23}$ und $R^{24}$ die oben angegebene Bedeutung von $R^{10}$ und $R^{11}$ haben und mit dieser gleich oder verschieden sind,

$R^{18}$ die oben angegebene Bedeutung von $R^{16}$ hat und mit dieser gleich oder verschieden ist,

$R^{19}$ ($C_1$-$C_4$)-Alkyl oder ($C_3$-$C_5$)-Alkenyl bedeutet, die ihrerseits gegebenenfalls durch Substituenten, ausgewählt aus der Gruppe Chlor, Phenyl, Hydroxy, Morpholinyl, Cyclopropyl, Cyclohexyl und durch eine Gruppe der Formel $-SiR^{25}R^{26}R^{27}$ substituiert sind,
worin
$R^{25}$, $R^{26}$ und $R^{27}$ gleich sind und Methyl bedeuten,

$R^{20}$ und $R^{21}$ gleich oder verschieden sind und Wasserstoff, Adamantyl, Cyclopropyl, Cyclopentyl, Cyclohexyl, Phenyl, Phenoxy-substituiertes Phenyl, Thiazolyl oder Pyrryl bedeuten, oder ($C_2$-$C_3$)-Alkenyl, ($C_1$-$C_7$)-Alkyl oder ($C_3$-$C_5$)-Alkinyl bedeuten, die gegebenenfalls durch Hydroxy, Cyclopropyl, Cyclopentyl, Cyclohexyl, ($C_1$-$C_3$)-Alkoxy, Hydroxy, Trifluormethyl, Phenyl, Pyridyl, Furyl, Thienyl oder Pyrryl substituiert sind, wobei die Ringsysteme gegebenenfalls bis zu 2-fach, gleich oder verschieden durch ($C_1$-$C_3$)-Alkoxy, ($C_1$-$C_6$)-Alkoxycarbonyl, Fluor, Chlor, Brom, Phenoxy, Hydroxy oder ($C_1$-$C_3$)-Alkyl substituiert sind, und/oder das unter $R^{20}$/$R^{21}$ aufgeführte Alkyl gegebenenfalls durch Reste der Formeln
$-SCF_3$,

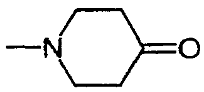 , 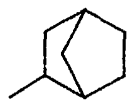 ,

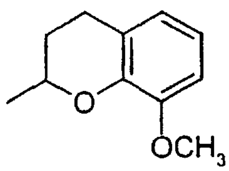

oder $-NR^{28}R^{29}$ substituiert ist,
worin

$R^{28}$ und $R^{29}$ gleich oder verschieden sind und Wasserstoff oder ($C_1$-$C_3$)-Alkyl bedeuten,

oder

$R^{20}$ oder $R^{21}$ einen Rest der Formel

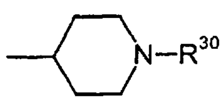

bedeutet,
worin

$R^{30}$ die oben angegebene Bedeutung von $R^{12}$ hat und mit dieser gleich oder verschieden ist,

oder

$R^{20}$ und $R^{21}$ gemeinsam mit dem Stickstoffatom einen Rest der Formel

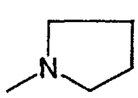 oder 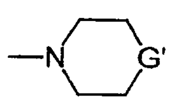

bilden,
worin

G' die oben angegebene Bedeutung von G hat und mit dieser gleich oder verschieden ist,

$R^2$ und $R^3$ gleich oder verschieden sind und für Wasserstoff oder Methyl stehen,
und
D und E gemeinsam Reste der Formeln

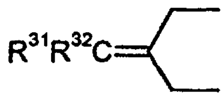
,
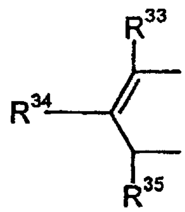

oder

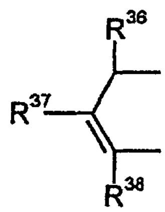

bilden,
worin
$R^{31}$, $R^{32}$, $R^{33}$, $R^{34}$, $R^{35}$, $R^{36}$, $R^{37}$ und $R^{38}$ gleich oder verschieden sind und Wasserstoff oder Methyl bedeuten,
und deren pharmazeutisch verträgliche Salze,
mit der Ausnahme von (3aS*, 6aR*)-6a-Methyl-3,3a,6,6a-tetrahydro-1Hcyclopenta*[c]*furan-1-on.

Ebenso besonders bevorzugt sind erfindungsgemäße Verbindungen der allgemeinen Formel (I), in denen A für die $-CH_2$-Gruppe steht,
mit der Ausnahme von (3aS*, 6aR*)-6a-Methyl-3,3a,6,6a-tetrahydro-1Hcyclopenta*[c]*furan-1-on.

Ganz besonders bevorzugt sind erfindungsgemäße Verbindungen der allgemeinen Formel (I), worin

A für $-CH_2-$ steht,
$R^1$ für Phenyl oder Naphthyl steht,
wobei alle oben aufgeführten Ringsysteme gegebenenfalls ein- bis mehrfach, gleich oder verschieden durch Substituenten, ausgewählt aus der Gruppe Fluor, Chlor, Brom, Jod, Cyano, Nitro, Trifluormethyl oder $(C_1\text{-}C_4)$-Alkoxy, substituiert sind,
und/oder durch $(C_1\text{-}C_4)$-Alkyl substituiert sind,
und/oder durch Gruppen der Formeln $-NR^{16}CO-R^{17}$, $-NR^{18}CO_2-R^{19}$ und $-CO-NR^{20}R^{21}$ substituiert sind,
worin

$R^{16}$ Wasserstoff bedeutet,

$R^{17}$ $(C_1\text{-}C_8)$-Alkyl bedeutet, das gegebenenfalls durch Cyclopropyl, Cyclopentyl, Cyclohexyl, Phenyl, Thienyl oder Furyl substituiert ist, wobei die Ringsysteme ihrerseits ein- bis mehrfach, gleich oder verschieden durch $(C_1\text{-}C_3)$-Alkyl, $(C_1\text{-}C_3)$-Alkoxy, Hydroxy, Nitro, Fluor, Chlor oder Brom substituiert sein können,

$R^{18}$ die oben angegebene Bedeutung von $R^{16}$ hat und mit dieser gleich oder verschieden ist,

$R^{19}$ $(C_1\text{-}C_4)$-Alkyl oder $(C_3\text{-}C_5)$-Alkenyl bedeutet,

$R^{20}$ und $R^{21}$ gleich oder verschieden sind und Wasserstoff, $(C_2\text{-}C_3)$-Alkenyl, $(C_1\text{-}C_7)$-Alkyl oder $(C_3\text{-}C_5)$-Alkinyl bedeuten, die gegebenenfalls durch Phenyl, Pyridyl, Furyl, Thienyl oder Pyrryl substituiert sind, wobei die Ringsysteme gegebenenfalls bis zu 2-fach, gleich oder verschieden durch $(C_1\text{-}C_3)$-Alkoxy, Fluor, Chlor, Brom oder $(C_1\text{-}C_3)$-Alkyl substituiert sind,

$R^2$ und $R^3$ für Wasserstoff oder Methyl stehen,
und
D und E gemeinsam Reste der Formeln

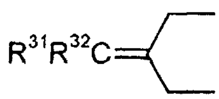
oder
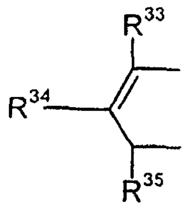
bilden,

worin
$R^{31}$, $R^{32}$, $R^{33}$, $R^{34}$, $R^{35}$ Wasserstoff bedeuten,
und deren pharmazeutisch verträgliche Salze.

Ganz besonders bevorzugt sind die in der folgenden Tabelle aufgeführten Strukturen, die racemisch oder enantiomerenrein vorliegen können:

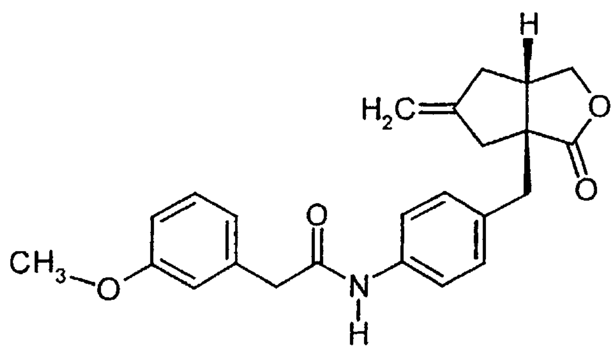

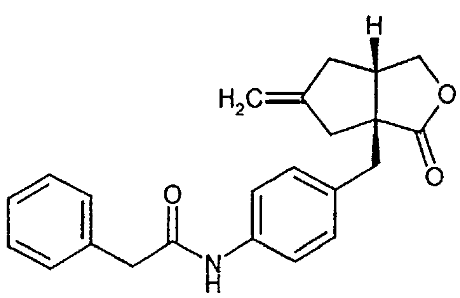

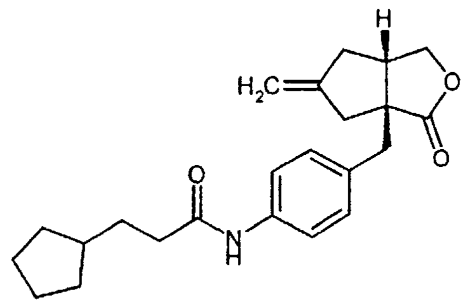

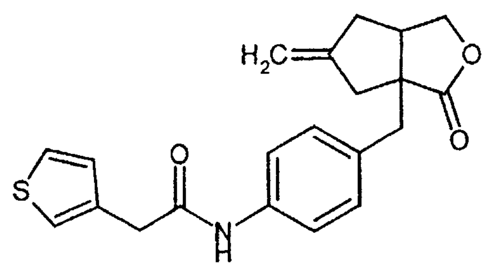

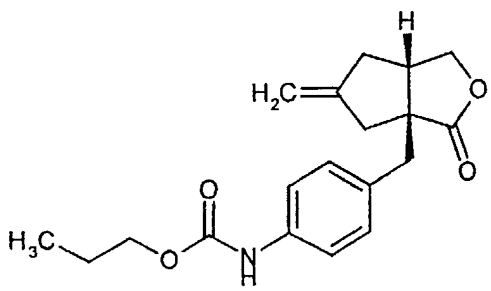

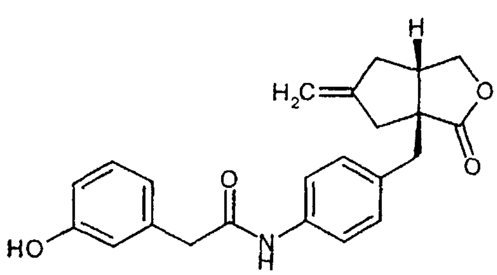

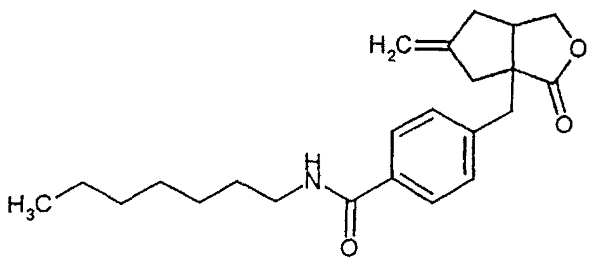

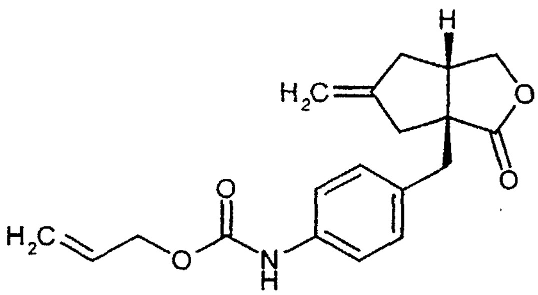

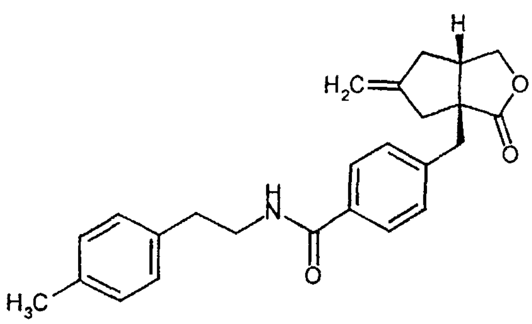

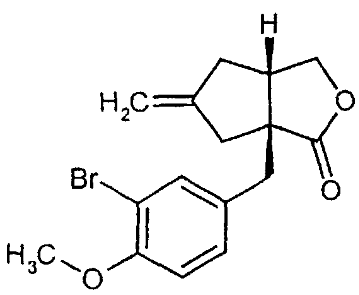

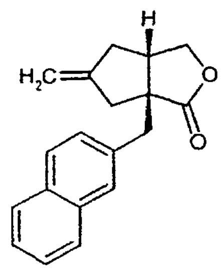

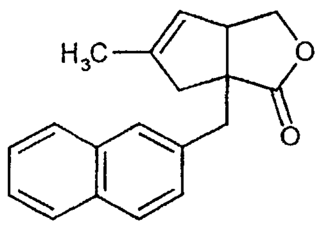

Außerdem wurde ein Verfahren zur Herstellung der erfindungsgemäßen Verbindungen der allgemeinen Formel (I) gefunden, dadurch gekennzeichnet, daß man

Verbindungen der allgemeinen Formel (II)

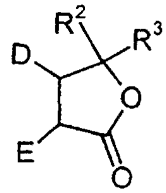

(II)

in welcher
D, E, $R^2$ und $R^3$ die oben angegebene Bedeutung haben,
mit Verbindungen der allgemeinen Formel (III),

$$T\text{-}A\text{-}R^1 \quad (III)$$

in welcher
T für Halogen, vorzugsweise für Brom steht,
und
A und $R^1$ die oben angegebene Bedeutung haben,
in inerten Lösemitteln und in Anwesenheit einer Base umsetzt,
und gegebenenfalls den Substituenten R' nach üblichen Methoden derivatisiert.

Das erfindungsgemäße Verfahren kann durch folgendes Formelschema beispielhaft erläutert werden:

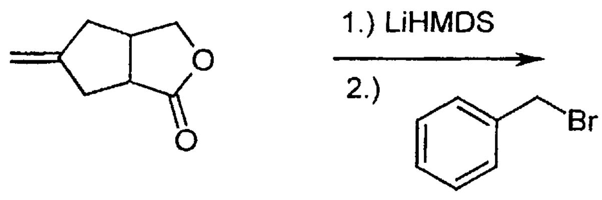


Als Lösemittel eignen sich alle inerten Lösemittel, die sich unter den Reaktionsbedingungen nicht verändern. Hierzu gehören bevorzugt Ether wie Diethylether, Dioxan, Tetrahydrofuran, Glykoldimethylether oder Diethylenglykoldimethylether. Besonders bevorzugt ist Tetrahydrofuran.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Natriumoder Kaliummethanolat, oder Natrium- oder Kaliumethanolat oder Kalium-tert.butylat, oder Amide wie Natriumamid, Lithium-bis-(trimethylsilyl)amid, Lithiumdiisopropylamid, oder metallorganische Verbindungen wie Butyllithium oder Phenyllithium. Bevorzugt sind Lithiumdiisopropylamid und Lithium-bis-(trimethylsilyl)amid.

Die Base wird hierbei in einer Menge von 1 bis 5, bevorzugt von 1 bis 2 Mol, bezogen auf 1 Mol der Verbindungen der allgemeinen Formel (II) eingesetzt.

Die Reaktion erfolgt im allgemeinen in einem Temperaturbereich von -78°C bis zu Rückflußtemperatur, bevorzugt von -78°C bis +20°C.

Die Umsetzung kann bei normalem, erhöhtem oder bei erniedrigtem Druck durchgeführt werden (z.B. von 0,5 bis 5 bar). Im allgemeinen arbeitet man bei Normaldruck.

Als Derivatisierungen im Rahmen der Erfindung seien vorzugsweise Umsetzungen am Rest $R^1$ mit Substituentengruppen $(C_1$-$C_6)$-Alkoxy, $-NR^{14}R^{15}$, $-NR^{16}$-$COR^{17}$-, $-NR^{18-}CO^2R^{19}$ und $-CO-NR^{20}R^{21}$ genannt. Ausgehend von den Carbonsäurensubstituierten Arylen werden diese mit den entsprechenden Aminen in inerten Lösemitteln und in Anwesenheit eines Hilfsstoffes umgesetzt. Ebenso ist eine Curtius-Umlagerung in Anwesenheit von $(C_6H_5O)_2$-$PON_3$ möglich. Ebenso ist es möglich, ausgehend von aminosubstituierten Arylen ($R^1$) durch die entsprechenden Säurechloride in Anwesenheit von Basen oder durch die entsprechenden Carbonsäuren in Anwesenheit eines Hilfstoffes, die Amidfunktion einzuführen.

Die Derivatisierungen können durch folgendes Formelschema beispielhaft erläutert werden:

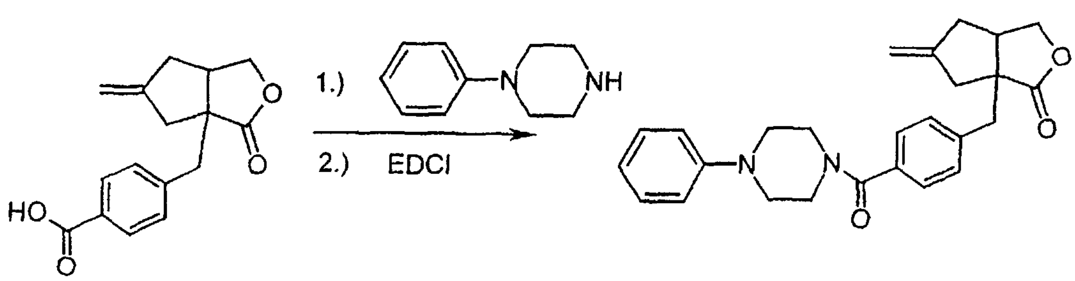


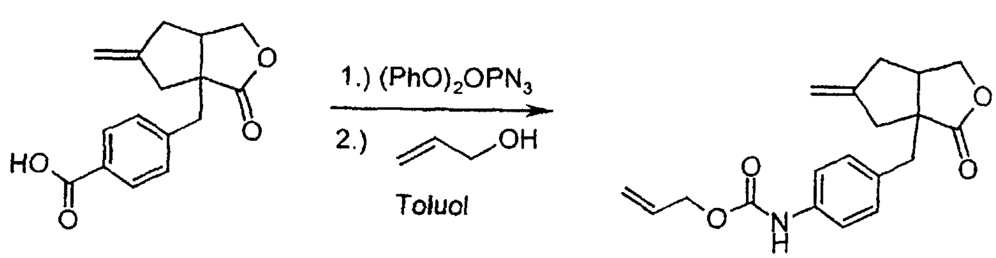

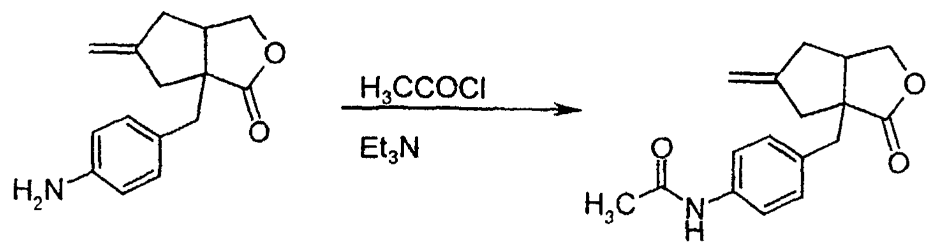


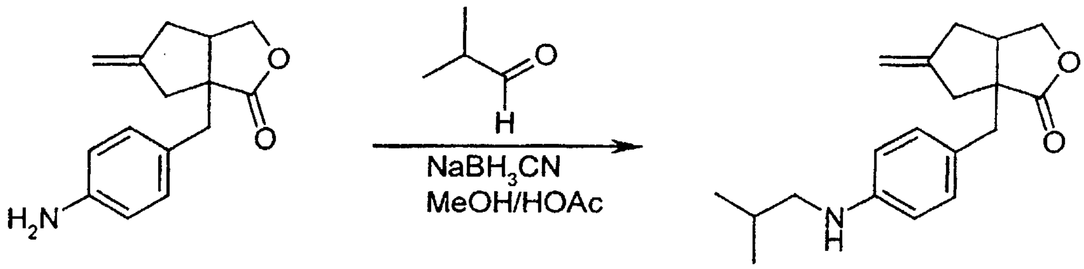


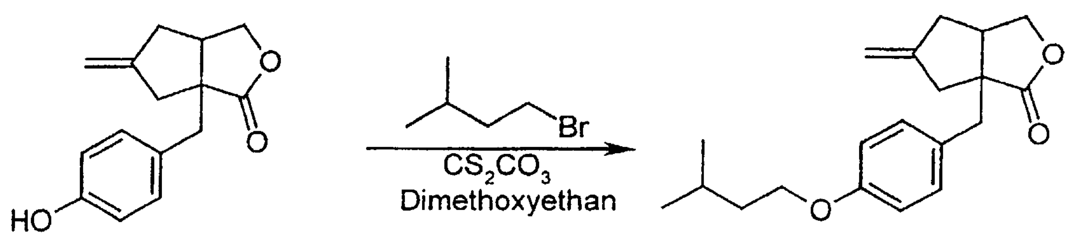


Die Amidierung erfolgt im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, Trichlorethan, Tetrachlorethan, 1,2-Dichlorethan oder Trichlorethylen, Kohlenwasserstoff wie Benzol, Xylol, Toluol, Hexan, Cyclohexan, oder Erdölfraktionen, Nitromethan, Dimethylformamid, Acetonitril oder Hexamethylphosphorsäuretriamid. Ebenso ist es möglich, Gemische der Lösemittel einzusetzen. Besonders bevorzugt ist Dichlormethan.

Als Basen für die Derivatisierungen eignen sich die üblichen basischen Verbindungen. Hierzu gehören vorzugsweise Alkali- oder Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.-butylat, oder organische Amine wie Benzyltrimethylammoniumhydroxid, Tetrabutylammoniumhydroxid, Pyridin, Triethylamin oder N-Methylpiperidin.

Die Derivatisierungen werden im allgemeinen in einem Temperaturbereich von -20°C bis 150°C, bevorzugt bei 0°C bis 25°C, durchgeführt.

Die Derivatisierungen werden im allgemeinen bei Normaldruck durchgeführt. Es ist aber auch möglich, die Verfahren bei Unterdruck oder bei Überdruck durchzuführen (z.B. in einem Bereich von 0,5 bis 5 bar).

Bei der Durchführung der Derivatisierungen werden die Basen im allgemeinen in einer Menge von 1 bis 3 Mol, bevorzugt von 1 bis 1,5 Mol, bezogen auf 1 mol der jeweiligen Carbonsäure, eingesetzt.

Als Dehydratisierungsreagenzien eignen sich Carbodiimide wie beispielsweise Diisopropylcarbodiimid, Dicyclohexylcarbodiimid oder N-(3-Dimethylaminopropyl)-N'ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexyfluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Außerdem wurde ein Verfahren gefunden zur Herstellung der erfindungsgemäßen Verbindungen der allgemeinen Formel (I')

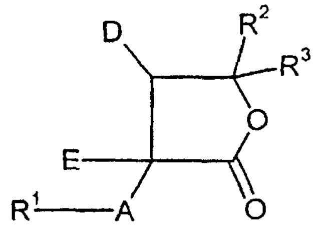

(I'),

in welcher
A, $R^1$, $R^2$ und $R^3$ die oben angegebene Bedeutung haben,
und
D und E gemeinsam Reste der Formeln

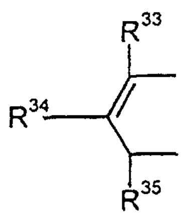

oder

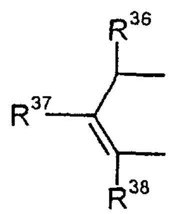

bilden,
worin
$R^{33}$, $R^{35}$, $R^{36}$ und $R^{38}$ für Wasserstoff stehen, und
$R^{34}$ und $R^{37}$ die oben angegebene Bedeutung haben,
dadurch gekennzeichnet, daß man
eine Verbindung der allgemeinen Formel (I")

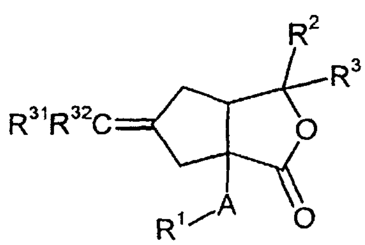

(I"),

in welcher
A, $R^1$, $R^2$ $R^3$, $R^{31}$ und $R^{32}$ die oben angegebene Bedeutung haben,
in Gegenwart eines Katalysators und gegebenenfalls eines Lösemittels isomerisiert.

Das erfindungsgemäße Verfahren kann durch folgendes Formelschema beispielhaft erläutert werden:

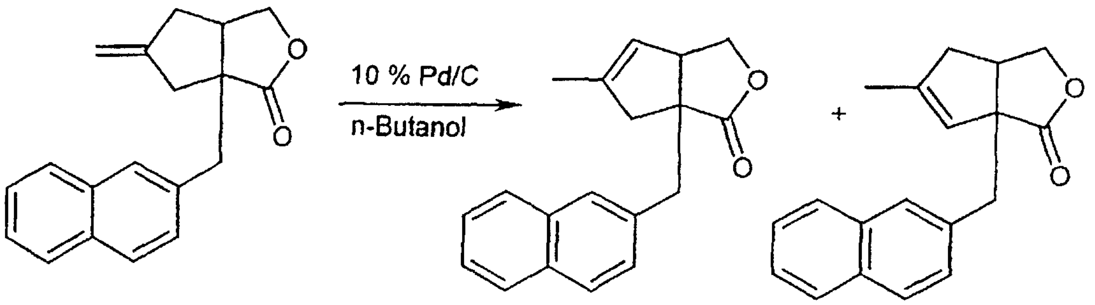


Als Lösemittel eignen sich beispielsweise Alkohole.

Bevorzugt ist n-Butanol.

Als Katalysatoren eignen sich Übergangsmetalle wie zum Beispiel Palladium, Platin oder Rhodium, bevorzugt Palladium, in einer Menge von 0,01 bis 1 Äquivalent bezogen auf die eingesetzte Masse der Verbindung der allgemeinen Formel (I''), bevorzugt 0,05 bis 0,2 Äquivalente.

Ganz besonders bevorzugt ist Palladium adsorbiert auf Aktivkohle.

Die Reaktion erfolgt im allgemeinen in einem Temperaturbereich von 80 bis 200°C, bevorzugt von 100 bis 150°C.

Die Umsetzung kann bei normalem, erhöhtem oder bei erniedrigtem Druck durchgeführt werden (z.B. 0,5 bis 5 bar). Im allgemeinen arbeitet man bei Normaldruck.

Die vorliegende Erfindung betrifft außerdem Verbindungen der allgemeinen Formel (II),

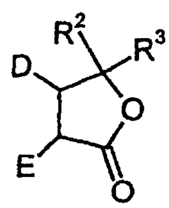

(II)

in welcher

$R^2$ und $R^3$ gleich oder verschieden sind und für Wasserstoff oder ($C_1$-$C_6$)-Alkyl stehen,

und

D und E gemeinsam Reste der Formeln

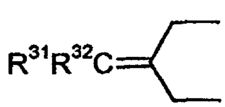

,

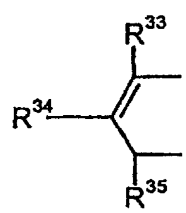

oder

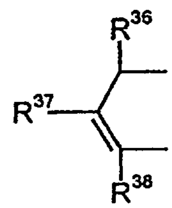

bilden,
worin
$R^{31}$, $R^{32}$, $R^{33}$, $R^{34}$, $R^{35}$, $R^{36}$, $R^{37}$ und $R^{38}$ gleich oder verschieden sind und Wasserstoff, Phenyl oder ($C_1$-$C_6$)-Alkyl bedeuten,
mit der Ausnahme von (3aS*, 6aR*)-, (3aS*, 6S*, 6aR*)-6-Methyl- und (3aS*, 6R*, 6aR*)-6-Methyl-3,3a,6,6a-tetrahydro-1H-cyclopenta*[c]*furan-1-on. Bevorzugt sind Verbindungen der allgemeinen Formel (II), in welcher

$R^2$ und $R^3$ für Wasserstoff oder Methyl stehen,

und
D und E gemeinsam Reste der Formeln

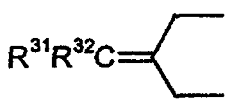

oder

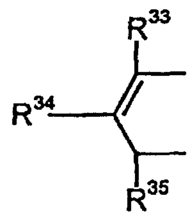

bilden,
worin
$R^{31}$, $R^{32}$, $R^{33}$, $R^{34}$ und $R^{35}$ Wasserstoff bedeuten,
mit der Ausnahme von (3aS*, 6aR*)-, (3aS*, 6S*, 6aR*)-6-Methyl- und (3aS*, 6R*, 6aR*)-6-Methyl-3,3a,6,6a-tetrahydro-1H-cyclopenta*[c]*furan-1-on.

Außerdem wurden Verfahren gefunden zur Herstellung der erfindungsgemäßen Verbindungen der allgemeinen Formel (II)

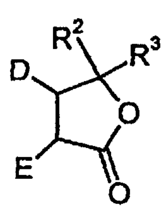

(II),

in welcher
D, E, $R^2$ und $R^3$ die oben angegebene Bedeutung haben,
sind dadurch gekennzeichnet, daß man bei

[A] Verbindungen der allgemeinen Formel (IV)

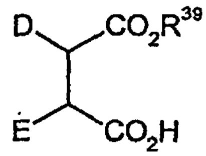

(IV),

in welcher

D und E die oben angegebene Bedeutung haben

und

$R^{39}$ oder für $(C_1-C_4)$-Alkyl oder $(C_2-C_4)$-Alkenyl steht, die gegebenenfalls durch Phenyl substituiert sind,

selektiv die Estergruppe reduziert und das Reaktionsprodukt unter sauren Bedingungen, gegebenenfalls nach Voraktivierung der Carboxylgruppe zum Lacton cyclisiert,
oder

[B] Verbindungen der allgemeinen Formel (V)

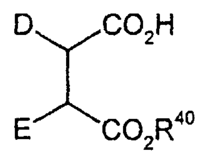

(V),

in welcher

D und E die oben angegebene Bedeutung haben

und

$R^{40}$ für $(C_1-C_4)$-Alkyl oder $(C_3-C_4)$-Alkenyl steht, die gegebenenfalls durch Phenyl substituiert sein können,

selektiv die Carboxylgruppe reduziert und das Reaktionsprodukt zum Lacton cyclisiert,
oder

[C] Verbindungen der allgemeinen Formel (VI)

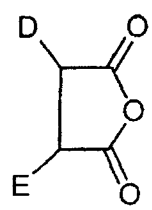

(VI),

in welcher

D und E die oben angegebene Bedeutung haben,

zuerst unter geeigneten Reduktionsbedingungen zu einem Hydroxylacton reduziert und anschließend in

einem inerten Lösemittel mit einer Verbindung der allgemeinen Formel (VII),

$$R^{2'}\text{-}Q \qquad (VII),$$

in welcher

$R^{2'}$ für ($C_1$-$C_6$)-Alkyl steht, und

Q für ein Alkali- oder Erdalkalimetallhalogenid, bevorzugt Mg-X, steht,

umsetzt und unter sauren Bedingungen zum entsprechenden Lacton cyclisiert,
oder

[D] Verbindungen der allgemeinen Formel (VI) in einem inerten Lösemittel mit mindestens zwei Mol-Äquivalenten einer Verbindung der allgemeinen Formel (VII) umsetzt und unter sauren Bedingungen zum entsprechenden Lacton cyclisiert,
oder

[E] Verbindungen der allgemeinen Formel (VI) in einem inerten Lösemittel zuerst mit einem Mol-Äquivalent einer Verbindung der allgemeinen Formel (VII) umsetzt, und dann mit mindestens einem weiteren Mol-Äquivalent einer Verbindung der allgemeinen Formel (VIII)

$$R^{3'}\text{-}Q' \qquad (VIII),$$

in welcher

$R^{3'}$ für ($C_1$-$C_6$)-Alkyl steht und

Q' die oben angegebene Bedeutung von Q hat und mit dieser gleich oder verschieden ist,

umsetzt und unter sauren Bedingungen zum entsprechenden Lacton cyclisiert.

Die erfindungsgemäßen Verfahren können durch folgende Formelschemata beispielhaft erläutert werden:

[A]

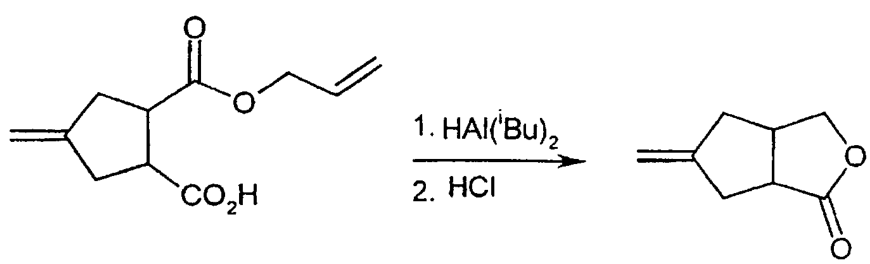


[B]

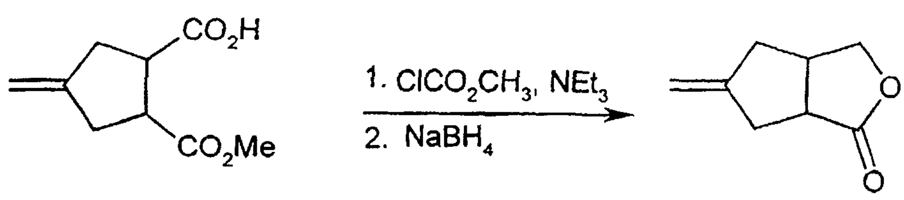

[C]

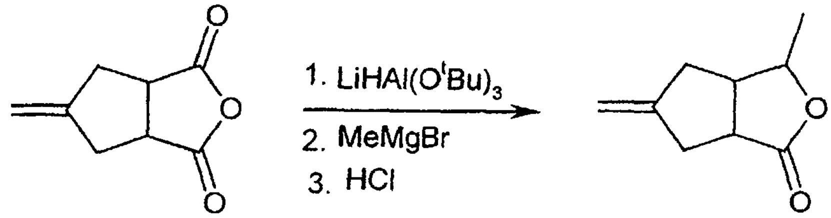


[D]

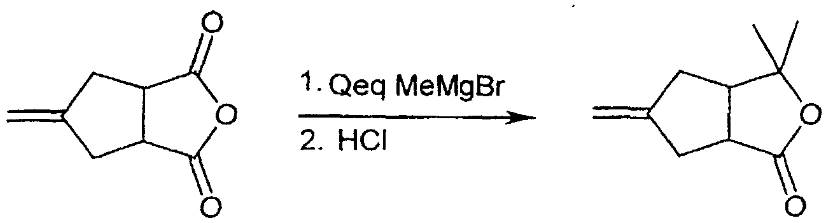


Als Reduktionsmittel für das Verfahren [A] eignen sich komplexe Metallhydride.

Bevorzugt ist Diisobutylaluminiumhydrid.

Als Lösemittel eignen sich inerte Lösemittel, wie zum Beispiel Methylenchlorid, THF, Dioxan, Diethylether, Toluol, 1,2-Dichlorethan.

Bevorzugt ist Methylenchlorid.

Die Reaktion erfolgt im allgemeinen in einem Temperaturbereich von -40°C bis zu Rückflußtemperatur des Lösemittels, bevorzugt von 0°C bis 30°C.

Gegebenenfalls kann die Cyclisierung der intermediären Hydroxycarbonsäure durch Aktivierung der Carboxylgruppe, zum Beispiel mit Chlorameisensäurealkylester, bevorzugt Chlorameisensäuremethylester, in Gegenwart einer Base, wie zum Beispiel Triethylamin, unterstützt werden.

Als Reduktionsmethode für Verfahren [B] eignet sich eine schrittweise Reduktion durch Voraktivierung der Carboxylgruppe mit Chlorameisensäurealkylester, bevorzugt Chlorameisensäuremethylester, in Gegenwart einer Base wie zum Beispiel Triethylamin, gefolgt von einer Reduktion mit einem komplexen Metallhydrid, wie zum Beispiel einem Borhydrid, bevorzugt Natriumborhydrid.

Als Lösemittel für die Aktivierung eignen sich inerte Lösemittel wie Diethylether, THF, Methylenchlorid. Als Lösemittel für die Reduktion mit Borhydriden eignen sich z.B. Alkohole, insbesondere Methanol.

Die Reaktion erfolgt im allgemeinen in einem Temperaturbereich von -40°C bis 40°C, bevorzugt von -20°C bis 30°C.

Als Reduktionsmittel für Verfahren [C] eignen sich komplexe Metallhydride mit reduzierter Reaktivität, wie z. B. Lithium-tris-tert.-butoxyaluminiumhydrid.

Als Lösemittel dafür eignen sich inerte Lösemittel, wie zum Beispiel Diethylether oder THF.

Die Reaktion erfolgt im allgemeinen in einem Temperaturbereich von -78°C bis 0°C, bevorzugt von -50°C bis -20°C.

Als inertes Lösemittel für die Umsetzung mit den Verbindungen der allgemeinen Formeln (VII) und (VIII) in Verfahren [C] bis [E] eignen sich Ether, bevorzugt Diethylether oder THF.

Die Reaktionen erfolgen im allgemeinen in einem Temperaturbereich von -78°C bis 35°C, bevorzugt bei -60°C bis 25°C.

Als Säuren für die Cyclisierung zu den Lactonen eignen sich insbesondere Mineralsäuren, wie z.B. verdünnte wäßrige Schwefelsäure oder Salzsäure.

Die Verbindungen der allgemeinen Formeln (IV) und (V) sind an sich bekannt oder nach üblichen Methoden herstellbar.

Die erfindungsgemäßen Verbindungen der allgemeinen Formel (I) eignen sich zur Verwendung als Medikamente in der Behandlung von Menschen und Tieren.

Die erfindungsgemäßen Verbindungen der allgemeinen Formel (I) eignen sich zur Modulation von metabotropen Glutamatrezeptoren und beeinflussen daher das glutamaterge Neurotransmittersystem.

Ein Modulator des metabotropen Glutamatrezeptors im Sinne der Erfindung ist ein Agonist oder Antagonist dieses Rezeptors.

Die erfindungsgemäßen Verbindungen sind besonders als Modulatoren des metabotropen Glutamatrezeptors vom Subtyp 1 geeignet, ganz besonders als Antagonisten dieses Rezeptorsubtyps.

Die erfindungsgemäßen Verbindungen können aufgrund ihrer pharmakologischen Eigenschaften allein oder in Kombination mit anderen Arzneimitteln zur Behandlung und/oder Prävention von neuronalen Schädigungen oder Erkrankungen, die mit pathophysiologischen Zuständen des glutamatergen Systems im zentralen und peripheren Nervensystem in Verbindung gebracht werden, eingesetzt werden.

Zur Behandlung und/oder Prävention von neuronalen Schädigungen beispielsweise verursacht durch ischämischen, thromb- und/oder thrombemolischen, und hämorrhagischen Schlaganfall, Zuständen nach direkten und indirekten Verletzungen im Bereich des Gehimes und des Schädels. Femer zur Behandlung und/oder Prävention von cerebralen Ischämien nach operativen Eingriffen am Gehirn oder peripheren Organen bzw. Körperteilen und damit einhergehenden oder vorausgehenden Zuständen krankhafter bzw. allergischer Natur, die primär und/oder sekundär zu einer neuronalen Schädigung führen können.

Gleichfalls eignen sich die erfindungsgemäßen Verbindungen auch zur Therapie von primären und/oder sekundären krankhaften Zuständen des Gehirnes, beispielsweise während oder nach cerebralen Vasospasmen, Hypoxie und/oder Anoxie nicht vorher genannter Genese, perinataler Asphyxie, Autoimmunerkrankungen, Stoffwechsel- und Organerkrankungen, die mit einer Schädigung des Gehirnes einhergehen können sowie Schädigungen des Gehimes infolge primärer Gehimerkrankungen beispielsweise Krampfleiden und artero- und/oder arteriosklerotischer Veränderungen. Zur Behandlung chronischer oder psychiatrischer Leiden wie beispielsweise Depression, neurodegenerativer Erkrankungen wie beispielsweise Alzheimersche, Parkinsonsche oder Huntingtonsche Erkrankung, Multiple Sklerose, amyotrophische laterale Sklerose, Neurodegeneration durch akute und/oder chronische virale oder bakterielle Infektionen und Multiinfarktdemenz.

Darüberhinaus können sie als Arzneimittel eingesetzt werden zur Behandlung von Demenzen unterschiedlichen Ursprungs, Hirnleistungsstörungen im Alter, Gedächtnisstörungen, Rückenmarksverletzungen, Schmerzzuständen, Angstzuständen unterschiedlichen Ursprungs, medikamentös bedingtem Parkinson-Syndrom, Psychosen (wie zum Beispiel Schizophrenie), Hirnödemen, neuronalen Schädigungen nach Hypoglykämie, Emesis, Übelkeit, Fettsucht, Suchterkrankungen und Entzugserscheinungen, ZNS-vermittelten Krämpfen, Sedation sowie Bewegungsstörungen.

Außerdem können die erfindungsgemäßen Verbindungen der allgemeinen Formel (I) verwendet werden zur Förderung der neuronalen Regeneration in der post-akuten Phase cerebraler Verletzungen oder chronischer Erkrankungen des Nervensystems.

Bevorzugt werden sie als Arzneimittel eingesetzt zur Behandlung von cerebralen Ischämien, Schädel-/Himtrauma, Schmerzzuständen oder ZNS-vermittelten Krämpfen (wie zum Beispiel Epilepsie).

Die Modulation von Substanzen am metabotropen Glutamatrezeptor (direkte oder indirekte Beeinflussung der Kopplungseffizienz des Glutamat-Rezeptors an G-Proteinen) kann an primären Körnerzellkulturen aus dem Kleinhirn überprüft werden. Elektrophysiologische Messungen an diesen Zellkulturen im "cell attached"-Modus zeigen, daß L-Typ-$Ca^{2+}$-Kanäle in dieser Präparation durch mGluR1-Rezeptoren aktiviert werden (J. Neurosci. 1995, 15, 135), wohingegen sie durch Gruppe II Rezeptoren blockiert werden (J. Neurosci. 1994, 14, 7067-7076). Durch entsprechende experimentelle Anordnung kann die modulatorische Wirkung von pharmakologischen Prüfsubstanzen an Glutamatrezeptoren kontrolliert werden. Eine detaillierte Überprüfung der Subtypspezifität unter kontrollierten Bedingungen kann an *Xenopus*-Oocyten durch Injektion der entsprechenden mGluR-Subtyp-DNA erfolgen (WO 92/10583).

**Permanente focale cerebrale Ischämie bei der Ratte (MCA-O)**

Unter Isofluran-Anästhesie wird die Arteria cerebri media einseitig freipräpariert, mittels Elektrokoagulation diese und deren Nebenäste irreversibel verschlossen. Als Folge des Eingriffs entsteht ein cerebraler Infarkt. Während der Operation wird die Körpertemperatur des Tieres auf 37°C gehalten. Nach Wundverschluß und Abklingen der Narkose werden die Tiere wieder in ihren Käfig entlassen. Die Substanzapplikation erfolgt nach unterschiedlichen zeitlichen Schemata und über unterschiedliche Applikationswege (i.v., i.p.) nach der Okklusion. Die Infarktgröße wird nach 7 Tagen bestimmt. Dazu wird das Gehirn entnommen, histologisch aufgearbeitet und mit Hilfe eines computergestützten Auswertsystemes das Infarktvolumen bestimmt.

| Wirksamkeit in dem Modell der permanenten focalen cerebralen Ischämie (MCA-O) | | |
|---|---|---|
| Beispiel | % Reduktion des Infarktvolumens | Dosis[a)] |
| 35 | 38 | 0,01 mg/kg/h |

a) Substanzgabe als intravenöse, kontinuierliche Infusion direkt bis 4 Stunden nach der Okklusion

**Subdurales Hämatom bei der Ratte (SDH)**

Unter Anästhesie wird den Tieren einseitig subdural Eigenblut injiziert. Unter dem Hämatom bildet sich ein Infarkt. Die Substanzapplikation erfolgt nach unterschiedlichen zeitlichen Schemata und über unterschiedliche Applikationswege (i.v., i.p.).

Die Bestimmung der Infarktgröße erfolgt wie beim Modell der Permanenten focalen Ischämie bei der Ratte (MCA-O) beschrieben.

| Wirksamkeit in dem Modell "Subdurales Hämatom bei der Ratte (SDH)" | | |
|---|---|---|
| Beispiel | % Reduktion des Infarktvolumens | Dosis[a)] |
| 35 | 51 | 0,01 mg/kg/h |

a) Substanzgabe als intravenöse, kontinuierliche Infusion direkt bis 4 Stunden post-Trauma

Auf antiepileptische Wirkung kann nach der in NeuroReport 1996, 7, 1469-1474 beschriebenen Methode getestet werden.

Die Eignung der erfindungsgemäßen Verbindungen zur Behandlung von Schizophrenie kann nach den in Science 1998, 218, 1349-1352 und Eur. J. Pharmacol. 1996, 316, 129-136 beschriebenen Methoden bestimmt werden.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfsoder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

**Allgemeines**

**Laufmittel zur Chromatographie**

I Dichlormethan / Methanol
II Dichlormethan / Ethanol
III Cyclohexan / Ethylacetat
IV Cyclohexan / Dichlormethan
V Butylacetat: Butanol:Essigsäure: Phosphat-Puffer (pH = 6) = 200:26:100:60

**Abkürzungen**

DME 1,2-Dimethoxyethan
HMPA Hexamethylphosphorsäuretriamid
LiHMDS Lithiumbistrimethylsilylamid
LDA Lithiumdiisopropylamid
MTBE Methyl-tert.butylether

THF Tetrahydrofuran
MPLC Mitteldruckflüssigchromatographie

**Ausgangsverbindungen**

**Beispiel 1A**

(3aS*,6aR*)-5-Methyliden-hexahydro-cyclopenta[c]furan-1-on

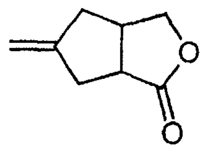

Eine Lösung von 2-Methoxycarbonyl-4-methylidencyclopentancarbonsäure (189,2 g; 1,027 mol) in THF (11) wurde bei -15°C mit Triethylamin(156,6 ml; 1,130 mol) und Chlorameisensäureethylester (18,2 ml; 1,027 mol) versetzt und das Reaktionsgemisch 1 h bei Raumtemperatur gerührt. Der Niederschlag wurde abgesaugt, das Filtrat eingeengt, in Methanol (11) aufgenommen, bei -15°C portionsweise $NaBH_4$ (97,146 g; 2,568 mol) zugegeben und 1 h bei Raumtemperatur gerührt. Zur Aufarbeitung wurde mit 1 N HCl versetzt, mit NaCl gesättigt und mit Essigester extrahiert. Die vereinigten organischen Phasen wurden getrocknet ($Na_2SO_4$), eingeengt und das Rohprodukt durch Chromatographie gereinigt.
Ausbeute: 82,03 g (58%)
$R_f$ (II, 50:1) = 0,42
MS (EI): m/z = 138 [$M^+$]

**Beispiel 2A**

(-)-(3aS,6aR)-5-Methyliden-hexahydro-cyclopenta[c]furan-1-on

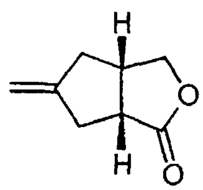

In Analogie zur Vorschrift des Beispiels 1A wurde aus (1R,2S)-4-Methyliden-2-(3-phenyl-2-propenyloxycarbonyl)-cyclopentancarbonsäure (31,5 g; 110,2 mmol; 97% ee; Beispiel 1 in EP 805 145A1, S. 9) die Zielverbindung dargestellt.
Ausbeute: 7,97 g (52 %; 97 % ee)
$R_f$ (I, 80:1) = 0,56

**Beispiel 3A**

(+)-(3aR, 6aS)-5-Methyliden-hexahydro-cyclopentan[c]furan-1-on

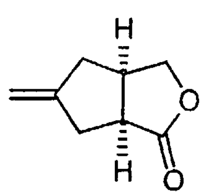

Zu einer Lösung von (1S,2R)-4-Methyliden-2-allyloxycarbonyl-cyclopentan-carbonsäure (2,0 g; 9,61 mmol; 75 % ee; Beispiel 5 in DOS 44 00 749, S. 11+12) in Dichlormethan (50 ml) wurde bei 10°C eine Lösung von Diisobutylaluminiumhydrid (1,5 M in $CH_2Cl_2$; 17,7 ml; 26,58 mmol) zugetropft und 1 h bei Raumtemperatur gerührt. Man versetzte mit 1 N HCl und Wasser und extrahierte mit Essigester. Die vereinigten organischen Phasen wurden getrocknet ($Na_2SO_4$), das Lösemittel abgezogen und der Rückstand in THF (200 ml) aufgenommen. Bei 0°C wurden Triethylamin (6,59 ml; 47,57 mmol) und Chlorameisensäureethylester (2,27 ml; 23,78 mmol) zugegeben und das Reaktionsgemisch 14 h bei 8°C stehen gelassen. Anschließend wurde mit Essigester und Wasser versetzt. Die organische Phase wurde mit 10 % wässriger HCl, gesättigter NaCl-Lösung, 10 % wässriger $NaHCO_3$-Lösung und gesättigter NaCl-Lösung gewaschen, getrocknet ($MgSO_4$), eingeengt und der Rückstand durch MPLC gereinigt.
Ausbeute: 260 mg (20 % , 89 % ee)
$R_f$(I, 10:1)=0,88

**Beispiel 4A**

(3S*,3aR*,6aS*)-3-Methyl-5-methyliden-hexahydro-cyclopenta[c]furan-1-on

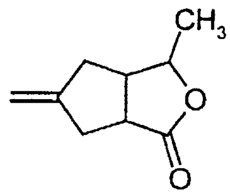

Zu einer Lösung von Lithiumtri-tert.-butoxyaluminiumhydrid (1M in THF, 82,16 ml) wurde bei -40°C eine Lösung von 4-Exomethylen-1,2-cyclopentandicarbonsäureanhydrid (10,0 g, 65,7 mmol; Ref. DE 4400749) in THF (130 ml) gegeben und das Reaktionsgemisch 14 h bei -30°C gerührt. Zur Aufarbeitung wurde mit 1 N HCl versetzt, das THF im Vakuum abgezogen, die wäßrige Phase mit NaCl gesättigt und mit Dichlormethan extrahiert. Die vereinigten organischen Phasen wurden getrocknet ($MgSO_4$), das Lösemittel abgezogen und der Rückstand durch MPLC gereinigt. Ausbeute: 2,24 g.
Das so erhaltene Zwischenprodukt (1 g; 6,47 mmol) in Diethylether (15 ml) wurde bei 0°C zu einer Lösung von Methylmagnesiumbromid (3M in Diethylether, 4,3 ml, 12,0 mmol) gegeben und das Reaktionsgemisch 2 h bei Raumtemperatur gerührt. Man versetzte mit 10 % wässriger HCl, rührte bei Raumtemperatur, extrahierte mit Essigester, trocknete die vereinigten organischen Phasen ($Na_2SO_4$), zog im Vakuum die Lösemittel ab und reinigte das Rohprodukt durch MPLC.
Ausbeute: 412 mg (33 %)
$R_f$ ($CH_2Cl_2$) = 0,69
MS (DCI / $NH_3$): m/z = 170 [$M+NH_4^+$]

**Beispiel 5A**

(3aS*,6aR*)-3,3-Dimethyl-5-methyliden-hexahydro-cyclopenta[c]furan-1-on

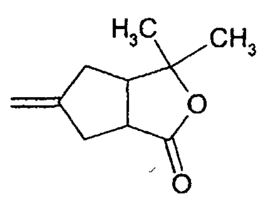

Zu einer Lösung von Methylmagnesiumbromid (3 M in Diethylether, 6,6 ml; 19,7 mmol) wurde bei 0°C eine Lösung von 4-Exomethylen-1,2-cyclopentandicarbon-säureanhydrid (1,0 g; 6,5 mmol) in Diethylether (40 ml) gegeben und das Reaktionsgemisch 14 h bei Raumtemperatur gerührt. Man versetzte mit 10 % wässriger HCl, sättigt mit Natriumchlorid, extrahiert mit Essigester, wäscht die vereinigten organischen Phasen mit 10 % wässriger $Na_2CO_3$ und gesättiger NaCl-Lösung und trocknet ($Na_2SO_4$). Nach Abziehen der Lösemittel erhält man die Titelverbindung.

Ausbeute: 929 mg (85 %)
$R_f$(I, 10:1)=0,24
MS (EI): m/z = 166[$M^+$]

**Herstellungsbeispiele**

**Beispiel 1**

(3aS*,6aS*)-6a-Benzyl-5-methyliden-hexahydro-cyclopenta[c]furan-1-on

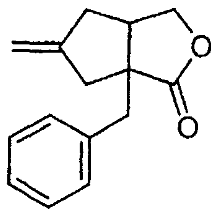

Zu einer Lösung von LiHMDS (1M in THF, 0,65 ml, 0,65 mmol) verdünnt mit Toluol (7 ml) wurde bei -78°C eine Lösung der Verbindung aus Beispiel 1A (90 mg, 0,65 mmol) in Toluol (3 ml) gegeben. Man ließ auf Raumtemperatur kommen, rührte weitere 60 min und versetzte anschließend mit dem Alkylierungsreagenz (Benzylbromid, 89,1 mg, 0,521 mmol). Nach 14h bei Raumtemperatur wurde Wasser (1 ml) zugegeben, das Reaktionsgemisch über eine Fritte, gefüllt mit Bitumenerde/Kieselgel filtriert, die Lösemittel abgezogen und das Rohprodukt gegebenenfalls durch MPLC gereinigt;
Ausbeute: 81 mg (68 %)
$R_f$ (I, 80:1) = 0,67
MS (EI): m/z = 229 [$M+H^+$]

In Analogie zur Vorschrift des Beispiels 1 wurden die in der folgenden Tabelle aufgeführten Beispiele 2 bis 97 dargestellt. Als Alkylierungsreagenzien wurden die entsprechenden Halogenide, Aldehyde bzw. Ester eingesetzt.

| Bsp. Nr. | Struktur | Edukt Bsp.-Nr. | Aus-beute [%] | Rf | MS |
|---|---|---|---|---|---|
| 2 | 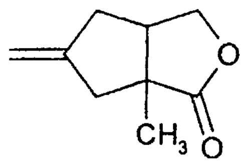 | 1A | 18 | 0,23 (III, 50:1) | 153 [M+H+] |
| 3 | 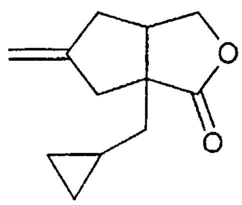 | 1A | 8 | 0,582 (I, 80:1) | 210 [M+NH4+] |
| 4 | 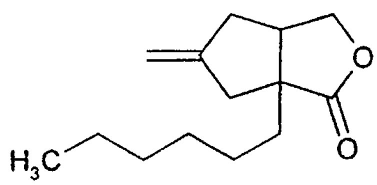 | 1A | 66 | 0,73 (I, 80:1) | 223 [M+H+] |
| 5 | 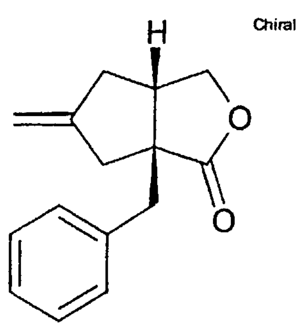  | 2A | 65 | 0,45 (III, 5:1) | 229 [M+H+] |
| 6 | 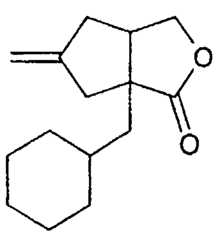 | 1A | 13 | 0,47 (III, 5:1) | 235 [M+H+] |

| Bsp. Nr. | Struktur | Edukt Bsp.-Nr. | Ausbeute [%] | Rf | MS |
|---|---|---|---|---|---|
| 7 | 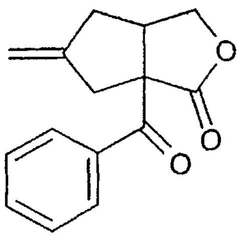 | 1A | 6 | 0,63 (CH2Cl2) | 260 [M+NH4+] |
| 8 | 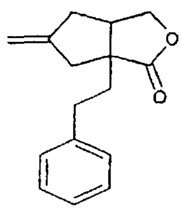 | 1A | 2 | 0,16 (III, 5:1) | 265 [M+Na+] |
| 9 | 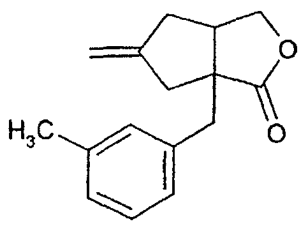 | 1A | 90 | 0,44 (III, 5:1) | 284 [M+CH3CN+H+] |
| 10 | 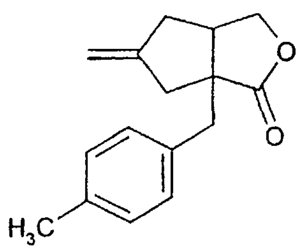 | 1A | 79 | 0,44 (III, 5:1) | 284 [M+CH3CN+H+] |
| 11 | 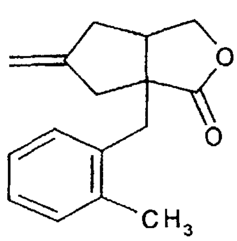 | 1A | 18 | 0,42 (III, 5:1) | 284 [M+CH3CN+H+] |

| Bsp. Nr. | Struktur | Edukt Bsp.-Nr. | Ausbeute [%] | Rf | MS |
|---|---|---|---|---|---|
| 12 | 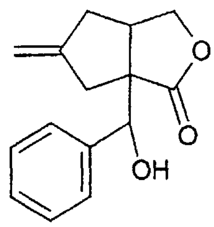 | 1A | 7 | 0,27 (CH2Cl2) | 265 [M+Na+] |
| 13 | 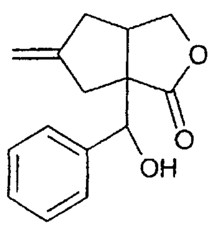 | 1A | 33 | 0,15 (CH2Cl2) | 262 [M+NH4+] |
| 14 | 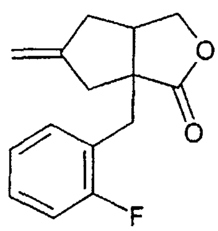 | 1A | 96 | 0,32 (III, 5:1) | 288 [M+CH3CN+ H+] |
| 15 | 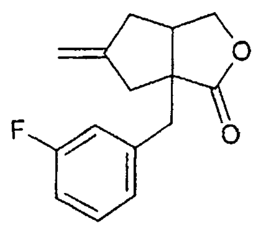 | 1A | 96 | 0,31 (III, 5:1) | 288 [M+CH3CN+ H+] |
| 16 | 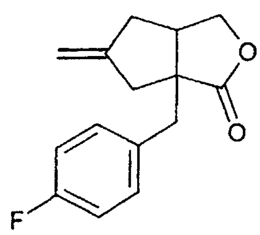 | 1A | 98 | 0,31 (III, 5:1) | 288 [M+CH3CN+ H+] |
| 17 | 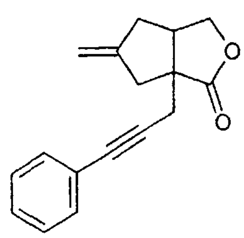 | 1A | 11 | 0,20 (III, 5:1) | 361 [M+H+] |

| Bsp. Nr. | Struktur | Edukt Bsp.-Nr. | Ausbeute [%] | Rf | MS |
|---|---|---|---|---|---|
| 18 | 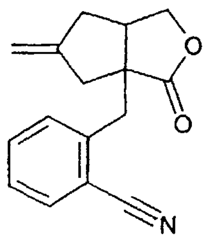 | 1A | 47 | 0,20 (III, 5:1) | 295 [M+CH3CN+H+] |
| 19 | 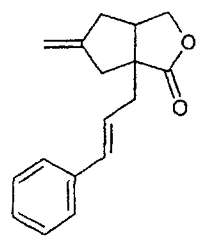 | 1A | 53 | 0,29 (III, 5:1) | 296 [M+CH3CN+H+] |
| 20 | 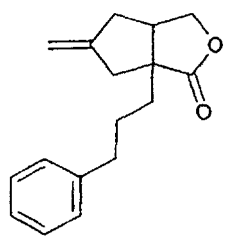 | 1A | 70 | 0,62 (I, 80:1) | 349 [M+H+] |
| 21 | 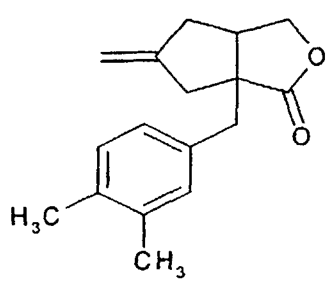 | 1A | 34 | 0,42 (III, 5:1) | 274 [M+NH4+] |
| 22 | 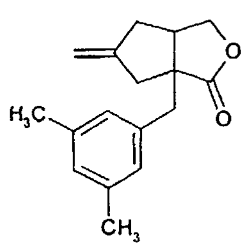 | 1A | 71 | 0,44 (III, 5:1) | 298 [M+CH3CN+H+] |

| Bsp. Nr. | Struktur | Edukt Bsp.-Nr. | Ausbeute [%] | Rf | MS |
|---|---|---|---|---|---|
| 23 | 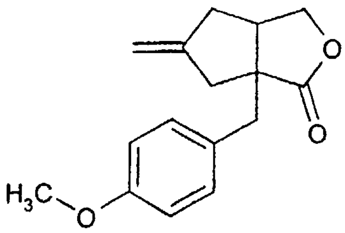 | 1A | 9 | 0,15 (III, 10:1) | 281 [M+Na+] |
| 24 | 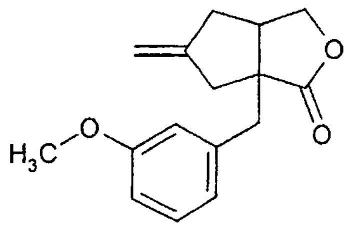 | 1A | 38 | 0,30 (III, 5:1) | 300 [M+CH3CN+ H+] |
| 25 | 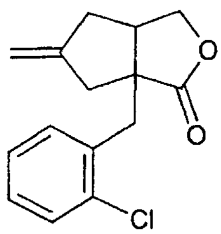 | 1A | 100 | 0,42 (III, 5:1) | 304 [M+CH3CN+ H+] |
| 26 | 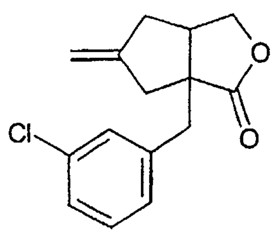 | 1A | 96 | 0,34 (III, 5:1) | 304 [M+CH3CN+ H+] |
| 27 | 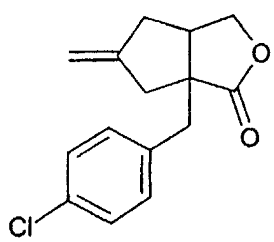 | 1A | 20 | 0,32 (III, 5:1) | 304 [M+CH3CN+ H+] |

| Bsp. Nr. | Struktur | Edukt Bsp.-Nr. | Ausbeute [%] | Rf | MS |
|---|---|---|---|---|---|
| 28 | 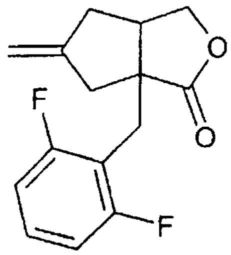 | 1A | 99 | 0,34 (III, 5:1) | 306 [M+CH3CN+H+] |
| 29 | 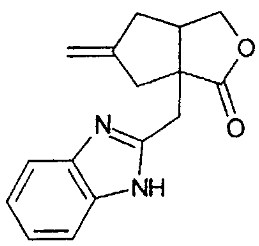 | 1A | 2 | 0,42 (III, 5:1) | 310 [M+CH3CN+H+] |
| 30 | 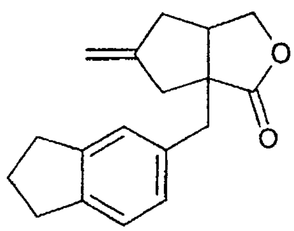 | 1A | 21 | 0,35 (III, 10:1) | 284 [M+NH4+] |
| 31 | 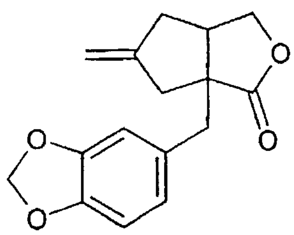 | 1A | 58 | 0,242 (III, 50:1) | 272 [M+] |
| 32 | 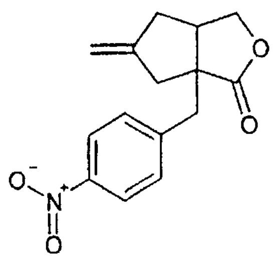 | 1A | 14 | 0,05 (IV, 1:1) | 291 [M+Na+] |

| Bsp. Nr. | Struktur | Edukt Bsp.-Nr. | Ausbeute [%] | Rf | MS |
|---|---|---|---|---|---|
| 33 | 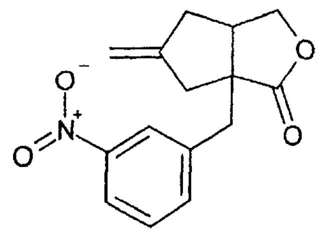 | 1A | 2 | 0,42 (III, 5:1) | 291 [M+NH4+] |
| 34 | 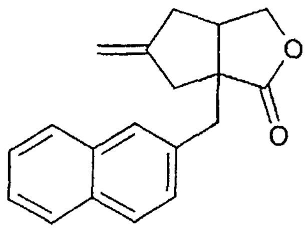 | 1A | 45 | 0,34 (III, 5:1) | 296 [M+NH4+] |
| 35 | 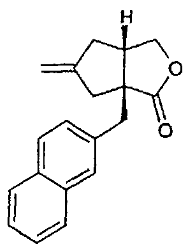 | 2A | 42 | 0,60 (CH2Cl2) | 296 [M+NH4+] |
| 36 | 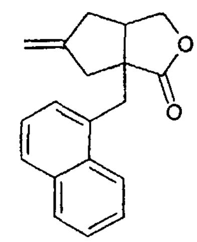 | 1A | 5 | 0,24 (III, 5:1) | 301 [M+Na+] |
| 37 | 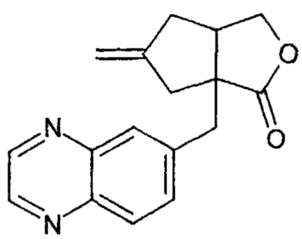 | 1A | 1 | 0,58 (III, 1:1) | 281 [M+H+] |

| Bsp. Nr. | Struktur | Edukt Bsp.-Nr. | Ausbeute [%] | Rf | MS |
|---|---|---|---|---|---|
| 38 | 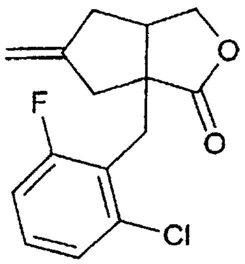 | 1A | 99 | 0,36 (III, 5:1) | 322 [M+CH3CN+H+] |
| 39 | 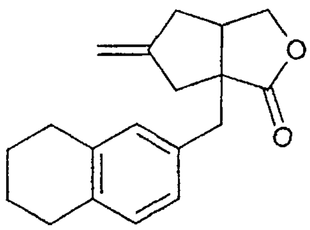 | 1A | 15 | 0,35 (III, 10:1) | 300 [M+NH4+] |
| 40 | 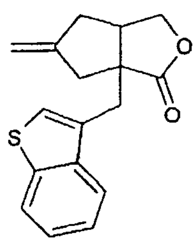 | 1A | 5 | 0,192 (III, 10:1) | 326 [M+CH3CN+H+] |
| 41 | 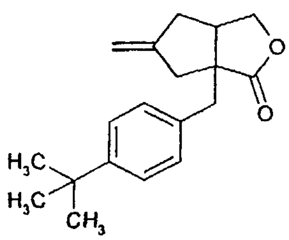 | 1A | 28 | 0,46 (III, 5:1) | 302 [M+NH4+] |
| 42 | 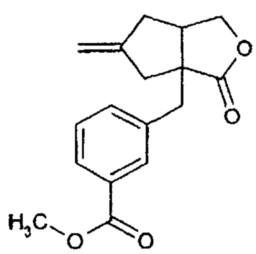 | 1A | 59 | 0,66 (I, 80:1) | 309 [M+Na+] |

| Bsp. Nr. | Struktur | Edukt Bsp.-Nr. | Aus-beute [%] | Rf | MS |
|---|---|---|---|---|---|
| 43 | 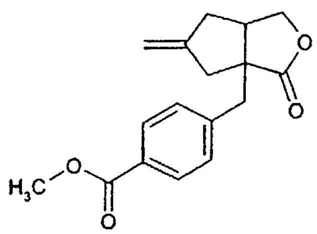 | 1A | 41 | 0,12 (III, 5:1) | 328 [M+CH3CN+ H+] |
| 44 | 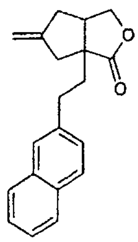 | 1A | 3 | 0,42 (III, 4:1) | 310 [M+NH4+] |
| 45 | 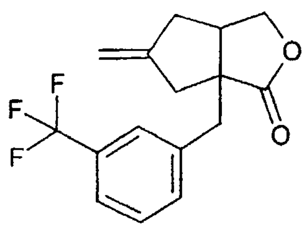 | 1A | 84 | 0,31 (III, 5:1) | 338 [M+CH3CN+ H+] |
| 46 | 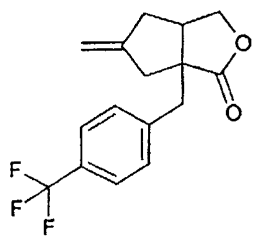 | 1A | 80 | 0,31 (III, 5:1) | 338 [M+CH3CN+ H+] |
| 47 | 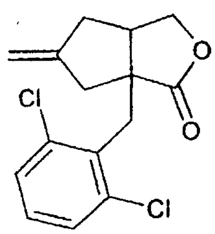 | 1A | 75 | 0,42 (III, 5:1) | 338 [M+CH3CN+ H+] |
| 48 | 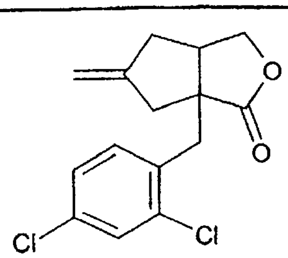 | 1A | 44 | 0,44 (III, 5:1) | - |

| Bsp. Nr. | Struktur | Edukt Bsp.-Nr. | Ausbeute [%] | Rf | MS |
|---|---|---|---|---|---|
| 49 | 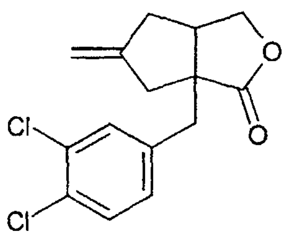 | 1A | 12 | 0,24 (III, 10:1) | 314 [M+NH4+] |
| 50 | 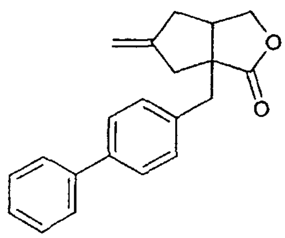 | 1A | 25 | 0,82 (I, 80:1) | 327 [M+Na+] |
| 51 | 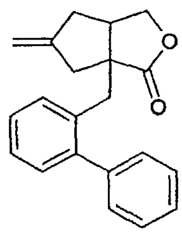 | 1A | 53 | 0,21 (III, 40:1) | 305 [M+H+] |
| 52 | 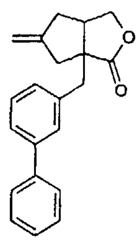 | 1A | 53 | 0,44 (III, 5:1) | 305 [M+H+] |
| 53 | 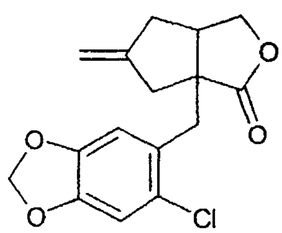 | 1A | 3 | 0,42 (III, 5:1) | 348 [M+CH3CN+H+] |
| 54 | 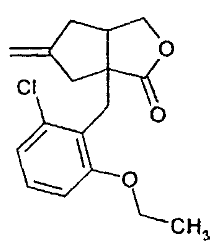 | 1A | 24 | 0,42 (III, 5:1) | 307 [M+H+] |

| Bsp. Nr. | Struktur | Edukt Bsp.-Nr. | Ausbeute [%] | Rf | MS |
|---|---|---|---|---|---|
| 55 | 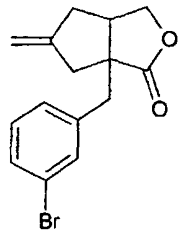 | 1A | 31 | 0,19 (III, 10:1) | 307 / 309 [M+H+] |
| 56 | 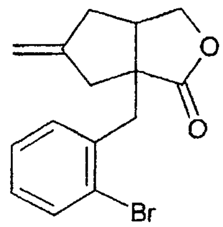 | 1A | 96 | 0,42 (III, 5:1) | 348 [M+CH3CN+ H+] |
| 57 | 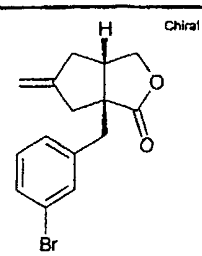 | 2A | 67 | 0,45 (III, 5:1) | 307/309 [M+H+] |
| 58 | 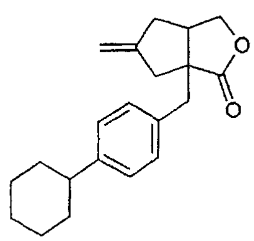 | 1A | 9 | 0,42 (III, 5:1) | 352 [M+CH3CN+ H+] |
| 59 | 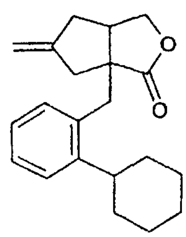 | 1A | 3 | 0,42 (III, 5:1) | 352 [M+CH3CN+ H+] |
| 60 | 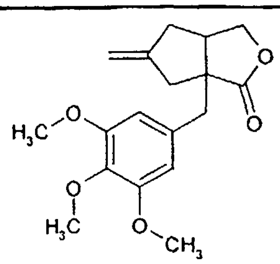 | 1A | 49 | 0,32 (III, 2:1) | 336 [M+H+] |

| Bsp. Nr. | Struktur | Edukt Bsp.-Nr. | Ausbeute [%] | Rf | MS |
|---|---|---|---|---|---|
| 61 | 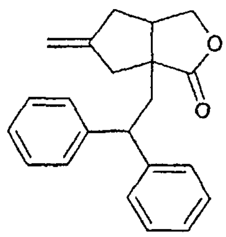 | 1A | 15 | 0,78 (I, 80:1) | - |
| 62 | 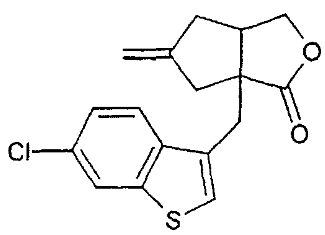 | 1A | 17 | 0,42 (III, 5:1) | 336 [M+NH4+] |
| 63 | 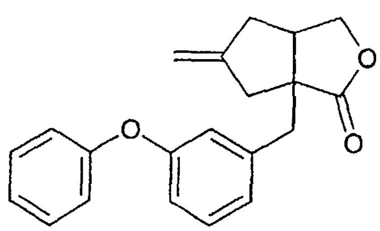 | 1A | 50 | 0,41 (III, 5:1) | 338 [M+NH4+] |
| 64 | 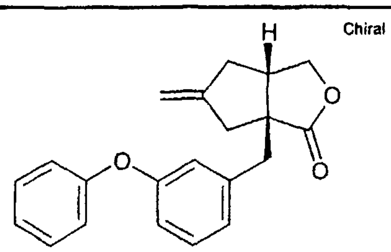 | 2A | 52 | 0,45 (III, 5:1) | 321 [M+H+] |
| 65 | 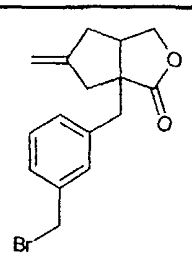 | 1A | 46 | 0,61 (CH2Cl2) | 323/321 [M+H+] |
| 66 | 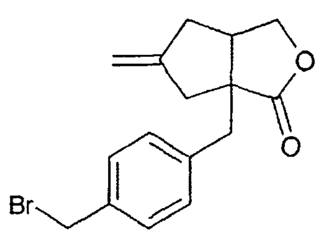 | 1A | 37 | 0,16 (III, 10:1) | 338 [M+NH4+] |

| Bsp. Nr. | Struktur | Edukt Bsp.-Nr. | Ausbeute [%] | Rf | MS |
|---|---|---|---|---|---|
| 67 | 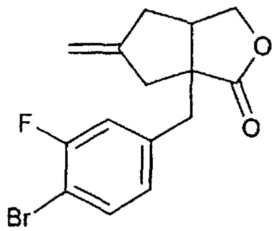 | 1A | 26 | 0,42 (III, 5:1) | 340/342 [M+NH4+] |
| 68 | 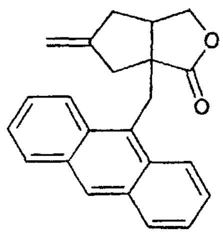 | 1A | 39 | 0,27 (III, 5:1) | 370 [M+CH3CN+H+] |
| 69 | 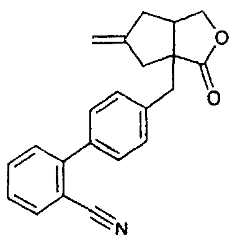 | 1A | 35 | 0,42 (III, 5:1) | - |
| 70 | 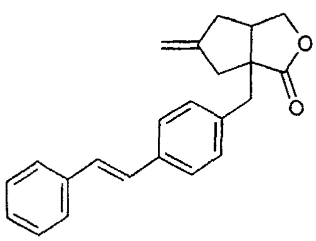 | 1A | 3 | 0,42 (III, 5:1) | 372 [M+CH3CN+H+] |
| 71 | 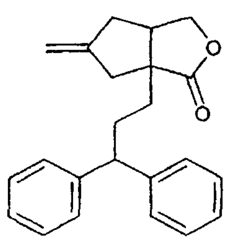 | 1A | 51 | 0,22 (IV, 1:1) | 355 [M+Na+] |
| 72 | 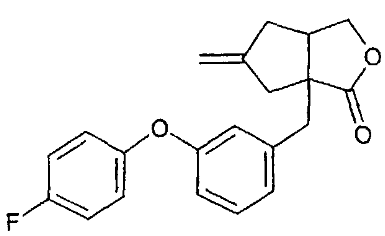 | 1A | 33 | 0,42 (III, 5:1) | 380 [M+CH3CN+H+] |

| Bsp. Nr. | Struktur | Edukt Bsp.-Nr. | Ausbeute [%] | Rf | MS |
|---|---|---|---|---|---|
| 73 | 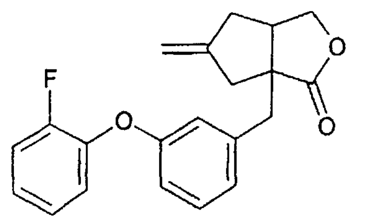 | 1A | 40 | 0,42 (III, 5:1) | 380 [M+CH3CN+H+] |
| 74 | 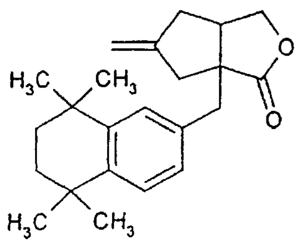 | 1A | 25 | 0,75 (III, 50:1) | 362 [M+H+] |
| 75 | 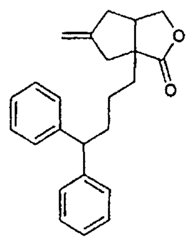 | 1A | 62 | 0,20 (IV, 5:1) | 347 [M+H+] |
| 76 | 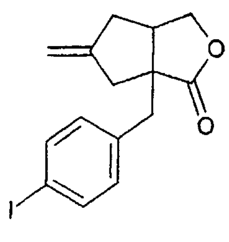 | 1A | 38 | 0,42 (III, 5:1) | 396 [M+CH3CN+H+] |
| 77 | 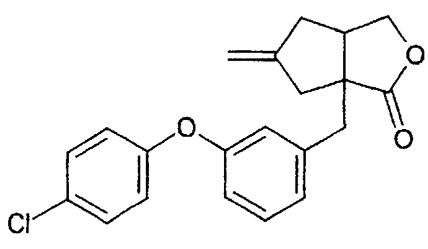 | 1A | 23 | 0,26 (III, 10:1) | 372 [M+NH4+] |
| 78 | 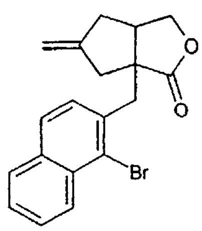 | 1A | 17 | 0,42 (III, 5:1) | 374/376 [M+NH4+] |

| Bsp. Nr. | Struktur | Edukt Bsp.-Nr. | Aus-beute [%] | Rf | MS |
|---|---|---|---|---|---|
| 79 | 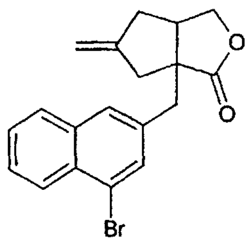 | 1A | 30 | 0,42 (III, 5:1) | 398 [M+CH3CN+ H+] |
| 80 | 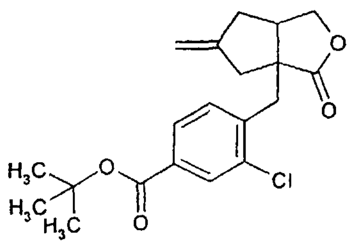 | 1A | 4 | 0,42 (III, 5:1) | 380 [M+NH4+] |
| 81 | 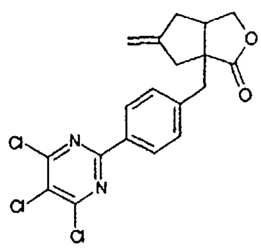 | 1A | 16 | 0,42 (III, 5:1) | 409 [M+H+] |

| Bsp.-Nr. | Struktur | Edukt | Ausbeute [%] | Rf | MS |
|---|---|---|---|---|---|
| 82 | 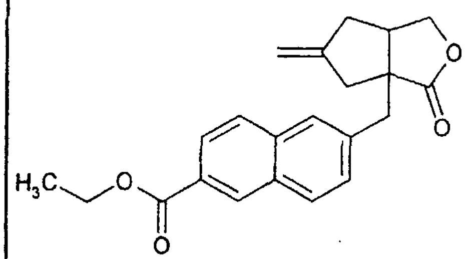 | 1A | 5 | 0,39 (III, 5:1) | 351 [M+H+] |
| 83 | 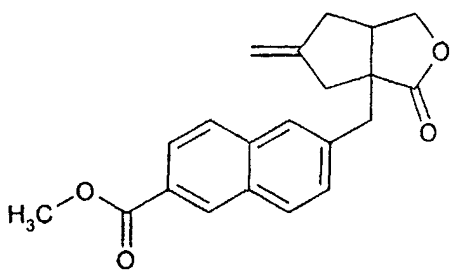 | 1A | 5 | 0,22 (III, 5:1) | 337 [M+H+] |
| 84 | 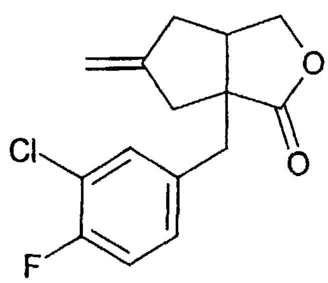 | 1A | 29 | 0,24 (III, 10:1) | 298 [M+NH4+] |
| 85 | 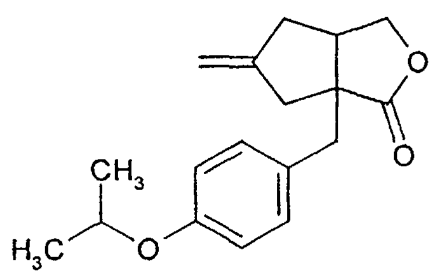 | 1A | 19 | 0,27 (III, 10:1) | 287 [M+H+] |

| Bsp.-Nr. | Struktur | Edukt | Ausbeute [%] | Rf | MS |
|---|---|---|---|---|---|
| 86 | 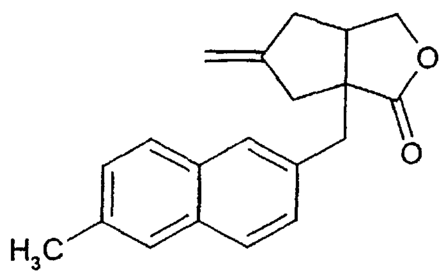 | 1A | 9 | 0,30 (III, 10:1) | 293 [M+H+] |
| 87 | 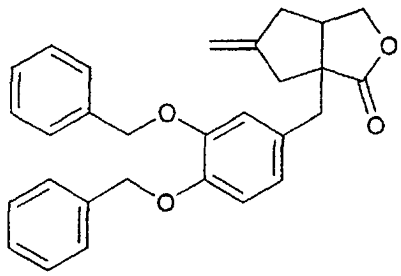 | 1A | 12 | 0,11 (III, 10:1) | 458 [M+NH4+] |
| 88 | 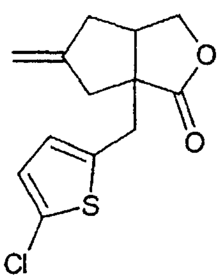 | 1A | 42 | 0,25 (III, 10;1) | 269 [M+H+] |
| 89 | 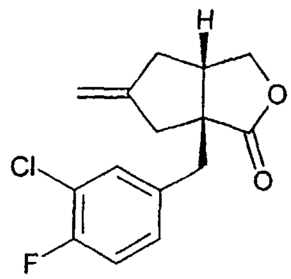 | 2A | 55 | 0,34 (III, 5:1) | - |

| Bsp.-Nr. | Struktur | Edukt | Ausbeute [%] | Rf | MS |
|---|---|---|---|---|---|
| 90 | 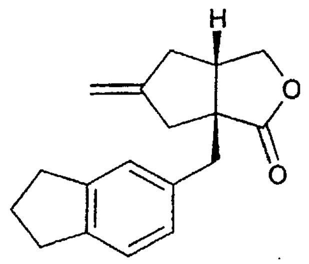 | 2A | 62 | 0,47 (III, 5:1) | 310 [M+H+H3CCN+] |
| 91 | 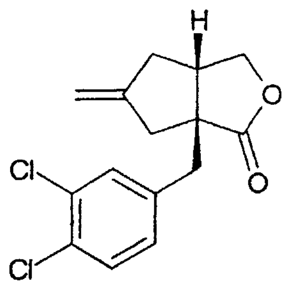 | 2A | 38 | 0,30 (III, 5:1) | - |
| 92 | 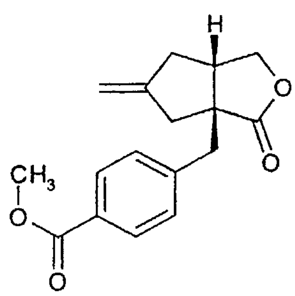 | 2A | 52 | 0,18 (III, 5:1) | 304 [M+NH4+] |
| 93 | 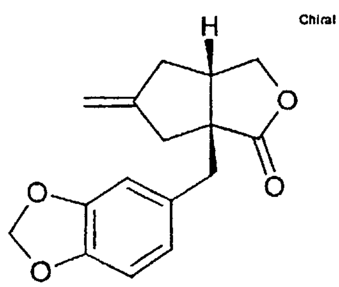 Chiral | 2A | 74 | 0,34 (III, 5:1) | 314 [M+H3CCN+H+] |

| Bsp.-Nr. | Struktur | Edukt | Ausbeute [%] | Rf | MS |
|---|---|---|---|---|---|
| 94 | 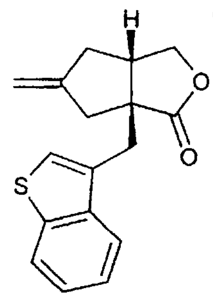 Chiral | 2A | 66 | 0,36 (III, 5:1) | 326 [M+H3CCN +H+] |
| 95 | 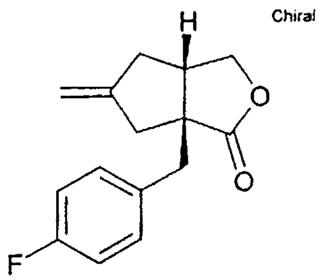 Chiral | 2A | 56 | 0,39 (III, 5:1) | 288 [M+H3CCN +H+] |
| 96 | 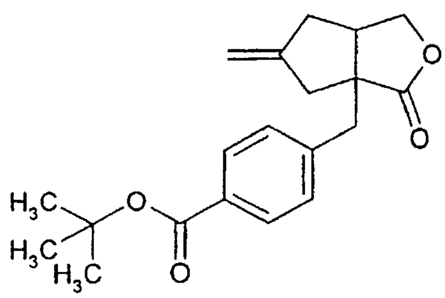 | 1A | 40 | 0,39 (III, 5:1) | 346 [M+NH4+] |
| 97 | 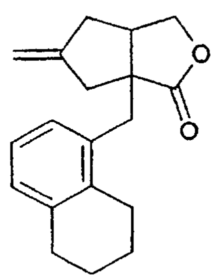 | 1A | 10 | 0,35 (III, 10:1) | 300 [M+NH4+] |

**Beispiel 98**

(3aS*,6aS*)-6a-(4-Allyloxycarbonylaminobenzyl)-5-methylen-hexahydrocyclopenta[c]-furan-1-on

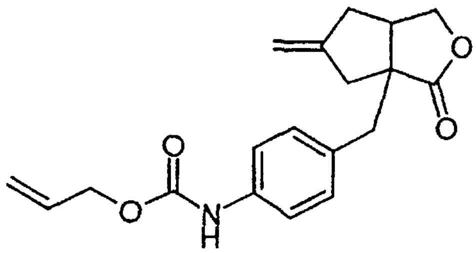

Eine Mischung aus der Verbindung aus Beispiel 124 (2,02 g; 7,5 mmol), Allylalkohol (4,3 g, 74,29 mmol), Diphenylphosphorylazid (2,25 g, 8,17 mmol), Diazabicyclooctan (DABCO, 0,17 g; 1,5 mmol) und Triethylamin (0,9 g, 8,92 mmol) in Toluol (70 ml) wurde 48 h unter Rückfluß gerührt. Das Reaktionsgemisch wurde mit 2 g Kieselgel versetzt, das Lösemittel abgezogen und der Rückstand durch MPLC gereinigt.
Ausbeute: 1,29 g (53 %)
$R_f$ (III, 5:1)=0,23
MS (DCI): m/z= 345 [$M+NH_4^+$]

Analog zur Vorschrift des Beispiels 98 wurden die in der folgenden Tabelle aufgeführten Beispiele 99 bis 103 hergestellt:

| Bsp.-Nr. | Struktur | Ausbeute | $R_f$ | MS |
|---|---|---|---|---|
| 99 | 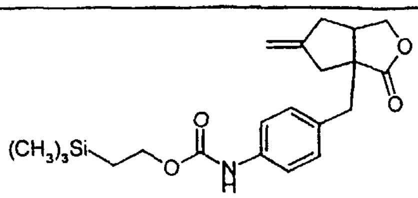 | 97 | 0,75 (III, 1:1) | / |

| Bsp.-Nr. | Struktur | Ausbeute | $R_f$ | MS |
|---|---|---|---|---|
| 100 | 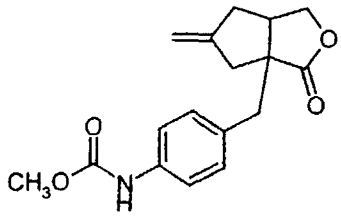 | 25 | 0,35 (III, 2:1) | / |
| 101 | 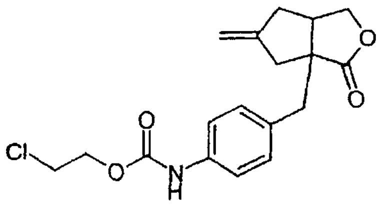 | 12 | 0,44 (III, 2:1) | / |
| 102 | 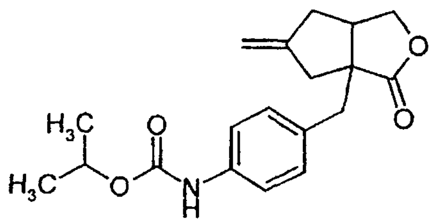 | 68 | 0,50 (III, 2:1) | / |
| 103 | 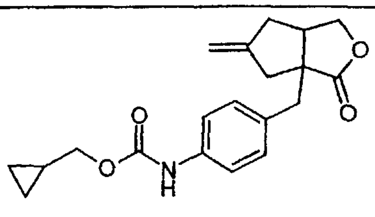 | 27 | 0,53 (III; 2:1) | / |

**Beispiel 104**

(3aS*,6aS*)-6a-(4-Aminobenzyl)-5-methyliden-hexahydro-cyclopenta[c]furan-1-on

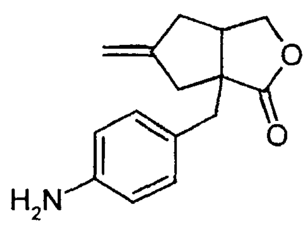

Beispiel 98 (1,275 g; 3,96 mmol) wurde zu einer Lösung von Tris(dibenzylidenaceton)dipalladium(0) (181 mg; 0,198 mmol), Bis(diphenyl-phosphino)ethan (315 mg; 0,79 mmol) und Dimedon (4,44 g; 31,65 mmol) in THF (320 ml) gegeben und das Reaktionsgemisch 1 h unter Rückfluß erhitzt. Das Reaktionsgemisch wird auf 1 N HCl gegeben, die

wäßrige Phase mit Essigester (2 x) gewaschen, anschließend auf pH = 9-10 gebracht und mit Essigester extrahiert (3 x). Die vereinigten Extrakte werden mit gesättigter NaCl-Lösung gewaschen, getrocknet ($MgSO_4$) und das Lösemittel im Vakuum abgezogen.
Ausbeute: 0,770 g (80 %)
$R_f$ (III, 1:1)=0,42

Analog wurden hergestellt:

| Bsp.-Nr. | Struktur | Ausbeute | $R_f$ |
|---|---|---|---|
| 105 | 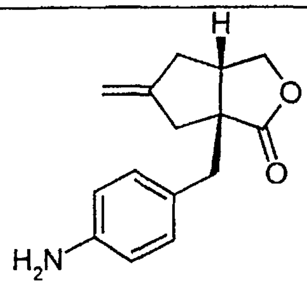 | 83 | 0,42 (II, 1:1) |

**Beispiel 106**

(3aS*,6aS*)-6a-(4-Acetylaminobenzyl)-5-methyliden-hexahydro-cyclopenta[c]furan-1-on

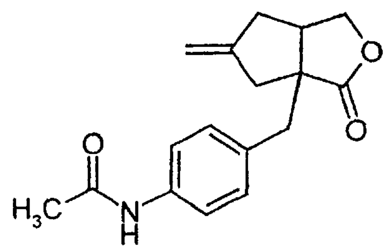

Zu einer Lösung der Verbindung aus Beispiel 104 (10 mg; 0,036 mmol) in Dichlormethan (5 ml) wurden Triethylamin (12 µl, 0,080 mmol) und Acetylchlorid (3,1 mg; 0,039 mmol) gegeben und das Reaktionsgemisch 20 h bei Raumtemperatur gerührt. Zur Aufarbeitung wurde eine gesättigte $NaHCO_3$-Lösung (1 ml) zugegeben und das Gemisch über eine Fritte, gefüllt mit Bitumenerde/Kieselgel, filtriert und mit Dichlormethan / Ethanol das Produkt eluiert.
Ausbeute: 12,2 mg (quant.)
$R_f$ (II, 50:1) = 0,18
MS (ESI): m/z = 286 $[M+H^+]$

Analog wurden hergestellt:

| Bsp.-Nr. | Struktur | Ausbeute | $R_f$ | MS |
|---|---|---|---|---|
| 107 | 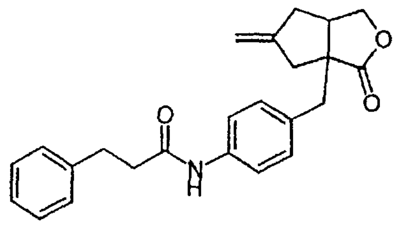 | quant. | 0,12 (II, 100:1) | 376 [M+H$^+$] |
| 108 | 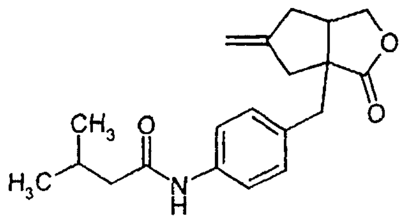 | quant. | 0,10 (II, 100:1) | 328 [M+H$^+$] |

**Beispiel 109**

(3aS'''*,6a'''S*)-N-[3-(5-Methyliden-hexahydro-cyclopenta[c]furan-1-on-6a-ylmethyl)-phenyl]-morpholin

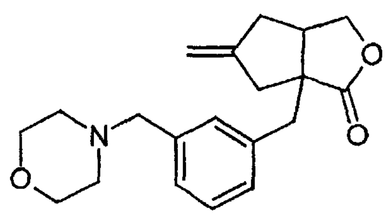

Eine Mischung aus Triethylamin (208 µl; 1,5 mmol), Morpholin (131 µl; 1,5 mmol) und der Verbindung aus Beispiel 65 (321 mg; 1,0 mmol) in Dichlormethan (1 ml) wurden 14 h bei Raumtemperatur gerührt. Der Ansatz wurde eingeengt und das Rohprodukt durch MPLC gereinigt.
Ausbeute: 230 mg (70 %)
$R_f$(I, 40:1) = 0,25
MS (DCI / $NH_3$): m/z = 328 [M+H$^+$]

In Analogie zum Beispiel 109 wurden die in der folgenden Tabelle aufgeführten Beispiele 110 bis 116 dargestellt:

| Bsp.-Nr. | Struktur | Edukt Bsp.-Nr. | Ausbeute (%) | $R_f$ | MS |
|---|---|---|---|---|---|
| 110 | 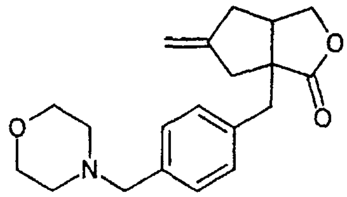 | 66 | 33 | 0,29 (I, 40:1) | 328 [M+H$^+$] |
| 111 | 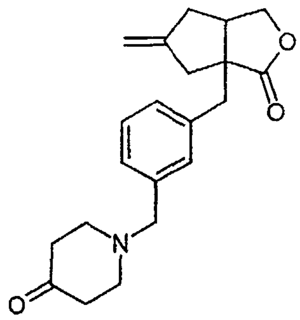 | 65 | 26 | 0,31 (I, 40:1) | 340 [M+H$^+$] |
| 112 | 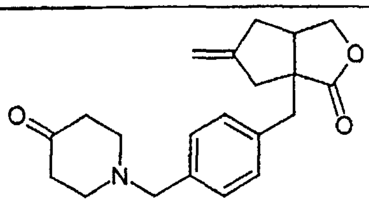 | 66 | 15 | 0,22 (I, 40:1) | 340 [M+H$^+$] |
| 113 | 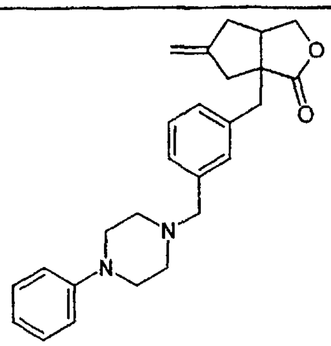 | 65 | 79 | 0,21 (I, 40:1) | 403 [M+H$^+$] |
| 114 | 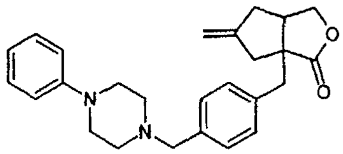 | 66 | 76 | 0,27 (I, 40:1) | 403 [M+H$^+$] |

| Bsp.-Nr. | Struktur | Edukt Bsp.-Nr. | Ausbeute (%) | $R_f$ | MS |
|---|---|---|---|---|---|
| 115 | 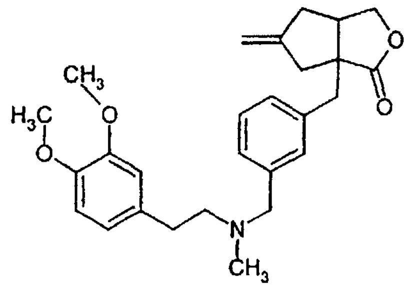 | 65 | 55 | 0,17 (I, 40:1) | 436 [M+H$^+$] |
| 116 | 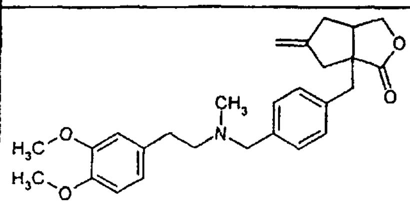 | 66 | 55 | 0,13 (I, 40:1) | 436 [M+H$^+$] |

**Beispiel 117 und Beispiel 118**

(3aS*,6aS*)-5-Methyl-6a-naphth-2-ylmethyl-3,3a,6,6a-tetrahydro-cyclopenta[c]-furan-1-on (Beispiel 117) und (3aS*,6aS*)-5-Methyl-6a-naphth-2-ylmethyl-3,3a,4,6a-tetrahydro-yclopenta[c]furan-1-on (Beispiel 118)

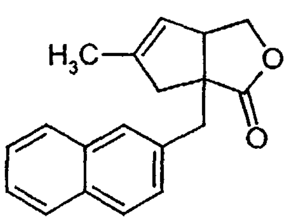

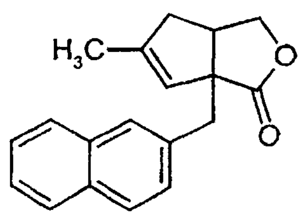

Eine Mischung der Verbindung aus Beispiel 34 (370 mg, 1,33 mmol) und Pd-C (10%, 370 mg) in n-Butanol (10 ml) wurde 48 h unter Rückfluß gerührt. Nach Filtration, Abdestillation des Lösemittels im Vakuum und Reinigung des Rohproduktes durch MPLC erhielt man die Titelverbindung; Ausbeute: 155 mg (42 %, Beispiel 117)
$R_f$ (III, 5:1) = 0,36
MS (EI): m/z = 279 [M+H$^+$]
Ausbeute: 75 mg (21 %, Beispiel 118)
$R_f$ (III, 5:1) = 0,30
MS (EI): m/z=301 [M+Na$^+$]

**Beispiel 119 und Beispiel 120**

(3aS*,3R*,6aS*)-3-Methyl-5-methyliden-6a-naphth-2-ylmethyl-hexahydro-cyclopenta(c)furan-1-on und (3aS*,3S*,6aS*)-3-Methyl-5-methylen-6a-naphth-2-ylmethyl-hexahydro-cyclopenta(c)furan-1-on

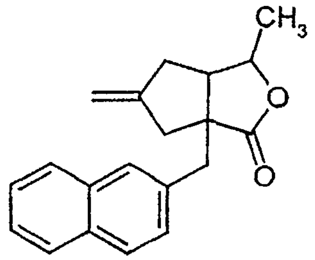

In Analogie zur Vorschrift des Beispiels 1 wurden aus der Verbindung aus Beispiel 4A (200 mg, 1,314 mmol) die Titelverbindungen dargestellt.
Ausbeute: 309 mg (93%, Gemisch der Diastereomeren)
$R_f$ ($CH_2Cl_2$) = 0,53
MS (DCI)/$NH_3$): m/z = 310 [$M+NH_4^+$]
Die Trennung der Diastereomeren erfolgte mittels HPLC (Kromasil 100 C 18, Acetonitril/Wasser 1:1)

Beispiel 121: Diastereomer A, Fraktion 1.

Beispiel 122: Diastereomer B, Fraktion 2.

**Beispiel 123**

(3 aS *,6aS* )-3,3-Dimethyl-5-methyliden-6a-naphth-2-ylmethyl-hexahydro-cyclopenta[c]furan-1-on

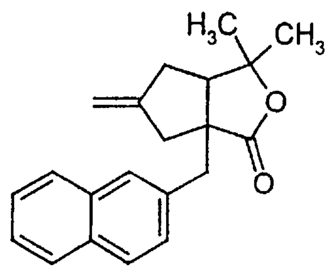

In Analogie zur Vorschrift des Beispiels 1 wurde aus der Verbindung aus Beispiel 5A (100 mg, 0,6 mmol) die Titelverbindung dargestellt;
Ausbeute: 116 mg (63 %)
$R_f$ (I, 10:1) = 0,47
MS (ESI): m/z = 307

**Beispiel 124**

(3a"S*,6"a)-4-(5-Methyliden-hexahydro-cyclopenta[c]furan-1-on-6a-ylmethyl)-benzoesäure

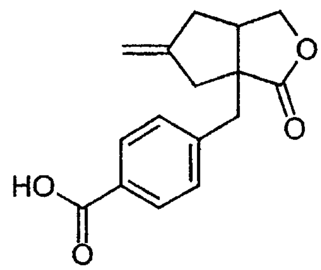

Eine Lösung der Verbindung aus Beispiel 43 (2,26g, 7,89 mmol) in THF (100 ml) und wäßriger NaOH (1 M, 127 ml) wurde 4h bei Raumtemperatur gerührt. Das Gemisch wurde mit 1 N wäßriger HCl auf pH = 2 gebracht, 1 h gerührt, mit NaCl gesättigt und mit Essigester extrahiert. Die vereinigten organischen Phasen wurden getrocknet ($Na_2SO_4$) und im Vakuum die Lösemittel abgezogen;
Ausbeute: 2,14g (quant.)
$R_f$ (II, 10:1)=0,47

**Beispiel 125**

(3a"S*,6a"S*)-3-(S-Methyliden-hexahydro-cyclopenta[c]furan-1-on-6aylmethyl)benzoesäure

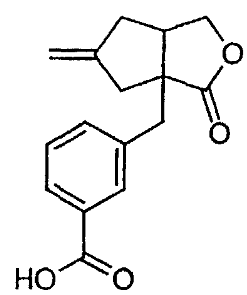

In Analogie zum Beispiel 124 wurde aus Beispiel 42 (2,30 g, 8,03 mmol) die Titelverbindung dargestellt;
Ausbeute: 1,38 g (63,1 %)
$R_f$ (II; 10:1) = 0,45
MS (DCI/$NH_3$): m/z = 290 [M+$NH_4^+$]

**Beispiel 126**

(3a"S,6a"S)-4-(5-Methyliden-hexahydro-cyclopenta[c]furan-1-on-6a-yimethyl)benzoesäure

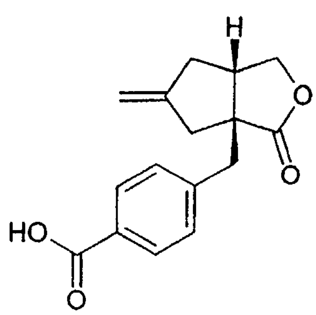

Eine Lösung der Verbindung aus Beispiel 92 (2,2 g, 7,68 mmol) in THF (100 ml) und wäßriger NaOH (1 M, 127 ml) wurde 4 h bei Raumtemperatur gerührt. Das Gemisch wurde mit 1 N wäßriger HCl auf pH = 2 gebracht, 1 h

gerührt, mit NaCl gesättigt und mit Essigester extrahiert. Die vereinigten organischen Phasen wurden getrocknet ($Na_2SO_4$) und im Vakuum die Lösemittel abgezogen;
Ausbeute: 2,1 g (quant.)

**Beispiel 127**

(3a"S*,6a"S*)-1-[(5-Methyliden-hexahydro-cyclopenta[c]furan-1-on-6a-ylmethyl)-benzoyl]-4-phenylpiperazin

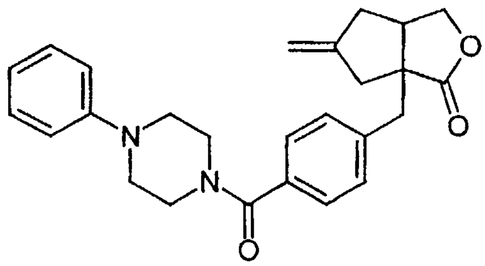

Eine Mischung der Verbindung aus Beispiel 124 (50 mg, 0,184 mmol), N-Phenylpiperazin (32,77 mg, 0,202 mmol), 1-Ethyl-3-(3'-dimethylamino)propyl)-carbodiimid Hydrochlorid (38,7 mg, 0,202 mmol) und Triethylamin (18,6 mg, 0,184 mmol) in Dichlormethan (10 ml) wurde 20 h bei Raumtemperatur gerührt. Zur Aufarbeitung wurde 10 % wäßrige $KHSO_4$ (1 ml) hinzugegeben, das Reaktionsgemisch über eine Fritte, gefüllt mit Bitumenerde/Kieselgel, filtriert, die Lösemittel abgezogen und das Rohprodukt durch Flash-Chromatographie gereinigt;
Ausbeute: 90 mg (quant.)
$R_f$ (II, 20:1) = 0,19
MS (ESI): m/z = 417 [M+H$^+$]

In Analogie zur Vorschrift des Beispiels 127 wurden die in der folgenden Tabelle aufgeführten Beispiele 128 bis 177 ausgehend von den Beispielen 124 bzw. 125, die Beispiele 178 bis 197 ausgehend von Beispiel 126 dargestellt:

| Bsp.-Nr. | Struktur | Edukt Bsp. Nr. | Ausbeute [%] | Rf | MS |
|---|---|---|---|---|---|
| 128 | 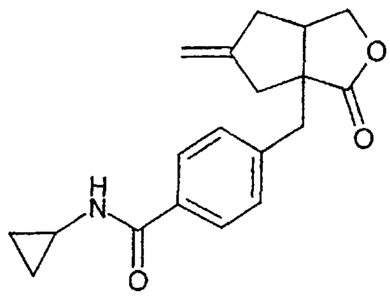 | 124 | 79 | 0,16 (II, 20:1) | 312 [M+H+] |
| 129 | 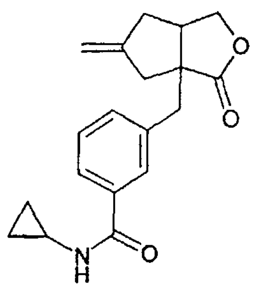 | 125 | 81 | 0,21 (II, 20:1) | 312 [M+H+] |
| 130 | 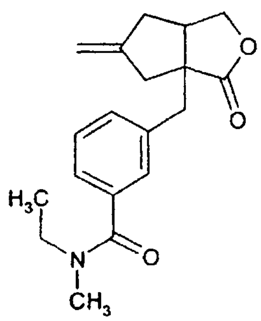 | 125 | 71 | 0,313 (II, 20:1) | 314 [M+H+] |
| 131 | 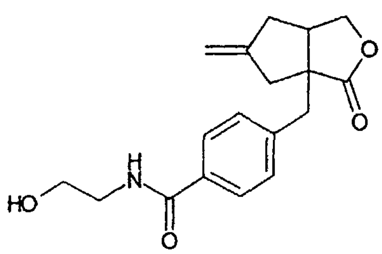 | 124 | 60 | 0,06 (II, 20:1) | 316 [M+H+] |

| Bsp.-Nr. | Struktur | Edukt Bsp. Nr. | Ausbeute [%] | Rf | MS |
|---|---|---|---|---|---|
| 132 | 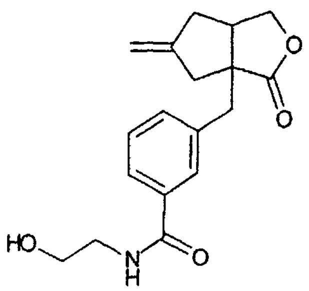 | 125 | 51 | 0,06 (II, 20:1) | 316 [M+H+] |
| 133 | 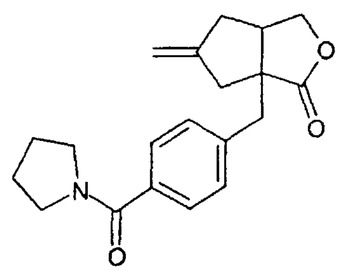 | 124 | 77 | 0,19 (II, 20:1) | 326 [M+H+] |
| 134 | 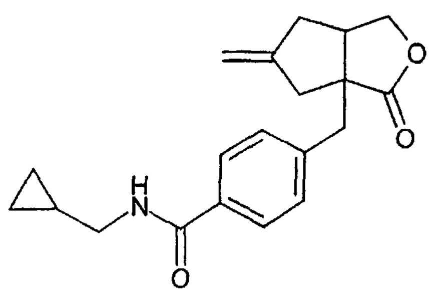 | 124 | 75 | 0,24 (II, 20:1) | 326 [M+H+] |
| 135 | 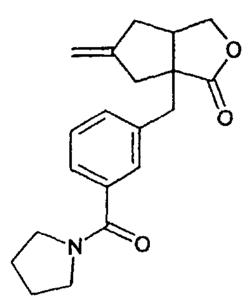 | 125 | 85 | 0,20 (II, 20:1) | 326 [M+H+] |

| Bsp.-Nr. | Struktur | Edukt Bsp. Nr. | Ausbeute [%] | Rf | MS |
|---|---|---|---|---|---|
| 136 | 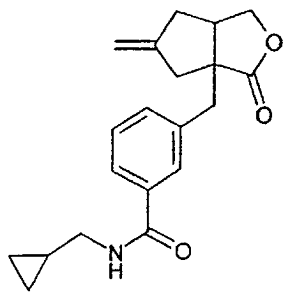 | 125 | 78 | 0,21 (II, 20:1) | 326 [M+H+] |
| 137 | 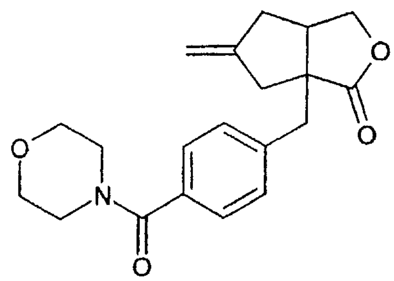 | 124 | 27 | 0,24 (III, 4:1) | 342 [M+H+] |
| 138 | 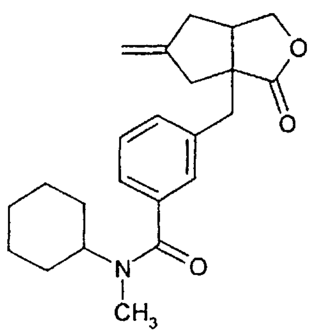 | 125 | 35 | 0,25 (II, 20:1) | 368 [M+H+] |
| 139 | 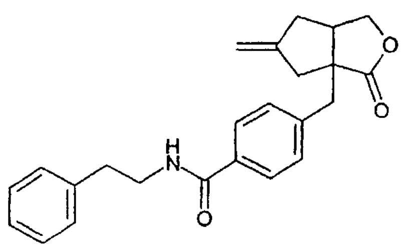 | 124 | 70 | 0,22 (II, 20:1) | 376 [M+H+] |

| Bsp.-Nr. | Struktur | Edukt Bsp. Nr. | Aus-beute [%] | Rf | MS |
|---|---|---|---|---|---|
| 140 | 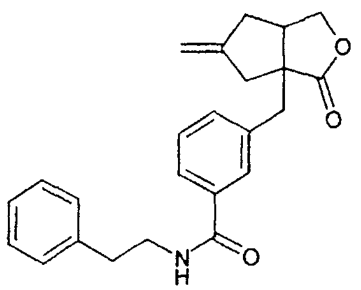 | 125 | 91 | 0,23 (II, 20:1) | 376 [M+H+] |
| 141 | 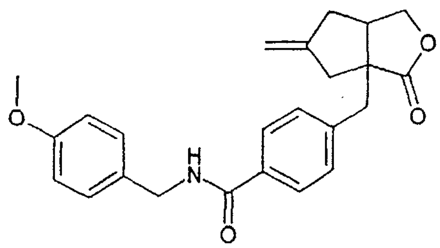 | 124 | 59 | 0,25 (II, 20:1) | 392 [M+H+] |
| 142 | 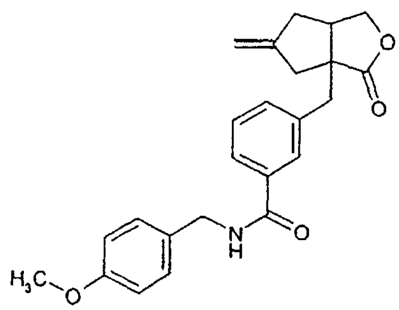 | 125 | 66 | 0,25 (II, 20:1) | 392 [M+H+] |
| 143 | 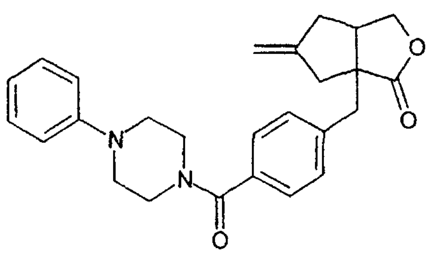 | 124 | 82 | 0,21 (II, 20:1) | 417 [M+H+] |

| Bsp.-Nr. | Struktur | Edukt Bsp. Nr. | Ausbeute [%] | Rf | MS |
|---|---|---|---|---|---|
| 144 | 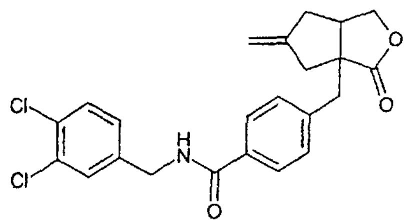 | 124 | 77 | 0,29 (II, 20:1) | 431 [M+H+] |
| 145 | 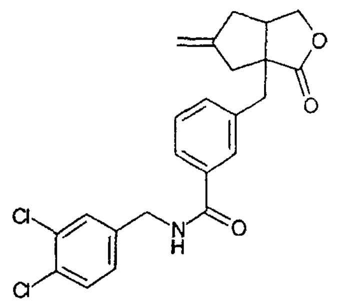 | 125 | 58 | 0,213 (II, 20:1) | 431 [M+H+] |
| 146 | 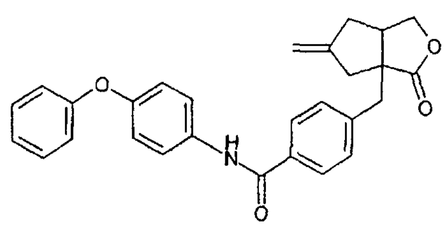 | 124 | 67 | 0,33 (II, 20:1) | 440 [M+H+] |
| 147 | 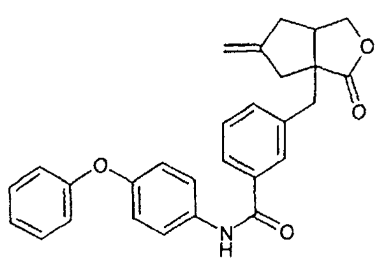 | 125 | 81 | 0,43 (II, 20:1) | 440 [M+H+] |

| Bsp.-Nr. | Struktur | Edukt Bsp. Nr. | Ausbeute [%] | Rf | MS |
|---|---|---|---|---|---|
| 148 | 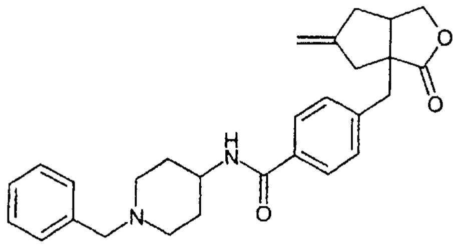 | 124 | 53 | 0,05 (II, 20:1) | 445 [M+H+] |
| 149 | 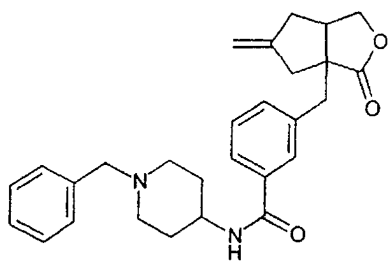 | 125 | 52 | 0,04 (II, 20:1) | 445 [M+H+] |
| 150 | 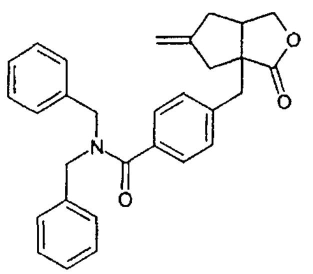 | 124 | 93 | 0,25 (II, 20:1) | 452 [M+H+] |
| 151 | 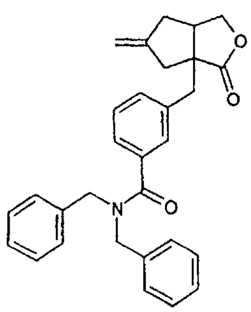 | 125 | 71 | 0,28 (II, 20:1) | 452 [M+H+] |

| Bsp. Nr. | Struktur | Edukt Bsp.Nr. | MG [g/mol] | HPLC-Area % bei 210 nm | MS [M+H+] |
|---|---|---|---|---|---|
| 152 | 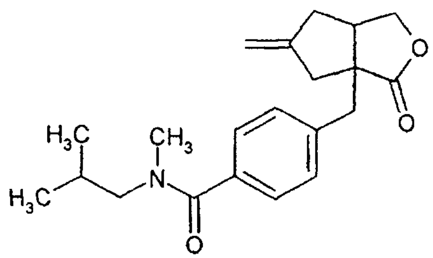 | 124 | 341,4542 | 97 | 342 |
| 153 | 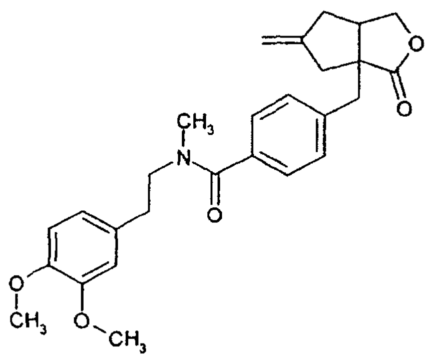 | 124 | 449,5518 | 97 | 450 |
| 154 | 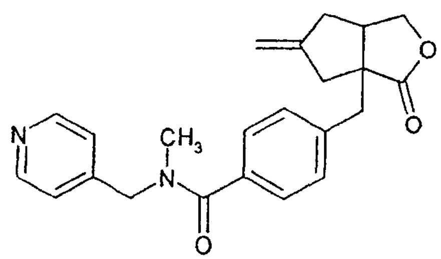 | 124 | 376,4593 | 92 | 377 |

| Bsp. Nr. | Struktur | Edukt Bsp.Nr. | MG [g/mol] | HPLC-Area % bei 210 nm | MS [M+H$^+$] |
|---|---|---|---|---|---|
| 155 | 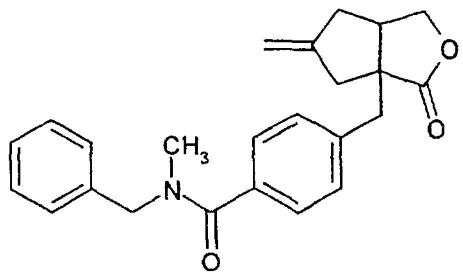 | 124 | 375,4718 | 96 | 376 |
| 156 | 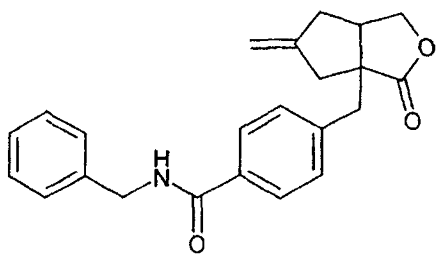 | 124 | 361,4447 | 96 | 362 |
| 157 | 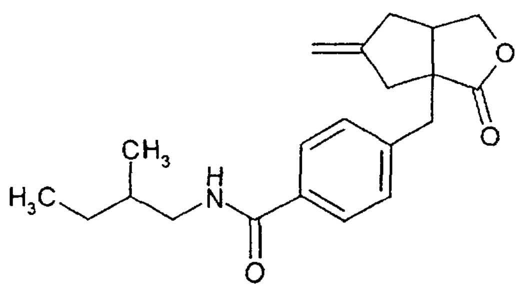 | 124 | 341,4542 | 96 | 342 |

| Bsp. Nr. | Struktur | Edukt Bsp.Nr. | MG [g/mol] | HPLC-Area % bei 210 nm | MS [M+H$^+$] |
|---|---|---|---|---|---|
| 158 | 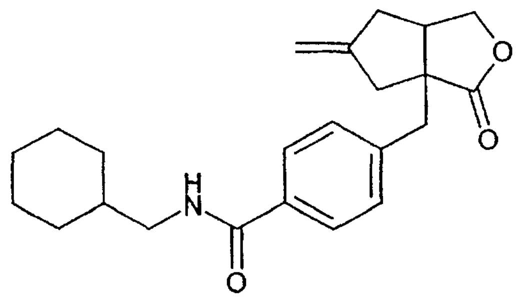 | 124 | 367,4925 | 95 | 368 |
| 159 | 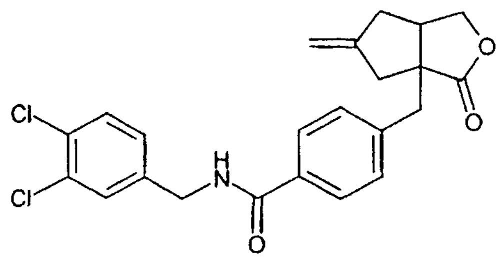 | 124 | 430,3347 | 96 | 430 |
| 160 | 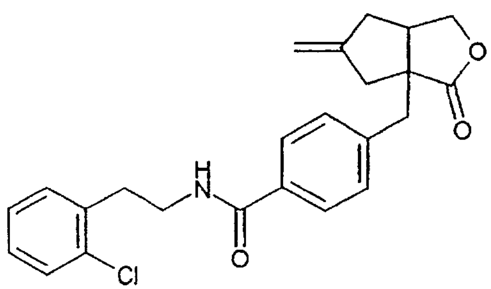 | 124 | 409,9168 | 95 | 410 |

| Bsp. Nr. | Struktur | Edukt Bsp.Nr. | MG [g/mol] | HPLC-Area % bei 210 nm | MS [M+H$^+$] |
|---|---|---|---|---|---|
| 161 | 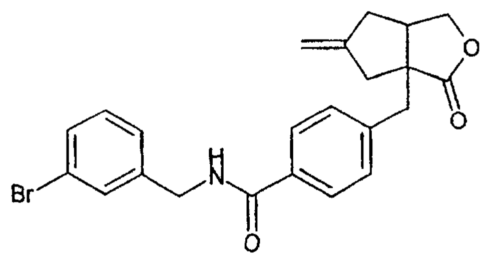 | 124 | 440,3407 | 96 | 440 |
| 162 | 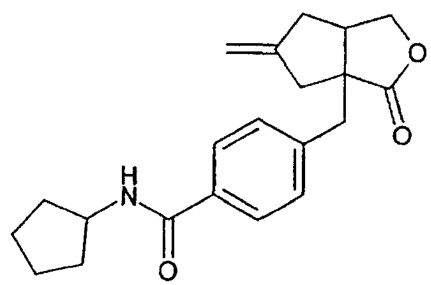 | 124 | 339,4383 | 96 | 340 |

| Bsp. Nr. | Struktur | Edukt Bsp.Nr. | MG [g/mol] | HPLC-Area % bei 210 nm | MS [M+H$^+$] |
|---|---|---|---|---|---|
| 163 | 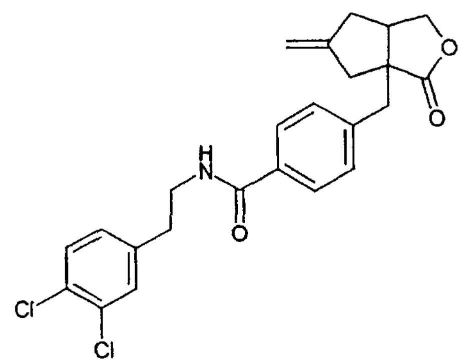 | 124 | 444,3618 | 95 | 444 |
| 164 | 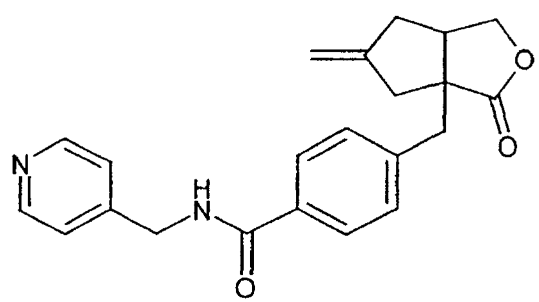 | 124 | 362,4322 | 97 | 363 |

| Bsp. Nr. | Struktur | Edukt Bsp.Nr. | MG [g/mol] | HPLC-Area % bei 210 nm | MS [M+H+] |
|---|---|---|---|---|---|
| 165 | 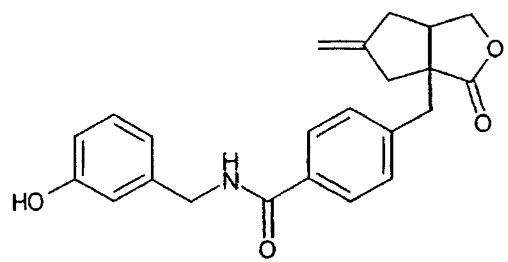 | 124 | 377,4441 | 98 | 378 |
| 166 | 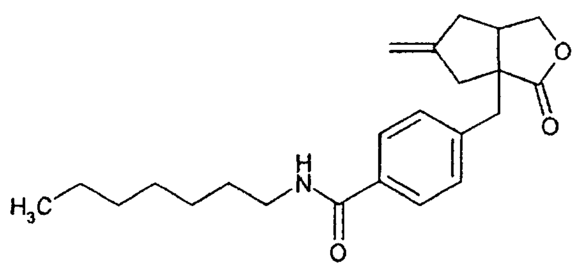 | 124 | 369,5084 | 94 | 370 |
| 167 | 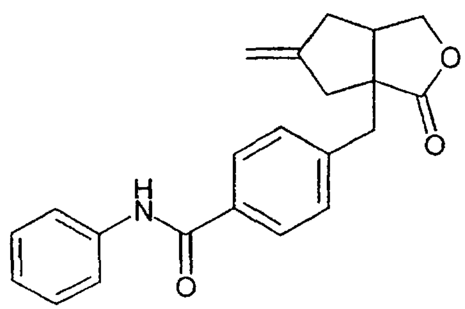 | 124 | 347,4176 | 94 | 348 |

| Bsp. Nr. | Struktur | Edukt Bsp.Nr. | MG [g/mol] | HPLC-Area % bei 210 nm | MS [M+H$^+$] |
|---|---|---|---|---|---|
| 168 | 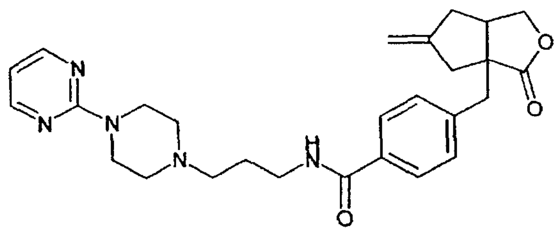 | 124 | 475,5958 | 94 | 476 |
| 169 | 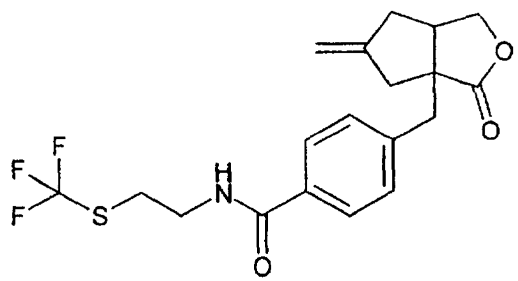 | 124 | 399,4354 | 96 | 400 |
| 170 | 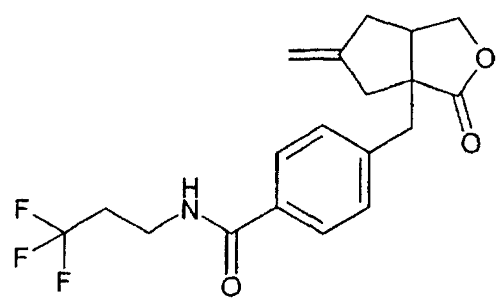 | 124 | 367,3714 | 93 | 368 |

| Bsp. Nr. | Struktur | Edukt Bsp.Nr. | MG [g/mol] | HPLC-Area % bei 210 nm | MS [M+H$^+$] |
|---|---|---|---|---|---|
| 171 | 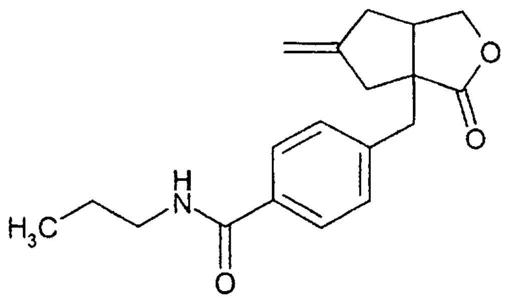 | 124 | 313,4001 | 96 | 314 |
| 172 | 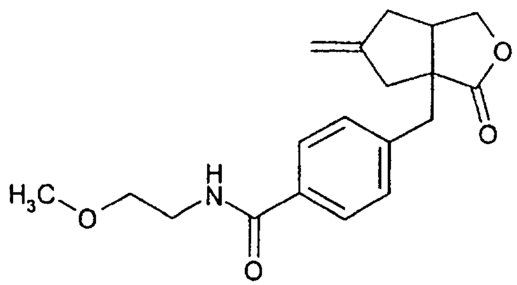 | 124 | 329,3995 | 99 | 330 |
| 173 | 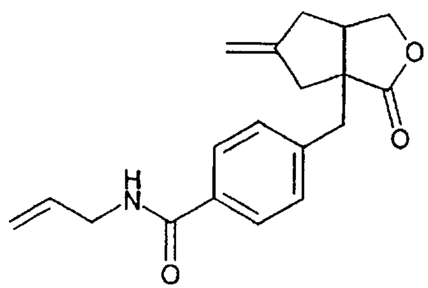 | 124 | 311,3841 | 95 | 312 |

| Bsp. Nr. | Struktur | Edukt Bsp.Nr. | MG [g/mol] | HPLC-Area % bei 210 nm | MS [M+H$^+$] |
|---|---|---|---|---|---|
| 174 | 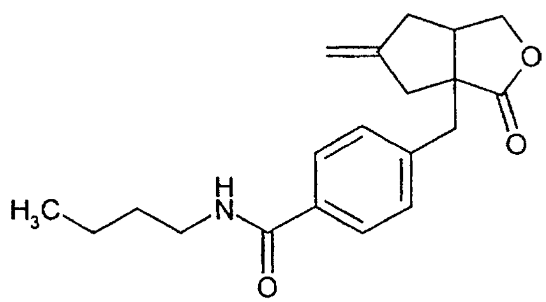 | 124 | 327,4272 | 94 | 328 |
| 175 | 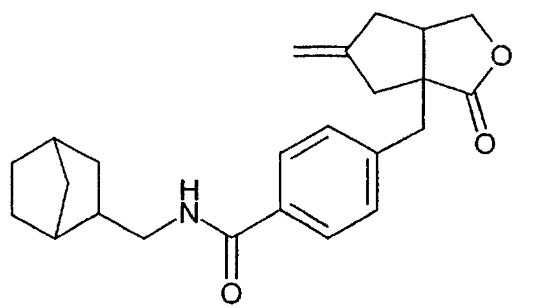 | 124 | 379,5036 | 95 | 380 |
| 176 | 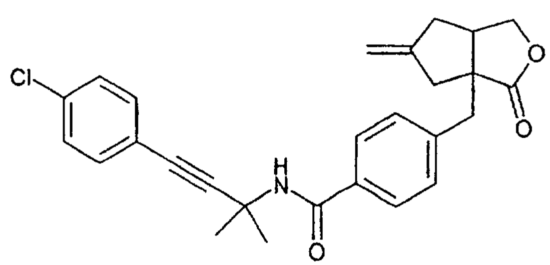 | 124 | 447,9662 | 64 | 448 |

| Bsp. Nr. | Struktur | Edukt Bsp.Nr. | MG [g/mol] | HPLC-Area % bei 210 nm | MS [M+H$^+$] |
|---|---|---|---|---|---|
| 177 | 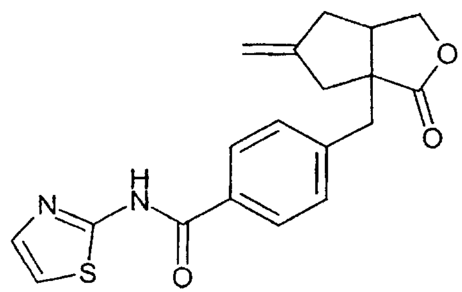 | 124 | 354,4309 | 51 | 355 |

| Bsp.-Nr. | Struktur | Molgewicht [g/mol] | HPLC-Area % bei 210 nm | MS [M+H$^+$] |
|---|---|---|---|---|
| 178 | 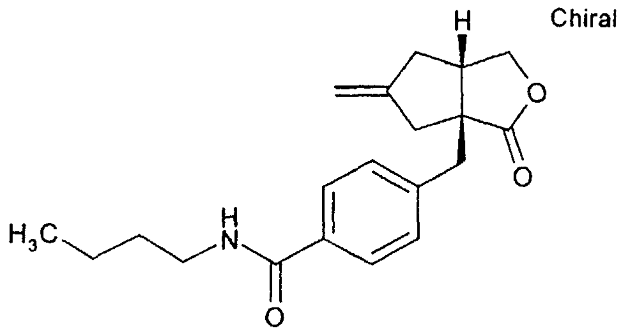 | 327,43 | 85 | 369 |
| 179 | 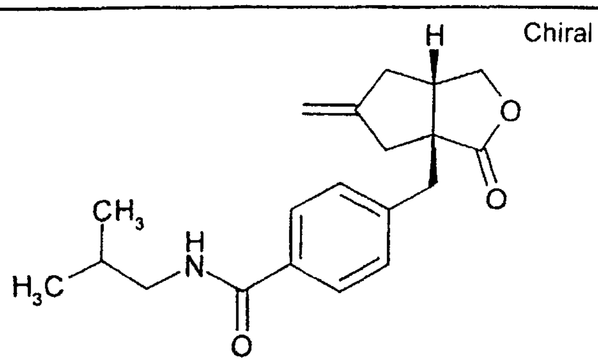 | 327,43 | 92 | 369 |
| 180 | 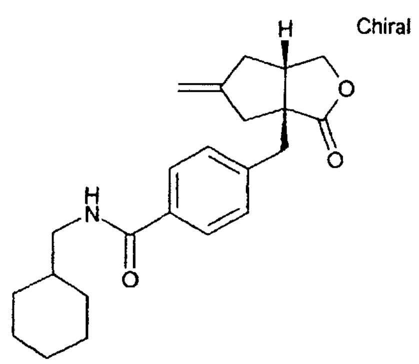 | 367,49 | 89 | 409 |
| 181 | 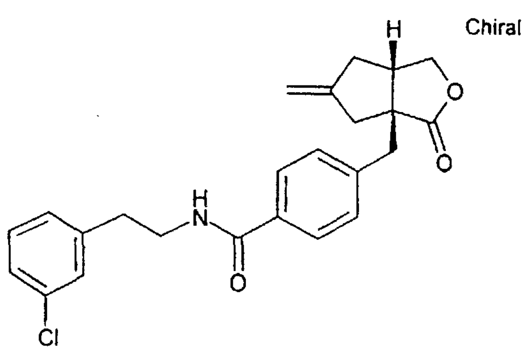 | 409,92 | 94 | 451 |

| Bsp.-Nr. | Struktur | Molgewicht [g/mol] | HPLC-Area % bei 210 nm | MS [M+H+] |
|---|---|---|---|---|
| 182 | 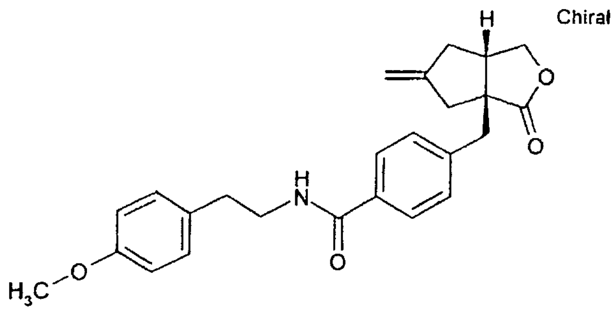 | 405,50 | 88 | 447 |
| 183 | 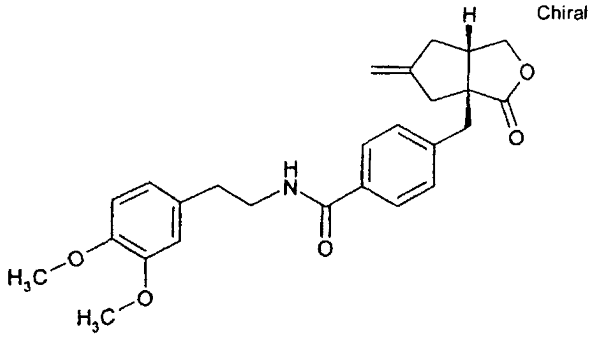 | 435,52 | 96 | 477 |

| Bsp.-Nr. | Struktur | Molgewicht [g/mol] | HPLC-Area % bei 210 nm | MS [M+H⁺] |
|---|---|---|---|---|
| 184 | 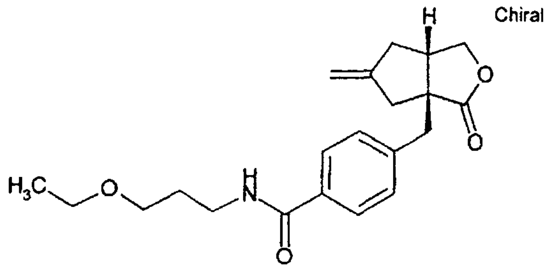 | 357,45 | 91 | 358 |
| 185 | 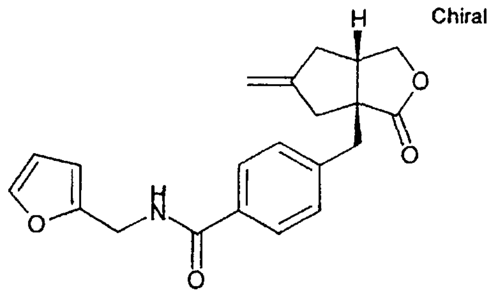 | 351,41 | 89 | 393 |
| 186 | 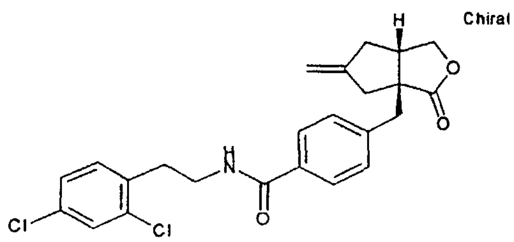 | 444,36 | 79 | 485 |
| 187 | 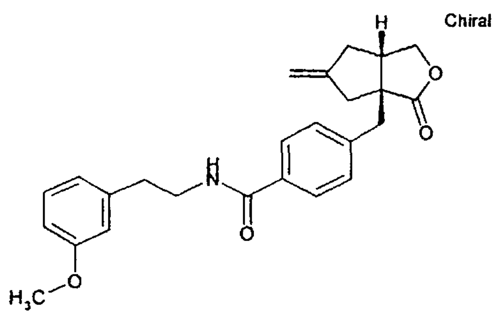 | 405,50 | 93 | 447 |

| Bsp.-Nr. | Struktur | Molgewicht [g/mol] | HPLC-Area % bei 210 nm | MS [M+H$^+$] |
|---|---|---|---|---|
| 188 | 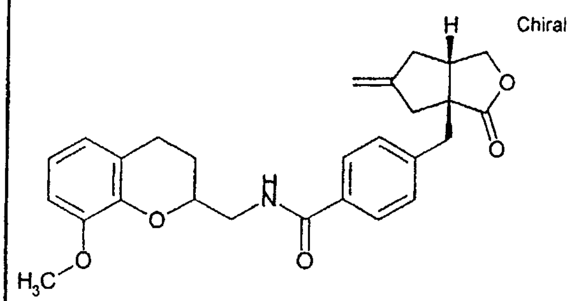 | 447,54 | 91 | 448 |
| 189 | 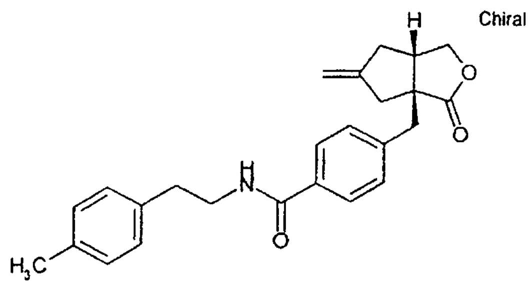 | 389,50 | 90 | 431 |
| 190 | 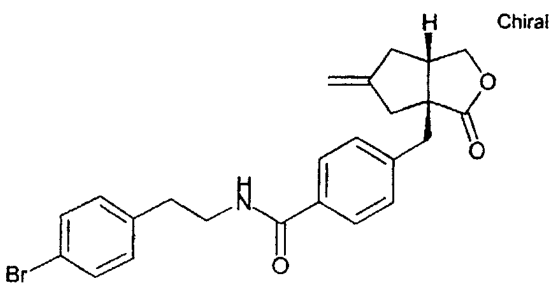 | 454,37 | 90 | 495 |
| 191 | 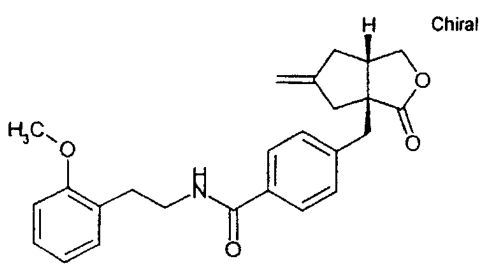 | 405,50 | 93 | 447 |

| Bsp.-Nr. | Struktur | Molgewicht [g/mol] | HPLC-Area % bei 210 nm | MS [M+H$^+$] |
|---|---|---|---|---|
| 192 | 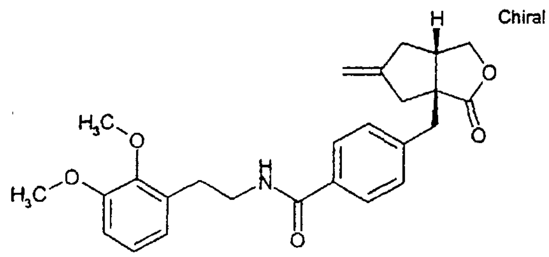 | 435,52 | 97 | 436 |
| 193 | 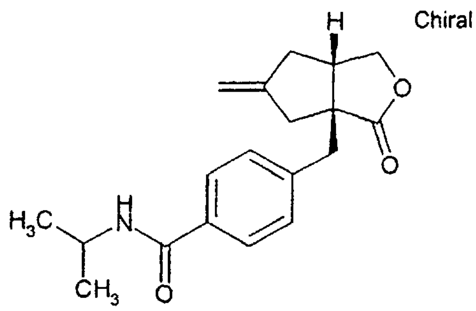 | 313,40 | 83 | 355 |
| 194 | 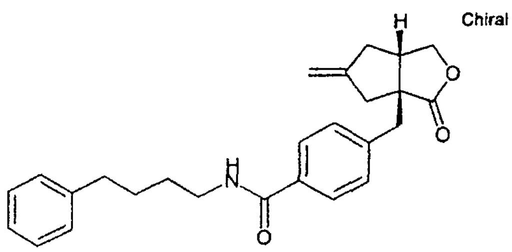 | 403,53 | 93 | 445 |
| 195 | 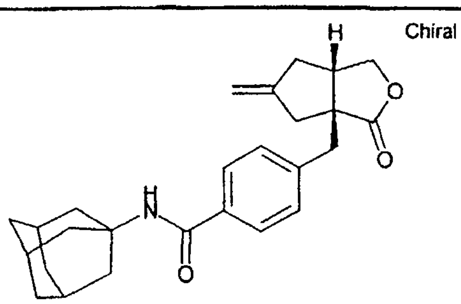 | 405,54 | 57 | 447 |

| Bsp.-Nr. | Struktur | Molgewicht [g/mol] | HPLC-Area % bei 210 nm | MS [M+H[+]] |
|---|---|---|---|---|
| 196 | 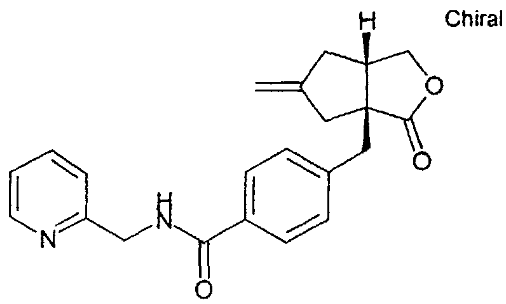 | 362,43 | 94 | 363 |
| 197 | 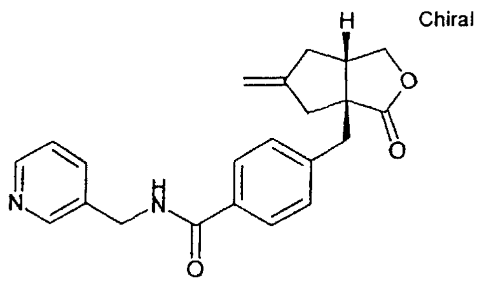 | 362,43 | 98 | 363 |

## Beispiel 198

(3a"S*,6a"S*)-4-(5-Methyliden-hexahydro-cyclopenta[c]furan-1-on-6a-ylmethyl)-benzoesäure-iso-propylester

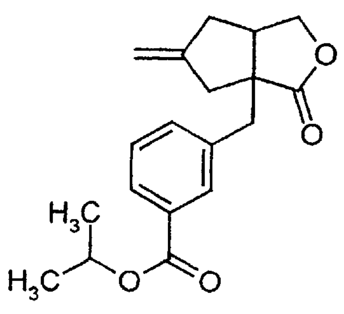

Zu einer Lösung der Verbindung aus Beispiel 125 (50,0 mg, 0,184 mmol) in Dichlormethan (2 ml) wurden bei Raumtemperatur Triethylamin (51 µl, 0,367 mmol) und Methansulfonsäurechlorid (14,2 µl, 0,184 mmol) gegeben. Nach 1 h wurden 2-Propanol (10,6 µl, 0,138 mmol) und Dimethylaminopyridin (4,5 mg, 0,037 mmol) zugegeben und weitere 20 h bei Raumtemperatur gerührt. Zur Aufarbeitung wurde mit 10 % wäßriger $NaHCO_3$-Lösung (1 ml) versetzt und über eine Fritte, gefüllt mit Bitumenerde/Kieselgel, filtriert, die Lösemittel abgezogen und das Rohprodukt durch Chromatographie gereinigt;

Ausbeute: 19 mg (32 %)
$R_f$ (II, 100:1)=0,47
MS (ESI): m/z = 337 [M+Na$^+$]

Die in der folgenden Tabelle aufgeführten Beispiele 199 bis 209 wurden analog der oben aufgeführten Vorschrift hergestellt.

| Bsp. Nr. | Struktur | Edukt Bsp. Nr. | Ausbeute [%] | Rf | MS |
|---|---|---|---|---|---|
| 199 | 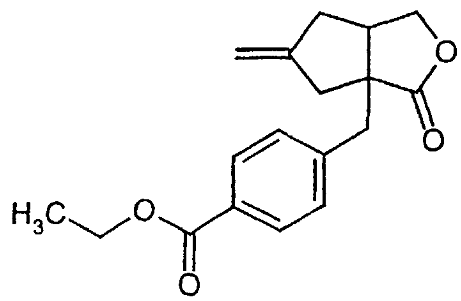 | 124 | 49 | 0,43 (II, 100:1) | 342 [M+CH3CN +H+] |
| 200 | 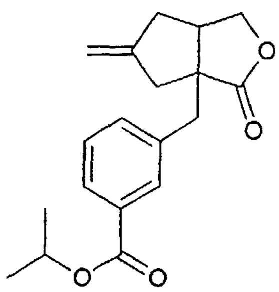 | 125 | 53 | 0,61 (II, 100:1) | 349 [M+H+] |
| 201 | 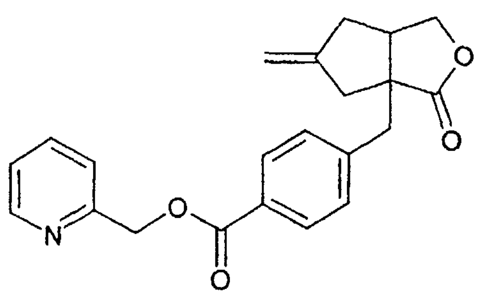 | 124 | 49 | 0,90 (II, 100:1) | 364 [M+H+] |
| 202 | 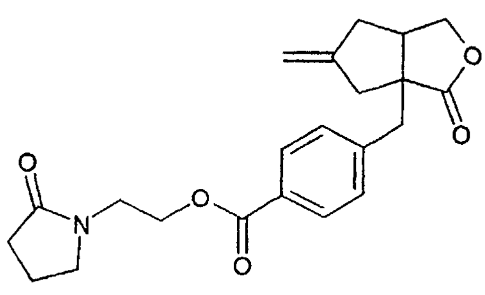 | 124 | 64 | 0,62 (II, 100:1) | 384 [M+H+] |

| Bsp. Nr. | Struktur | Edukt Bsp. Nr. | Ausbeute [%] | Rf | MS |
|---|---|---|---|---|---|
| 203 | 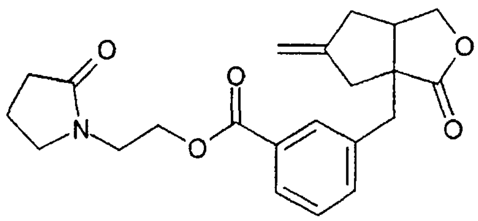 | 125 | 61 | 0,27 (II, 20:1) | - |
| 204 | 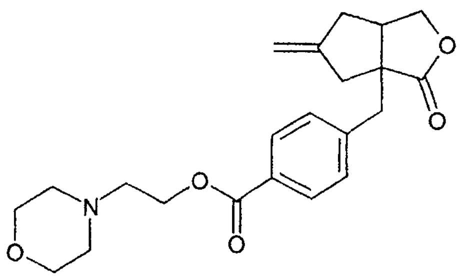 | 124 | 71 | 0,63 (II, 100:1) | 386 [M+H+] |
| 205 | 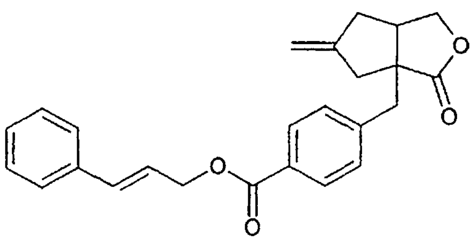 | 124 | 79 | 0,54 (II,100:1) | 411 [M+Na+] |
| 206 | 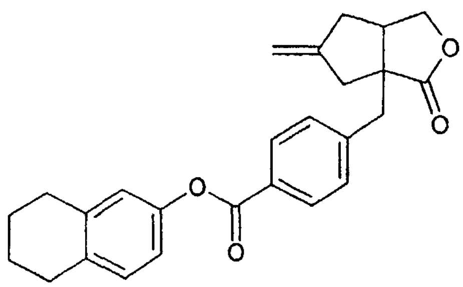 | 124 | 19 | 0,60 (II, 100:1) | 403 [M+H+] |
| 207 | 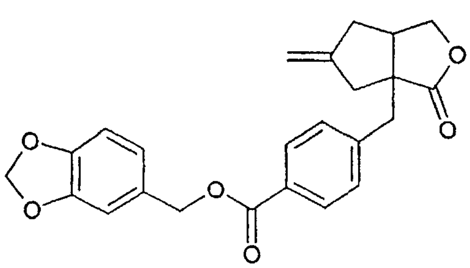 | 124 | 50 | 0,43 (II, 100:1) | 429 [M+Na+] |

| Bsp. Nr. | Struktur | Edukt Bsp. Nr. | Aus-beute [%] | Rf | MS |
|---|---|---|---|---|---|
| 208 | 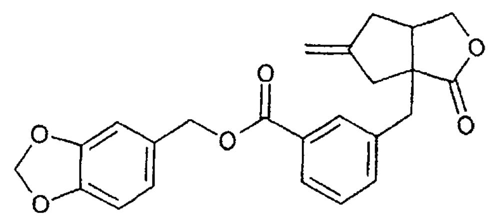 | 125 | 62 | 0,46 (II, 100:1) | 424 [M+NH4+] |
| 209 | 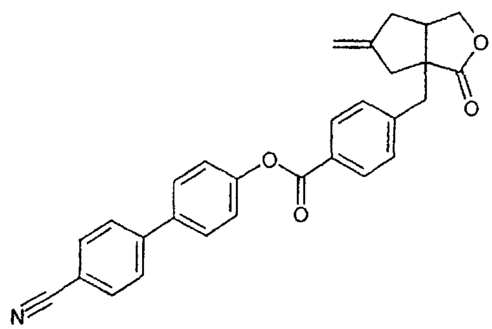 | 124 | 56 | 0,41 (II, 100:1) | 450 [M+H+] |

In Analogie zur Vorschrift des Beispiels 98 wurden die in der folgenden Tabelle aufgeführten Beispiele 210 bis 214 hergestellt:

| Bsp.-Nr. | Struktur | Edukt Bsp.Nr. | Ausbeute (%) | $R_f$ | MS [M+$NH_4^+$] |
|---|---|---|---|---|---|
| 210 | 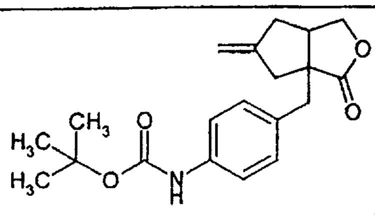 | 124 | 56 | 0,80 (III, 1:1) | 361 |
| 211 | 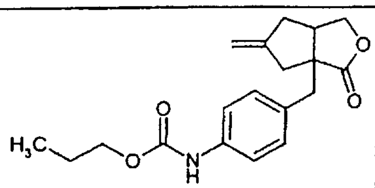 | 124 | 45 | 0,52 (III, 2:1) | 347 |
| 212 | 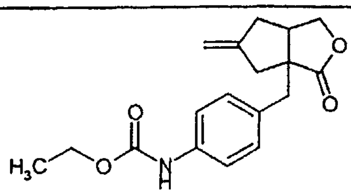 | 124 | 44 | 0,48 (III, 2:1) | 333 |
| 213 | 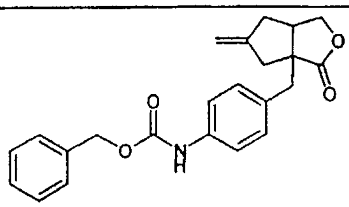 | 124 | 15 | 0,51 (III, 2:1) | 342 |
| 214 | 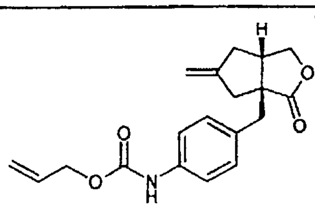 Chiral | 126 | 64 | 0,23 (III, 5:1) | 345 |

Analog der Vorschrift des Beispiels 1 wurden die in der folgenden Tabelle aufgeführten Beispiele 215 bis 231 hergestellt:

| Bsp.-Nr. | Struktur | Edukt Bsp.-Nr. | Ausbeute (%) | $R_f$ | MS |
|---|---|---|---|---|---|
| 215 | 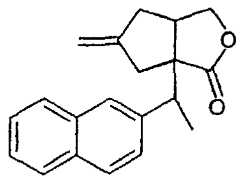 | 1A | 30 | 0,52 (III; 5:1) | 404 [$M+H^+$] |
| 216 | 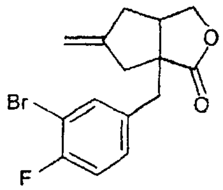 | 1A | 29 | 0,61 ($CH_2Cl_2$) | 342 [$M+H^+$] |
| 217 | 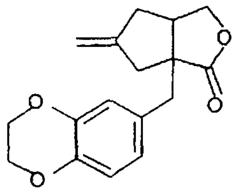 | 1A | 15 | 0,52 (I, 80:1) | 348 [$M+H^+$] |
| 218 | 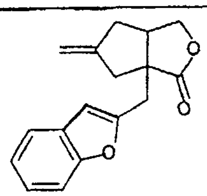 | 1A | 5 | 0,17 (I, 80:1) | 269 [$M+H^+$] |
| 219 | 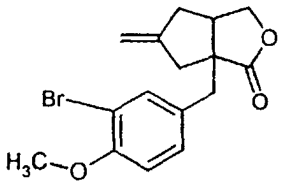 | 1A | 11 | 0,47 (III, 5:1) | 339 [$M+H^+$] |
| 220 | 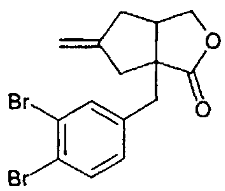 | 1A | 30 | 0,20 (IV, 5:1) | 404 [$M+NH_4^+$] |

| Bsp.-Nr. | Struktur | Edukt Bsp.-Nr. | Ausbeute (%) | $R_f$ | MS |
|---|---|---|---|---|---|
| 221 | 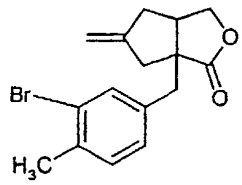 | 1A | 20 | 0,50 (IV, 1:1) | 322 $[M+Na^+]$ |
| 222 | 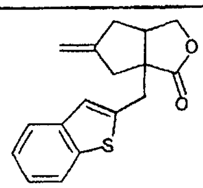 | 1A | 35 | 0,82 (I, 80:1) | 227 $[M+NH_4^+]$ |
| 223 | 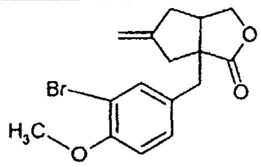 | 1A | 18 | 0,42 (III, 50;1) | 339 $[M+NH_4^+]$ |
| 224 | 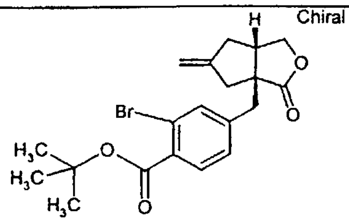 | 2A | 40 | 0,16 ($CH_2Cl_2$) | - |
| 225 | 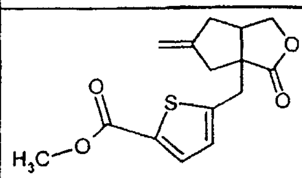 | 1A | 9 | 0,62 ($CH_2Cl_2$) | 310 $[MN+NH_4^+]$ |
| 226 | 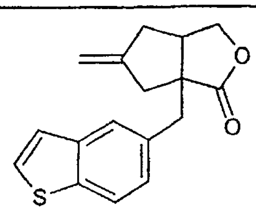 | 1A | 31 | 0,35 ($CH_2Cl_2$) | 285 $[M+H^+]$ |
| 227 | 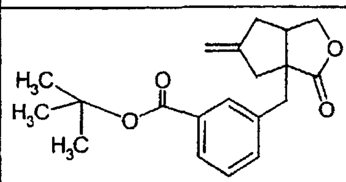 | 1A | 7 | 0,24 (III, 100:1) | 329 $[M+H^+]$ |

| Bsp.-Nr. | Struktur | Edukt Bsp.-Nr. | Ausbeute (%) | $R_f$ | MS |
|---|---|---|---|---|---|
| 228 | 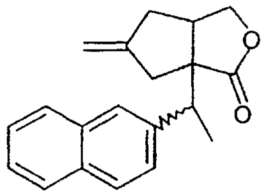 | 1A | 15 | 0,52 (III, 5:1)[a)] | 404 $[M+H^+]$ |
| 229 | 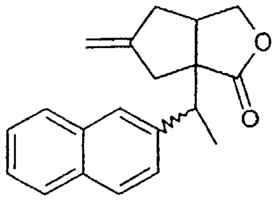 | 1A | 15 | 0,52 (III; 5:1)[b)] | 404 $[M+H^+]$ |
| 230 | 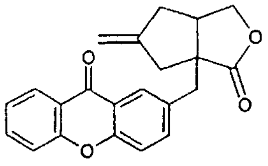 | 1A | 25 | 0,31 (I, 40:1) | 347 $[M+H^+]$ |
| 231 | 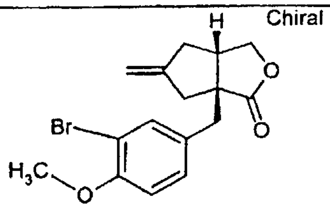 | 2A | 52 | 0,42 (I, 40:1) | 339 $[M+H^+]$ |

[a)] Diastereomer A: Fraktion 1 (HPLC, Kromasil 100C 18, Methanol/$H_2O$ 65:35)

[b)] Diastereomer B: Fraktion 2 (HPLC, Kromasil 100C 18, Methanol/$H_2O$ 65:35)

Analog der Verbindung aus Beispiel 106 wurden die in der folgenden Tabelle aufgeführten Beispiele 232 bis 255 hergestellt:

| Bsp.-Nr. | Struktur | MG (g/mol) | HPLC-Area % bei 210 nm | Mz+H |
|---|---|---|---|---|
| 232 | 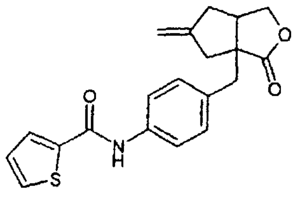 | 353,44 | 92 | 354 |
| 233 | 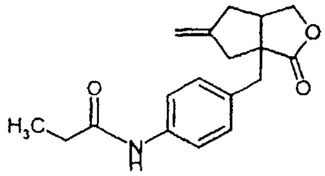 | 299,37 | 75 | 300 |
| 234 | 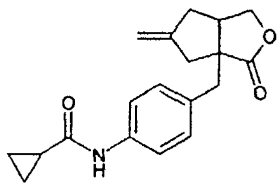 | 311,38 | 96 | 312 |
| 235 | 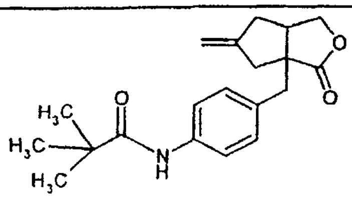 | 327,42 | 98 | 328 |
| 236 | 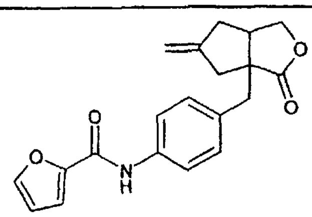 | 337,37 | 97 | 338 |
| 237 | 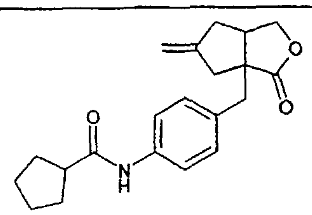 | 339,43 | 89 | 340 |

| Bsp.-Nr. | Struktur | MG (g/mol) | HPLC-Area % bei 210 nm | Mz+H |
|---|---|---|---|---|
| 238 | 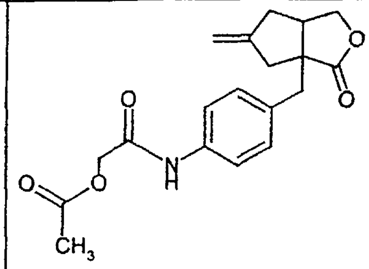 | 343,38 | 96 | 344 |
| 239 | 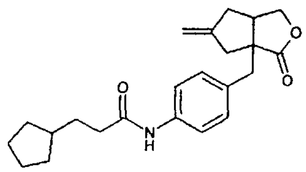 | 367,49 | 88 | 368 |
| 240 | 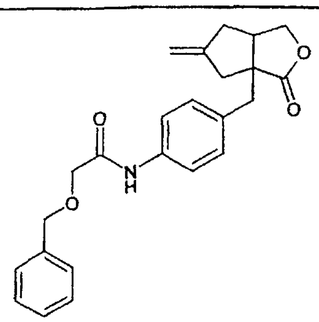 | 391,47 | 97 | 392 |
| 241 | 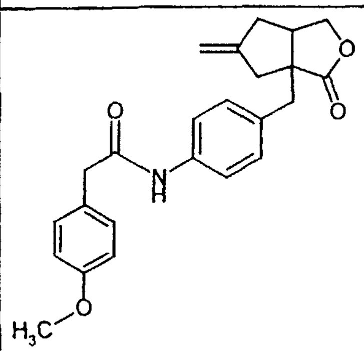 | 391,47 | 97 | 392 |
| 242 | 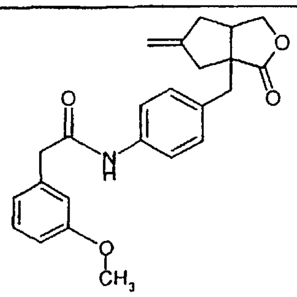 | 391,47 | 95 | 392 |

| Bsp.-Nr. | Struktur | MG (g/mol) | HPLC-Area % bei 210 nm | Mz+H |
|---|---|---|---|---|
| 243 | 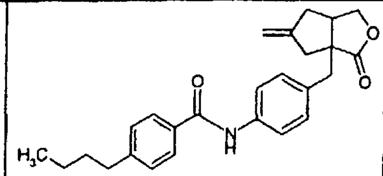 | 403,52 | 91 | 404 |
| 244 | 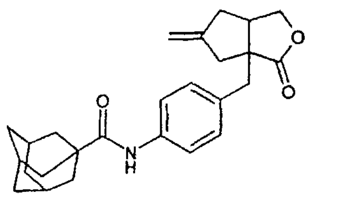 | 405,54 | 92 | 244 |
| 245 | 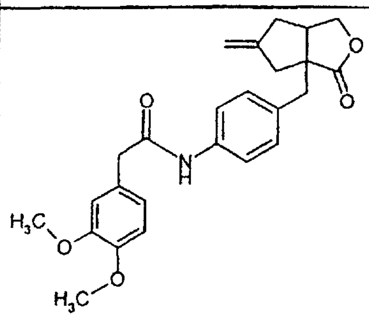 | 421,49 | 82 | 422 |
| 246 | 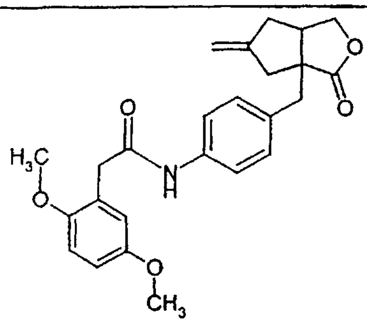 | 421,49 | 95 | 422 |
| 247 | 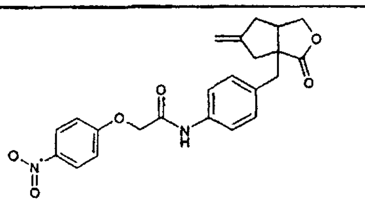 | 422,44 | 6 | 423 |

| Bsp.-Nr. | Struktur | MG (g/mol) | HPLC-Area % bei 210 nm | Mz+H |
|---|---|---|---|---|
| 248 | 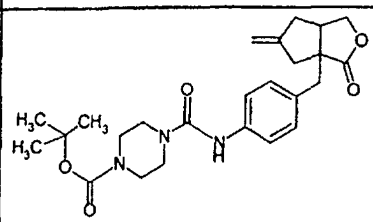 | 455,55 | 61 | 456 |
| 249 | 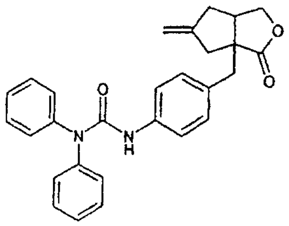 | 438,53 | 16 | 439 |
| 250 | 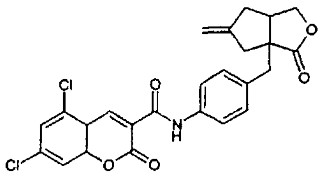 | 486,35 | 49 | 486 |
| 251 | 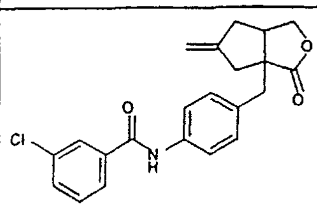 | 381,86 | 95 | 382 |
| 252 | 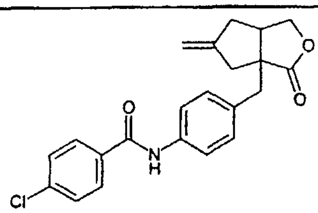 | 381,86 | 94 | 382 |
| 253 | 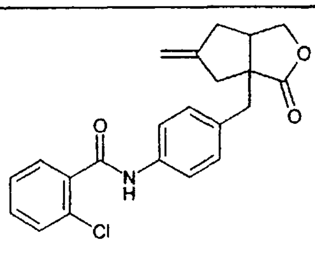 | 381,86 | 94 | 382 |

| Bsp.-Nr. | Struktur | MG (g/mol) | HPLC-Area % bei 210 nm | Mz+H |
|---|---|---|---|---|
| 254 | 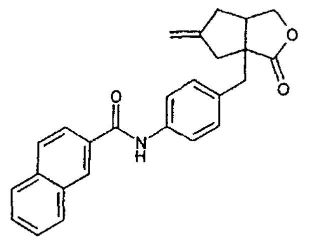 | 397,47 | 92 | 398 |
| 255 | 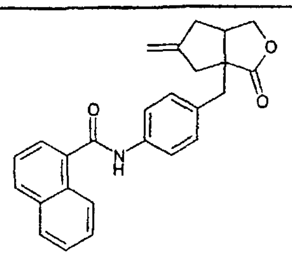 | 397,47 | 98 | 398 |

Analog Beispiel 106 wurden die in der folgenden Tabelle aufgeführten Verbindungen hergestellt:

| Bsp.-Nr. | Struktur | Ausbeute (%) | MS |
|---|---|---|---|
| 256 | 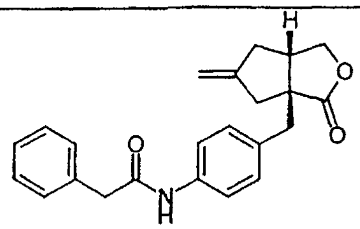 | 87 | 362 $[M+H^+]$ |
| 257 | 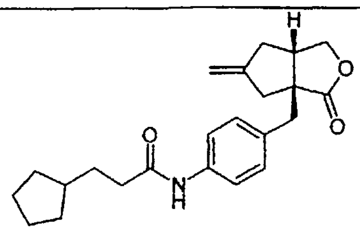 | 80 | 409 $[M+H_3CCN+H^+]$ |
| 258 | 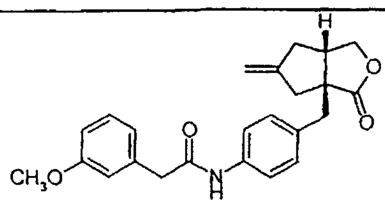 | 97 | 392 $[M+H^+]$ |

**Beispiel 259**

N[(3a"S*,6a"S)-4-(5-Methyliden-hexahydro-cyclopenta[c]furan-1-on-6a-ylmethylphenyl]-4-methylpentancarbonsäureamid

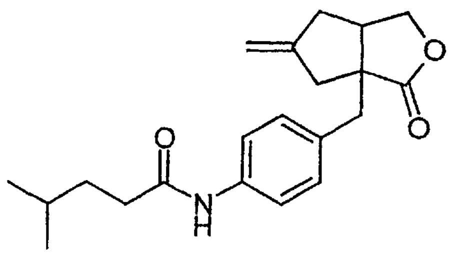

Zu einer Lösung der Verbindung aus Beispiel 104 (20,0 mg, 0,082 mmol) in Dichlormethan (5 ml) wurden bei 0°C, N,N-Dimethylaminopyridin (10,1 mg, 0,09 mmol), 1-Ethyl-3-(3'-dimethylamino)propyl)-carbodiimidhydrochlorid (17,3 mg, 0,09 mmol) und 4-Methylpentancarbonsäure (10,5 mg, 0,09 mmol) gegeben und das Reaktionsgemisch 6 Stunden bei Raumtemperatur gerührt. Zur Aufarbeitung wurde 1 M wäßrige HCl zugegeben (0,7 ml), das Gemisch über eine Fritte, gefüllt mit Bitumenerde/Kieselgel, mit Dichlormethan filtriert, das Filtrat mit 10% wäßriger $NaHCO_3$ (0,7 ml) versetzt, erneut über eine Fritte, gefüllt mit Bitumenerde, filtriert und im Vakuum das Lösemittel abgezogen.
Ausbeute: 27,6 mg (98 %)
$R_f$ (III, 2:1) = 0,38
MS(ESI): m/z = 342 [M+H$^+$]

**Beispiel 260**

(3aS*,6aS*)-6a-(4-(2-Methylpropylaminobenzyl)-5-methyliden-hexahydro-cyclopenta[c]furan-1-on

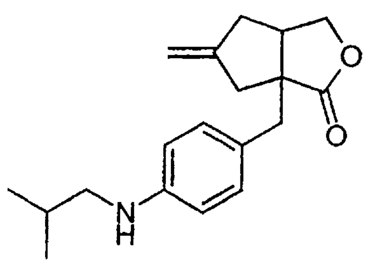

Eine Lösung der Verbindung aus Beispiel 104 (15 mg, 0,062 mmol), 2-Methylpropanal (4,5 mg, 0,062 mmol) in Methanol/Essigsäure (3:1, 1,2 ml) wurde 20 Minuten bei Raumtemperatur gerührt und anschließend mit Natriumcyanoborhydrid (5,0 mg, 0,08 mmol) in Methanol (0,6 ml) versetzt. Nach 20 Stunden wurde mit Ether versetzt, die organische Phase mit ges. $NaHCO_3$ gewaschen und getrocknet ($MgSO_4$). Der Rückstand wurde durch Chromatografie gereinigt.
Ausbeute: 3,7 mg (20 %)
$R_f$ (II, 50:1) = 0,35
MS (ESI): m/z = 300 [M+H$^+$]

**Beispiel 261**

(3aS*,6aS*)-6a-(4-Hydroxybenzyl)-5-methyliden-hexahydro-cyclopenta[c]furan-1-on

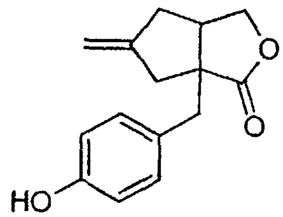

Eine Mischung der Verbindung aus Beispiel 279 (192 mg, 0,59 mmol), $K_2CO_3$ (486 mg, 3,52 mmol) in Wasser (4,7 ml) und Methanol (7,1 ml) wurde 30 Minuten bei Raumtemperatur gerührt. Das Gemisch wurde mit 1 N wäßriger HCl auf pH = 2 gebracht, mit NaCl gesättigt und mit Essigester extrahiert. Die vereinigten organischen Phasen wurde getrocknet ($Na_2SO_4$) und im Vakuum die Lösemittel abgezogen. Ausbeute: 157 mg (quant.)
$R_f$ (III, 2:1) = 0,31
MS (ESI): m/z = 286 [$M+H^+$]

**Beispiel 262**

(3aS*,6aS*)-6a-(4-(3-Methylbutyloxy)benzyl)-5-methyliden-hexahydro-cyclopenta[c]furan-1-on

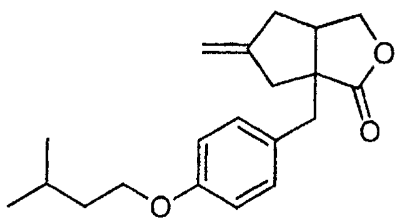

Eine Mischung der Verbindung aus Beispiel 261 (13,3 mg, 0,054 mol), 1-Brom-3-methylbutan (6,8 mg, 0,045 mmol), $Cs_2CO_3$ (44,4 mg, 0,136 mmol) in Dimethoxyethan (5 ml) wurde 48 Stunden bei 75°C gerührt. Zur Aufarbeitung wurde mit Dichlormethan und 1 M wäßriger NaOH versetzt und das Gemisch über eine Fritte, gefüllt mit Bitumenerde, filtriert, das Lösemittel abgezogen und der Rückstand durch Chromatografie gereinigt.
Ausbeute: 2,8 mg (16%)
$R_f$ (III, 5:1) = 0,49
LCMS: m/z = 356 [$M+H_3CCN+H^+$]

**Beispiel 263 und 264**

N-[(3a"R*, 6a"R*)-4-(5-Methyliden-hexahydro-cyclopenta[c]furan-1-on-6a-ylmethyl)-phenyl]-(3-methoxyphenyl)essigsäureamid (264) und
N-[(3a"S*, 6a"S*)-4-(5-Methyl-3,3a,6,6a-tetrahydro-cyclopenta[c]furan-1-on-6a-ylmethyl)-phenyl]-(3-methoxyphenyl) essigsäureamid (265)

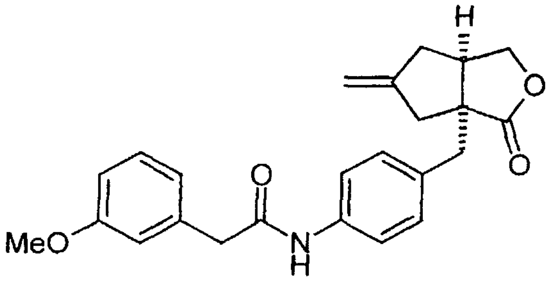 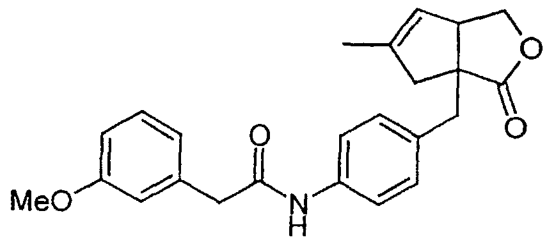

Analog der Vorschrift des Beispiels 106 wurden die Verbindung aus Beispiel 104 und 3-Methoxyphenylessigsäurechlorid umgesetzt. Das Rohprodukt wurde mittels HPLC (Chiralpak AS, Ethanol/iso-Hexan 20:80) in die Enantiomeren getrennt.

| | |
|---|---|
| Fraktion I (Beispiel 265): | Enantiomer A, $[\alpha]^{20}_{D}$ = -46° (c = 0,473, $CHCl_3$) |
| Fraktion II: | nicht identifiziert |
| Fraktion III (Beispiel 264): | Enantiomer A, $[\alpha]^{20}_{D}$ = -22,2° (c = 0,200, $CHCl_3$) |
| Fraktion IV (Beispiel ...): | Enantiomer B |

Analog der Vorschrift des Beispiels 1 wurden die in der Tabelle aufgeführten Verbindungen dargestellt:

| Bsp. | Struktur | Edukt Bsp.-Nr. | Ausbeute [%] | Rf (Cyclohexan - Ethylacetat 3:1) | MS (CI) [M⁺ + 1] |
|---|---|---|---|---|---|
| 265 | 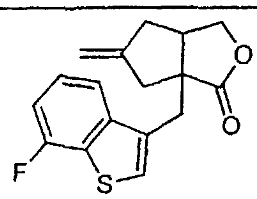 | 1 A | 9.9 | 0.50 | 303 |
| 266 | 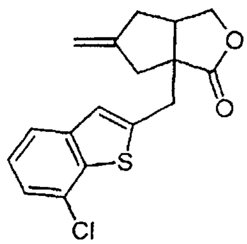 | 1 A | 3.5 | 0.51 | 319 |
| 267 | 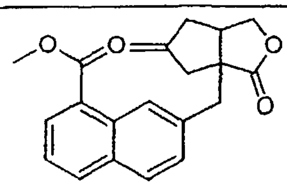 | 1 A | 6.8 | 0.42 | 337 |
| 268 | 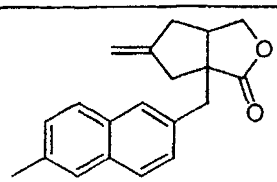 | 1 A | 10.2 | 0.63 | 335 |
| 269 | 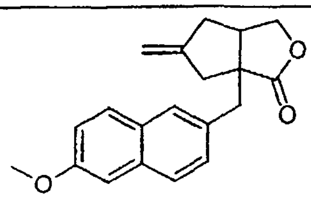 | 1 A | 29.2 | 0.46 | 309 |
| 270 | 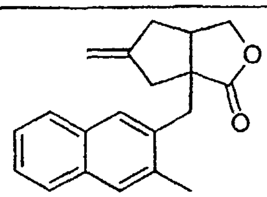 | 1 A | 15.4 | 0.54 | 293 |

| Bsp. | Struktur | Edukt Bsp.-Nr. | Ausbeute [%] | Rf (Cyclohexan - Ethylacetat 3:1) | MS (CI) [$M^+ + 1$] |
|---|---|---|---|---|---|
| 271 | 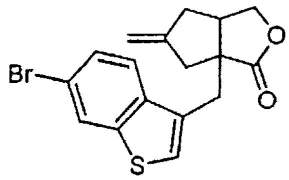 | 1 A | 9.1 | 0.52 | 364 |
| 272 | 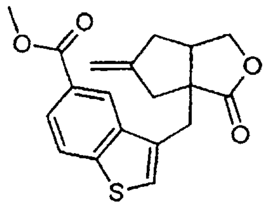 | 1 A | 8.2 | 0.39 | 343 |
| 273 | 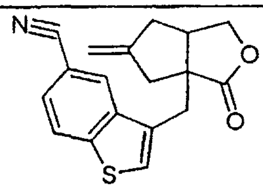 | 1 A | 21.0 | 0.35 | 310 |
| 274 | 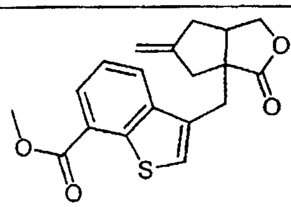 | 1 A | 10.5 | 0.36 | 343 |
| 275 | 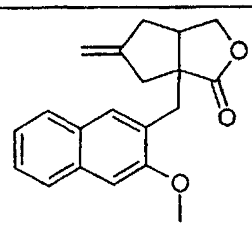 | 1 A | 13.0 | 0.46 | 309 |

| Bsp. Nr. | Struktur | Ausbeute % | Edukt | Vorschrift analog Bsp. | Rf | MS |
|---|---|---|---|---|---|---|
| 276 | 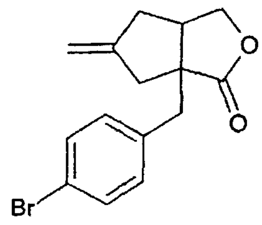 | 39 | 1A | 1 | 0,5 (CH2Cl2) | 324 [M+NH4+] |
| 277 | 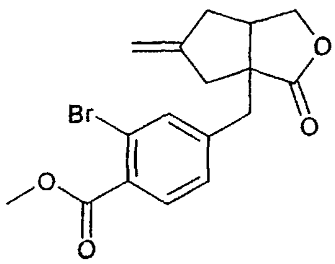 | 22 | 1A | 1 | 0,50 (II, 1:1) | 382/384 [M+NH4+] |
| 278 | 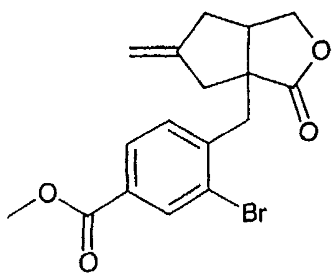 | 17 | 1A | 1 | 0,53 (III, 2:1) | 382/384 [M+NH4+] |
| 279 | 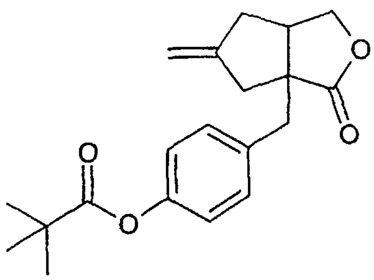 | 30 | 1A | 1 | 0,20 (III, 5:1) | |
| 280 | 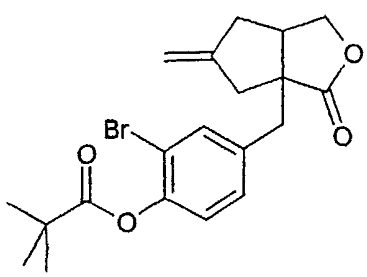 | 43 | 1A | 1 | | 407 [M+H*] |

Die in der folgenden Tabelle aufgeführten Beispiele wurden analog der angegebenen Vorschriften dargestellt.

| Bsp. Nr. | Struktur | Aus-beute % | Edukt | Vor-schrift analog Bsp. | Rf | MS |
|---|---|---|---|---|---|---|
| 281 | 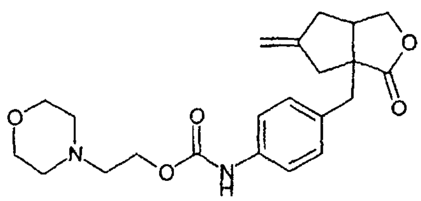 | 30 | 124 | 98 | 0,23 (II, 20:1) | 401 [M+H+9] |
| 282 | 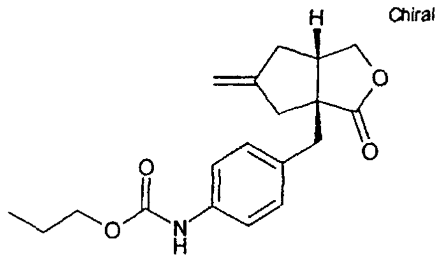 | 27 | 124 | 98 | 0,47 (III, 2:1) | 330 [M+H+] |
| 283 | 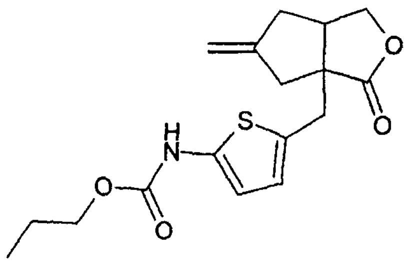 | 26 | 318 | 98 | 0,36 (I, 40:1) | 336 [M+H+] |
| 284 | 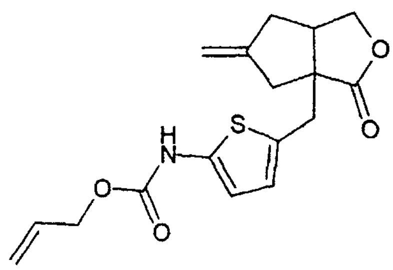 | 20 | 318 | 98 | 0,34 (I, 40:1) | 351 [M+NH4+] |
| 285 | 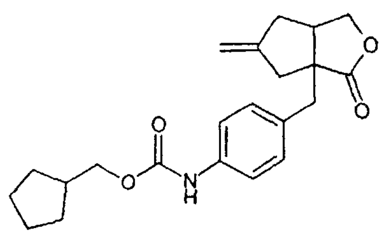 | 88 | 124 | 98 | 0,23 (III, 5:1) | 370 [M+H+] |

| Bsp. Nr. | Struktur | Ausbeute % | Edukt | Vorschrift analog Bsp. | Rf | MS |
|---|---|---|---|---|---|---|
| 286 | 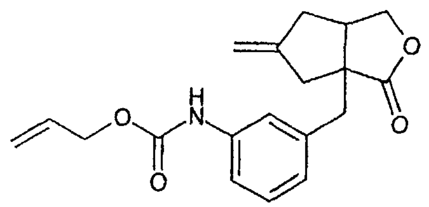 | 73 | 125 | 98 | 0,15 (CH2Cl2) | |
| 287 | 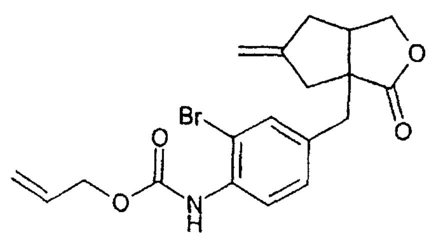 | 56 | 327 | 98 | 0,54 (III, 1:1) | 423/425 [M+NH4+] |
| 288 | 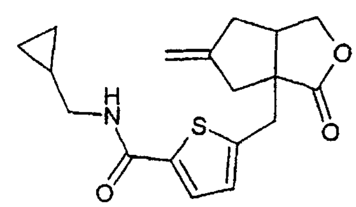 | 73 | 318 | 127 | 0,43 (I, 40:1) | 332 [M+H+] |
| 289 | 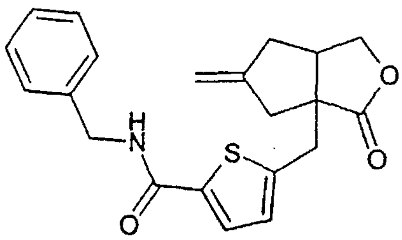 | 91 | 318 | 127 | 0,46 (I, 40:1) | 368 [M+H+] |
| 290 | 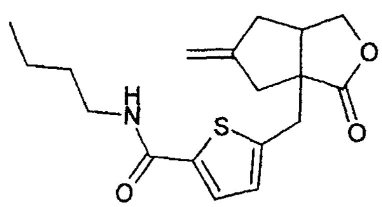 | 92 | 318 | 127 | 0,45 (I, 40:1) | 334 [M+H+] |

| Bsp. Nr. | Struktur | Aus-beute % | Edukt | Vor-schrift analog Bsp. | Rf | MS |
|---|---|---|---|---|---|---|
| 291 | 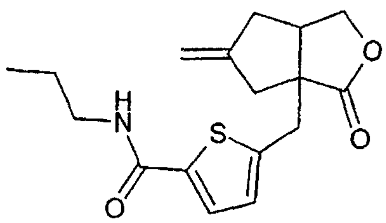 | 93 | 318 | 127 | 0,37 (I, 40:1) | 320 [M+H+] |
| 292 | 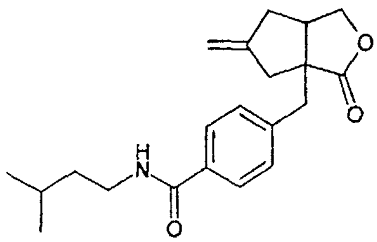 | 98 | 124 | 127 | 0,20 (III, 2:1) | 383 [M+H3CCN +H+] |
| 293 | 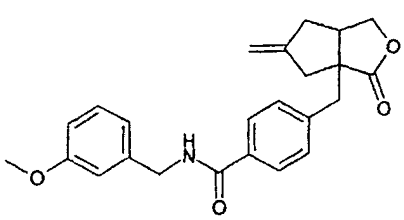 | quant. | 124 | 127 | 0,37 (I, 40:1) | |
| 294 | 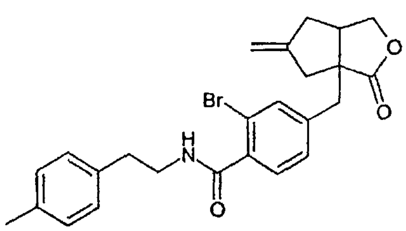 | 72 | 327 | 127 | 0,79 (I, 10:1) | 509/511 [M+H3CCN +H+] |
| 295 | 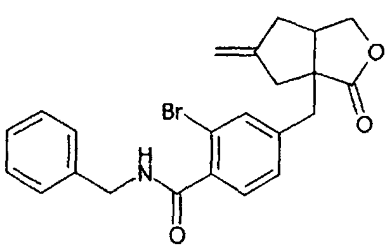 | 66 | 327 | 127 | 0,79 (I, 10:1) | 481/483 [M+H3CCN +H+] |

| Bsp. Nr. | Struktur | Ausbeute % | Edukt | Vorschrift analog Bsp. | Rf | MS |
|---|---|---|---|---|---|---|
| 296 | 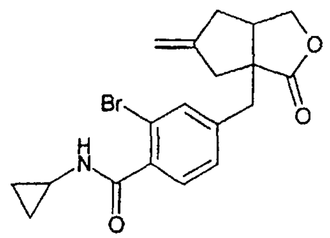 | 47 | 327 | 127 | 0,66 (I, 10:1) | 421/423 [M+H3CCN +H+] |
| 297 | 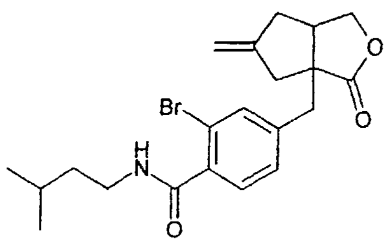 | 59 | 327 | 127 | 0,77 (I, 10:1) | 459/461 [M+H3CCN +H+] |
| 298 | 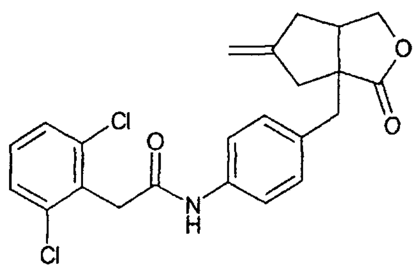 | 91 | 104 | 259 | 0,38 (II, 2:1) | 430 [M+H+] |
| 299 | 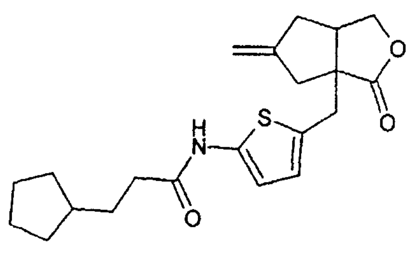 | 7 | 325 | 106 | 0,43 (III, 1:1) | 374 [M+H+] |
| 300 | 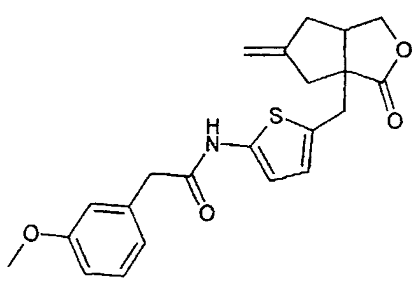 | 7 | 325 | 106 | 0,31 (III, 1:1) | 398 [M+H+] |

| Bsp. Nr. | Struktur | Ausbeute % | Edukt | Vorschrift analog Bsp. | Rf | MS |
|---|---|---|---|---|---|---|
| 301 | 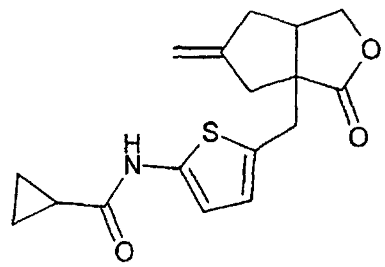 | 7 | 325 | 106 | 0,29 (III, 1:1) | 318 [M+H+] |
| 302 | 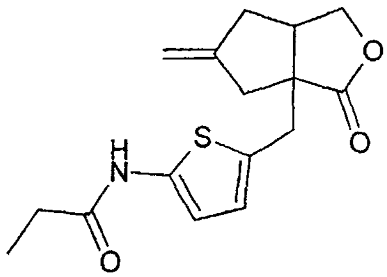 | 17 | 325 | 106 | 0,22 (III, 1:1) | 306 [M+H+] |
| 303 | 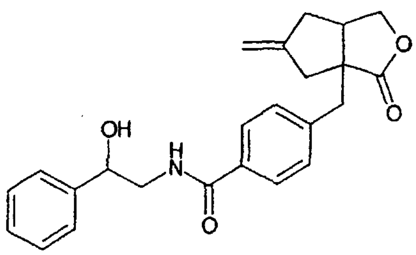 | 73 | 124 | 127 | 0,21 (II, 20:1) | 392 [M+H+] |
| 304 | 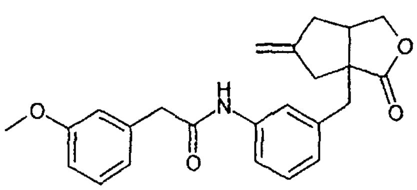 | 17 | 326 | 106 | 0,38 (III, 1:1) | 392 [M+H+] |
| 305 | 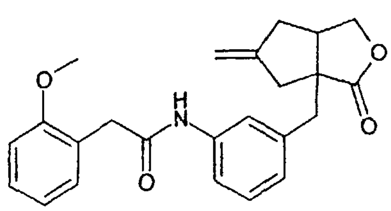 | 86 | 326 | 259 | 0,35 (III, 1:1) | 392 [M+H+] |

| Bsp. Nr. | Struktur | Ausbeute % | Edukt | Vorschrift analog Bsp. | Rf | MS |
|---|---|---|---|---|---|---|
| 306 | 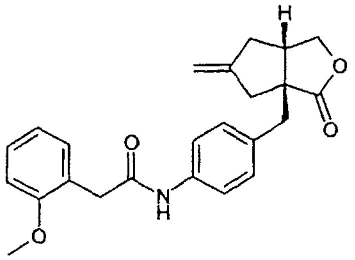 | 41 | 105 | 259 | 0,40 (III, 1:1) | |
| 307 | 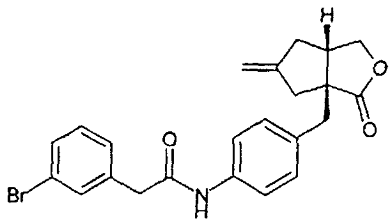 | 80 | 105 | 259 | 0,33 (III, 1:1) | |
| 308 | 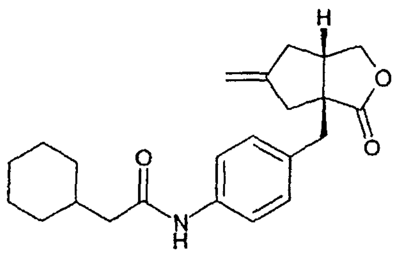 | 53 | 105 | 259 | 0,48 (III, 1:1) | |
| 309 | 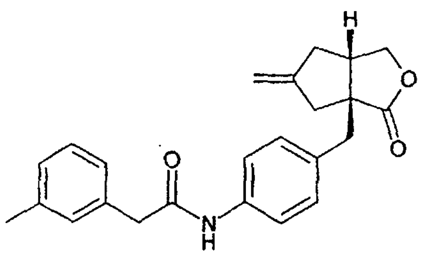 | 64 | 105 | 259 | 0,38 (III, 1:1) | |
| 310 | 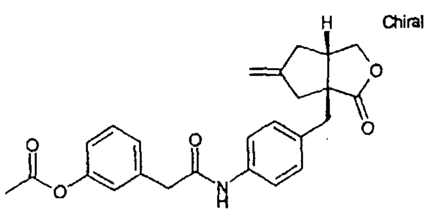 | 18 | 105 | 259 | 0,17 (III, 1:1) | 437 [M+H+] |

| Bsp. Nr. | Struktur | Ausbeute % | Edukt | Vorschrift analog Bsp. | Rf | MS |
|---|---|---|---|---|---|---|
| 311 | 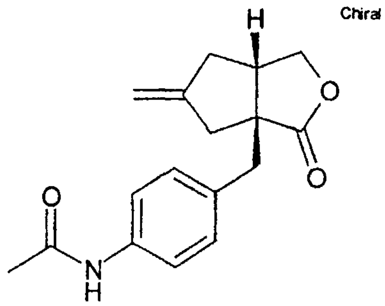 | 33 | 105 | 259 | 0,04 (III, 1:1) | |
| 312 | 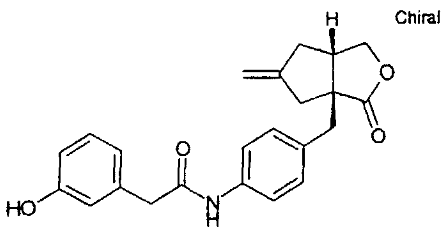 | 95 | 310 | 261 | 0,58 (I, 10:1) | 395 [M+NH4+] |
| 313 | 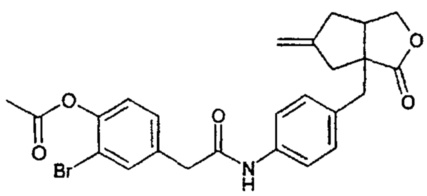 | 27 | 104 | 259 | | 498 [M+H+] |
| 314 | 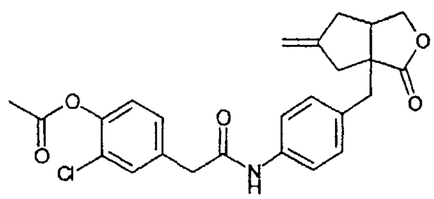 | 26 | 104 | 259 | | 453 [M+H+] |
| 315 | 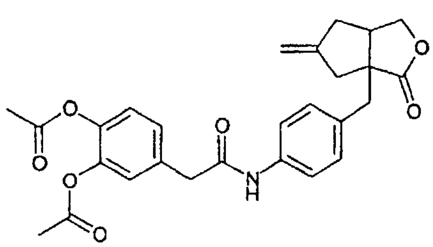 | 7 | 104 | 259 | | 478 [M+H+] |

| Bsp. Nr. | Struktur | Aus-beute % | Edukt | Vor-schrift analog Bsp. | Rf | MS |
|---|---|---|---|---|---|---|
| 316 | 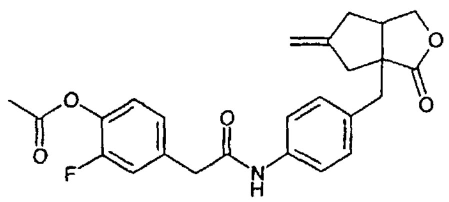 | 28 | 104 | 259 | | 438 [M+H+] |
| 317 | 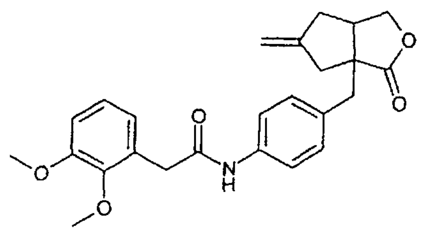 | quant. | 104 | 259 | 0,26 (II, 50:1) | 422 [M+H+] |
| 318 | 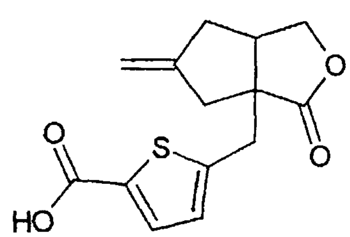 | 98 | 1A | 1(Alky-lierung) 124(Ver-seifung des Esters) | | 296 [M+NH4+] |
| 319 | 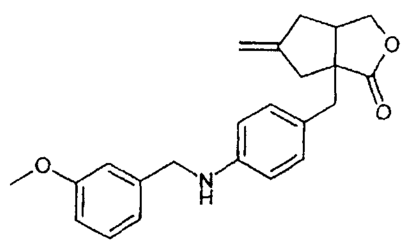 | 98 | 104 | 260 | 0,61 (II, 20:1) | 405 [M+H3CCN +H+] |
| 320 | 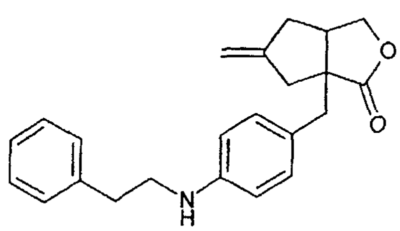 | 35 | 104 | 260 | 0,25 (III, 2:1) | 389 [M+H3CCN +H+] |

| Bsp. Nr. | Struktur | Aus-beute % | Edukt | Vor-schrift analog Bsp. | Rf | MS |
|---|---|---|---|---|---|---|
| 321 | 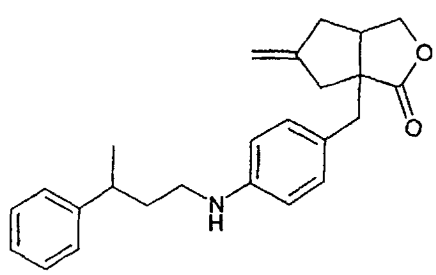 | 53 | 104 | 260 | 0,62 (III, 2:1) | 376 [M+H+] |
| 322 | 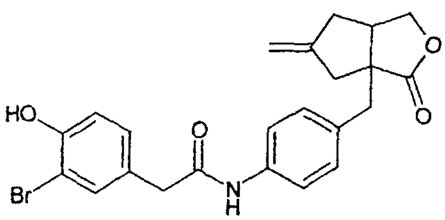 | 79 | 313 | 261 | 0,52 (I, 10:1) | 456 [M+H+] |
| 323 | 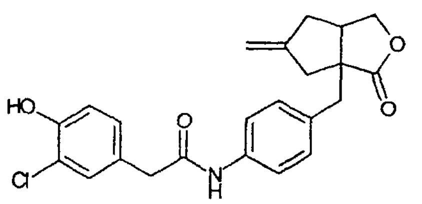 | 69 | 314 | 261 | 0,4 (I, 10:1) | 454 [M+H+] |
| 324 | 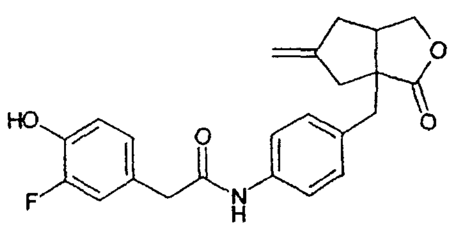 | 83 | 316 | 261 | 0,51 (I, 10:1) | 437 [M+H3CCN +H+] |
| 325 | 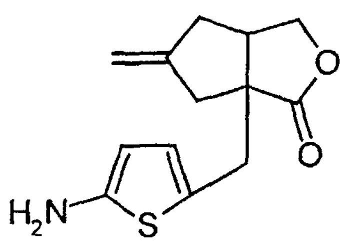 | quant. | 284 | 104 | 0,33 (III, 1:1) | - |

| Bsp. Nr. | Struktur | Aus-beute % | Edukt | Vor-schrift analog Bsp. | Rf | MS |
|---|---|---|---|---|---|---|
| 326 | 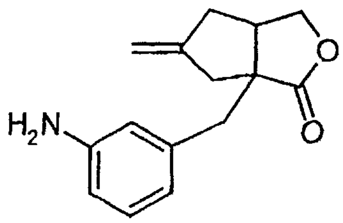 | 5 | 286 | 104 | 0,32 (III, 1:1) | - |
| 327 | 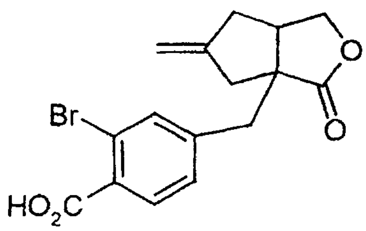 | quant. | 277 | 124 | - | 351 [M+H+] |
| 328 | 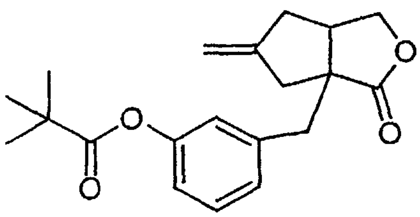 | 40 | 1A | 1 | 0,49 (III, 2:1) | 346 [M+NH4+] |
| 329 | 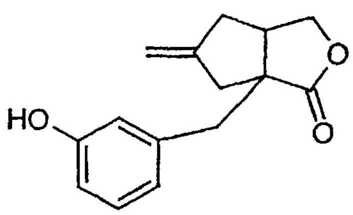 | 83 | 328 | 261 | 0,31 (II, 20:1) | 262 [M+NH4+] |
| 330 | 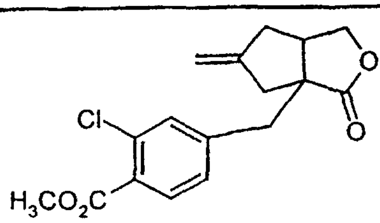 | 28 | 1A | 1 | 0,39 (CH2Cl2) | 321 [M+NH4+] |

| Bsp. Nr. | Struktur | Ausbeute % | Edukt | Vorschrift analog Bsp. | Rf | MS |
|---|---|---|---|---|---|---|
| 331 | 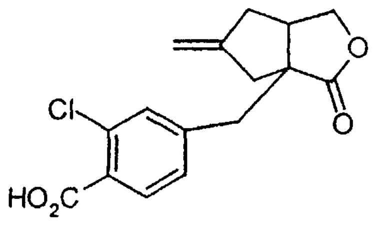 | quant. | 330 | 124 | - | 307 [M+H+] |
| 332 | 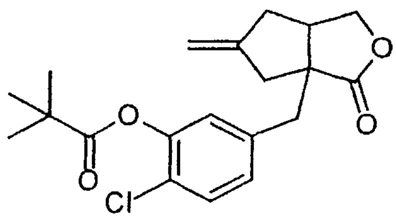 | | 1 | 1A | | |
| 333 | 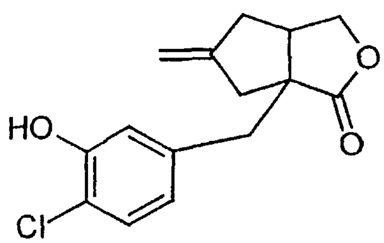 | | 332 | 261 | | |
| 334 | 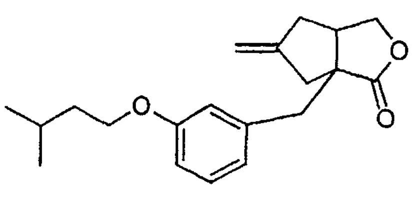 | | 329 | 262 | | |
| 335 | 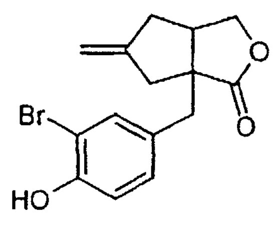 | quant. | 280 | 261 | 0.35 (II, 20:1) | - |
| 336 | 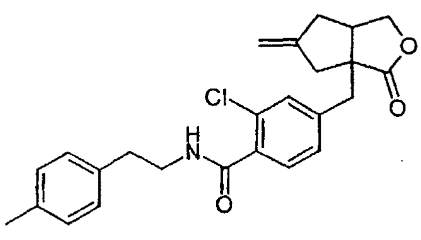 | 90 | 331 | 127 | | 465 [M+H+H3CCN]+ |

| Bsp. Nr. | Struktur | Aus-beute % | Edukt | Vor-schrift analog Bsp. | Rf | MS |
|---|---|---|---|---|---|---|
| 337 | 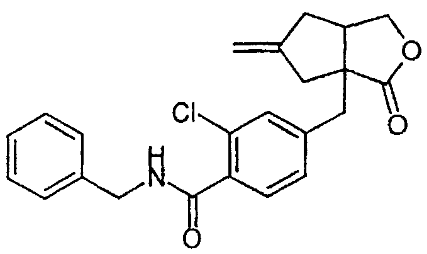 | 87 | 331 | 127 | | 437 [M+H3CCN +H]+ |
| 338 | 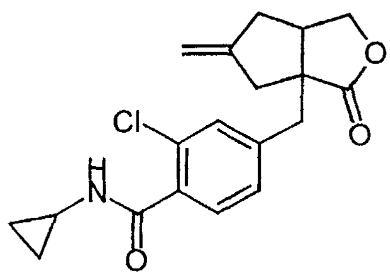 | 98 | 331 | 127 | | 387 [M+H3CCN +H]+ |
| 339 | 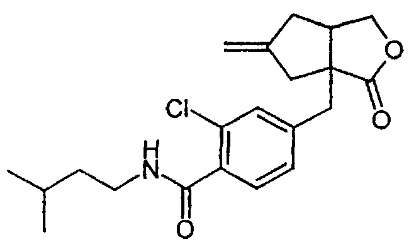 | 78 | 331 | 127 | | 417 [M+H3CCN +H]+ |

| Bsp.-Nr. | Struktur | Ausbeute in % | Edukt | Vorschrift analog Bsp. | Rf | MS |
|---|---|---|---|---|---|---|
| 340 | 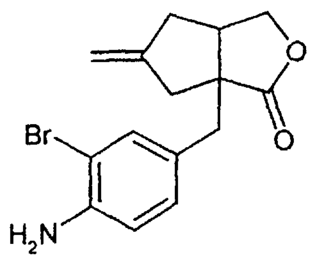 | 63 | 287 | 104 | - | 363/365 [M+H3CCN +H+] |
| 341 | 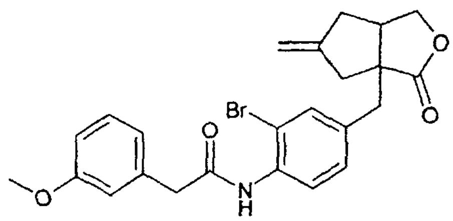 | 41 | 340 | 259 | 0,42 (III, 1:1) | 487/489 [M+H+] |
| 342 | 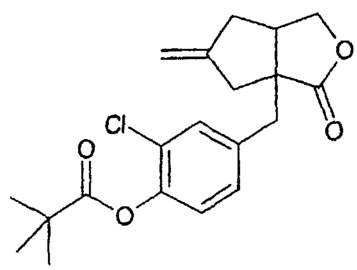 | 41 | 1A | 1 | 0,35 (CH2Cl2) | - |
| 343 | 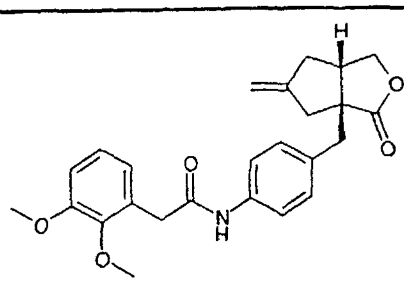 Chiral | 96 | 105 | 259 | 0,37 (III, 1:1) | 422 [M+H+] |
| 344 | 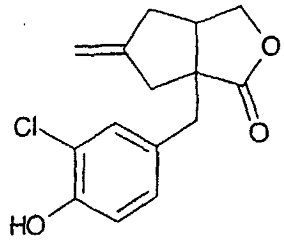 | 79 | 342 | 261 | 0,21 (CH2Cl2) | - |

In Analogie zur Vorschrift des Beispiels 127 wurden die in der folgenden Tabelle aufgeführten Beispiele dar-

gestellt.

| Bsp.-Nr. | Struktur | Formel | Molgewicht [g/mol] | Mz+H |
|---|---|---|---|---|
| 345 | 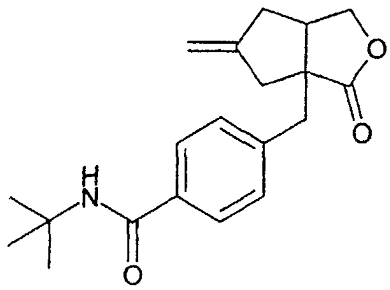 | C20 H25 N O3 | 327,4272 | 328 |
| 346 | 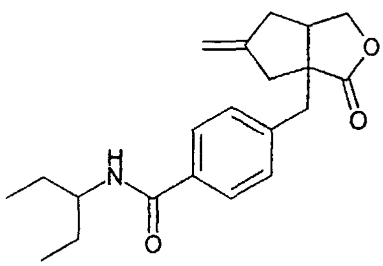 | C21 H27 N O3 | 341,4543 | 342 |
| 347 | 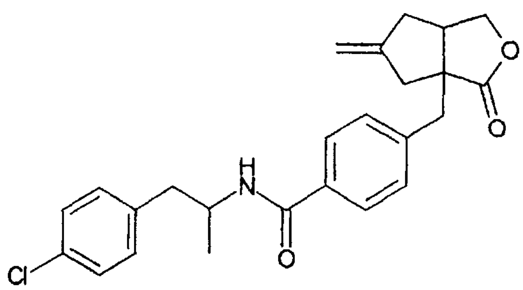 | C25 H26 Cl N O3 | 423,9439 | 424 |
| 348 | 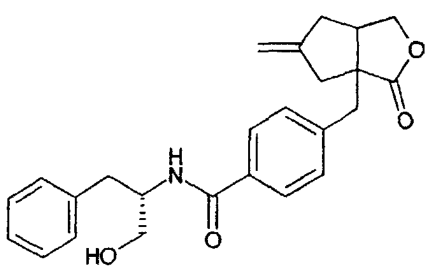 | C25 H27 N O4 | 405,4982 | 406 |

| Bsp.-Nr. | Struktur | Formel | Molgewicht [g/mol] | Mz+H |
|---|---|---|---|---|
| 349 | 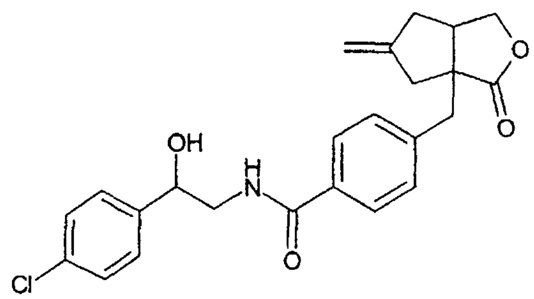 | C24 H24 Cl N O4 | 425,9162 | 426 |
| 350 | 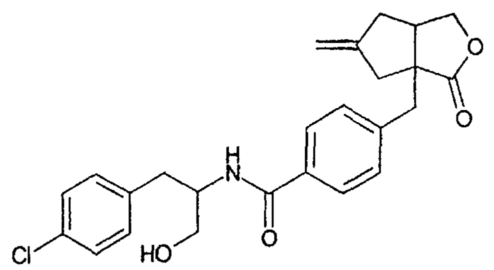 | C25 H26 Cl N O4 | 439,9433 | 440 |
| 351 | 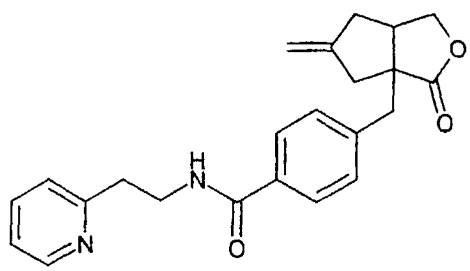 | C23 H24 N2 O3 | 376,4594 | 377 |
| 352 | 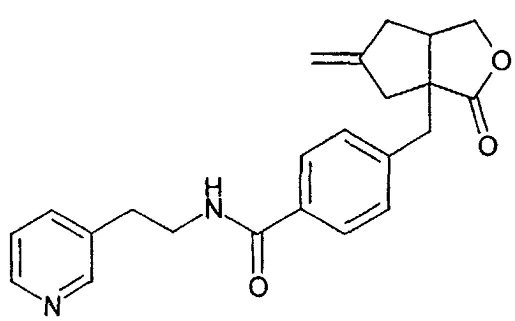 | C23 H24 N2 O3 | 376,4594 | 377 |

| Bsp.-Nr. | Struktur | Formel | Molgewicht [g/mol] | Mz+H |
|---|---|---|---|---|
| 353 | 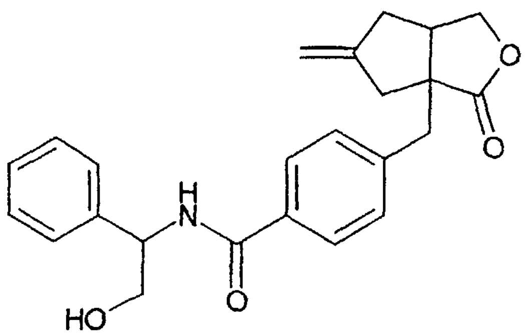 | C24 H25 N O4 | 391,4712 | 392 |

In Analogie zur Vorschrift des Beispiels 259 wurden die in der Tabelle aufgeführten Verbindungen dargestellt.

| Bsp.-Nr. | Struktur | Formel | Molge- wicht [g/mol] | Mz + H |
|---|---|---|---|---|
| 354 | 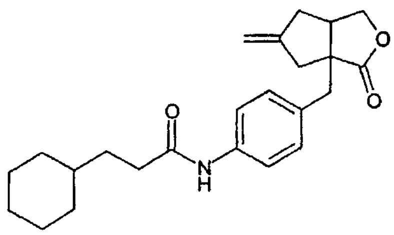 | C24H31NO3 | 381,51957 | 382 |
| 355 | Chiral 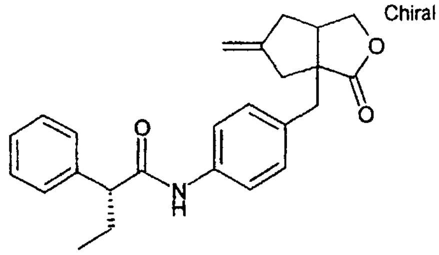 | C25H27NO3 | 389,49884 | 390 |
| 356 | Chiral 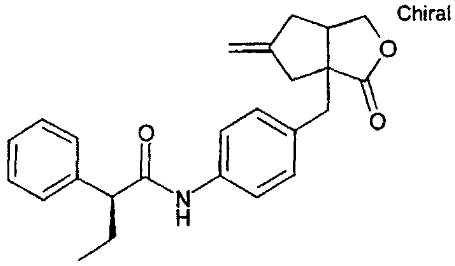 | C25H27NO3 | 389,49884 | 390 |
| 357 | 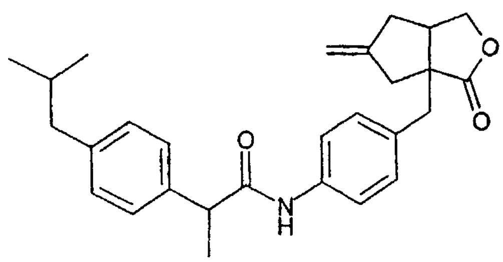 | C28H33NO3 | 431,58011 | 432 |
| 358 | 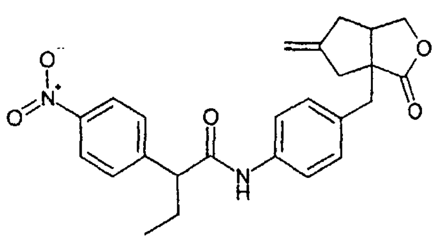 | C25H26N2O5 | 434,49637 | 435 |

| Bsp.-Nr. | Struktur | Formel | Molge- wicht [g/mol] | Mz + H |
|---|---|---|---|---|
| 359 | 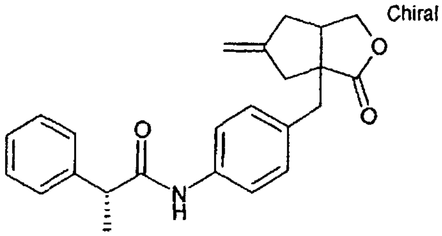 Chiral | C24H25NO3 | 375,47175 | 376 |
| 360 | 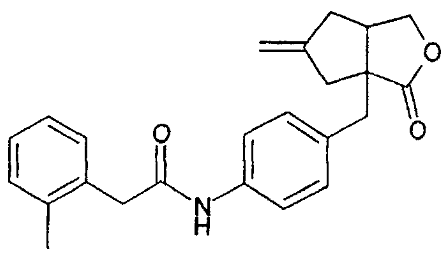 | C24H25NO3 | 375,47175 | 376 |
| 361 | 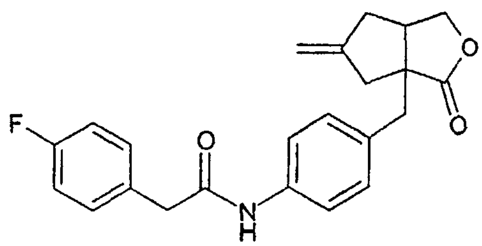 | C23H22FNO3 | 379,43509 | 380 |
| 362 | 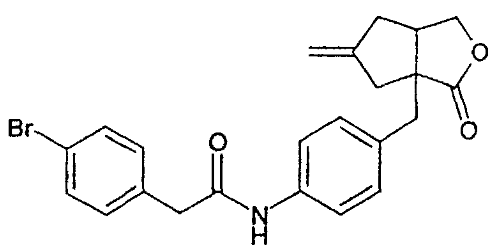 | C23H22BrNO3 | 440,34069 | 440 |
| 363 | 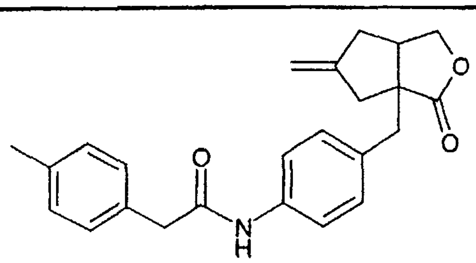 | C24H25NO3 | 375,47175 | 376 |

| Bsp.-Nr. | Struktur | Formel | Molge- wicht [g/mol] | Mz + H |
|---|---|---|---|---|
| 364 | 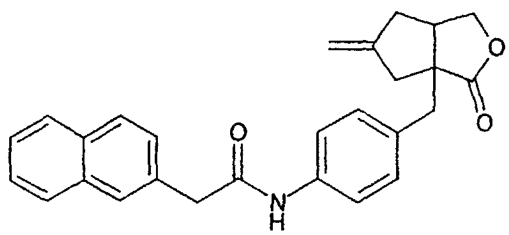 | C27H25NO3 | 411,5052 | 412 |
| 365 | 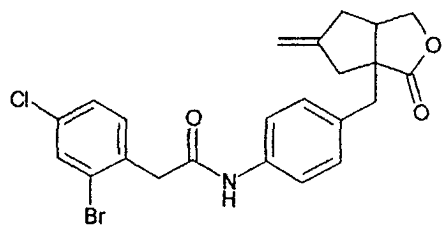 | C23H21BrClNO3 | 474,78572 | 474 |
| 366 | 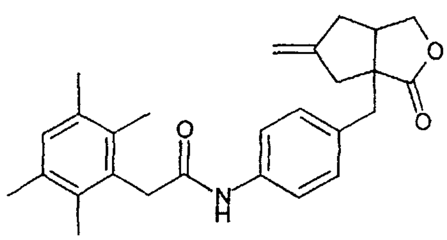 | C27H31NO3 | 417,55302 | 418 |
| 367 | 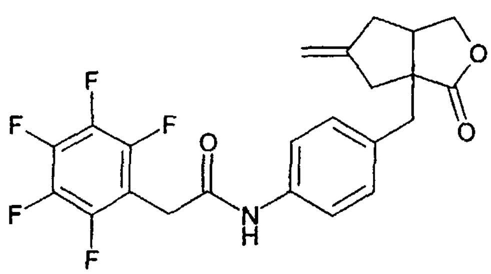 | C23H18F5NO3 | 451,39681 | 452 |
| 368 | 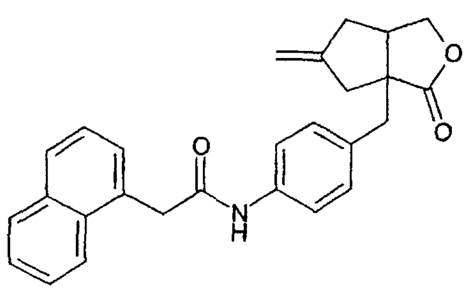 | C27H25NO3 | 411,5052 | 412 |

| Bsp.-Nr. | Struktur | Formel | Molge- wicht [g/mol] | Mz + H |
|---|---|---|---|---|
| 369 | 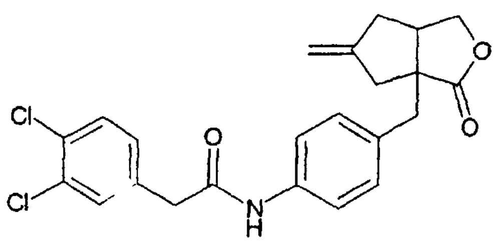 | C23H21Cl2NO3 | 430,33472 | 430 |
| 370 | 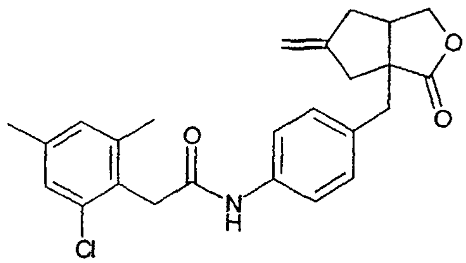 | C25H26ClNO3 | 423,94387 | 424 |
| 371 | 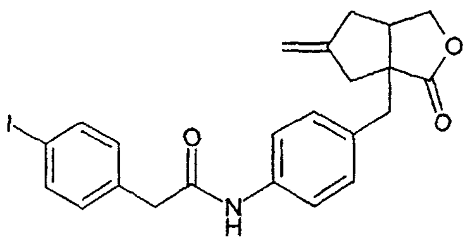 | C23H22INO3 | 487,34109 | 488 |
| 372 | 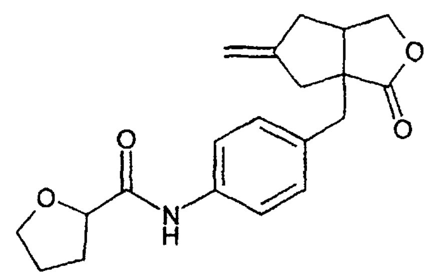 | C20H23NO4 | 341,41061 | 342 |
| 373 | 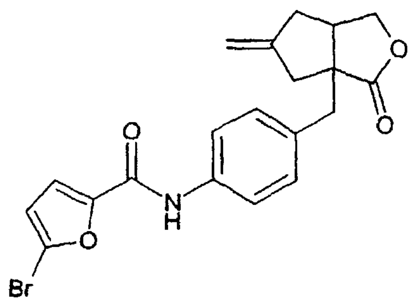 | C20H18BrNO4 | 416,27476 | 416 |

| Bsp.-Nr. | Struktur | Formel | Molge- wicht [g/mol] | Mz + H |
|---|---|---|---|---|
| 374 | 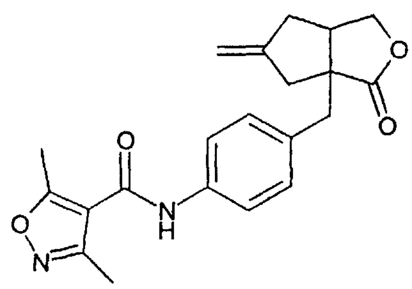 | C21H22N2O4 | 366,42049 | 367 |
| 375 | 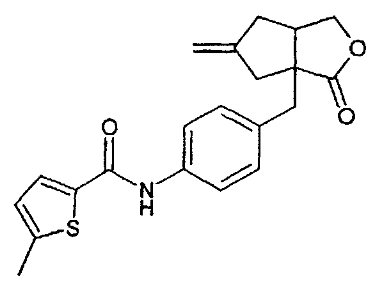 | C21H21NO3S | 367,47042 | 368 |
| 376 | 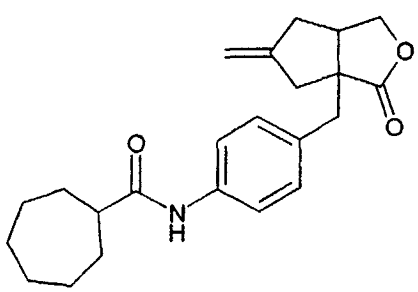 | C23H29NO3 | 367,49248 | 368 |
| 377 | 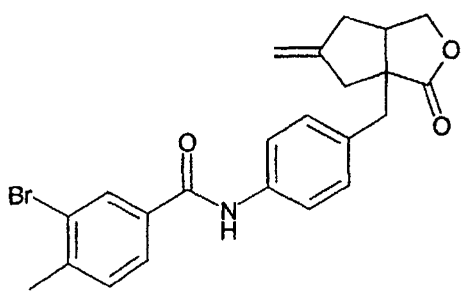 | C23H22BrNO3 | 440,34069 | 441 |

| Bsp.-Nr. | Struktur | Formel | Molge- wicht [g/mol] | Mz + H |
|---|---|---|---|---|
| 378 | 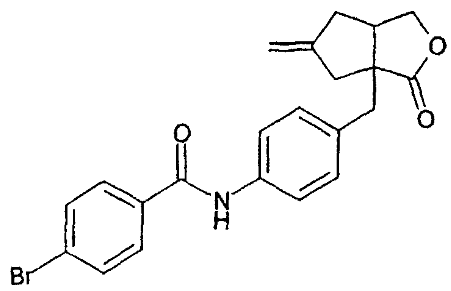 | C22H20BrNO3 | 426,3136 | 426 |
| 379 | 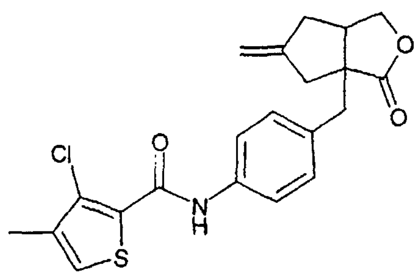 | C21H20ClNO3S | 401,91545 | 402 |
| 380 | 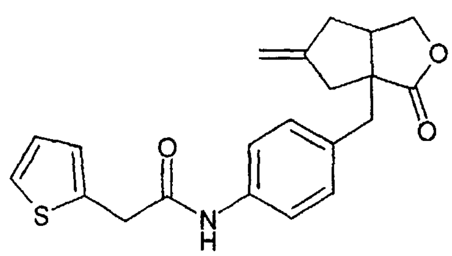 | C21H21NO3S | 367,47042 | 368 |
| 381 | 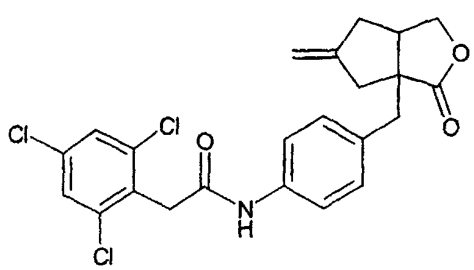 | C23H20Cl3NO3 | 464,77975 | 464 |
| 382 | 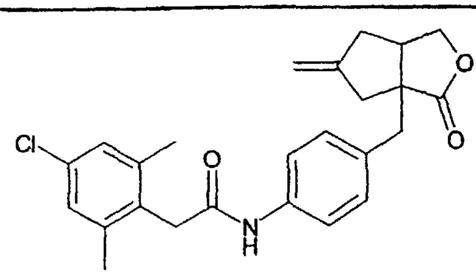 | C25H26ClNO3 | 423,94387 | 424 |

| Bsp.-Nr. | Struktur | Formel | Molge- wicht [g/mol] | Mz + H |
|---|---|---|---|---|
| 383 | 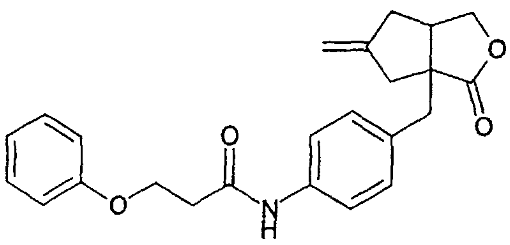 | C24H25NO4 | 391,47115 | 392 |
| 384 | 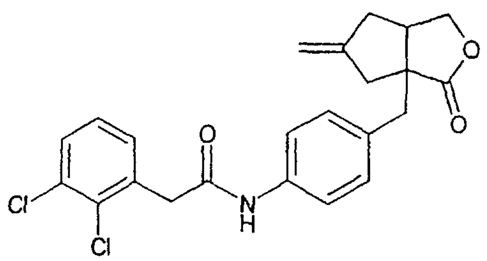 | C23H21Cl2NO3 | 430,33472 | 430 |
| 385 | 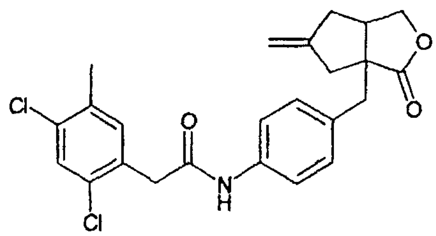 | C24H23Cl2NO3 | 444,36181 | 444 |
| 386 | 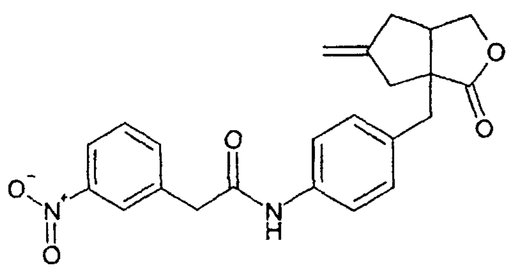 | C23H22N2O5 | 406,44219 | 407 |
| 387 | 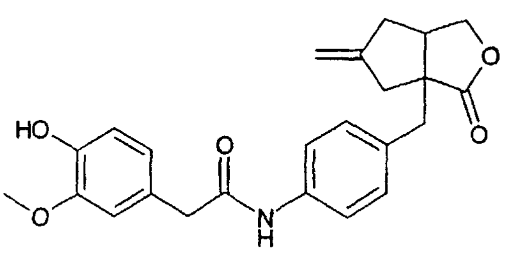 | C24H25NO5 | 407,47055 | 408 |

| Bsp.-Nr. | Struktur | Formel | Molge- wicht [g/mol] | Mz + H |
|---|---|---|---|---|
| 388 | 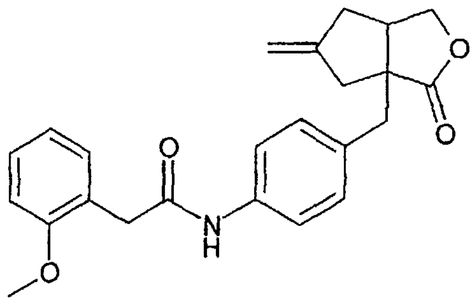 | C24H25NO4 | 391,47115 | 392 |
| 389 | 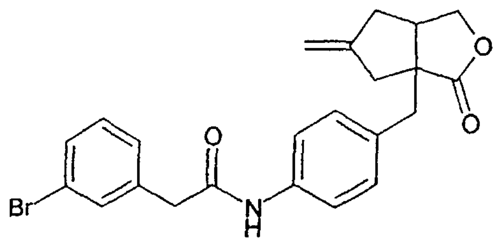 | C23H22BrNO3 | 440,34069 | 441 |
| 390 | 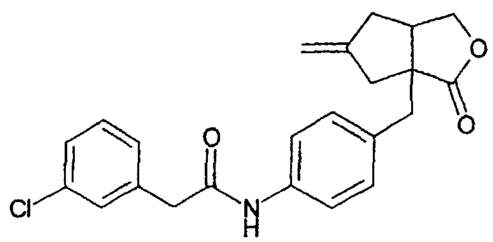 | C23H22ClNO3 | 395,88969 | 396 |
| 391 | 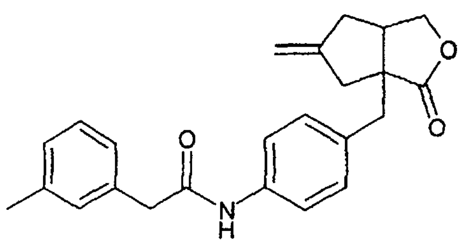 | C24H25NO3 | 375,47175 | 376 |
| 392 | 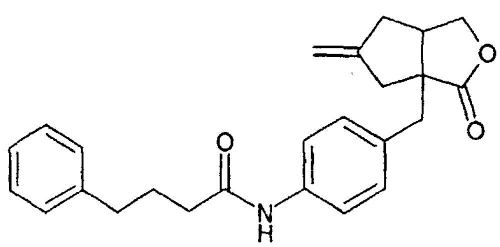 | C25H27NO3 | 389,49884 | 390 |

| Bsp.-Nr. | Struktur | Formel | Molge- wicht [g/mol] | Mz + H |
|---|---|---|---|---|
| 393 | 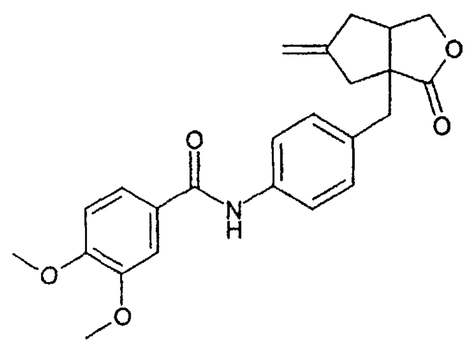 | C24H25NO5 | 407,47055 | 408 |
| 394 | 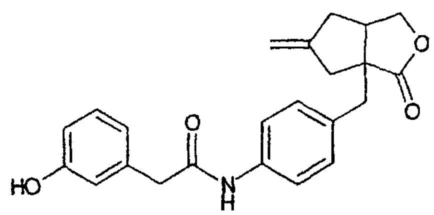 | C23H23NO4 | 377,44406 | 378 |
| 395 | 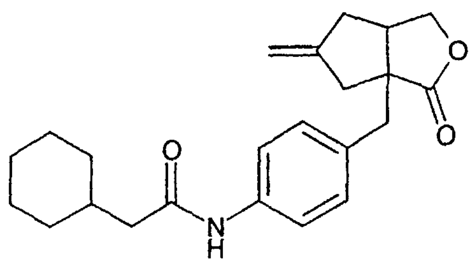 | C23H29NO3 | 367,49248 | 368 |
| 396 | 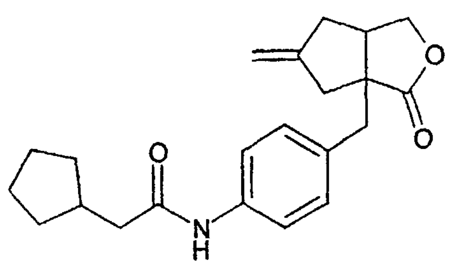 | C22H27NO3 | 353,46539 | 354 |
| 397 | 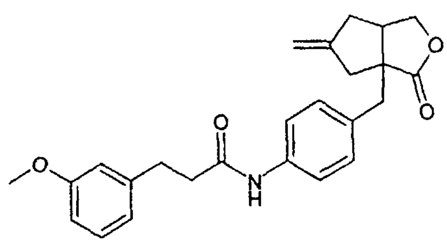 | C25H27NO4 | 405,49824 | 406 |

| Bsp.-Nr. | Struktur | Formel | Molge- wicht [g/mol] | Mz + H |
|---|---|---|---|---|
| 398 | 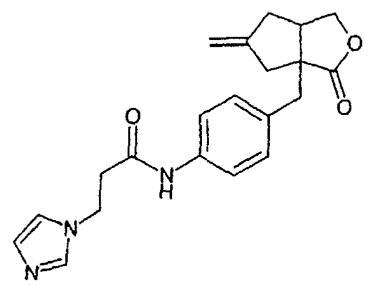 | C21H23N3O3 | 365,43576 | 366 |
| 399 | 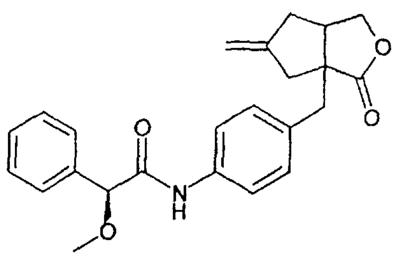 | C24H25NO4 | 391,47115 | 392 |
| 400 | 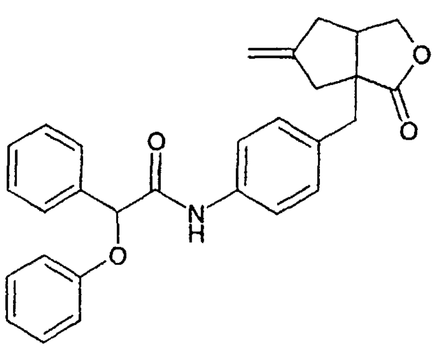 | C29H27NO4 | 453,54284 | 454 |
| 401 | 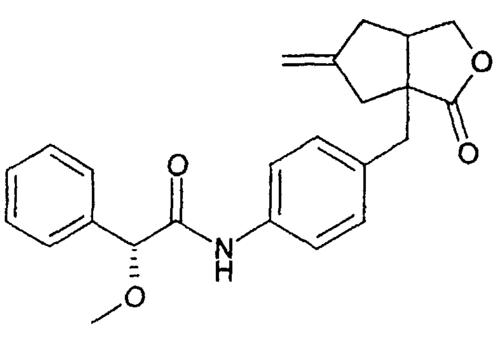 | C24H25NO4 | 391,47115 | 392 |

| Bsp.-Nr. | Struktur | Formel | Molge- wicht [g/mol] | Mz + H |
|---|---|---|---|---|
| 402 | 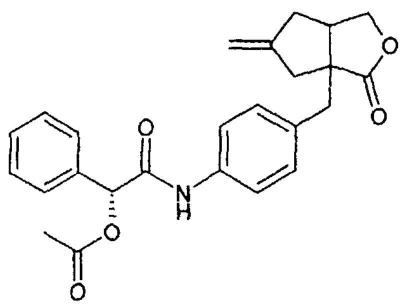 | C25H25NO5 | 419,4817 | 420 |
| 403 | 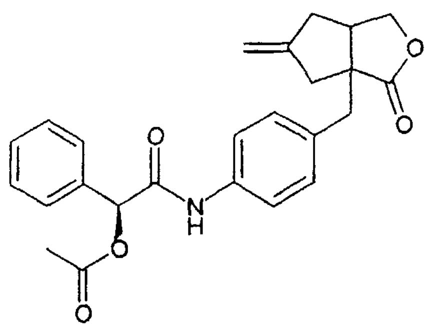 | C25H25NO5 | 419,4817 | 420 |
| 404 | 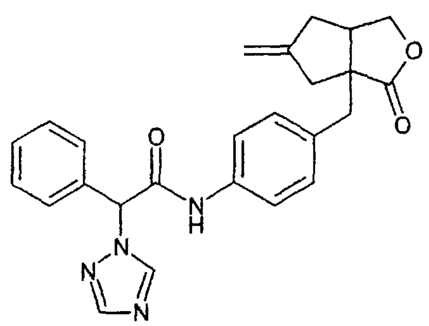 | C25H24N4O3 | 428,49503 | 429 |
| 405 | 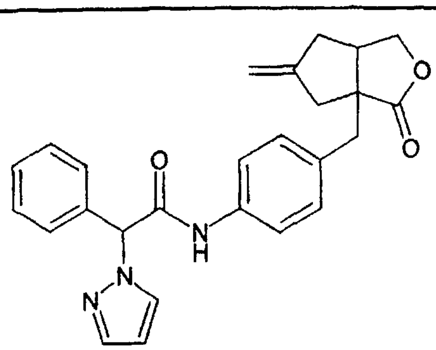 | C26H25N3O3 | 427,50745 | 428 |

| Bsp.-Nr. | Struktur | Formel | Molge- wicht [g/mol] | Mz + H |
|---|---|---|---|---|
| 406 | 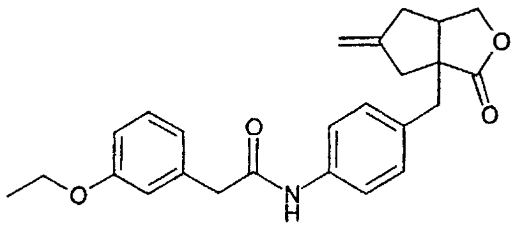 | C25H27NO4 | 405,49824 | 406 |
| 407 | 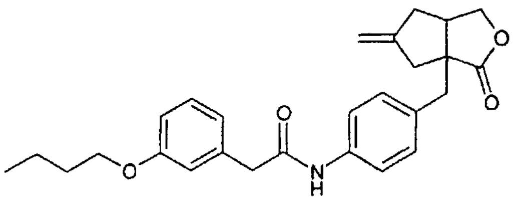 | C27H31NO4 | 433,55242 | 434 |
| 408 | 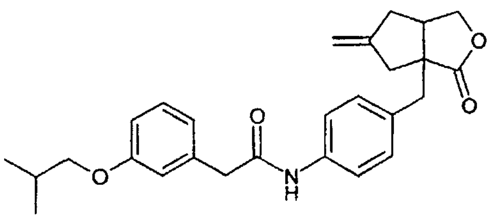 | C27H31NO4 | 433,55242 | 434 |
| 409 | 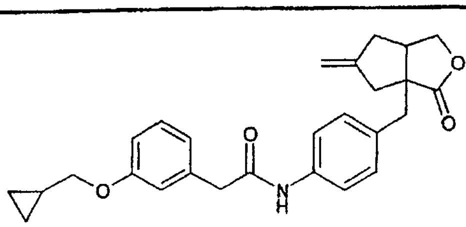 | C27H29NO4 | 431,53648 | 432 |
| 410 | 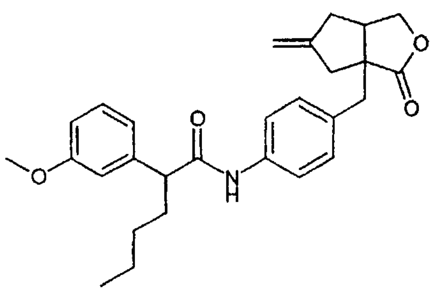 | C28H33NO4 | 447,57951 | 448 |

| Bsp.-Nr. | Struktur | Formel | Molge- wicht [g/mol] | Mz + H |
|---|---|---|---|---|
| 411 | 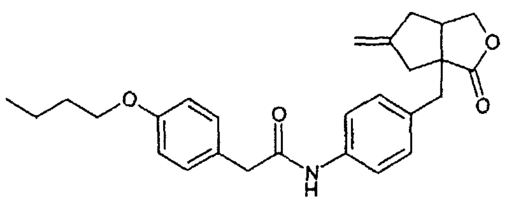 | C27H31NO4 | 433,55242 | 434 |
| 412 | 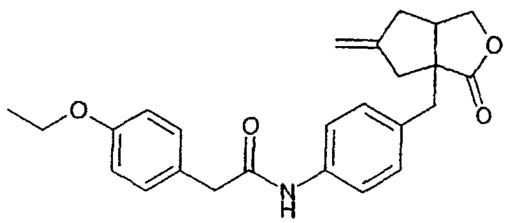 | C25H27NO4 | 405,49824 | 406 |
| 413 | 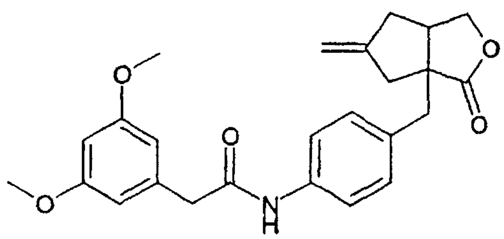 | C25H27NO5 | 421,49764 | 422 |
| 413a | 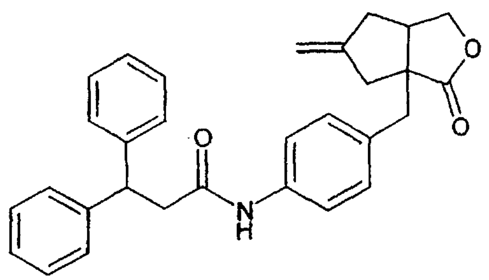 | C30H29NO3 | 451,57053 | 452 |
| 414 | 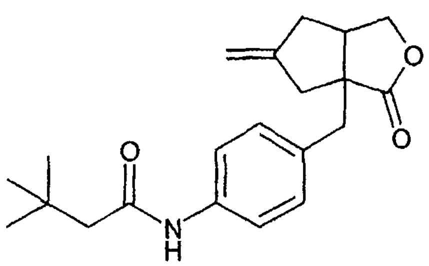 | C21H27NO3 | 341,45424 | 342 |

| Bsp.-Nr. | Struktur | Formel | Molge- wicht [g/mol] | Mz + H |
|---|---|---|---|---|
| 415 | 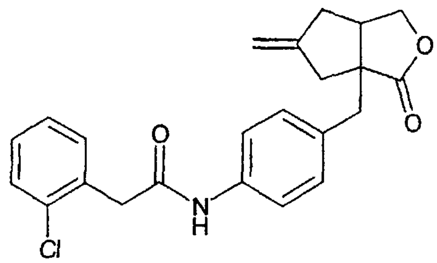 | C23H22ClNO3 | 395,8897 | 396 |
| 416 | 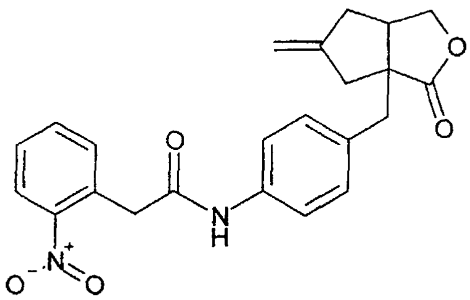 | C23H22N2O5 | 406,4422 | 407 |
| 417 | 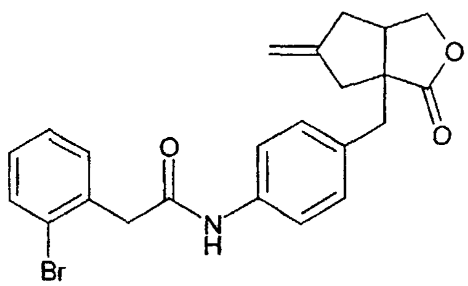 | C23H22BrNO3 | 440,3407 | 441 |
| 418 | 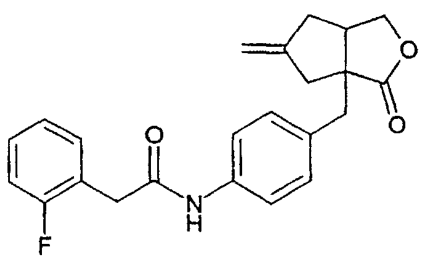 | C23H22FNO3 | 379,4352 | 380 |
| 419 | 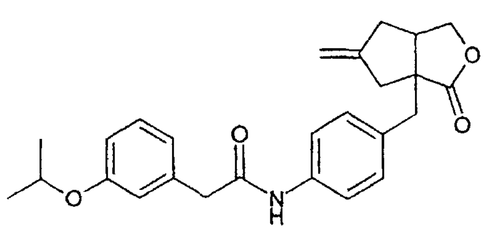 | C26 H29 N O4 | 419,5254 | 419 |

| Bsp.-Nr. | Struktur | Formel | Molge- wicht [g/mol] | Mz + H |
|---|---|---|---|---|
| 420 | 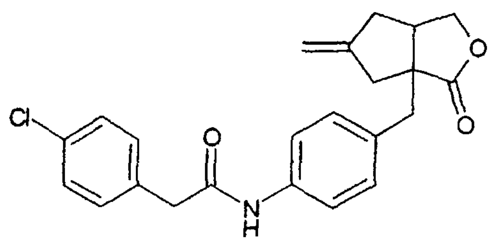 | C23H22ClNO3 | 395,8897 | 396 |
| 421 | 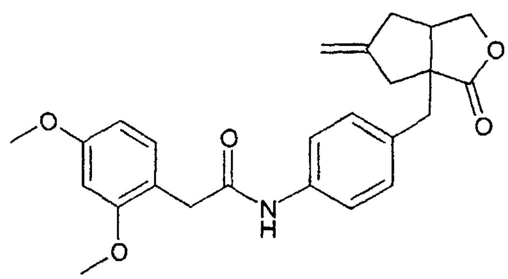 | C25 H27 N O5 | 421,4977 | 422 |
| 422 | 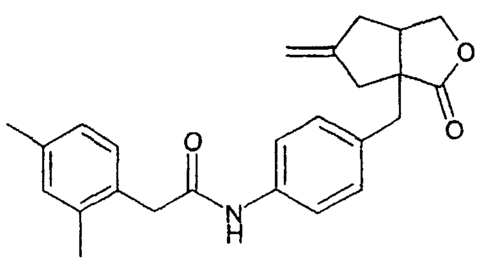 | C25 H27 N O3 | 389,4988 | 390 |
| 423 | 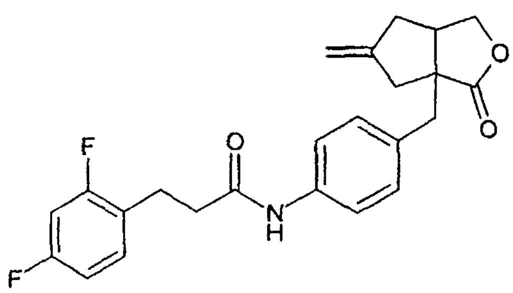 | C24H23F2NO3 | 411,4526 | 412 |

| Bsp.-Nr. | Struktur | Formel | Molge- wicht [g/mol] | Mz + H |
|---|---|---|---|---|
| 424 | 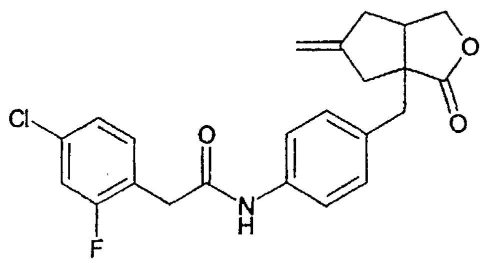 | C23H21ClFNO3 | 413,8802 | 414 |
| 425 | 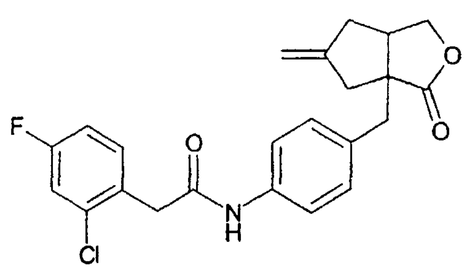 | C23H21ClFNO3 | 413,8802 | 414 |
| 426 | 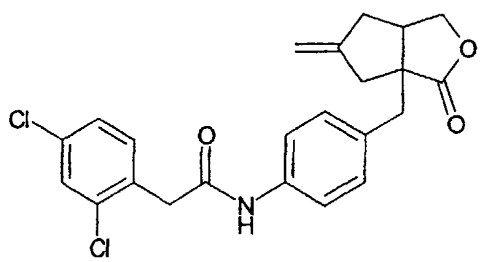 | C23H21Cl2NO3 | 430,3347 | 430 |
| 427 | 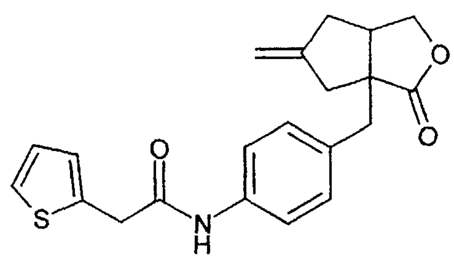 | C21H21NO3S | 367,47042 | 368 |
| 428 | 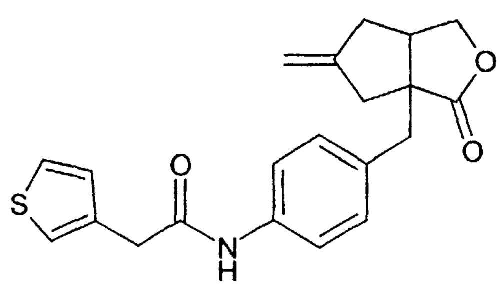 | C21 H21 N 03 S | 367,4704 | 368 |

| Bsp.-Nr. | Struktur | Formel | Molge- wicht [g/mol] | Mz + H |
|---|---|---|---|---|
| 429 | 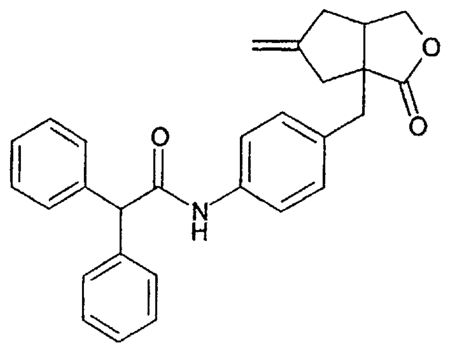 | C29 H27 N O3 | 437,5435 | 438 |
| 430 | 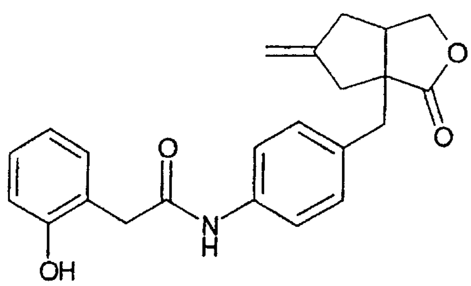 | C23 H23 N O4 | 377,4441 | 378 |
| 431 | 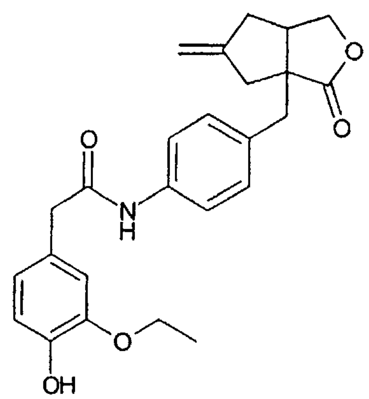 | C25 H27 N O5 | 421,4977 | 422 |

| Bsp.-Nr. | Struktur | Formel | Molge- wicht [g/mol] | Mz + H |
|---|---|---|---|---|
| 432 | 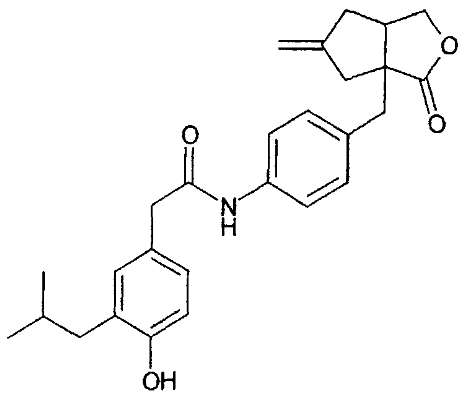 | C27H31NO4 | 433,55242 | 434 |
| 433 | 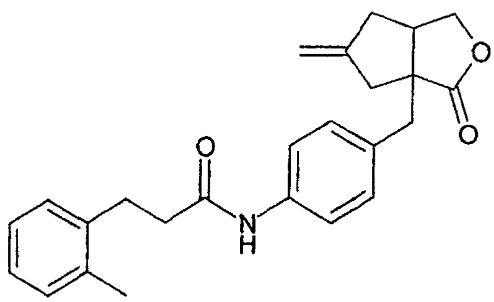 | C25 H27 N O3 | 389,4988 | 390 |
| 434 | 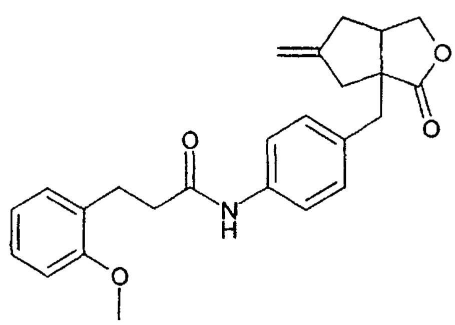 | C25 H27 N O4 | 405,4982 | 406 |
| 435 | 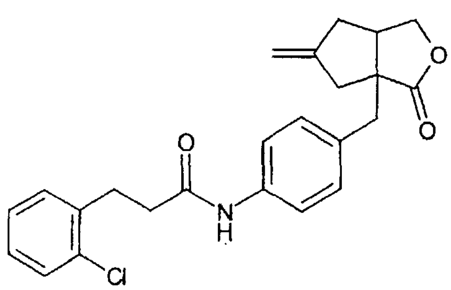 | C24H24ClNO3 | 409,9168 | 410 |

| Bsp.-Nr. | Struktur | Formel | Molge- wicht [g/mol] | Mz + H |
|---|---|---|---|---|
| 436 | 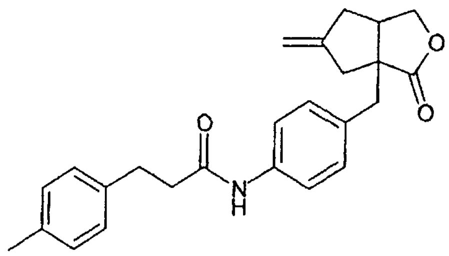 | C25H27NO3 | 389,4988 | 390 |
| 437 | 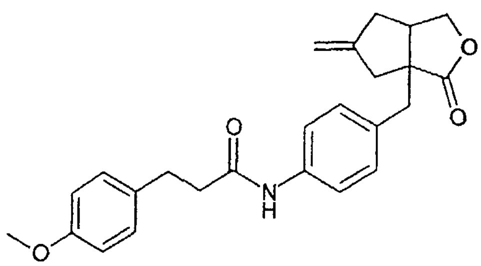 | C25 H27 N O4 | 405,4982 | 406 |
| 438 | 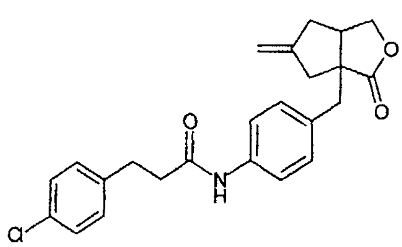 | C24H24ClNO3 | 409,9168 | 410 |
| 439 | 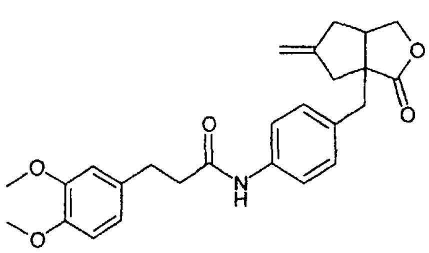 | C26 H29 N O5 | 435,5248 | 436 |
| 440 | 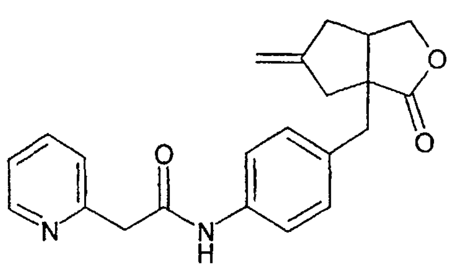 | C22 H22 N2 O3 | 362,4323 | 363 |

**Beispiel 441**

N[(3a"S*,6a"S)-4-(5-Methyliden-hexahydro-cyclopenta[c]furan-1-on6a-ylmethyl)]-phenyl-N'-(isopropyl)-harnstoff

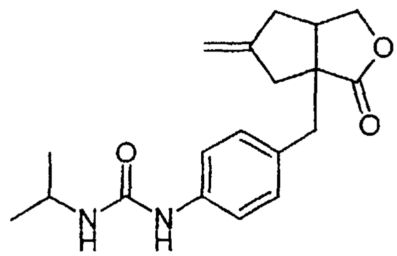

Eine Lösung der Verbindung aus Beispiel 104 (20 mg, 0,084 mmol) und Isopropylisocyanat (7,8 mg, 0,092 mmol) in Toluol (3 ml) wurde 24 Stunden bei Raumtemperatur gerührt. Zur Aufarbeitung wurden Essigester (3 ml), Dichlormethan (2 ml) und 1 M wäßrige HCl (0,6 ml) hinzugegeben, das Gemisch über eine Fritte, gefüllt mit Bitumenerde, filtriert und im Vakuum die Lösemittel abgezogen.
Ausbeute: 7,2 mg (26 %)
$R_f$(II, 2:1) = 0,18
MS (ECI) = 329 [M+H$^+$]

In Analogie zur Vorschrift des Beispiels 441 wurden die in der Tabelle aufgeführten Verbindungen dargestellt.

| Bsp.-Nr | Struktur | Formel | Molgewicht [g/mol] | Mz + H |
|---|---|---|---|---|
| 442 | 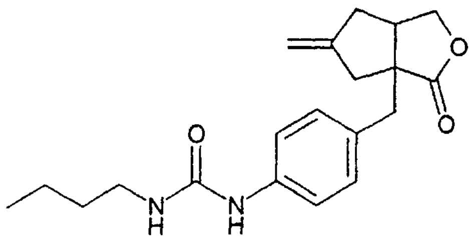 | C20 H26 N2 O3 | 342,4418 | 343 |
| 443 | 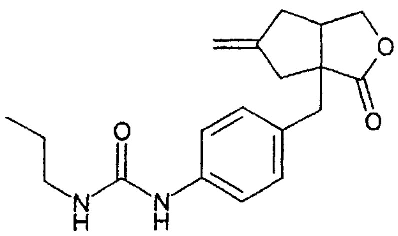 | C19 H24 N2 O3 | 328,4147 | 329 |
| 444 | 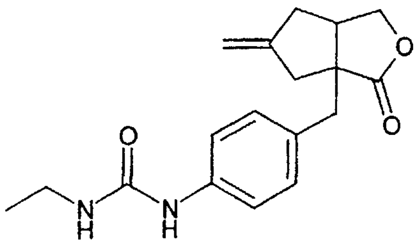 | C18 H22 N2 O3 | 314,3877 | 315 |

In Analogie zur Vorschrift des Beispiels 262 wurden die in folgender Tabelle aufgeführten Verbindungen dargestellt.

| Bsp.-Nr. | Struktur | Formel | Molgewicht [g/mol] | Mz + H |
|---|---|---|---|---|
| 445 | 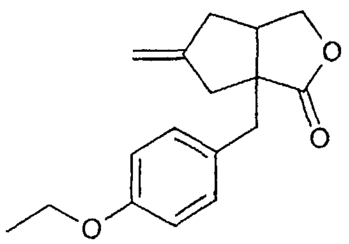 | C17 H20 O3 | 272,3472 | 273 |
| 446 | 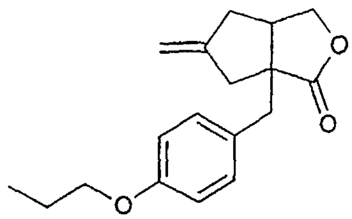 | C18 H22 O3 | 286,3743 | 287 |
| 447 | 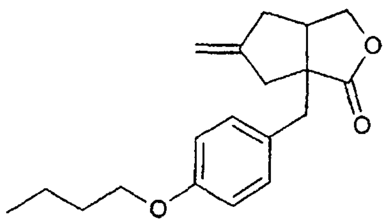 | C19 H24 O3 | 300,4013 | 301 |

In Analogie zur Vorschrift des Beispiels 262, ausgehend von Beispiel 335, wurden die in der Tabelle aufgeführten Verbindungen dargestellt.

| Bsp.-Nr. | Struktur | Formel | Molgewicht [g/mol] | Mz |
|---|---|---|---|---|
| 448 | 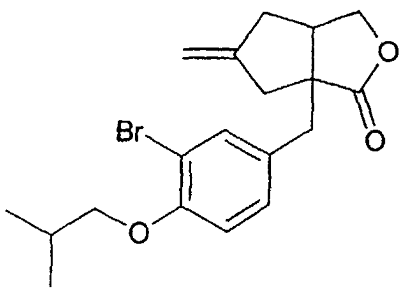 | C19 H23 Br O3 | 379,2974 | 378 |
| 449 | 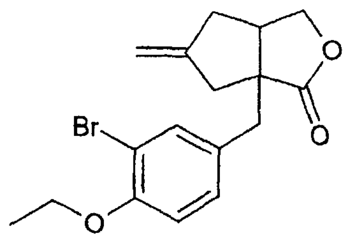 | C17H19BrO3 | 351,24318 | 350 |
| 450 | 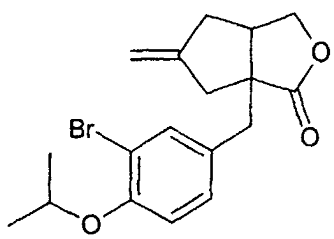 | C18H21BrO3 | 365,27027 | 364 |
| 451 | 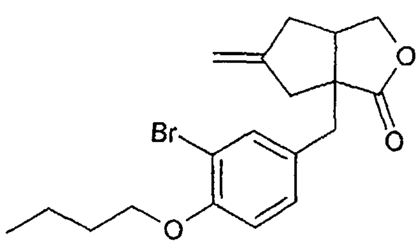 | C19H23BrO3 | 379,29736 | 378 |

In Analogie zur Vorschrift des Beispiels 262, ausgehend von Beispiel 344, wurden die in der Tabelle aufgeführten Verbindungen dargestellt.

| Bsp.-Nr. | Struktur | Formel | Molgewicht [g/mol] | Mz + H |
|---|---|---|---|---|
| 452 | 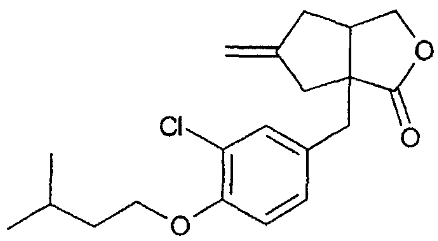 | | 348,8735 | 349,1 |
| 453 | 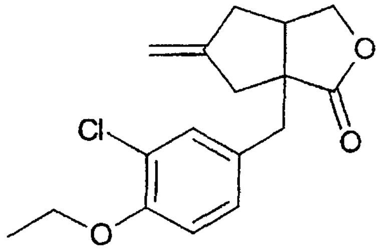 | | 306,7922 | 307,1 |
| 454 | 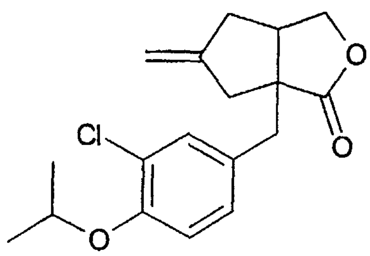 | | 320,8193 | 321,1 |
| 455 | 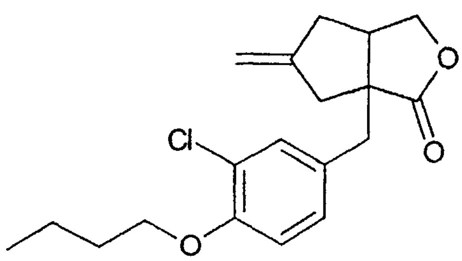 | | 334,8464 | 335,1 |

In Analogie zur Vorschrift des Beispiels 262, ausgehend von Beispiel 329, wurden die in der Tabelle aufgeführten Verbindungen dargestellt.

| Bsp.-Nr. | Struktur | Formel | Molgewicht [g/mol] | Mz + H |
|---|---|---|---|---|
| 456 | 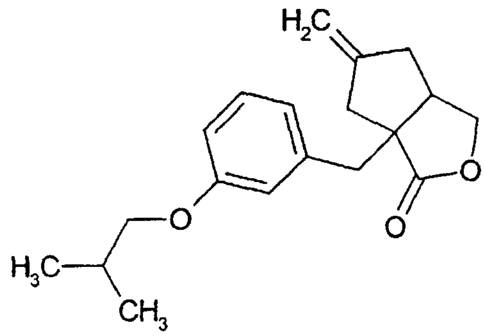 | C19H24O3 | 300,401 | 301 |
| 457 | 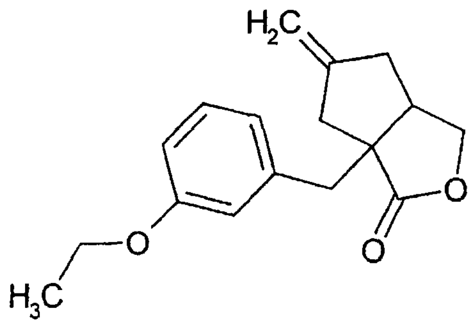 | C17H20O3 | 272,347 | 273 |
| 458 | 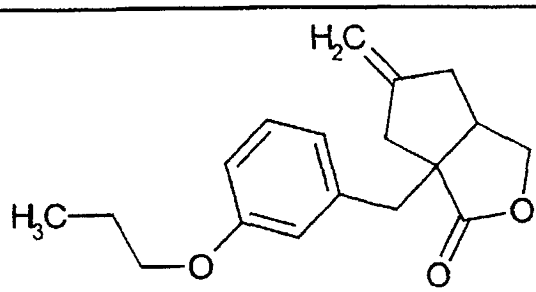 | C18H22O3 | 286,374 | 287 |

| Bsp.-Nr. | Struktur | Formel | Molgewicht [g/mol] | Mz + H |
|---|---|---|---|---|
| 459 | 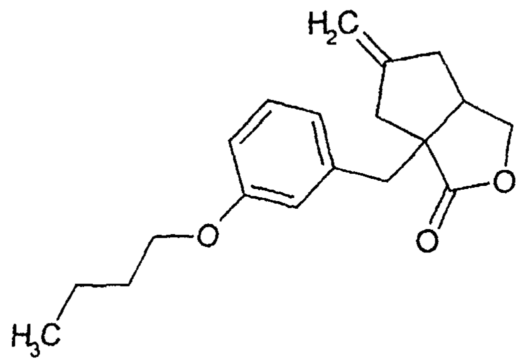 | C19H24O3 | 300,401 | 301 |

Analog der Vorschriften des Beispiels 260 wurden die in der Tabelle aufgeführten Verbindungen dargestellt.

| Bsp.-Nr. | Struktur | Edukt Bsp.-Nr. | Ausbeute [%] | Rf (Cyclohexan - Ethylacetat 3:1) | MS (CI) [$M^+ + 1$] |
|---|---|---|---|---|---|
| 460 | 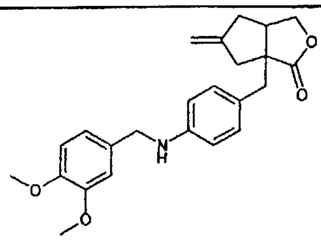 | 104 | | 0.16 | 394 |
| 461 | 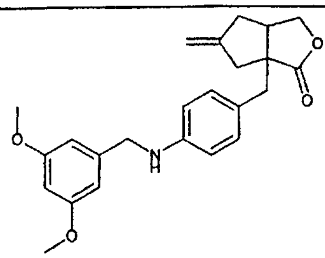 | 104 | | 0.29 | 394 |
| 462 | 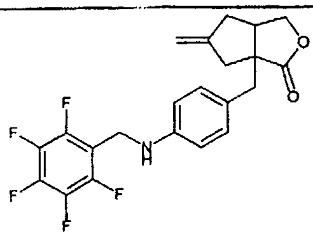 | 104 | | 0.13 | 424 |
| 463 | 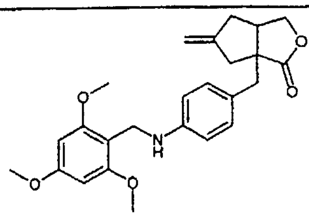 | 104 | | 0.24 | 424 |

**Beispiel 464**

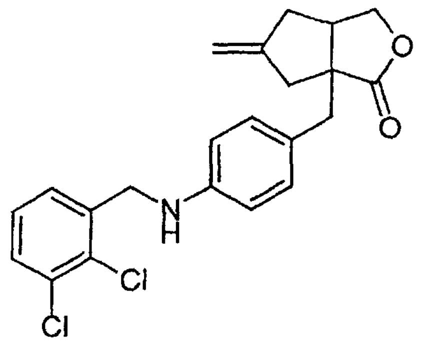

Eine Lösung von 60 µl 3 M Schwefelsäure und 40 mg (0,23 mmol) 2,3-Dichlorbenzaldehyd in 400 µl THF wird bei 0°C langsam in einen offenen Kolben mit 49 mg (0,2 mmol) der Verbindung aus Beispiel 104, gelöst in 1 ml THF und 350µl Methanol, gegeben. Nach 5 Minuten werden 14 mg Natriumborhydrid bei 0°C zu der gut gerührten Lösung gegeben. Die Mischung wird noch 10 Minuten bei Raumtemperatur gerührt.

Zur Aufarbeitung wird der Ansatz mit 400 µl Wasser verdünnt, mit festem NaOH unter Eiskühlung alkalisiert und sofort mit MTB-Ether extrahiert. Die vereinigten Etherphasen werden mit gesättigter Kochsalzlösung gewaschen, über Magnesiumsulfat getrocknet, filtriert und eingedampft. Die Reinigung erfolgt säulenchromatografisch (Fließmittel: Cyclohexan:Ethylacetat 3:1).
Ausbeute: 2,4 mg (3,0 %)
$R_f$: 0,40 (Cyclohexan:Ethylacetat 3:1)
MS (EI): m/z = 401 [$M^+$]

| Bsp-Nr. | Struktur | Ausbeute (%) | Edukt | Vorschrift analog Beispiel | Rf (Cyclohexan: Ethylacetat 3:1) | MS (EI: $M^+$; CI: $M+H^+$) |
|---|---|---|---|---|---|---|
| 465 | 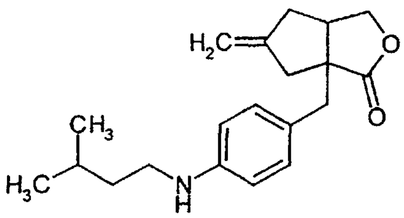 | 40,7 | 104 | 464 | 0,52 | 313 (EI) |
| 466 | 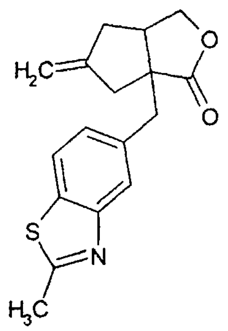 | 3,5 | 1A | 1 | 0,19 | 299 (EI) |
| 467 | 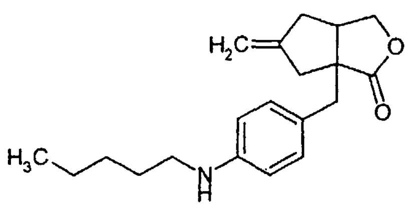 | 16 | 104 | 464 | 0,5 | 313 (EI) |
| 468 | 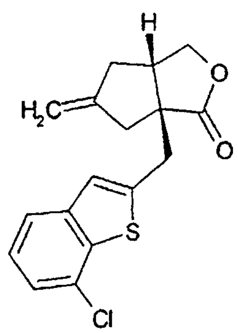 | 8,1 | 1A | 1 | 0,52 | 318 (EI) |
| 469 | 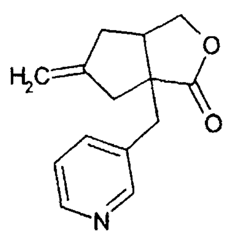 | 3,1 | 1A | 1 | 0,14 (1:1) | 229 (EI) |

| Bsp-Nr. | Struktur | Ausbeute (%) | Edukt | Vorschrift analog Beispiel | Rf (Cyclohexan: Ethylacetat 3:1) | MS (EI: $M^+$; CI: $M+H^+$) |
|---|---|---|---|---|---|---|
| 470 | 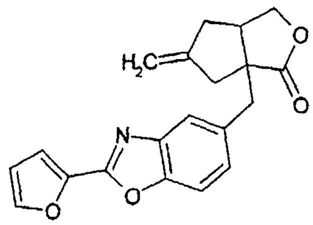 | 35,6 | 1A | 1 | 0,3 | - |
| 471 | 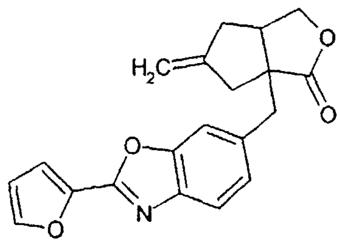 | 21,5 | 1A | 1 | 0,31 | 353 (CI) [M + NH4+] |
| 472 | 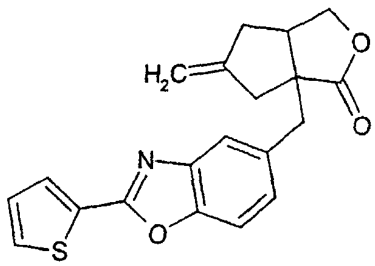 | 3,1 | 1A | 1 | 0,21 | 352 (CI) |
| 473 | 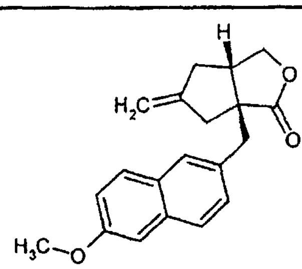 | 24,1 | 2A | 1 | 0,38 | 309 (CI) |
| 474 | 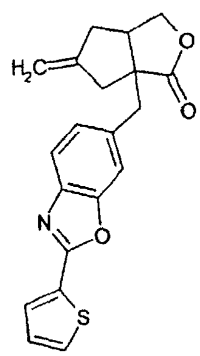 | 18,4 | 1A | 1 | 0,31 | 351 (EI) |

| Bsp. Nr. | Struktur | Ausbeute (%) | Edukt | Vorschrift analog Beispiel | Rf (Cyclohexan:Ethylacetat 3:1) | MS (EI: $M^+$; CI: $M+H^+$) |
|---|---|---|---|---|---|---|
| 475 | 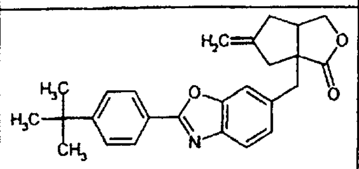 | 14,7 | 1A | 1 | 0,4 | 402 (CI) |
| 476 | 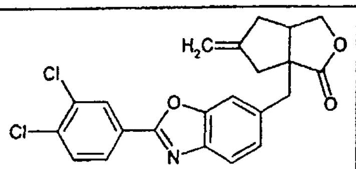 | 0,8 | 1A | 1 | 0,43 | - |

In Analogie zur Vorschrift des Beispiels 127 wurden die in der folgenden Tabelle aufgeführten Beispiele dargestellt:

| Bsp.-Nr. | Struktur | Formel | Molgewicht (g/mol) | Mz+H |
|---|---|---|---|---|
| 477 | 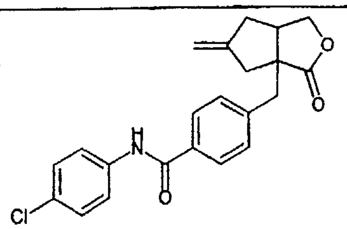 | C22 H20 Cl N O3 | 381,8626 | 382 |
| 478 | 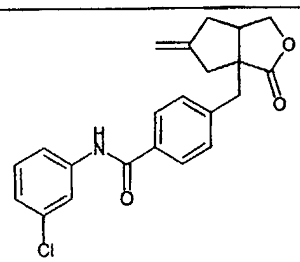 | C22 H20 Cl N O3 | 381.8626 | 382 |

## Patentansprüche

1. Verbindungen der allgemeinen Formel (I)

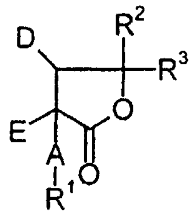

(I)

in welcher

A für Reste der Formeln $-CH_2-$, -CO-, $-CR^4(OH)-$ oder $-(CH_2)_a-CHR^5$steht, worin

a eine Zahl 0, 1, 2, 3 oder 4 bedeutet,

$R^4$ Wasserstoff oder $(C_1-C_6)$-Alkyl bedeutet

und

$R^5$ Phenyl bedeutet,

oder
für $(C_2-C_8)$-Alkandiyl, $(C_2-C_6)$-Alkendiyl oder $(C_2-C_6)$-Alkindiyl steht,

$R^1$ für Wasserstoff, $(C_3-C_6)$-Cycloalkyl oder
für einen 5- bis 6-gliedrigen Heterocyclus steht, der bis zu 3 Heteroatome aus der Reihe S, O, N und/oder einen Rest der Formel $-NR^6$ enthalten kann,
worin

$R^6$ Wasserstoff oder Methyl bedeutet,

oder
für einen 5- bis 6-gliedrigen, benzokondensierten Heterocyclus steht, der bis zu 2 Heteroatome aus der Reihe S, O, N und/oder einen Rest der Formel $-NR^7$ enthalten kann, und der sowohl über den Phenylring als auch über den Heterocyclus gebunden sein kann,
worin

$R^7$ die oben angegebene Bedeutung von $R^6$ hat und mit dieser gleich oder verschieden ist,

oder
für Reste der Formeln

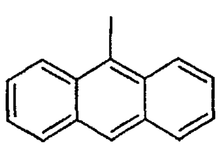

,

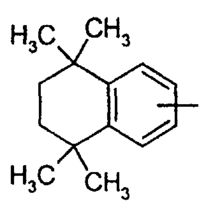

,

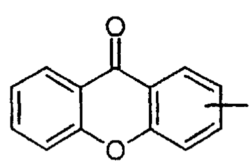

,

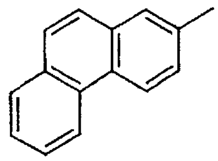 , 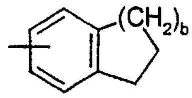 oder 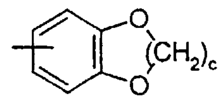

steht,

worin

b und c gleich oder verschieden sind und eine Zahl 1 oder 2 bedeuten,

oder

für $(C_6-C_{10})$-Aryl steht,

wobei alle oben aufgeführten Ringsysteme gegebenenfalls ein- bis mehrfach, gleich oder verschieden durch Substituenten, ausgewählt aus der Gruppe Halogen, Cyano, Nitro, Trifluormethyl, Hydroxy, $(C_1-C_6)$-Alkoxy, $(C_1-C_6)$-Alkyl-carbonyl und $(C_3-C_6)$-Cycloalkyl, Phenyl, Phenoxy, Benzyloxy und einen 5- bis 6-gliedrigen aromatischen Heterocyclus mit bis zu 3 Heteroatomen aus der Reihe S, N und/oder O substituiert sind, die ihrerseits bis zu dreifach gleich oder verschieden durch Cyano oder Halogen substituiert sein können,

und/oder durch $(C_1-C_6)$-Alkyl und $(C_2-C_6)$-Alkylen substituiert sind, die ihrerseits durch Halogen, $(C_6-C_{10})$-Aryl oder durch Reste der Formel $-SR^8$, $-OR^9$ oder $-NR^{10}R^{11}$ oder

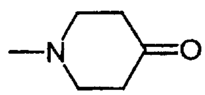

substituiert sein können,

worin

$R^8$ $(C_1-C_6)$-Alkyl oder Phenyl bedeutet,

$R^9$ Wasserstoff oder $(C_1-C_6)$-Alkyl bedeutet,

und

$R^{10}$ und $R^{11}$ gleich oder verschieden sind und Wasserstoff, Phenyl oder $(C_1-C_6)$-Alkyl bedeuten, das gegebenenfalls durch Phenyl substituiert ist, das seinerseits ein- bis mehrfach, gleich oder verschieden durch Halogen, Nitro, Hydroxy oder $(C_1-C_6)$-Alkoxy substituiert sein kann,

oder

$R^{10}$ und $R^{11}$ gemeinsam mit dem Stickstoffatom einen Rest der Formel

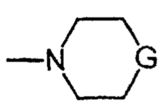

bilden,

worin

G ein Sauerstoffatom, eine $-CH_2$-Gruppe oder einen Rest der Formel $-NR^{12}$- bedeutet,

worin

$R^{12}$ Wasserstoff, Phenyl, Benzyl, $(C_1-C_6)$-Alkyl, $(C_1-C_6)$-Alkoxy-carbonyl oder einen 5- bis 6-gliedrigen aromatischen Heterocyclus mit bis zu 3 Heteroatomen aus der Reihe S, N und/oder O bedeutet,

und/oder durch Gruppen der Formeln $-CO_2-R^{13}$, $-NR^{14}R^{15}$, $-NR^{16}CO-R^{17}$, $-NR^{18}CO_2-R^{19}$ und $-CO-NR^{20}R^{21}$ sub-

stituiert sind,
worin

$R^{13}$ Wasserstoff bedeutet, oder
$(C_1\text{-}C_9)$-Alkyl oder $(C_2\text{-}C_6)$-Alkenyl bedeutet, die ihrerseits durch Reste der Formeln

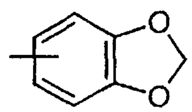 , 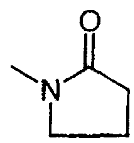 ,

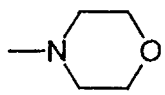 , 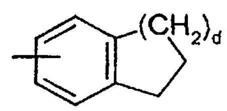

oder
$(C_6\text{-}C_{10})$-Aryl oder durch einen 5- bis 7-gliedrigen aromatischen Heterocyclus mit bis zu 3 Heteroatomen aus der Reihe S, N und/oder O substituiert sein können,
worin

d eine Zahl 1 oder 2 bedeutet,

oder
$(C_6\text{-}C_{10})$-Aryl bedeutet, das gegebenenfalls durch Phenyl substituiert ist, das seinerseits durch Cyano oder Halogen substituiert sein kann,

$R^{14}$ und $R^{15}$ gleich oder verschieden sind und Wasserstoff, $(C_3\text{-}C_6)$-Cycloalkyl, Phenyl oder $(C_1\text{-}C_6)$-Alkyl bedeuten, das gegebenenfalls durch $(C_3\text{-}C_6)$-Cycloalkyl oder Phenyl substituiert ist, das seinerseits ein- bis mehrfach, gleich oder verschieden durch Halogen, Hydroxy oder $(C_1\text{-}C_6)$-Alkoxy substituiert sein kann,

$R^{16}$ Wasserstoff oder $(C_1\text{-}C_6)$-Alkyl bedeutet,

$R^{17}$ Wasserstoff, Adamantyl, $(C_3\text{-}C_8)$-Cycloalkyl, $(C_2\text{-}C_6)$-Alkenyl oder $(C_1\text{-}C_{12})$-Alkyl bedeutet, das gegebenenfalls
durch Adamantyl, $(C_3\text{-}C_6)$-Cycloalkyl, $(C_6\text{-}C_{10})$-Aryl, Phenoxy oder einen 5- bis 6-gliedrigen aromatischen Heterocyclus mit bis zu 4 Heteroatomen aus der Reihe S, N und/oder O substituiert ist, wobei Aryl und der Heterocyclus ihrerseits ein- bis mehrfach, gleich oder verschieden durch $(C_1\text{-}C_6)$-Alkyl, $(C_1\text{-}C_6)$-Alkoxy, Hydroxy, Nitro oder Halogen substituiert sein können,
und/oder Alkyl gegebenenfalls durch einen Rest der Formel

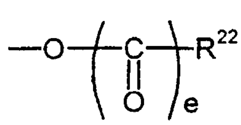

substituiert ist,
worin

e eine Zahl 0 oder 1 bedeutet und
$R^{22}$ $(C_1\text{-}C_6)$-Alkyl oder $(C_6\text{-}C_{10})$-Aryl bedeutet, das gegebenenfalls ein- bis mehrfach,

gleich oder verschieden durch Halogen, Nitro, Hydroxy oder ($C_1$-$C_6$)-Alkoxy substituiert ist,

oder

($C_6$-$C_{10}$)-Aryl oder einen 5- bis 6-gliedrigen aromatischen Heterocyclus mit bis zu 3 Heteroatomen aus der Reihe S, N und/oder O bedeutet, die ihrerseits gegebenenfalls ein- bis mehrfach, gleich oder verschieden durch ($C_1$-$C_6$)-Alkoxy, ($C_1$-$C_6$)-Alkyl, Hydroxy, Nitro oder Halogen substituiert sein können,

oder

einen Rest der Formel

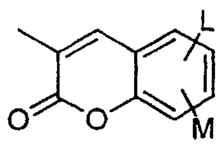

oder -$NR^{23}R^{24}$ bedeutet,

worin

L und M gleich oder verschieden sind und Wasserstoff oder Halogen bedeuten,

$R^{23}$ und $R^{24}$ die oben angegebene Bedeutung von $R^{10}$ und $R^{11}$ haben und mit dieser gleich oder verschieden sind,

$R^{18}$ die oben angegebene Bedeutung von $R^{16}$ hat und mit dieser gleich oder verschieden ist,

$R^{19}$ ($C_3$-$C_8$)-Cycloalkyl bedeutet, oder ($C_1$-$C_8$)-Alkyl oder ($C_2$-$C_8$)-Alkenyl bedeutet, die ihrerseits gegebenenfalls durch Substituenten, ausgewählt aus der Gruppe Halogen, Phenyl, Hydroxy, Morpholinyl, ($C_3$-$C_8$)-Cycloalkyl und durch eine Gruppe der Formel -$SiR^{25}R^{26}R^{27}$ substituiert sind,

worin

$R^{25}$, $R^{26}$ und $R^{27}$ gleich oder verschieden sind und ($C_1$-$C_6$)-Alkyl bedeuten,

$R^{20}$ und $R^{21}$ gleich oder verschieden sind und Wasserstoff, Adamantyl, ($C_3$-$C_8$)-Cycloalkyl, Phenyl, Phenoxy-substituiertes Phenyl oder einen 5- bis 6-gliedrigen, aromatischen Heterocyclus mit bis zu 3 Heteroatomen aus der Reihe S, N und/oder O bedeuten, oder ($C_2$-$C_8$)-Alkenyl, ($C_1$-$C_{12}$)-Alkyl oder ($C_2$-$C_6$)-Alkinyl bedeuten, die gegebenenfalls durch Hydroxy, ($C_3$-$C_6$)-Cycloalkyl, ($C_1$-$C_6$)-Alkoxy, Halogen, Hydroxy, Trifluormethyl, Phenyl oder durch einen 5- bis 6-gliedrigen aromatischen Heterocyclus mit bis zu 3 Heteroatomen aus der Reihe S, N und/oder O substituiert sind, wobei die Ringsysteme gegebenenfalls bis zu 2-fach gleich oder verschieden durch ($C_1$-$C_6$)-Alkoxy, ($C_1$-$C_6$)-Alkoxycarbonyl, Halogen, Phenoxy, Hydroxy oder (C,-$C_6$)-Alkyl substiutiert sind,

und/oder das unter $R^{20}$/$R^{21}$ aufgeführte Alkyl gegebenenfalls durch Reste der Formeln

-$SCF_3$

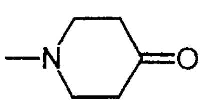 , 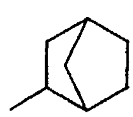 ,

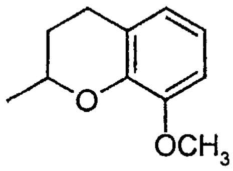

oder $-NR^{28}R^{29}$ substituiert ist,
worin

$R^{28}$ und $R^{29}$ gleich oder verschieden sind und Wasserstoff oder $(C_1-C_6)$-Alkyl bedeuten,

oder
einen Rest der Formel

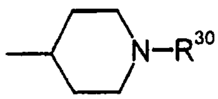

bedeutet,
worin

$R^{30}$ die oben angegebene Bedeutung von $R^{12}$ hat und mit dieser gleich oder verschieden ist,

oder

$R^{20}$ und $R^{21}$ gemeinsam mit dem Stickstoffatom einen Rest der Formel

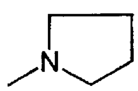 oder 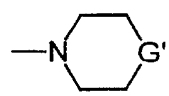

bilden,
worin

G' die oben angegebene Bedeutung von G hat und mit dieser gleich oder verschieden ist,

$R^2$ und $R^3$ gleich oder verschieden sind und für Wasserstoff oder $(C_1-C_6)$-Alkyl stehen,

und
D und E gemeinsam Reste der Formeln

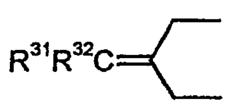 , 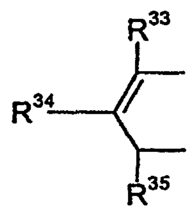

oder

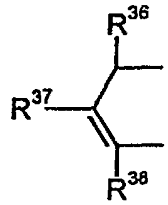

bilden,
worin
$R^{31}$, $R^{32}$, $R^{33}$, $R^{34}$, $R^{35}$, $R^{36}$, $R^{37}$ und $R^{38}$ gleich oder verschieden sind und Wasserstoff, Phenyl oder $(C_1-C_6)$-Alkyl bedeuten,
und deren pharmazeutisch verträgliche Salze,
mit der Ausnahme von (3aS*, 6aR*)-6a-Methyl-3,3a,6,6a-tetrahydro-1H-cyclopenta*[c]*furan-1-on.

**2.** Verbindungen der Formel (I) nach Anspruch 1, worin

A für Reste der Formeln $-CH_2-$, -CO-, $-CR^4(OH)-$ oder $-(CH_2)_a-CHR^5$steht,
worin

a eine Zahl 0, 1, 2 oder 3 bedeutet,

$R^4$ Wasserstoff oder $(C_1-C_4)$-Alkyl bedeutet

und

$R^5$ Phenyl bedeutet,

oder
für $(C_2-C_6)$-Alkandiyl, $(C_2-C_4)$-Alkendiyl oder $(C_2-C_4)$-Alkindiyl steht,

$R^1$ für Wasserstoff, Cyclopropyl, Cyclopentyl oder Cyclohexyl steht, oder
für Benzofuranyl, Benzothiophenyl, Benzimidazolyl, Thienyl, Furyl, Chinazolyl, Chinoxalinyl oder Chinolyl steht,

oder
für Reste der Formeln

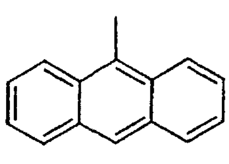 , 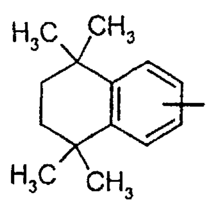 , 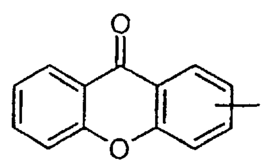 ,

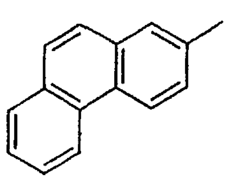 , 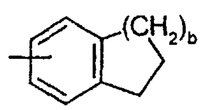 oder 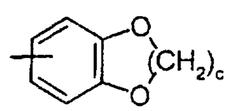

steht,

worin
b und c gleich oder verschieden sind und eine Zahl 1 oder 2 bedeuten,
oder
für Phenyl oder Naphthyl steht,
wobei alle oben aufgeführten Ringsysteme gegebenenfalls ein- bis mehrfach, gleich oder verschieden durch Substituenten, ausgewählt aus der Gruppe Fluor, Chlor, Brom, Jod, Cyano, Nitro, Trifluormethyl, Hydroxy oder $(C_1-C_5)$-Alkoxy, $(C_1-C_5)$-Alkyl-carbonyloxy, Cyclopropyl, Cyclopentyl, Cyclohexyl, Phenyl, Phenoxy, Pyridyl, Pyrimidyl, Pyridazinyl, Thienyl, Furyl oder Benzyloxy substituiert sind, die ihrerseits bis zu dreifach gleich oder verschieden durch Cyano, Fluor, Chlor, Brom oder Jod substituiert sein können,
und/oder durch $(C_1-C_5)$-Alkyl und $(C_2-C_4)$-Alkenyl substituiert sind, die ihrerseits durch Fluor, Chlor, Brom, Jod, Phenyl, Naphthyl oder durch Reste der Formel $-SR^8$, $-OR^9$ oder $-NR^{10}R^{11}$ oder

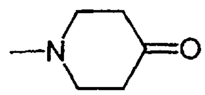

substituiert sein können,
worin

$R^8$ $(C_1-C_4)$-Alkyl oder Phenyl bedeutet,

$R^9$ Wasserstoff oder $(C_1-C_4)$-Alkyl bedeutet,

und

$R^{10}$ und $R^{11}$ gleich oder verschieden sind und Wasserstoff, Phenyl oder $(C_1-C_4)$-Alkyl bedeuten, das gegebenenfalls durch Phenyl substituiert ist, das seinerseits ein- bis mehrfach, gleich oder verschieden durch Fluor, Chlor, Brom, Nitro, Hydroxy oder $(C_1-C_4)$-Alkoxy substituiert sein kann,

oder

$R^{10}$ und $R^{11}$ gemeinsam mit dem Stickstoffatom einen Rest der Formel

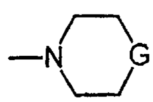

bilden,
worin

G ein Sauerstoffatom, eine $-CH_2$-Gruppe oder einen Rest der Formel $-NR^{12}-$ bedeutet,
worin

$R^{12}$ Wasserstoff, Phenyl, Benzyl, $(C_1-C_4)$-Alkyl, $(C_1-C_4)$-Alkoxycarbonyl, Pyridyl, Pyrimidyl, Pyridazinyl oder Furyl bedeutet,

und/oder durch Gruppen der Formeln $-CO_2-R^{13}$, $-NR^{14}R^{15}$, $-NR^{16}CO-R^{17}$, $-NR^{18}CO_2-R^{19}$ und $-CO-NR^{20}R^{21}$ substituiert sind,
worin

$R^{13}$ Wasserstoff bedeutet, oder
$(C_1-C_8)$-Alkyl oder $(C_2-C_5)$-Alkenyl bedeutet, die ihrerseits durch Reste der Formeln

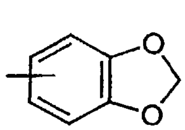 , 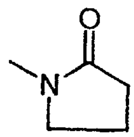 ,

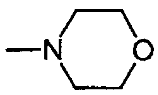 , 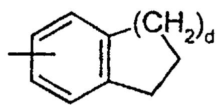

oder
Phenyl, Naphthyl, Pyridyl, Thienyl oder Furyl substituiert sein können,
worin

d eine Zahl 1 oder 2 bedeutet,

oder
Phenyl oder Naphthyl bedeutet, die gegebenenfalls durch Phenyl substituiert sind, das seinerseits durch Cyano, Fluor, Chlor oder Brom substituiert sein kann,

$R^{14}$ und $R^{15}$ gleich oder verschieden sind und Wasserstoff, Cyclopropyl, Cyclopentyl, Cyclohexyl, Phenyl oder ($C_1$-$C_5$)-Alkyl bedeuten, das gegebenenfalls durch Cyclopropyl, Cyclopentyl, Cyclohexyl oder Phenyl substituiert ist, das seinerseits ein- bis mehrfach, gleich oder verschieden durch Fluor, Chlor, Brom, Hydroxy oder ($C_1$-$C_4$)-Alkoxy substituiert sein kann,

$R^{16}$ Wasserstoff oder ($C_1$-$C_3$)-Alkyl bedeutet,

$R^{17}$ Wasserstoff, Adamantyl, Cyclopropyl, Cyclopentyl oder Cyclohexyl bedeutet, oder ($C_2$-$C_4$)-Alkenyl oder ($C_1$-$C_{10}$)-Alkyl bedeutet, das gegebenenfalls durch Adamantyl, Cyclopropyl, Cyclopentyl, Cyclohexyl, Phenyl, Phenoxy-naphthyl, Pyridyl, Thienyl, Tetrazolyl oder Furyl substituiert ist, wobei die Ringsysteme ihrerseits ein- bis mehrfach, gleich oder verschieden durch ($C_1$-$C_4$)-Alkyl, ($C_1$-$C_4$)-Alkoxy, Hydroxy, Nitro, Fluor, Chlor oder Brom substituiert sein können,
und/oder Alkyl gegebenenfalls durch einen Rest der Formel

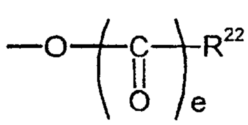

substituiert ist,
worin

e eine Zahl 0 oder 1 bedeutet und
$R^{22}$ ($C_1$-$C_4$)-Alkyl, Phenyl oder Naphthyl bedeutet, die gegebenenfalls ein- bis mehrfach, gleich oder verschieden durch Fluor, Chlor, Brom, Nitro, Hydroxy oder ($C_1$-$C_4$)-Alkoxy substituiert sind,

oder
Phenyl, Naphthyl, Thienyl, Furyl oder Pyridyl bedeutet, die ihrerseits gegebenenfalls ein- bis mehrfach, gleich oder verschieden durch ($C_1$-$C_4$)-Alkoxy, ($C_1$-$C_4$)-Alkyl, Hydroxy, Nitro, Fluor, Chlor oder Brom substituiert sein können,
oder
einen Rest der Formel

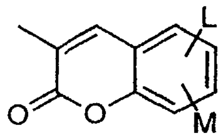

oder -$NR^{23}R^{24}$ bedeutet,
worin

L und M gleich oder verschieden sind und Wasserstoff, Fluor, Chlor oder Brom bedeuten,

$R^{23}$ und $R^{24}$ die oben angegebene Bedeutung von $R^{10}$ und $R^{11}$ haben und mit dieser gleich oder verschieden sind,

$R^{18}$ die oben angegebene Bedeutung von $R^{16}$ hat und mit dieser gleich oder verschieden ist,

$R^{19}$ Cyclopropyl, Cyclopentyl oder Cyclohexyl bedeutet, oder ($C_1$-$C_7$)-Alkyl oder ($C_2$-$C_6$)-Alkenyl bedeutet, die ihrerseits gegebenenfalls durch Substituenten, ausgewählt aus der Gruppe Fluor, Chlor, Brom, Phenyl, Hydroxy, Morpholinyl, Cyclopropyl, Cyclopentyl, Cyclohexyl und durch eine Gruppe der Formel -$SiR^{25}R^{26}R^{27}$ substituiert sind,
worin

$R^{25}$, $R^{26}$ und $R^{27}$ gleich oder verschieden sind und ($C_1$-$C_4$)-Alkyl bedeuten,

$R^{20}$ und $R^{21}$ gleich oder verschieden sind und Wasserstoff, Adamantyl, Cyclopropyl, Cylcopentyl, Cyclohexyl, Phenyl, Phenoxy-substituiertes Phenyl, Pyridyl, Furyl, Thienyl, Thiazolyl oder Pyrryl bedeuten, oder
($C_2$-$C_6$)-Alkenyl, ($C_1$-$C_{10}$)-Alkyl oder ($C_3$-$C_6$)-Alkinyl bedeuten, die gegebenenfalls durch Hydroxy, Cyclopropyl, Cyclopentyl, Cyclohexyl, ($C_1$-$C_5$)-Alkoxy, ($C_1$-$C_6$)-Alkoxycarbonyl, Fluor, Chlor, Brom, Hydroxy, Trifluormethyl, Phenyl, Pyridyl, Furyl, Thienyl oder Pyrryl substituiert sind, wobei die Ringsysteme gegebenenfalls bis zu 2-fach gleich oder verschieden durch ($C_1$-$C_4$)-Alkoxy, Fluor, Chlor, Brom, Phenoxy, Hydroxy oder ($C_1$-$C_4$)-Alkyl substituiert sind, und/oder das unter $R^{20}$/$R^{21}$ aufgeführte Alkyl gegebenenfalls durch Reste der Formeln
-$SCF_3$,

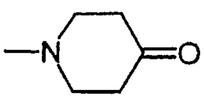
,
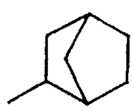
,

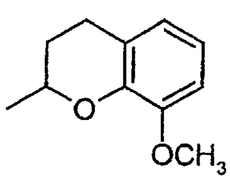

oder -$NR^{28}R^{29}$ substituiert ist,
worin

$R^{28}$ und $R^{29}$ gleich oder verschieden sind und Wasserstoff oder ($C_1$-$C_4$)-Alkyl bedeuten, oder

oder

einen Rest der Formel

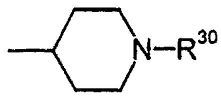

bedeutet,
worin

$R^{30}$ die oben angegebene Bedeutung von $R^{12}$ hat und mit dieser gleich oder verschieden ist,

oder

$R^{20}$ und $R^{21}$ gemeinsam mit dem Stickstoffatom einen Rest der Formel

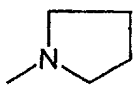

oder

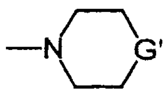

bilden,
worin

G' die oben angegebene Bedeutung von G hat und mit dieser gleich oder verschieden ist,

$R^2$ und $R^3$ gleich oder verschieden sind und für Wasserstoff oder $(C_1-C_3)$-Alkyl stehen,

und

D und E gemeinsam Reste der Formeln

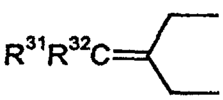

,

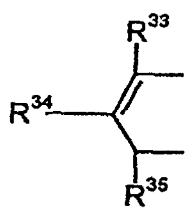

oder

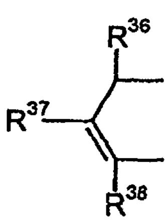

bilden,

worin

$R^{31}$, $R^{32}$, $R^{33}$, $R^{34}$, $R^{35}$, $R^{36}$, $R^{37}$ md $R^{38}$ gleich oder verschieden sind und Wasserstoff, Phenyl oder $(C_1-C_3)$ -Alkyl bedeuten,

und deren pharmazeutisch verträgliche Salze,

mit der Ausnahme von (3aS*, 6aR*)-6a-Methyl-3,3a,6,6a-tetrahydro-1H-cyclopenta*[c]*furan-1-on.

**3.** Verbindungen der Formel (I) nach Anspruch 1 oder 2, worin

A für Reste der Formeln $-CH_2-$, -CO-, $-CR^4(OH)$ oder $-(CH_2)_a-CHR^5$steht, worin

a eine Zahl 0, 1, 2 oder 3 bedeutet,

$R^4$ Wasserstoff oder $(C_1-C_3)$-Alkyl bedeutet

und

$R^5$ Phenyl bedeutet,

oder

für $(C_2-C_4)$-Alkandiyl, Propendiyl oder $(C_2-C_3)$-Alkindiyl steht,

$R^1$ für Wasserstoff, Cyclopropyl oder Cyclohexyl steht, oder für Benzofuranyl, Benzothiophenyl, Benzimidazolyl, Thienyl, Chinazolyl oder Chinoxalinyl steht,

oder

für Reste der Formeln

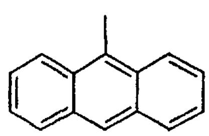 , 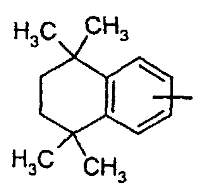 , 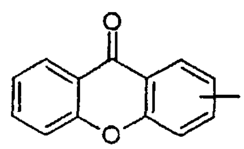 ,

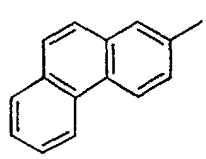 , 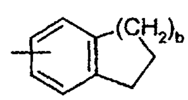 oder 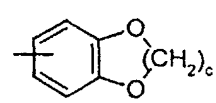

steht,

worin

b und c gleich oder verschieden sind und eine Zahl 1 oder 2 bedeuten,

oder

für Phenyl oder Naphthyl steht,

wobei alle oben aufgeführten Ringsysteme gegebenenfalls ein- bis mehrfach, gleich oder verschieden durch Substituenten, ausgewählt aus der Gruppe Fluor, Chlor, Brom, Jod, Cyano, Nitro, Trifluormethyl oder $(C_1-C_4)$-Alkoxy, $(C_1-C_4)$-Alkyl-carbonyloxy, Cyclohexyl, Phenyl, Phenoxy, Pyridyl, Pyrimidyl, Pyridazinyl oder Benzyloxy substituiert sind, die ihrerseits bis zu dreifach, gleich oder verschieden durch Cyano, Fluor, Chlor, Brom, oder Jod substituiert sein können,

und/oder durch $(C_1-C_4)$-Alkyl und $(C_2-C_3)$-Alkenyl substituiert sind, die ihrerseits durch Chlor, Brom, Jod oder

Phenyl oder durch Reste der Formel $-OR^9$ oder $-NR^{10}R^{11}$ oder

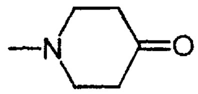

substituiert sein können,
worin

$R^9$ Wasserstoff oder $(C_1-C_3)$-Alkyl bedeutet,

und

$R^{10}$ und $R^{11}$ gleich oder verschieden sind und Wasserstoff, Phenyl oder $(C_1-C_3)$-Alkyl bedeuten, das gegebenenfalls durch Phenyl substituiert ist, das seinerseits durch Chlor, Brom, Hydroxy oder $(C_1-C_3)$-Alkoxy substituiert sein kann,

oder

$R^{10}$ und $R^{11}$ gemeinsam mit dem Stickstoffatom einen Rest der Formel

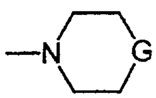

bilden,
worin

G ein Sauerstoffatom oder einen Rest der Formel $-NR^{12}$ bedeutet,

worin

$R^{12}$ Wasserstoff, Phenyl, Benzyl, $(C_1-C_3)$-Alkyl, $(C_1-C_3)$-Alkoxycarbonyl, Pyridyl, Pyrimidyl, Pyridazinyl oder Furyl bedeutet,

und/oder durch Gruppen der Formeln $-CO_2-R^{13}$, $-NR^{14}R^{15}$, $-NR^{16}CO-R^{17}$, $-NR^{18}CO_2-R^{19}$ und $-CO-NR^{20}R^{21}$ substituiert sind,
worin

$R^{13}$ Wasserstoff bedeutet, oder
$(C_1-C_6)$-Alkyl oder Allyl bedeutet, die ihrerseits durch Reste der Formeln

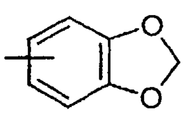
,
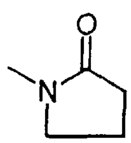
,

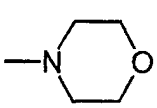
,
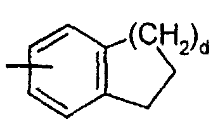

oder
Phenyl, Naphthyl oder Pyridyl substituiert sein können,
worin

d eine Zahl 1 oder 2 bedeutet,

oder
Phenyl bedeutet, das gegebenenfalls durch Phenyl substituiert ist, das seinerseits durch Cyano, Chlor oder Brom substituiert sein kann,

$R^{14}$ und $R^{15}$ gleich oder verschieden sind und Wasserstoff, Cyclohexyl, Phenyl oder $(C_1-C_4)$-Alkyl bedeuten, das gegebenenfalls durch Cyclopropyl, Cyclohexyl oder Phenyl substituiert ist, das seinerseits ein- bis mehrfach, gleich oder verschieden durch Chlor oder $(C_1-C_3)$-Alkoxy substituiert sein kann,

$R^{16}$ Wasserstoff, Methyl oder Ethyl bedeutet,

$R^{17}$ Wasserstoff, Adamantyl, Cyclopentyl oder Cyclohexyl bedeutet, oder
$(C_2-C_3)$-Alkenyl oder $(C_1-C_8)$-Alkyl bedeutet, das gegebenenfalls durch Adamantyl, Cyclopropyl, Cyclopentyl, Cyclohexyl, Phenyl, Phenoxy, Thienyl oder Furyl substituiert ist, wobei die Ringsysteme ihrerseits ein- bis mehrfach, gleich oder verschieden durch $(C_1-C_3)$-Alkyl, $(C_1-C_3)$-Alkoxy, Hydroxy, Nitro, Fluor, Chlor oder Brom substituiert sein können,
und/oder Alkyl gegebenenfalls durch einen Rest der Formel

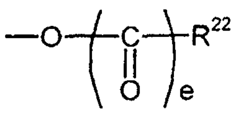

substituiert ist,
worin

e eine Zahl 0 oder 1 bedeutet und

$R^{22}$ $(C_1-C_3)$-Alkyl, Phenyl oder Naphthyl bedeutet, die gegebenenfalls ein- bis mehrfach, gleich oder verschieden durch Fluor, Chlor, Brom, Nitro, Hydroxy oder $(C_1-C_3)$-Alkoxy substituiert sind,

oder
Phenyl, Naphthyl, Thienyl oder Furyl bedeutet, die ihrerseits gegebenenfalls ein- bis mehrfach, gleich oder verschieden durch $(C_1-C_3)$-Alkoxy, $(C_1-C_3)$-Alkyl, Nitro, Fluor, Chlor oder Brom substituiert sein können,
oder
einen Rest der Formel

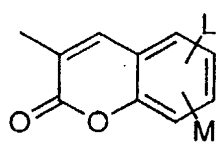

oder $-NR^{23}R^{24}$ bedeutet,
worin

L und M gleich oder verschieden sind und Wasserstoff, Fluor oder Chlor bedeuten,

$R^{23}$ und $R^{24}$ die oben angegebene Bedeutung von $R^{10}$ und $R^{11}$ haben und mit dieser gleich oder verschieden sind,

$R^{18}$ die oben angegebene Bedeutung von $R^{16}$ hat und mit dieser gleich oder verschieden ist,

$R^{19}$ $(C_1-C_4)$-Alkyl oder $(C_3-C_5)$-Alkenyl bedeutet, die ihrerseits gegebenenfalls durch Substituenten, ausgewählt aus der Gruppe Chlor, Phenyl, Hydroxy, Morpholinyl, Cyclopropyl, Cyclohexyl und durch eine Gruppe der Formel $-SiR^{25}R^{26}R^{27}$ substituiert sind,
worin
$R^{25}$, $R^{26}$ und $R^{27}$ gleich sind und Methyl bedeuten,

$R^{20}$ und $R^{21}$ gleich oder verschieden sind und Wasserstoff, Adamantyl, Cyclopropyl, Cyclopentyl, Cyclohexyl, Phenyl, Phenoxy-substituiertes Phenyl, Thiazolyl oder Pyrryl bedeuten, oder $(C_2-C_3)$-Alkenyl, $(C_1-C_7)$-Alkyl oder $(C_3-C_5)$-Alkinyl bedeuten, die gegebenenfalls durch Hydroxy, Cyclopropyl, Cyclopentyl, Cyclohexyl, $(C_1-C_3)$-Alkoxy, Hydroxy, Trifluormethyl, Phenyl, Pyridyl, Furyl, Thienyl oder Pyrryl substituiert sind, wobei die Ringsysteme gegebenenfalls bis zu 2-fach, gleich oder verschieden durch $(C_1-C_3)$-Alkoxy, $(C_1-C_6)$-Alkoxycarbonyl, Fluor, Chlor, Brom, Phenoxy, Hydroxy oder $(C_1-C_3)$-Alkyl substituiert sind,
und/oder das unter $R^{20}/R^{21}$ aufgeführte Alkyl gegebenenfalls durch Reste der Formeln
$-SCF_3$,

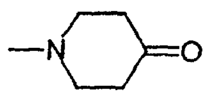 , 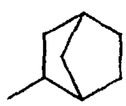 ,

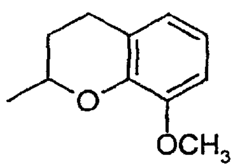

oder $-NR^{28}R^{29}$ substituiert ist,
worin

$R^{28}$ und $R^{29}$ gleich oder verschieden sind und Wasserstoff oder $(C_1-C_3)$-Alkyl bedeuten, oder

oder

$R^{20}$ oder $R^{21}$ einen Rest der Formel

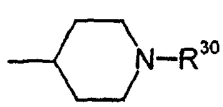

bedeutet,
worin

$R^{30}$ die oben angegebene Bedeutung von $R^{12}$ hat und mit dieser gleich oder verschieden ist,

oder

$R^{20}$ und $R^{21}$ gemeinsam mit dem Stickstoffatom einen Rest der Formel

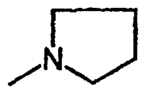 oder 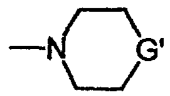

bilden,
worin

G' die oben angegebene Bedeutung von G hat und mit dieser gleich oder verschieden ist,

$R^2$ und $R^3$ gleich oder verschieden sind und für Wasserstoff oder Methyl stehen,

und

D und E gemeinsam Reste der Formeln

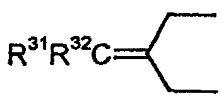 , 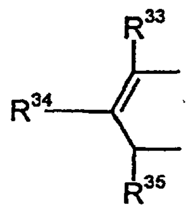

oder

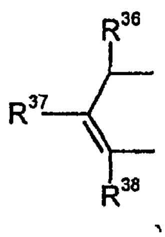

bilden,

worin
$R^{31}$, $R^{32}$, $R^{33}$, $R^{34}$, $R^{35}$, $R^{36}$, $R^{37}$ und $R^{38}$ gleich oder verschieden sind und Wasserstoff oder Methyl bedeuten,
und deren pharmazeutisch verträgliche Salze,
mit der Ausnahme von (3aS*, 6aR*)-6a-Methyl-3,3a,6,6a-tetrahydro-1H-cyclopenta*[c]*furan-1-on.

**4.** Verbindungen der allgemeinen Formel (I) nach irgendeinem der Ansprüche 1 bis 3, in denen A für die $-CH_2$-Gruppe steht,
mit der Ausnahme von (3aS*, 6aR*)-6a-Methyl-3,3a,6,6a-tetrahydro-1H-cyclopenta*[c]*furan-1-on.

**5.** Verbindungen der Formel (I) nach Anspruch 1 bis 4, worin

A für $-CH_2$- steht,

$R^1$ für Phenyl oder Naphthyl steht,
wobei alle oben aufgeführten Ringsysteme gegebenenfalls ein- bis mehrfach, gleich oder verschieden durch Substituenten, ausgewählt aus der Gruppe Fluor, Chlor, Brom, Jod, Cyano, Nitro, Trifluormethyl oder $(C_1-C_4)$-Alkoxy substituiert sind,

und/oder durch ($C_1$-$C_4$)-Alkyl substituiert sind,
und/oder durch Gruppen der Formeln $-NR^{16}CO-R^{17}$, $-NR^{18}CO_2-R^{19}$ und $-CO-NR^{20}R^{21}$ substituiert sind,
worin

$R^{16}$ Wasserstoff bedeutet,

$R^{17}$ ($C_1$-$C_8$)-Alkyl bedeutet, das gegebenenfalls durch Cyclopropyl, Cyclopentyl, Cyclohexyl, Phenyl, Thienyl oder Furyl substituiert ist, wobei die Ringsysteme ihrerseits ein- bis mehrfach, gleich oder verschieden durch ($C_1$-$C_3$)-Alkyl, ($C_1$-$C_3$)-Alkoxy, Hydroxy, Nitro, Fluor, Chlor oder Brom substituiert sein können,

$R^{18}$ die oben angegebene Bedeutung von $R^{16}$ hat und mit dieser gleich oder verschieden ist,

$R^{19}$ ($C_1$-$C_4$)-Alkyl oder ($C_3$-$C_5$)-Alkenyl bedeutet,

$R^{20}$ und $R^{21}$ gleich oder verschieden sind und Wasserstoff, ($C_2$-$C_3$)-Alkenyl, ($C_1$-$C_7$)-Alkyl oder ($C_3$-$C_5$)-Alkinyl bedeuten, die gegebenenfalls durch Phenyl, Pyridyl, Furyl, Thienyl oder Pyrryl substituiert sind, wobei die Ringsysteme gegebenenfalls bis zu 2-fach, gleich oder verschieden durch ($C_1$-$C_3$)-Alkoxy, Fluor, Chlor, Brom oder ($C_1$-$C_3$)-Alkyl substituiert sind,

$R^2$ und $R^3$ für Wasserstoff oder Methyl stehen,

und

D und E gemeinsam Reste der Formeln

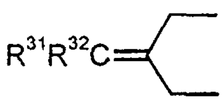

oder

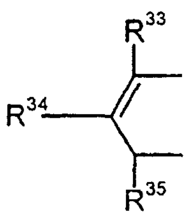

bilden,
worin
$R^{31}$, $R^{32}$, $R^{33}$, $R^{34}$, $R^{35}$ Wasserstoff bedeuten,

und deren pharmazeutisch verträgliche Salze.

**6.** Verbindungen nach irgendeinem der Ansprüche 1 bis 3, ausgewählt aus der Gruppe bestehend aus

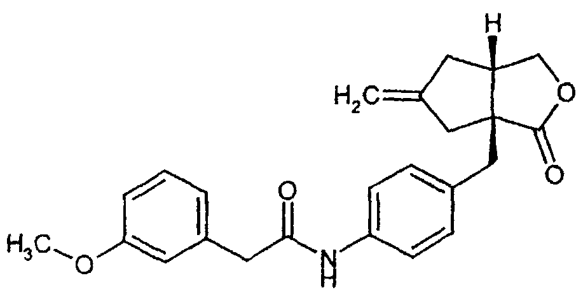

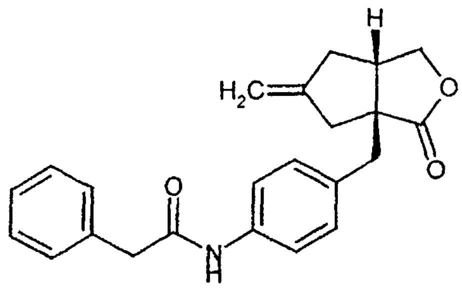

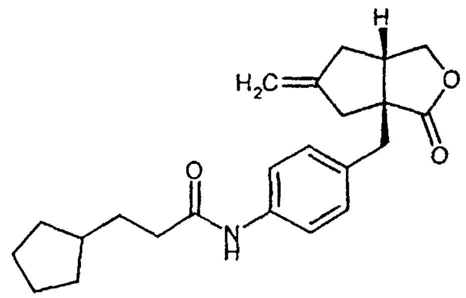

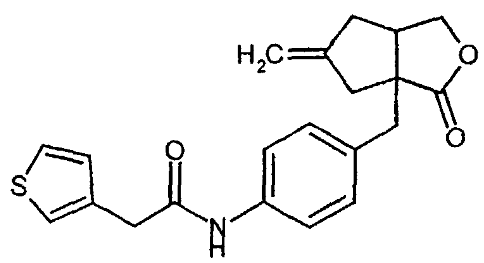

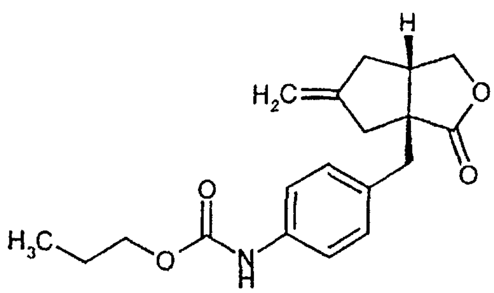

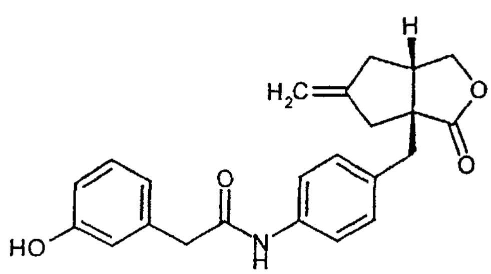

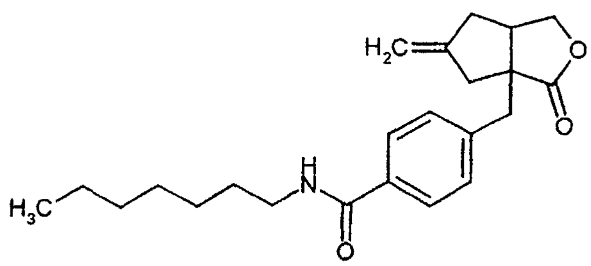

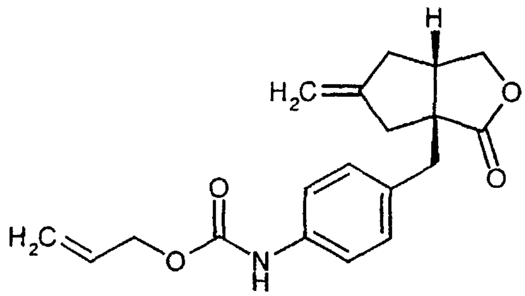

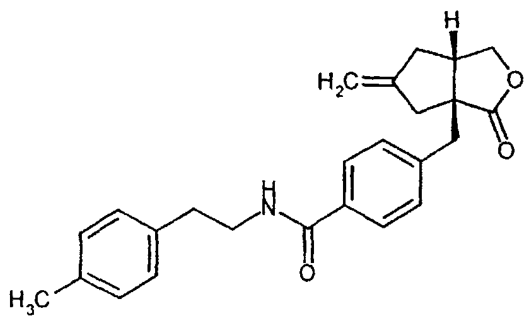

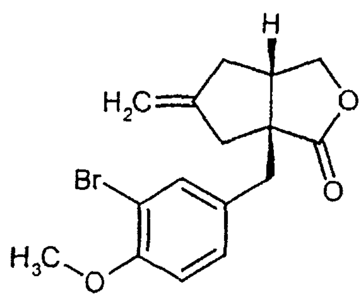

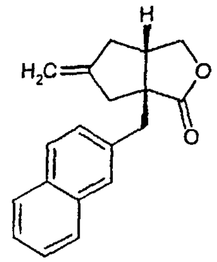

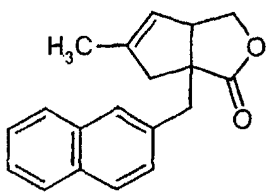

wobei diese Verbindungen als Racemat oder reines Enantiomer vorliegen können.

**7.** Verfahren zur Herstellung der Verbindungen der Formel (I) nach irgendeinem der Ansprüche 1 bis 6, indem man Verbindungen der allgemeinen Formel (II)

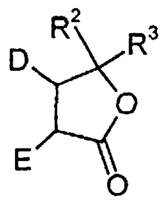

(II)

in welcher
D, E, $R^2$ und $R^3$ die oben angegebene Bedeutung haben,
mit Verbindungen der allgemeinen Formel (III),

$$T\text{-}A\text{-}R^1 \quad \text{(III)}$$

in welcher

T für Halogen, vorzugsweise für Brom steht,

und
A und $R^1$ die oben angegebene Bedeutung haben,
in inerten Lösemitteln und in Anwesenheit einer Base umsetzt,
und gegebenenfalls den Substituenten R' nach üblichen Methoden derivatisiert.

**8.** Verfahren zur Herstellung der erfindungsgemäßen Verbindungen der allgemeinen Formel (I') nach einem der Ansprüche 1 bis 6

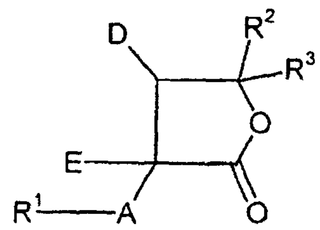

(I'),

in welcher
A, $R^1$, $R^2$ und $R^3$ die oben angegebene Bedeutung haben,
und
D und E gemeinsam Reste der Formein

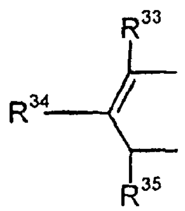

oder

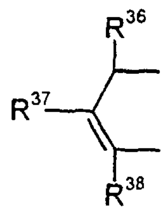

bilden,
worin
$R^{33}$, $R^{35}$, $R^{36}$ und $R^{38}$ für Wasserstoff stehen, und
$R^{34}$ und $R^{37}$ die oben angegebene Bedeutung haben,
**dadurch gekennzeichnet, daß** man
eine Verbindung der allgemeinen Formel (I")

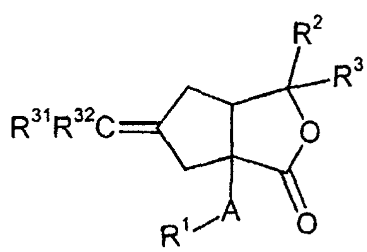

(I"),

in welcher
A, $R^1$, $R^2$ $R^3$, $R^{31}$ und $R^{32}$ die oben angegebene Bedeutung haben,
in Gegenwart eines Katalysators und gegebenenfalls eines Lösemittels isomerisiert.

**9.** Verbindungen der allgemeinen Formel (II),

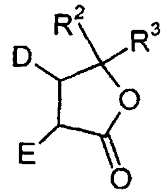

(II)

in welcher

$R^2$ und $R^3$ gleich oder verschieden sind und für Wasserstoff oder $(C_1-C_6)$-Alkyl stehen,

und

D und E gemeinsam Reste der Formeln

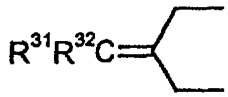

,

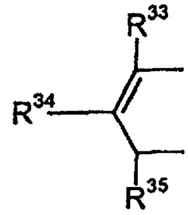

oder

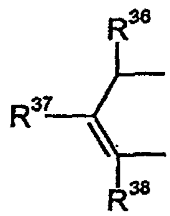

bilden,

worin

$R^{31}$, $R^{32}$, $R^{33}$, $R^{34}$, $R^{35}$, $R^{36}$, $R^{37}$ und $R^{38}$ gleich oder verschieden sind und Wasserstoff, Phenyl oder $(C_1-C_6)$-Alkyl bedeuten,

mit der Ausnahme von (3aS*, 6aR*)-, (3aS*, 6S*, 6aR*)-6-Methyl- und (3aS*, 6R*, 6aR*)-6-Methyl-3,3a,6,6a-tetrahydro-1H-cyclopenta*[c]*furan-1-

**10.** Verbindungen der allgemeinen Formel (II) nach Anspruch 9, in welcher

$R^2$ und $R^3$ für Wasserstoff oder Methyl stehen,

und

D und E gemeinsam Reste der Formeln

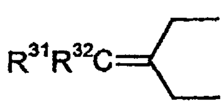

oder

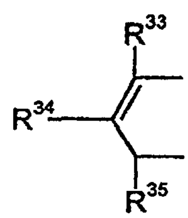

bilden,

worin

$R^{31}$, $R^{32}$, $R^{33}$, $R^{34}$ und $R^{35}$ Wasserstoff bedeuten,

mit der Ausnahme von (3aS*, 6aR*)-, (3aS*, 6S*, 6aR*)-6-Methyl- und (3aS*, 6R*, 6aR*)-6-Methyl-3,3a,6,6a-tetrahydro-1H-cyclopenta*[c]*furan-1-o

**11.** Verbindungen nach Anspruch 1 bis 6 oder (3aS*, 6aR*)-6a-Methyl-3,3a,6,6atetrahydro-1H-cydopenta[c]furan-1-on zur Verwendung als Medikament in der Behandlung von Menschen und Tieren.

**12.** Pharmazeutische Zusammensetzungen, die als aktiven Bestandteil mindestens eine Verbindung gemäß Anspruch 1 bis 6 oder (3aS*, 6aR*)-6a-Methyl-, 3,3a,6,6a-tetrahydro-1H-cyclopenta[c]furan-1-on in Zusammenmischung mit mindestens einem pharmazeutisch verträglichen, im wesentlichen nicht giftigen Träger oder Exzipianten umfaßt

**13.** Verwendung der Verbindungen gemäß Anspruch 1 bis 6 oder (3aS*, 6aR*)-6a-Methyl-3,3a,6,6a- tetrahydro-1H-cyclopenta[c]furan-1-on für die Herstellung eines Medikaments zur Prävention und/oder Behandlung von Erkrankungen, die durch eine Über- oder Unterfunktion des glutamatergen Systems ausgelöst werden.

**14.** Verwendung der Verbindungen gemäß Anspruch 1 bis 6 oder (3aS*, 6aR*)-6a-Methyl-3,3a,6,6a-tetrahydro-1H-cyclopenta[c]furan-1-on für die Herstellung eines Medikaments zur Prävention und/oder Behandlung von cerebralen Ischämien, Schädel-/Himtrauma, Schmerzzuständen oder ZNS-vermittelten Krämpfen.

**Claims**

**1.** Compounds of the general formula (I)

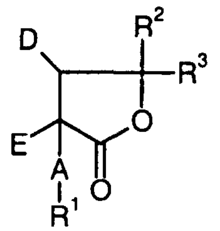

(I)

in which

A represents radicals of the formulae $-CH_2-$, -CO-, $-CR^4(OH)-$ or $-(CH_2)_a-CHR^5-$,
in which

a represents a number 0, 1, 2, 3 or 4,

$R^4$ represents hydrogen or $(C_1-C_6)$-alkyl

and

$R^5$ represents phenyl,

or

represents $(C_2-C_8)$-alkanediyl, $(C_2-C_6)$-alkenediyl or $(C_2-C_6)$-alkinediyl,

$R^1$ represents hydrogen, $(C_3-C_6)$-cycloalkyl or represents a 5- to 6-membered heterocycle which may contain up to 3 heteroatoms from the group consisting of S, O, N and/or a radical of the formula $-NR^6$,
in which

$R^6$ represents hydrogen or methyl,

or

represents a 5- to 6-membered benzo-fused heterocycle which may contain up to 2 heteroatoms from the group consisting of S, O, N and/or a radical of the formula $-NR^7$, and which may be attached both via the phenyl ring and via the heterocycle,
in which

$R^7$ has the meaning of $R^6$ given above and is identical to or different from this meaning,

or

represents radicals of the formulae

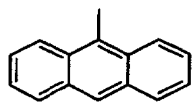 , 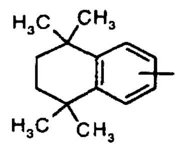 , 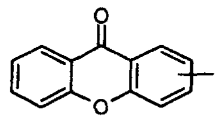 ,

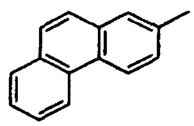 , 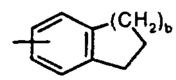 or 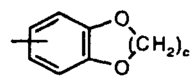 ,

in which

b and c are identical or different and represent a number 1 or 2,

or

represents ($C_6$-$C_{10}$)-aryl,

where all of the ring systems listed above are optionally mono- to polysubstituted by identical or different substituents selected from the group consisting of halogen, cyano, nitro, trifluoromethyl, hydroxyl, ($C_1$-$C_6$)-alkoxy, ($C_1$-$C_6$)-alkyl-carbonyl and ($C_3$-$C_6$)-cycloalkyl, phenyl, phenoxy, benzyloxy and a 5- to 6-membered aromatic heterocycle having up to 3 heteroatoms from the group consisting of S, N and/or O, which for their part may be substituted up to three times by identical or different substituents from the group consisting of cyano and halogen,

and/or are substituted by ($C_1$-$C_6$)-alkyl and ($C_2$-$C_6$)-alkylene, which for their part may be substituted by halogen, ($C_6$-$C_{10}$)-aryl or by radicals of the formula $-SR^8$, $-OR^9$ or $-NR^{10}R^{11}$ or

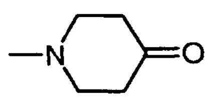 ,

in which

$R^8$ represents ($C_1$-$C_6$)-alkyl or phenyl,

$R^9$ represents hydrogen or ($C_1$-$C_6$)-alkyl,

and

$R^{10}$ and $R^{11}$ are identical or different and represent hydrogen, phenyl or ($C_1$-$C_6$)-alkyl, which is optionally substituted by phenyl, which for its part may be mono- to polysubstituted by identical or different substituents from the group consisting of halogen, nitro, hydroxyl and ($C_1$-$C_6$)-alkoxy,

or

$R^{10}$ and $R^{11}$ together with the nitrogen atom form a radical of the formula

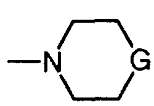 ,

in which

G represents an oxygen atom, a $-CH_2-$ group or a radical of the formula $-NR^{12}-$, in which

$R^{12}$ represents hydrogen, phenyl, benzyl, $(C_1-C_6)$-alkyl, $(C_1-C_6)$-alkoxy-carbonyl or a 5- to 6-membered aromatic heterocycle having up to 3 heteroatoms from the group consisting of S, N and/or O,

and/or are substituted by groups of the formulae $-CO_2-R^{13}$, $-NR^{14}R^{15}$, $-NR^{16}CO-R^{17}$, $-NR^{18}CO_2-R^{19}$ and $-CO-NR^{20}R^{21}$,
in which

$R^{13}$ represents hydrogen, or
represents $(C_1-C_9)$-alkyl or $(C_2-C_6)$-alkenyl, which for their part may be substituted by radicals of the formulae

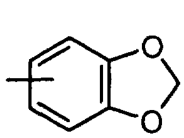 , 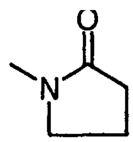 ,

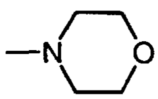 , 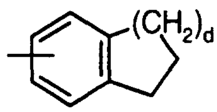

or
$(C_6-C_{10})$-aryl or by a 5- to 7-membered aromatic heterocycle having up to 3 heteroatoms from the group consisting of S, N and/or O,
in which

d represents a number 1 or 2,

or
represents $(C_6-C_{10})$-aryl, which is optionally substituted by phenyl, which for its part may be substituted by cyano or halogen,

$R^{14}$ and $R^{15}$ are identical or different and represent hydrogen, $(C_3-C_6)$-cycloalkyl, phenyl or $(C_1-C_6)$-alkyl, which is optionally substituted by $(C_3-C_6)$-cycloalkyl or phenyl, which for its part may be mono- to polysubstituted by identical or different substituents from the group consisting of halogen, hydroxyl or $(C_1-C_6)$-alkoxy,

$R^{16}$ represents hydrogen or $(C_1-C_6)$-alkyl,

$R^{17}$ represents hydrogen, adamantyl, $(C_3-C_8)$-cycloalkyl, $(C_2-C_6)$-alkenyl or $(C_1-C_{12})$-alkyl which is optionally substituted by adamantyl, $(C_3-C_6)$-cycloalkyl, $(C_6-C_{10})$-aryl, phenoxy or a 5-to 6-membered aromatic heterocycle having up to 4 heteroatoms from the group consisting of S, N and/or O, where aryl and the heterocycle for their part may be mono- to polysubstituted by identical or different substituents from the group consisting of $(C_1-C_6)$-alkyl, $(C_1-C_6)$-alkoxy, hydroxyl, nitro or halogen, and/or alkyl is optionally substituted by a radical of the formula

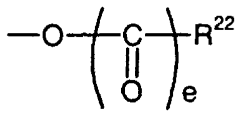

in which

e represents a number 0 or 1 and

$R^{22}$ represents $(C_1-C_6)$-alkyl or $(C_6-C_{10})$-aryl, which is optionally mono- to polysubsituted by identical or different substituents from the group consisting of halogen, nitro, hydroxyl and $(C_1-C_6)$-alkoxy,

or

represents $(C_6-C_{10})$-aryl or a 5- to 6-membered aromatic heterocycle having up to 3 heteroatoms from the group consisting of S, N and/or O, which for their part may optionally be mono- to polysubstituted by identical or different substituents from the group consisting of $(C_1-C_6)$-alkoxy, $(C_1-C_6)$-alkyl, hydroxyl, nitro and halogen,

or

represents a radical of the formula

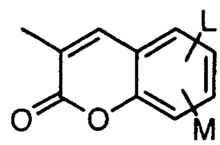

or $-NR^{23}R^{24}$,

in which

L and M are identical or different and represent hydrogen or halogen,

$R^{23}$ and $R^{24}$ have the meaning of $R^{10}$ and $R^{11}$ given above and are identical to or different from this meaning,

$R^{18}$ has the meaning of $R^{16}$ given above and is identical to or different from this meaning,

$R^{19}$ represents $(C_3-C_8)$-cycloalkyl, or represents $(C_1-C_8)$-alkyl or $(C_2-C_8)$-alkenyl, which for their part are optionally substituted by substituents selected from the group consisting of halogen, phenyl, hydroxyl, morpholinyl, $(C_3-C_8)$-cycloalkyl and by a group of the formula $-SiR^{25}R^{26}R^{27}$,

in which

$R^{25}$, $R^{26}$ and $R^{27}$ are identical or different and represent $(C_1-C_6)$-alkyl,

$R^{20}$ and $R^{21}$ are identical or different and represent hydrogen, adamantyl, $(C_3-C_8)$-cycloalkyl, phenyl, phenoxy-substituted phenyl or a 5- to 6-membered, aromatic heterocycle having up to 3 heteroatoms from the group consisting of S, N and/or O, or

represent $(C_2-C_8)$-alkenyl, $(C_1-C_{12})$-alkyl or $(C_2-C_6)$-alkinyl, which are optionally substituted by hydroxyl, $(C_3-C_6)$-cycloalkyl, $(C_1-C_6)$-alkoxy, halogen, hydroxyl, trifluoromethyl, phenyl or by a 5- to 6-membered aromatic heterocycle having up to 3 heteroatoms from the group consisting of S, N and/or O, where the ring systems are optionally substiutted up to 2 times by identical or different substituents from the group consisting of $(C_1-C_6)$-alkoxy, $(C_1-C_6)$-alkoxycarbonyl, halogen, phenoxy, hydroxyl and $(C_1-C_6)$-alkyl,

and/or the alkyl listed under $R^{20}/R^{21}$ is optionally substituted by radicals of the formulae

$-SCF_3$,

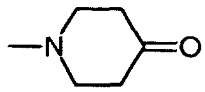 , 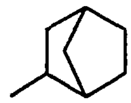 ,

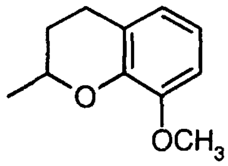

or $-NR^{28}R^{29}$,
in which

$R^{28}$ and $R^{29}$ are identical or different and represent hydrogen or $(C_1-C_6)$-alkyl,

or
represents a radical of the formula

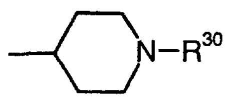 ,

in which

$R^{30}$ has the meaning of $R^{12}$ given above and is identical to or different from this meaning,

or

$R^{20}$ and $R^{21}$ together with the nitrogen atom form a radical of the formula

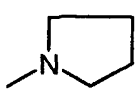 or 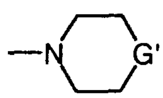 ,

in which

G' has the meaning of G given above and is identical to or different from this meaning,

$R^2$ and $R^3$ are identical or different and represent hydrogen or $(C_1-C_6)$-alkyl,

and
D and E together form radicals of the formulae

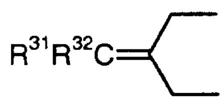
,
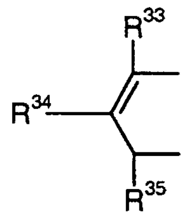

or

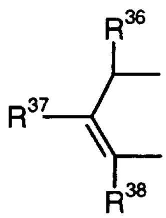
,

in which
$R^{31}$, $R^{32}$, $R^{33}$, $R^{34}$, $R^{35}$, $R^{36}$, $R^{37}$ and $R^{38}$ are identical or different and represent hydrogen, phenyl or $(C_1-C_6)$-alkyl,
and their pharmaceutically acceptable salts,
with the exception of (3aS*, 6aR*)-6a-methyl-3,3a,6,6a-tetrahydro-1H-cyclopenta[c]furan-1-one.

**2.** Compounds of the formula (I) according to Claim 1, in which

A represents radicals of the formulae $-CH_2-$, -CO-, $-CR^4(OH)-$ or $-(CH_2)_a-CHR^5-$, in which

a represents a number 0, 1, 2 or 3,

$R^4$ represents hydrogen or $(C_1-C_4)$-alkyl

and

$R^5$ represents phenyl,

or
represents $(C_2-C_6)$-alkanediyl, $(C_2-C_4)$-alkenediyl or $(C_2-C_4)$-alkinediyl,

$R^1$ represents hydrogen, cyclopropyl, cyclopentyl or cyclohexyl, or represents benzofuranyl, benzothiophenyl, benzimidazolyl, thienyl, furyl, quinazolyl, quinoxalinyl or quinolyl,

or
represents radicals of the formulae

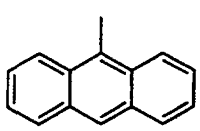
,
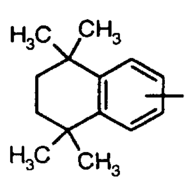
,
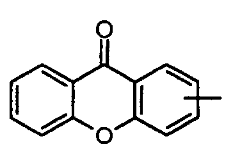
,

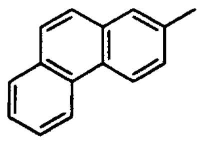 , 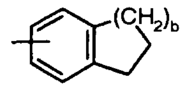 or 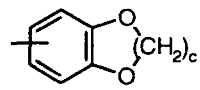 ,

in which

b and c are identical or different and represent a number 1 or 2,

or

represents phenyl or naphthyl,

where all of the ring systems listed above are optionally mono- to polysubstituted by identical or different substituents selected from the group consisting of fluorine, chlorine, bromine, iodine, cyano, nitro, trifluoromethyl, hydroxyl or $(C_1-C_5)$-alkoxy, $(C_1-C_5)$-alkyl-carbonyloxy, cyclopropyl, cyclopentyl, cyclohexyl, phenyl, phenoxy, pyridyl, pyrimidyl, pyridazinyl, thienyl, furyl or benzyloxy, which for their part may be substituted up to three times by identical or different substituents from the group consisting of cyano, fluorine, chlorine, bromine and iodine,

and/or are substituted by $(C_1-C_5)$-alkyl and $(C_2-C_4)$-alkenyl, which for their part may be substituted by fluorine, chlorine, bromine, iodine, phenyl, naphthyl or by radicals of the formula $-SR^8$, $-OR^9$ or $-NR^{10}R^{11}$ or

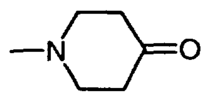 ,

in which

$R^8$ represents $(C_1-C_4)$-alkyl or phenyl,

$R^9$ represents hydrogen or $(C_1-C_4)$-alkyl,

and

$R^{10}$ and $R^{11}$ are identical or different and represent hydrogen, phenyl or $(C_1-C_4)$-alkyl, which is optionally substituted by phenyl, which for its part may be mono- to polysubstituted by identical or different substituents from the group consisting of fluorine, chlorine, bromine, nitro, hydroxyl and $(C_1-C_4)$-alkoxy,

or

$R^{10}$ and $R^{11}$ together with the nitrogen atom form a radical of the formula

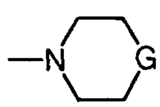 ,

in which

G represents an oxygen atom, a $-CH_2-$ group or a radical of the formula $-NR^{12}-$,
in which

$R^{12}$ represents hydrogen, phenyl, benzyl, $(C_1-C_4)$-alkyl, $(C_1-C_4)$-alkoxycarbonyl, pyridyl, pyrimidyl, pyridazinyl or fury],

and/or are substituted by groups of the formulae $-CO_2-R^{13}$, $-NR^{14}R^{15}$, $-NR^{16}CO-R^{17}$, $-NR^{18}CO_2-R^{19}$ and $-CO-NR^{20}R^{21}$,

in which

$R^{13}$ represents hydrogen, or

represents ($C_1$-$C_8$)-alkyl or ($C_2$-$C_5$)-a]kenyl, which for their part may be substituted by radicals of the formulae

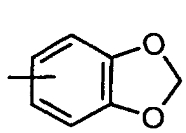
,
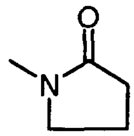
,

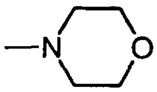
,
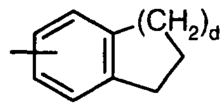

or
phenyl, naphthyl, pyridyl, thienyl or furyl,
in which

d represents a number 1 or 2,

or
represents phenyl or naphthyl, which are optionally substituted by phenyl, which for its part may be substituted by cyano, fluorine, chorine or bromine,

$R^{14}$ and $R^{15}$ are identical or different and represent hydrogen, cyclopropyl, cyclopentyl, cyclohexyl, phenyl or ($C_1$-$C_5$)-alkyl, which is optionally substituted by cyclopropyl, cyclopentyl, cyclohexyl or phenyl, which for its part may be mono- to polysubstituted by identical or different substituents from the group consisting of fluorine, chlorine, bromine, hydroxyl or ($C_1$-$C_4$)-alkoxy,

$R^{16}$ represents hydrogen or ($C_1$-$C_3$)-alkyl,

$R^{17}$ represents hydrogen, adamantyl, cyclopropyl, cyclopentyl or cyclohexyl, or represents ($C_2$-$C_4$)-alkenyl or ($C_1$-$C_{10}$)-alkyl, which is optionally substituted by adamantyl, cyclopropyl, cyclopentyl, cyclohexyl, phenyl, phenoxy-naphthyl, pyridyl, thienyl, tetrazolyl or furyl, where the ring systems for their part may be mono- to polysubstituted by identical or different substituents from the group consisting of ($C_1$-$C_4$)-alkyl, ($C_1$-$C_4$)-alkoxy, hydroxyl, nitro, fluorine, chlorine and bromine,
and/or alkyl is optionally substituted by a radical of the formula

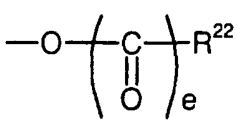
,

in which

e represents a number 0 or 1 and

$R^{22}$ represents ($C_1$-$C_4$)-alkyl, phenyl or naphthyl, which are optionally mono- to polysubstituted by identical or different substituents from the group consisting of fluorine, chlorine, bromine, nitro, hydroxyl and ($C_1$-$C_4$)-alkoxy,

or

represents phenyl, naphthyl, thienyl, furyl or pyridyl, which for their part may optionally be mono- to polysubstituted by identical or different substituents from the group consisting of $(C_1-C_4)$-alkoxy, $(C_1-C_4)$-alkyl, hydroxyl, nitro, fluorine, chlorine and bromine,

or

represents a radical of the formula

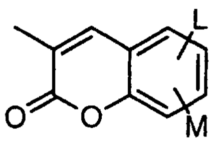

or -$NR^{23}R^{24}$,
in which

L and M are identical or different and represent hydrogen, fluorine, chlorine or bromine,

$R^{23}$ and $R^{24}$ have the meaning of $R^{10}$ and $R^{11}$ given above and are identical to or different from this meaning,

$R^{18}$ has the meaning of $R^{16}$ given above and is identical to or different from this meaning,

$R^{19}$ represents cyclopropyl, cyclopentyl or cyclohexyl, or represents $(C_1-C_7)$-alkyl or $(C_2-C_6)$-alkenyl, which for their part are optionally substituted by substituents selected from the group consisting of fluorine, chlorine, bromine, phenyl, hydroxyl, morpholinyl, cyclopropyl, cyclopentyl, cyclohexyl and by a group of the formula -$SiR^{25}R^{26}R^{27}$,
in which

$R^{25}$, $R^{26}$ and $R^{27}$ are identical or different and represent $(C_1-C_4)$-alkyl,

$R^{20}$ and $R^{21}$ are identical or different and represent hydrogen, adamantyl, cyclopropyl, cyclopentyl, cyclohexyl, phenyl, phenoxy-substituted phenyl, pyridyl, furyl, thienyl, thiazolyl or pyrryl, or represent $(C_2-C_6)$-alkenyl, $(C_1-C_{10})$-alkyl or $(C_3-C_6)$-alkinyl, which are optionally substituted by hydroxyl, cyclopropyl, cyclopentyl, cyclohexyl, $(C_1-C_5)$-alkoxy, $(C_1-C_6)$-alkoxycarbonyl, fluorine, chlorine, bromine, hydroxyl, trifluoromethyl, phenyl, pyridyl, furyl, thienyl or pyrryl, where the ring systems are optionally substituted up to 2 times by identical or different substituents from the group consisting of $(C_1-C_4)$-alkoxy, fluorine, chlorine, bromine, phenoxy, hydroxyl or $(C_1-C_4)$-alkyl,
and/or the alkyl listed under $R^{20}$/$R^{21}$ is optionally substituted by radicals of the formulae
-$SCF_3$,

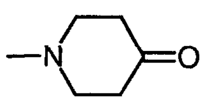
,
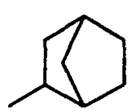
,

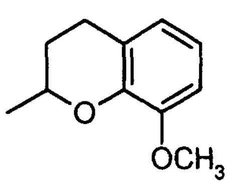

or -$NR^{28}R^{29}$,

in which

$R^{28}$ and $R^{29}$ are identical or different and represent hydrogen or $(C_1-C_4)$-alkyl,

or

represents a radical of the formula

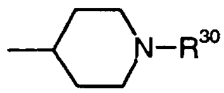

,

in which

$R^{30}$ has the meaning of $R^{12}$ given above and is identical to or different from this meaning,

or

$R^{20}$ and $R^{21}$ together with the nitrogen atom form a radical of the formula

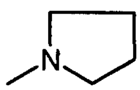

or

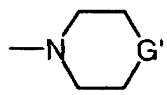

,

in which

G' has the meaning of G given above and is identical to or different from this meaning,

$R^2$ and $R^3$ are identical or different and represent hydrogen or $(C_1-C_3)$-alkyl,

and

D and E together form radicals of the formulae

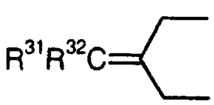

,

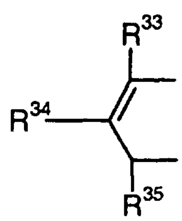

or

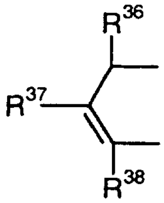

,

in which

$R^{31}$, $R^{32}$, $R^{33}$, $R^{34}$, $R^{35}$, $R^{36}$, $R^{37}$ and $R^{38}$ are identical or different and represent hydrogen, phenyl or ($C_1$-$C_3$)-alkyl,

and their pharmaceutically acceptable salts,

with the exception of (3aS*, 6aR*)-6a-methyl-3,3a,6,6a-tetrahydro-1H-cyclopenta[c]furan-1-one,

**3.** Compounds of the formula (I) according to Claim 1 or 2, in which

A represents radicals of the formulae $-CH_2-$, -CO-, $-CR^4(OH)-$ or $-(CH_2)_a-CHR^5-$,
in which

a represents a number 0, 1, 2 or 3,

$R^4$ represents hydrogen or ($C_1$-$C_3$)-alkyl

and

$R^5$ represents phenyl,

or

represents ($C_2$-$C_4$)-alkanediyl, propenediyl or ($C_2$-$C_3$)-alkinediyl,

$R^1$ represents hydrogen, cyclopropyl or cyclohexyl, or represents benzofuranyl, benzothiophenyl, benzimidazolyl, thienyl, quinazolyl or quinoxalinyl,

or

represents radicals of the formulae

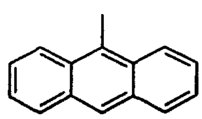 , 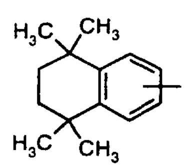 , 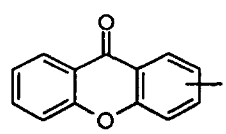 ,

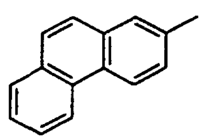 , 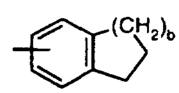 or 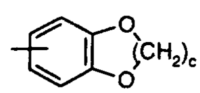 ,

in which

b and c are identical or different and represent a number 1 or 2,

or

represents phenyl or naphthyl,

where all of the ring systems listed above are optionally mono- to polysubstituted by identical or different substituents selected from the group consisting of fluorine, chlorine, bromine, iodine, cyano, nitro, trifluoromethyl or ($C_1$-$C_4$)-alkoxy, ($C_1$-$C_4$)-alkyl-carbonyloxy, cyclohexyl, phenyl, phenoxy, pyridyl, pyrimidyl, pyridazinyl or benzyloxy, which for their part may be substituted up to three times by identical or different substituents from the group consisting of cyano, fluorine, chlorine, bromine and iodine,

and/or are substituted by ($C_1$-$C_4$)-alkyl and ($C_2$-$C_3$)-alkenyl, which for their part may be substituted by chlorine, bromine, iodine or phenyl or by radicals of the formula $-OR^9$ or $-NR^{10}R^{11}$ or

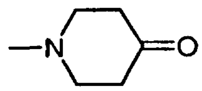 ,

in which

$R^9$ represents hydrogen or ($C_1$-$C_3$)-alkyl,

and

$R^{10}$ and $R^{11}$ are identical or different and represent hydrogen, phenyl or ($C_1$-$C_3$)-alkyl, which is optionally substituted by phenyl, which for its part may be substituted by chlorine, bromine, hydroxyl or ($C_1$-$C_3$)-alkoxy,

or
$R^{10}$ and $R^{11}$ together with the nitrogen atom form a radical of the formula

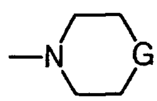 ,

in which

G represents an oxygen atom or a radical of the formula -$NR^{12}$,

in which

$R^{12}$ represents hydrogen, phenyl, benzyl, ($C_1$-$C_3$)-alkyl, ($C_1$-$C_3$)-alkoxycarbonyl, pyridyl, pyrimidyl, pyridazinyl or furyl,

and/or are substituted by groups of the formulae -$CO_2$-$R^{13}$, -$NR^{14}R^{15}$, -$NR^{16}CO$-$R^{17}$, -$NR^{18}CO_2$-$R^{19}$ and -CO-$NR^{20}R^{21}$,
in which

$R^{13}$ represents hydrogen, or
represents ($C_1$-$C_6$)-alkyl or allyl, which for their part may be substituted by radicals of the formulae

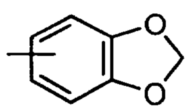 , 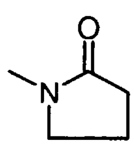 ,

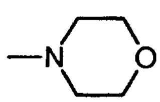 , 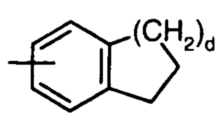

or
phenyl, naphthyl or pyridyl,
in which

d represents a number 1 or 2,

or

represents phenyl, which is optionally substituted by phenyl, which for its part may be substituted by cyano, chlorine or bromine,

$R^{14}$ and $R^{15}$ are identical or different and represent hydrogen, cyclohexyl, phenyl or ($C_1$-$C_4$)-alkyl, which is optionally substituted by cyclopropyl, cyclohexyl or phenyl, which for its part may be mono- to polysubstituted by identical or different substituents from the group consisting of chlorine and ($C_1$-$C_3$)-alkoxy,

$R^{16}$ represents hydrogen, methyl or ethyl,

$R^{17}$ represents hydrogen, adamantyl, cyclopentyl or cyclohexyl, or represents ($C_2$-$C_3$)-alkenyl or ($C_1$-$C_8$)-alkyl, which is optionally substituted by adamantyl, cyclopropyl, cyclopentyl, cyclohexyl, phenyl, phenoxy, thienyl or furyl, where the ring systems for their part may be mono- to polysubstituted by identical or different substituents from the group consisting of ($C_1$-$C_3$)-alkyl, ($C_1$-$C_3$)-alkoxy, hydroxyl, nitro, fluorine, chlorine and bromine,
and/or alkyl is optionally substituted by a radical of the formula

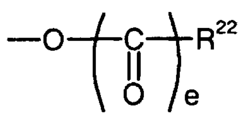

,

in which

e is a number 0 or 1 and

$R^{22}$ represents ($C_1$-$C_3$)-alkyl, phenyl or naphthyl, which are optionally mono- to polysubstituted by identical or different substituents from the group consisting of fluorine, chlorine, bromine, nitro, hydroxyl and ($C_1$-$C_3$)-alkoxy,

or

represents phenyl, naphthyl, thienyl or furyl, which for their part may optionally be mono- to polysubstituted by identical or different substituents from the group consisting of ($C_1$-$C_3$)-alkoxy, ($C_1$-$C_3$)-alkyl, nitro, fluorine, chlorine and bromine,

or

represents a radical of the formula

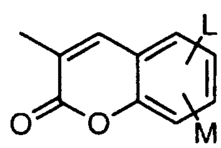

or -$NR^{23}R^{24}$,

in which

L and M are identical or different and represent hydrogen, fluorine or chlorine,

$R^{23}$ and $R^{24}$ have the meaning of $R^{10}$ and $R^{11}$ given above and are identical to or different from this meaning,

$R^{18}$ has the meaning of $R^{16}$ given above and is identical to or different from this meaning,

$R^{19}$ represents ($C_1$-$C_4$)-alkyl or ($C_3$-$C_5$)-alkenyl, which for their part are optionally substituted by substituents selected from the group consisting of chlorine, phenyl, hydroxyl, morpholinyl, cyclopropyl, cyclohexyl and by a group of the formula -$SiR^{25}R^{26}R^{27}$,
in which

$R^{25}$, $R^{26}$ and $R^{27}$ are identical and represent methyl,

$R^{20}$ and $R^{21}$ are identical or different and represent hydrogen, adamantyl, cyclopropyl, cyclopentyl, cyclohexyl, phenyl, phenoxy-substituted phenyl, thiazolyl or pyrryl, or represent ($C_2$-$C_3$)-alkenyl, ($C_1$-$C_7$)-alkyl or ($C_3$-$C_5$)-alkinyl, which are optionally substituted by hydroxyl, cyclopropyl, cyclopentyl, cyclohexyl, ($C_1$-$C_3$)-alkoxy, hydroxyl, trifluoromethyl, phenyl, pyridyl, furyl, thienyl or pyrryl, where the ring systems are optionally substituted up to 2 times by identical or different substituents from the group consisting of ($C_1$-$C_3$)-alkoxy, ($C_1$-$C_6$)-alkoxycarbonyl, fluorine, chlorine, bromine, phenoxy, hydroxyl and ($C_1$-$C_3$)-alkyl,
and/or the alkyl listed under $R^{20}$/$R^{21}$ is optionally substituted by radicals of the formulae

$-SCF_3$,

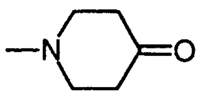 , 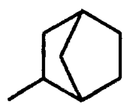 ,

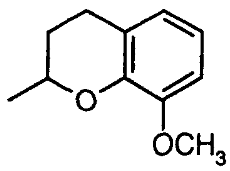

or $-NR^{28}R^{29}$,
in which

$R^{28}$ and $R^{29}$ are identical or different and represent hydrogen or ($C_1$-$C_3$)-alkyl, or

or

$R^{20}$ or $R^{21}$ represents a radical of the formula

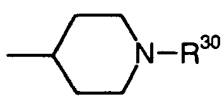 ,

in which

$R^{30}$ has the meaning of $R^{12}$ given above and is identical to or different from this meaning,

or

$R^{20}$ and $R^{21}$ together with the nitrogen atom form a radical of the formula

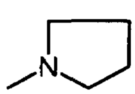 or 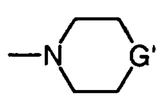 ,

in which

G' has the meaning of G given above and is identical to or different from this meaning,

$R^2$ and $R^3$ are identical or different and represent hydrogen or methyl,

and

D and E together form radicals of the formulae

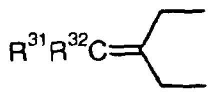

,

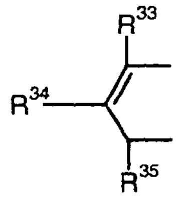

or

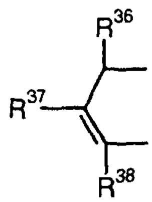

,

in which

$R^{31}$, $R^{32}$, $R^{33}$, $R^{34}$, $R^{35}$, $R^{36}$, $R^{37}$ and $R^{38}$ are identical or different and represent hydrogen or methyl,

and their pharmaceutically acceptable salts,

with the exception of (3aS*, 6aR*)-6a-methyl-3,3a,6,6a-tetrahydro-1H-cyclopenta[c]furan-1-one,

**4.** Compounds of the general formula (I) according to any of Claims 1 to 3, in which A represents the $-CH_2-$ group, with the exception of (3aS*, 6aR*)-6a-methyl-3,3a,6,6a-tetrahydro-1H-cyclopenta[c]furan-1-one,

**5.** Compounds of the formula (I) according to Claims 1 to 4, in which

A represents $-CH_2-$,

$R^1$ represents phenyl or naphthyl,
where all of the abovementioned ring systems are optionally mono- to polysubstituted by identical or different substituents selected from the group consisting of fluorine, chlorine, bromine, iodine, cyano, nitro, trifluoromethyl or $(C_1-C_4)$-alkoxy,
and/or are substituted by $(C_1-C_4)$-alkyl,
and/or are substituted by groups of the formulae $-NR^{16}CO-R^{17}$, $-NR^{18}CO_2-R^{19}$ and $-CO-NR^{20}R^{21}$, in which

$R^{16}$ is hydrogen,

$R^{17}$ is $(C_1-C_8)$-alkyl, which is optionally substituted by cyclopropyl, cyclopentyl, cyclohexyl, phenyl, thienyl or furyl, where the ring systems for their part may be mono- to polysubstituted by identical or different substituents from the group consisting of $(C_1-C_3)$-alkyl, $(C_1-C_3)$-alkoxy, hydroxyl, nitro, fluorine, chlorine and bromine,

$R^{18}$ has the meaning of $R^{16}$ given above and is identical to or different from this meaning,

$R^{19}$ represents $(C_1-C_4)$-alkyl or $(C_3-C_5)$-alkenyl,

$R^{20}$ and $R^{21}$ are identical or different and represent hydrogen, $(C_2-C_3)$-alkenyl, $(C_1-C_7)$-alkyl or $(C_3-C_5)$-alkinyl, which are optionally substituted by phenyl, pyridyl, furyl, thienyl or pyrryl, where the ring systems are optionally substituted up to 2 times by identical or different substituents from the group con-

sisting of ($C_1$-$C_3$)-alkoxy, fluorine, chlorine, bromine and ($C_1$-$C_3$)-alkyl,

$R^2$ and $R^3$ represent hydrogen or methyl,

and

D and E together form radicals of the formulae

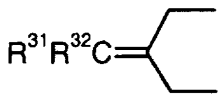 or 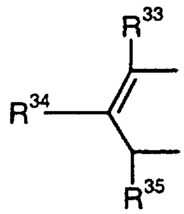 ,

in which
$R^{31}$, $R^{32}$, $R^{33}$, $R^{34}$, $R^{35}$ represent hydrogen,

and their pharmaceutically acceptable salts.

**6.** Compounds according to any of Claims 1 to 3, selected from the group consisting of

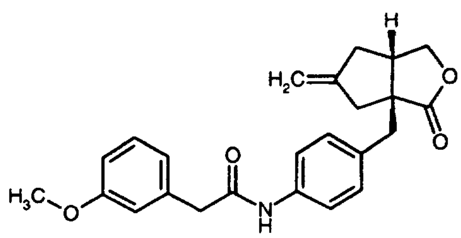

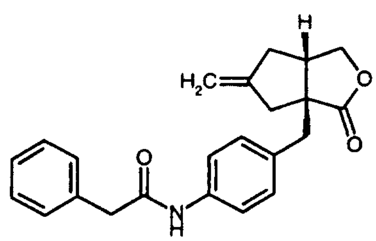

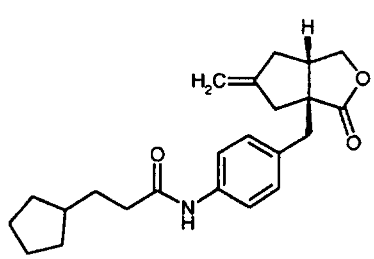

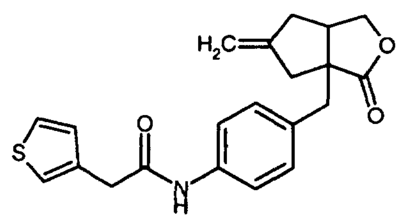

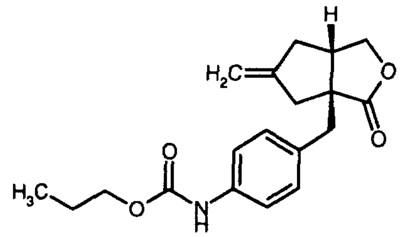

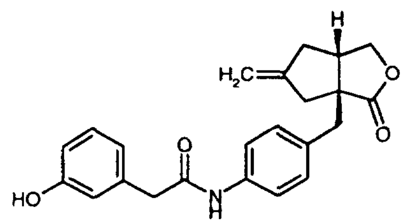

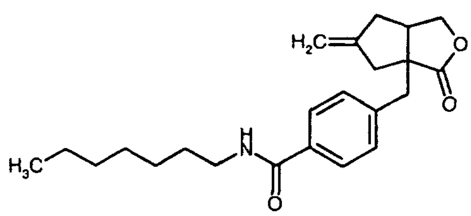

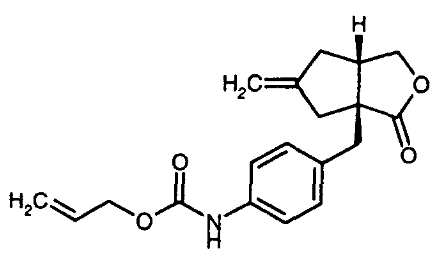

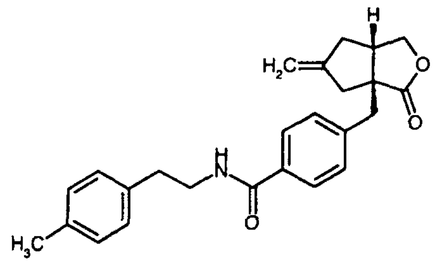

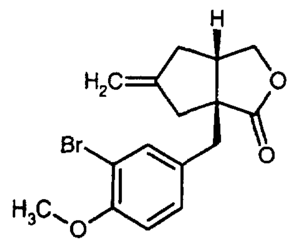

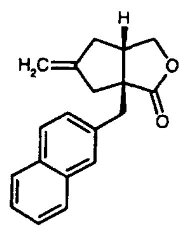

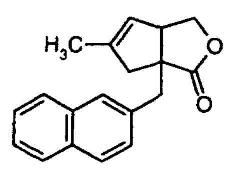

where these compounds can be present as a racemate or as a pure enantiomer.

**7.** Process for preparing the compounds of the formula (I) according to any one of Claims 1 to 6, by reacting compounds of the general formula (II)

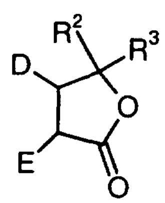

(II)

in which
D, E, $R^2$ and $R^3$ are as defined above,
with compounds of the general formula (III),

$$T\text{-}A\text{-}R^1 \qquad \text{(III)}$$

in which

T represents halogen, preferably bromine,

and
A and $R^1$ are as defined above,
in inert solvents and in the presence of a base,
and, if appropriate, derivatizing the substituent $R^1$ by customary methods.

**8.** Process for preparing the compounds of the general formula (I') according to the invention according to any of Claims 1 to 6

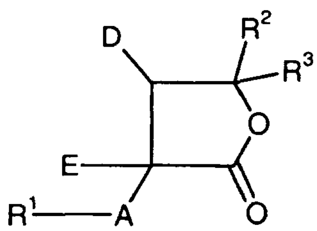

(I') ,

in which
A, $R^1$, $R^2$ and $R^3$ are as defined above,
and
D and E together form radicals of the formulae

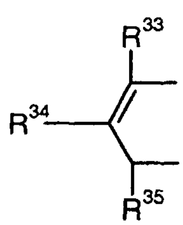

or

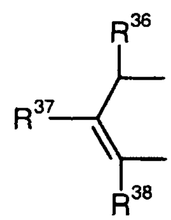

,

in which
$R^{33}$, $R^{35}$, $R^{36}$ and $R^{38}$ represent hydrogen, and
$R^{34}$ and $R^{37}$ are as defined above,
**characterized in that**
a compound of the general formula (I")

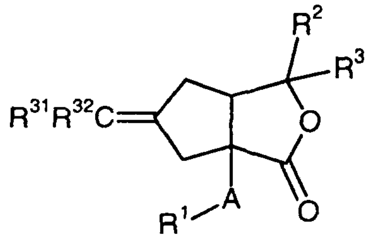

(I"),

in which
A, $R^1$, $R^2$ $R^3$, $R^{31}$ and $R^{32}$ are as defined above,
is isomerized in the presence of a catalyst and, if appropriate, a solvent.

**9.** Compounds of the general formula (II),

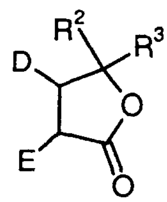

(II)

in which

$R^2$ and $R^3$ are identical or different and represent hydrogen or $(C_1\text{-}C_6)$-alkyl,

and
D and E together form radicals of the formulae

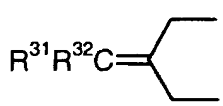

,

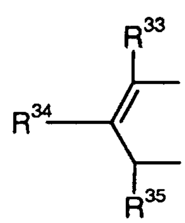

or

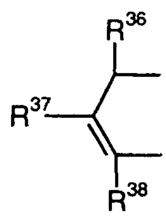

,

in which
$R^{31}$, $R^{32}$, $R^{33}$, $R^{34}$, $R^{35}$, $R^{36}$, $R^{31}$ and $R^{38}$ are identical or different and represent hydrogen, phenyl or $(C_1\text{-}C_6)$-alkyl.
With the exception of (3aS*, 6aR*)-, (3aS*, 6S*, 6aR*)-6-methyl- and (3aS*, 6R*, 6aR*)-6-methyl-3,3a,6,6a-tetrahydro-1H-cyclopenta[c]furan-1-one.

**10.** Compounds of the general formula (II) according to Claim 9, in which

$R^2$ and $R^3$ represent hydrogen or methyl,

and
D and E together form radicals of the formulae

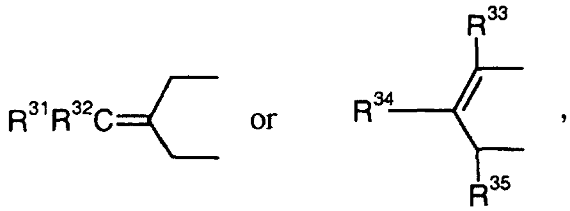

in which
$R^{31}$, $R^{32}$, $R^{33}$, $R^{34}$ and $R^{35}$ represent hydrogen,
With the exception of (3aS*, 6aR*)-, (3aS*, 6S*, 6aR*)-6-methyl- and (3aS*, 6R*, 6aR*)-6-methyl-3,3a,6,6a-tetrahydro-1H-cyclopenta[c]furan-1-one.

**11.** Compounds according to Claims 1 to 6 or (3aS*, 6aR*)-6a-methyl-3,3a,6,6a-tetrahydro-1H-cyclopenta[c]furan-1-one for use as medicaments in the treatment of humans and animals.

**12.** Pharmaceutical composition, comprising as active component at least one compound according to Claims 1 to 6 or (3aS*, 6aR*)-6a-methyl-3,3a,6,6a-tetrahydro-1H-cyclopenta[c]furan-l-one mixed together with at least one pharmaceutically acceptable, essentially non-toxic vehicle or excipient.

**13.** Use of the compounds according to Claims 1 to 6 or (3aS*, 6aR*)-6a-methyl-3,3a,6,6a-tetrahydro-1H-cyclopenta [c]furan-1-one for preparing a medicament for the prevention and/or treatment of disorders caused by hyper- or hypofunction of the glutamatergic system.

**14.** Use of the compounds according to Claims 1 to 6 or (3aS*, 6aR*)-6a-methyl-3,3a,6,6a-tetrahydro-lH-cyclopenta [c]furan-1-one for preparing a medicament for the prevention and/or treatment of cerebral ischaemias, craniocerebral trauma, states of pain or CNS-mediated spasms.

**15.** Use of the compounds according to Claims 1 to 6 for preparing a medicament for the prevention and/or treatment of cerebral ischaemias, craniocerebral trauma, states of pain or CNS-mediated spasms.

**Revendications**

**1.** Composés de formule générale (I)

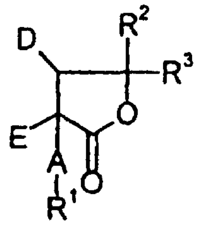

(I)

dans laquelle

A représente des restes de formules $-CH_2-$, -CO-, $-CR^4(OH)-$ ou $-(CH_2)_a-CHR^5-$, où

a représente le nombre 0, 1, 2, 3 ou 4, et
$R^4$ est l'hydrogène ou un groupe alkyle en $C_1$ à $C_6$,

et

$R^5$ représente un groupe phényle,

ou bien
des restes alcanediyle en $C_2$ à $C_8$, alcènediyle en $C_2$ à $C_6$ ou alcynediyle en $C_2$ à $C_6$,

$R^1$ est l'hydrogène, un groupe cycloalkyle en $C_3$ à $C_6$ ou un hétérocycle pentagonal ou hexagonal qui peut contenir jusqu'à 3 hétéroatomes de la série S, O, N et/ou un reste de formule $-NR^6$, dans laquelle

$R^6$ est l'hydrogène ou un groupe méthyle,

ou bien
un hétérocycle pentagonal à hexagonal condensé au benzène, qui peut contenir jusqu'à 2 hétéroatomes de la série S, O, N et/ou un reste de formule $-NR^7$ et qui peut être lié tant par l'intermédiaire du noyau phényle que par l'intermédiaire de l'hétérocycle,
où

$R^7$ a la définition indiquée ci-dessus pour $R^6$ et y est identique ou en est différent,

ou bien
des restes de formules

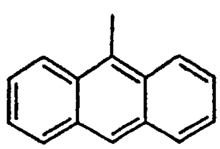 , 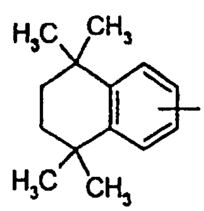 , 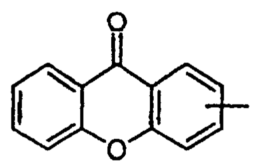 ,

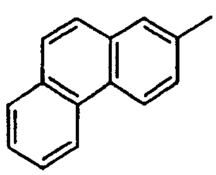 , 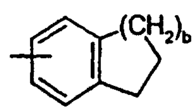 ou 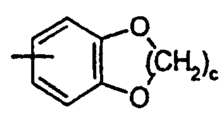 ,

dans lesquelles
b et c sont égaux ou différents et ont la valeur 1 ou 2,
ou bien
un reste aryle en $C_6$ à $C_{10}$,
tous les noyaux indiqués ci-dessus étant éventuellement substitués une ou plusieurs fois identiques ou différentes par des substituants choisis dans le groupe halogéno, cyano, nitro, trifluorométhyle, hydroxy, alkoxy en $C_1$ à $C_6$, (alkyle en $C_1$ à $C_6$)-carbonyle, cycloalkyle en $C_3$ à $C_6$, phényle, phénoxy, benzyloxy et par un hétérocycle aromatique pentagonal ou hexagonal ayant jusqu'à 3 hétéroatomes de la série S, N, et/ou O, qui peuvent de même être substitués jusqu'à trois fois identiques ou différentes par un radical cyano ou halogéno,
et/ou portant des substituants alkyle en $C_1$ à $C_6$ et alkylène en $C_2$ à $C_6$ qui peuvent eux-mêmes être substitués

par un halogène, un reste aryle en $C_6$ à $C_{10}$ ou par des restes de formules $-SR^8$, $-OR^9$ ou $-NR^{10}R^{11}$ ou

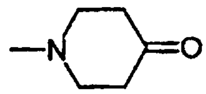

où

$R^8$ désigne un reste alkyle en $C_1$ à $C_6$ ou phényle,
$R^9$ est l'hydrogène ou un groupe alkyle en $C_1$ à $C_6$,

et

$R^{10}$ et $R^{11}$ sont identiques ou différents et représentent l'hydrogène, un reste phényle ou alkyle en $C_1$ à $C_6$, qui est éventuellement substitué par un groupe phényle pouvant lui-même être substitué une ou plusieurs fois identiques ou différentes par un halogène, un groupe nitro, hydroxy ou alkoxy en $C_1$ à $C_6$,

ou bien

$R^{10}$ et $R^{11}$ forment conjointement avec l'atome d'azote un reste de formule

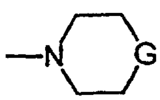

dans laquelle

G est un atome d'oxygène, un groupe $-CH_2-$ ou un reste de formule $-NR^{12}-$, dans laquelle

$R^{12}$ représente l'hydrogène, un groupe phényle ou benzyle, alkyle en $C_1$ à $C_6$, (alkoxy en $C_1$ à $C_6$)-carbonyle ou un hétérocycle aromatique pentagonal ou hexagonal ayant jusqu'à 3 hétéroatomes de la série S, N et/ou O,

et/ou sont substitués par des groupes de formules $-CO_2-R^{13}$, $-NR^{14}R^{15}$, $-NR^{16}CO-R^{17}$, $-NR^{18}CO_2-R^{19}$ et $-CO-NR^{20}R^{21}$,
dans lesquelles,
$R^{13}$ désigne l'hydrogène, ou
représente un groupe alkyle en $C_1$ à $C_9$ ou alcényle en $C_2$ à $C_6$, pouvant eux-mêmes être substitués par des restes de formules

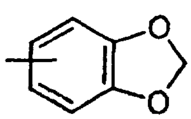

,

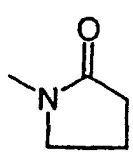

,

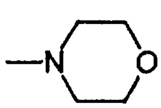

,

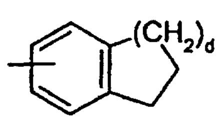

ou
aryle en $C_6$ à $C_{10}$ ou par un hétérocycle aromatique pentagonal à heptagonal ayant jusqu'à 3 hétéroatomes de la série S, N et/ou O,
où

d désigne le nombre 1 ou 2,

ou bien
désigne un reste aryle en $C_6$ à $C_{10}$ qui est éventuellement substitué par un groupe phényle pouvant lui-même être substitué par un radical cyano ou un halogène,

$R^{14}$ et $R^{15}$ sont identiques ou différents et représentent l'hydrogène, un reste cycloalkyle en $C_3$ à $C_6$, phényle ou alkyle en $C_1$ à $C_6$, qui est éventuellement substitué par un groupe cycloalkyle en $C_3$ à $C_6$ ou phényle pouvant lui-même être substitué une ou plusieurs fois identiques ou différentes par un halogène, un radical hydroxy ou alkoxy en $C_1$ à $C_6$,
$R^{16}$ est l'hydrogène ou un groupe alkyle en $C_1$ à $C_6$,
$R^{17}$ est l'hydrogène, un groupe adamantyle, cycloalkyle en $C_3$ à $C_8$, alcényle en $C_2$ à $C_6$ ou alkyle en $C_1$ à $C_{12}$, qui est éventuellement substitué par un groupe adamantyle, cycloalkyle en $C_3$ à $C_8$, aryle en $C_6$ à $C_{10}$, phénoxy ou par un hétérocycle aromatique en $C_5$ ou $C_6$ ayant jusqu'à 4 hétéroatomes de la série S, N et/ou O, le groupe aryle et l'hétérocycle pouvant eux-mêmes être substitués une ou plusieurs fois identiques
ou différentes par un radical alkyle en $C_1$ à $C_6$, alkoxy en $C_1$ à $C_6$, hydroxy, nitro ou halogéno, et/ou alkyle éventuellement substitué par un reste de formule

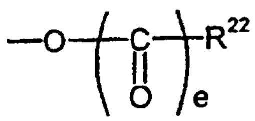

dans laquelle

e désigne le nombre 0 ou 1 et
$R^{22}$ est un reste alkyle en $C_1$ à $C_6$ ou aryle en $C_6$ à $C_{10}$ qui est éventuellement substitué une ou plusieurs fois identiques ou différentes par un radical halogéno, nitro, hydroxy ou alkoxy en $C_1$ à $C_6$,

ou
un reste aryle en $C_6$ à $C_{10}$ ou un hétérocycle aromatique pentagonal ou hexagonal ayant jusqu'à 3 hétéroatomes de la série S, N et/ou O, ces restes pouvant eux-mêmes être substitués le cas échéant une ou plusieurs fois identiques ou différentes par un radical alkoxy en $C_1$ à $C_6$, alkyle en $C_1$ à $C_6$, hydroxy, nitro ou halogéno,
ou bien
un reste de formule

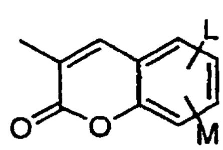

ou $-NR^{23}R^{24}$
dans laquelle
L et M sont identiques ou différents et représentent l'hydrogène ou un halogène,
$R^{23}$ et $R^{24}$ ont les définitions indiquées ci-dessus pour $R^{10}$ et $R^{11}$ et y sont identiques ou en sont différents,

$R^{18}$ a la définition indiquée ci-dessus pour $R^{16}$ et y est identique ou en est différent,

$R^{19}$ désigne un reste cycloalkyle et $C_3$ à $C_8$, ou un reste alkyle en $C_1$ à $C_8$ ou alcényle en $C_2$ à $C_8$, qui sont eux-mêmes substitués le cas échéant par des substituants choisis dans le groupe halogéno, phényle, hydroxy, morpholinyle, cycloalkyle en $C_3$ à $C_8$ ou par un groupe de formule $-SiR^{25}R^{26}R^{27}$,

où

$R^{25}$, $R^{26}$ et $R^{27}$ sont identiques ou différents et désignent des groupes alkyle en $C_1$ à $C_6$,

$R^{20}$ et $R^{21}$ sont identiques ou différents et représentent l'hydrogène, un reste adamantyle, cycloalkyle en $C_3$ à $C_8$, phényle, phényle à substituant phénoxy ou un hétérocycle aromatique pentagonal ou hexagonal ayant jusqu'à 3 hétéroatomes de la série S, N et/ou O,

ou bien

des restes alcényle en $C_2$ à $C_8$, alkyle en $C_1$ à $C_{12}$ ou alcynyle en $C_2$ à $C_6$ qui sont éventuellement substitués par des radicaux hydroxy, cycloalkyle en $C_3$ à $C_6$, alkoxy en $C_1$ à $C_6$, halogéno, hydroxy, trifluorométhyle, phényle ou par un hétérocycle aromatique pentagonal ou hexagonal ayant jusqu'à 3 hétéroatomes de la série S, N et/ou O, les noyaux étant éventuellement substitués jusqu'à deux fois identiques ou différentes par des radicaux alkoxy en $C_1$ à $C_6$, (alkoxy en $C_1$ à $C_6$)-carbonyle, halogéno, phénoxy, hydroxy ou alkyle en $C_1$ à $C_6$,

et/ou le reste alkyle indiqué pour $R^{20}/R^{21}$ est éventuellement substitué par des restes de formules $-SCF_3$.

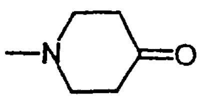 , 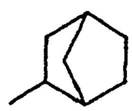 ,

ou $-NR^{28}R^{29}$,

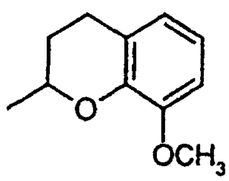

où

$R^{28}$ et $R^{29}$ sont identiques ou différents et représentent l'hydrogène ou des groupes alkyle en $C_1$ à $C_6$,

ou bien

un reste de formule

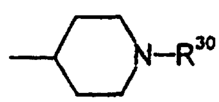

dans laquelle

$R^{30}$ a la définition indiquée ci-dessus pour $R^{12}$ et y est identique ou en est différent,

ou bien,

$R^{20}$ et $R^{21}$ forment conjointement avec l'atome d'azote un reste de formule

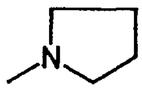 ou 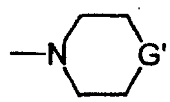

dans laquelle

G' a la définition indiquée ci-dessus pour G et y est identique ou en est différent,

$R^2$ et $R^3$ sont identiques ou différents et représentent l'hydrogène ou un groupe alkyle en $C_1$ à $C_6$,

et

D et E forment ensemble des restes de formules

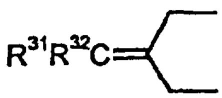 , 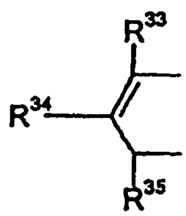

ou

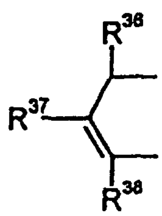

dans lesquelles

$R^{31}$, $R^{32}$, $R^{33}$, $R^{34}$, $R^{35}$, $R^{36}$, $R^{37}$ et $R^{38}$ sont identiques ou différents et représentent l'hydrogène, un groupe phényle ou alkyle en $C_1$ à $C_6$,

et leurs sels acceptables du point de vue pharmaceutique, à l'exception de la (3aS*, 6aR*)-6a-méthyl-3,3a,6,6a-tétrahydro-1H-cyclopénta[c]furanne-1-one.

**2.** Composés de formule (I) suivant la revendication 1, dans laquelle

A représente des restes de formules $-CH_2-$, -CO-, $-CR^4(OH)-$ ou $-(CH_2)_a-CHR^5-$, où

a représente le nombre 0, 1, 2 ou 3, et

$R^4$ est l'hydrogène ou un groupe alkyle en $C_1$ à $C_4$,

et

$R^5$ représente un groupe phényle,

ou bien

un groupe alcanediyle en $C_2$ à $C_6$, alcènediyle en $C_2$ à $C_4$ ou alcynediyle en $C_2$ à $C_4$,

$R^1$ est l'hydrogène, un groupe cyclopropyle, cyclopentyle ou cyclohexyle,

ou

un groupe benzofurannyle, benzothiophényle, benzimidazolyle, thiényle, furyle, quinazolyle, quinoxalinyle ou quinolyle,

ou bien

des restes de formules

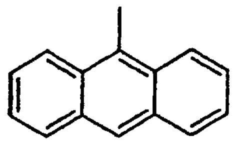 , 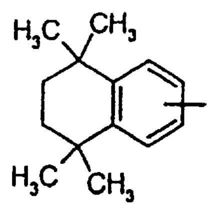 , 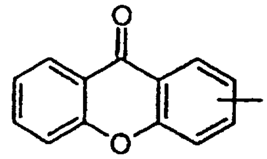 ,

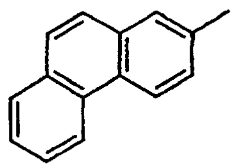 , 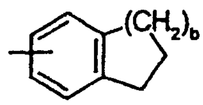 ou 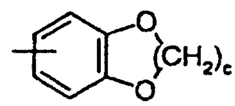

dans lesquelles

b et c sont identiques ou différents et désignent le nombre 1 ou 2,

ou bien

un reste phényle ou naphtyle,

tous les noyaux indiqués ci-dessus étant éventuellement substitués une ou plusieurs fois identiques ou différentes par des substituants choisis dans le groupe fluor, chlore, brome, iode, cyano, nitro, trifluorométhyle, hydroxy ou alkoxy en $C_1$ à $C_5$, (alkyle en $C_1$ à $C_5$)-carbonyloxy, cyclopropyle, cyclopentyle, cyclohexyle, phényle, phénoxy, pyridyle, pyrimidyle, pyridazinyle, thiényle, furyle ou benzyloxy, qui peuvent eux-mêmes être substitués jusqu'à trois fois identiques ou différentes par un radical cyano, du fluor, du chlore, du brome ou de l'iode,

et/ou substitués par des restes alkyle en $C_1$ à $C_5$ et alcényle en $C_2$ à $C_4$ qui peuvent eux-mêmes être substitués par du fluor, du chlore, du brome, de l'iode, un reste phényle, naphtyle ou des restes de formules $-SR^8$, $-OR^9$ ou $-NR^{10}R^{11}$ ou

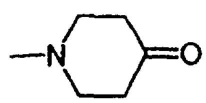

où

$R^8$ est un reste alkyle en $C_1$ à $C_4$ ou phényle,

$R^9$ est l'hydrogène ou un radical alkyle en $C_1$ à $C_4$,

et

$R^{10}$ et $R^{11}$ sont identiques ou différents et désignent l'hydrogène, un groupe phényle ou alkyle en $C_1$ à $C_4$, qui est éventuellement substitué par un reste phényle pouvant lui-même être substitué une ou plusieurs fois identiques ou différentes par du fluor, du chlore, du brome, un radical nitro, hydroxy ou alkoxy en $C_1$ à $C_4$,

ou bien

$R^{10}$ et $R^{11}$ forment conjointement avec l'atome d'azote un reste de formule

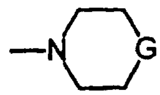

dans laquelle

G est un atome d'oxygène, un groupe $-CH_2-$ ou un reste de formule $-NR^{12}$,

dans laquelle

$R^{12}$ représente l'hydrogène, un groupe phényle, benzyle, alkyle en $C_1$ à $C_4$, (alkoxy en $C_1$ à $C_4$)-carbonyle, pyridyle, pyrimidyle, pyridazinyle ou furyle,

et/ou sont substitués par des groupes de formules $-CO_2-R^{13}$, $-NR^{14}R^{15}$, $-NR^{16}CO-R^{17}$, $-NR^{18}CO_2-R^{19}$ et $-CO-NR^{20}R^{21}$,
où,

$R^{13}$ désigne l'hydrogène, ou désigne
un groupe alkyle en $C_1$ à $C_8$ ou un groupe alcényle en $C_2$ à $C_5$, qui peuvent eux-mêmes être substitués par des restes de formules

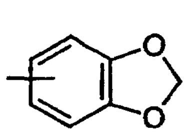
,
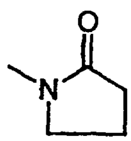
,

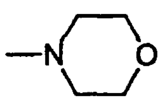
,
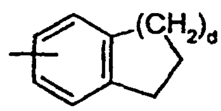

ou

des restes phényle, naphtyle, pyridyle, thiényle ou furyle,
formules dans lesquelles

d désigne le nombre 1 ou 2,

ou bien représente
des restes phényle ou naphtyle qui sont éventuellement substitués par un reste phényle pouvant lui-même être substitué par un radical cyano, fluoro, chloro ou bromo,

$R^{14}$ et $R^{15}$ sont identiques ou différents et représentent l'hydrogène, un groupe cyclopropyle, cyclopentyle, cyclohexyle, phényle ou alkyle en $C_1$ à $C_5$ qui est éventuellement substitué par un radical cyclopropyle, cyclopentyle, cyclohexyle ou phényle pouvant lui-même porter un ou plusieurs substituants, identiques ou différents, fluor, chlore, brome, hydroxy ou alkoxy en $C_1$ à $C_4$,

$R^{16}$ est l'hydrogène ou un groupe alkyle en $C_1$ à $C_3$,

$R^{17}$ est l'hydrogène, un groupe adamantyle, cyclopropyle, cyclopentyle ou cyclohexyle, ou désigne un groupe alcényle en $C_2$ à $C_4$ ou alkyle en $C_1$ à $C_{10}$, qui est substitué par un radical adamantyle, cyclopropyle, cyclopentyle, cyclohexyle, phényle, phénoxy-naphtyle, pyridyle, thiényle, tétrazolyle ou furyle, les noyaux pouvant eux-mêmes porter un ou plusieurs substituants, identiques ou différents, alkyle en $C_1$ à $C_4$, alkoxy en $C_1$ à $C_4$, hydroxy, nitro, fluoro, chloro ou bromo,
et/ou un groupe alkyle éventuellement substitué par un reste de formule

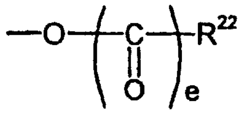

dans laquelle

e désigne le nombre 0 ou 1 et

$R^{22}$ désigne un groupe alkyle en $C_1$ à $C_4$, un groupe phényle ou un groupe naphtyle qui sont éventuellement substitués une ou plusieurs fois identiques ou différentes par un radical fluoro, chloro, bromo, nitro, hydroxy ou alkoxy en $C_1$ à $C_4$,

ou désigne

un groupe phényle, naphtyle, thiényle, furyle ou pyridyle, pouvant chacun être substitué éventuellement une ou plusieurs fois identiques ou différentes par un radical alkoxy en $C_1$ à $C_4$, alkyle en $C_1$ à $C_4$, hydroxy, nitro, fluoro, chloro ou bromo,

ou bien désigne

un reste de formule

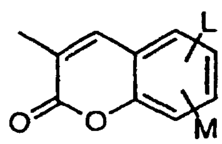

ou $-NR^{23}R^{24}$,

où

L et M sont identiques ou différents et représentent l'hydrogène, le fluor, le chlore ou le brome,

$R^{23}$ et $R^{24}$ ont la définition indiquée ci-dessus pour $R^{10}$ et

$R^{11}$ et y sont identiques ou en sont différents,

$R^{18}$ a la définition indiquée ci-dessus pour $R^{16}$ et y est identique ou en est différent,

$R^{19}$ désigne un reste cyclopropyle, cyclopentyle ou cyclohexyle, ou désigne un reste alkyle en $C_1$ à $C_7$ ou un reste alcényle en $C_2$ à $C_6$ qui portent eux-mêmes éventuellement des substituants choisis dans le groupe fluor, chlore, brome, phényle, hydroxy, morpholinyle, cyclopropyle, cyclopentyle, cyclohexyle, et un groupe de formule $-SiR^{25}R^{26}R^{27}$,

dans lequel

$R^{25}$, $R^{26}$ et $R^{27}$ sont identiques ou différents et désignent des radicaux alkyle en $C_1$ à $C_4$,

$R^{20}$ et $R^{21}$ sont identiques ou différents et représentent l'hydrogène, un groupe adamantyle, cyclopropyle, cyclopentyle, cyclohexyle, phényle, phényle à substituant phénoxy, pyridyle, furyle, thiényle, thiazolyle ou pyrryle, ou désignent des restes alcényle en $C_2$ à $C_6$, alkyle en $C_1$ à $C_{10}$ ou alcynyle en $C_3$ à $C_6$ qui sont éventuellement substitués par des radicaux hydroxy, cyclopropyle, cyclopentyle, cyclohexyle, alkoxy en $C_1$ à $C_5$, (alkoxy en $C_1$ à $C_6$)-carbonyle, fluoro, chloro, bromo, hydroxy, trifluorométhyle, phényle, pyridyle, furyle, thiényle ou pyrryle, les noyaux étant éventuellement substitués jusqu'à deux fois identiques ou différentes par des radicaux alkoxy en $C_1$ à $C_4$, fluoro, chloro, bromo, phénoxy, hydroxy ou alkyle en $C_1$ à $C_4$,

et/ou le groupe alkyle indiqué pour $R^{20}/R^{21}$ est éventuellement substitué par des restes de formules $-SCF_3$,

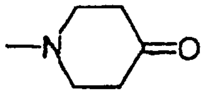 , 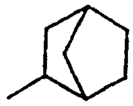 ,

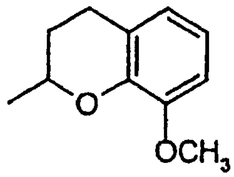

ou $-NR^{28}R^{29}$,

où

$R^{28}$ et $R^{29}$ sont identiques ou différents et représentent l'hydrogène ou des groupes alkyle en $C_1$ à $C_4$,

ou désigne

un reste de formule

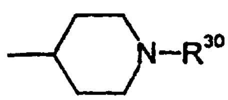

dans laquelle

$R^{30}$ a la définition indiquée ci-dessus pour

$R^{12}$ et y est identique ou en est différent,

ou bien,

$R^{20}$ et $R^{21}$ forment conjointement avec l'atome d'azote un reste de formule

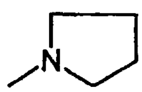 ou 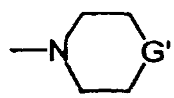

dans laquelle

G' a la définition indiquée ci-dessus pour

G et y est identique ou en est différent,

$R^2$ et $R^3$ sont identiques ou différents et représentent l'hydrogène ou un groupe alkyle en $C_1$ à $C_3$,

et

D et E forment ensemble des restes de formules

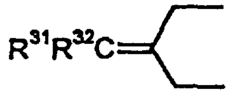 , 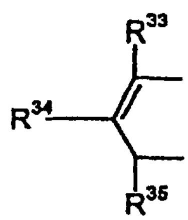

ou

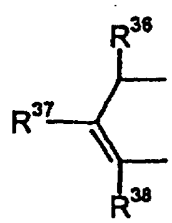

dans lesquelles

$R^{31}$, $R^{32}$, $R^{33}$, $R^{34}$, $R^{35}$, $R^{36}$, $R^{37}$ et $R^{38}$ sont identiques ou différents et représentent l'hydrogène, un groupe phényle ou un groupe alkyle en $C_1$ à $C_3$,

et leurs sels acceptables du point de vue pharmaceutique, à l'exception de la (3aS*, 6aR*)-6a-méthyl-3,3a,6,6a-tétrahydro-1H-cyclopénta[c]furanne-1-one.

**3.** Composés de formule (I) suivant la revendication 1 ou 2, formule dans laquelle

A désigne des restes de formules $-CH_2-$, -CO-, $-CR^4(OH)$-ou $-(CH_2)_a-CHR^5-$,
où

a représente le nombre 0, 1, 2 ou 3,
$R^4$ est l'hydrogène ou un groupe alkyle en $C_1$ à $C_3$,

et

$R^5$ représente le groupe phényle,

ou bien

un reste alcanediyle en $C_2$ à $C_4$, propènediyle ou alcynediyle en $C_2$ ou $C_3$,

$R^1$ est l'hydrogène, un groupe cyclopropyle ou cyclohexyle, ou un groupe benzofurannyle, benzothiophényle, benzimidazolyle, thiényle, quinazolyle ou quinoxalinyle,

ou bien

des restes de formules

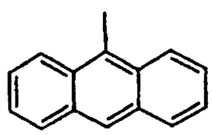 , 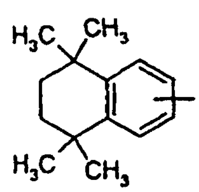 , 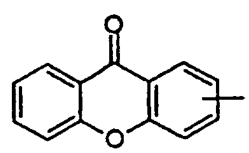 ,

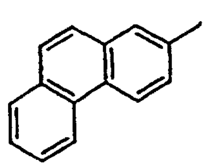 , 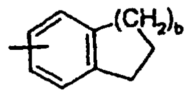 ou 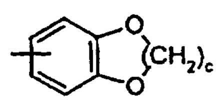

dans lesquelles

b et c sont identiques ou différents et représentent le nombre 1 ou 2,

ou bien

un groupe phényle ou naphtyle,

tous les noyaux indiqués ci-dessus étant éventuellement substitués une ou plusieurs fois identiques ou différentes par des substituants choisis dans le groupe fluor, chlore, brome, iode, cyano, nitro, trifluoro-méthyle, ou des substituants alkoxy en $C_1$ à $C_4$, (alkyle en $C_1$ à $C_4$)-carbonyloxy, cyclohexyle, phényle, phénoxy, pyridyle,

pyrimidyle, pyridazinyle ou benzyloxy, qui peuvent eux-mêmes être substitués jusqu'à trois fois identiques ou différentes par des radicaux cyano, fluoro, chloro, bromo ou iodo,

et/ou substitués par des restes alkyle en $C_1$ à $C_4$ ou alcényle en $C_2$ ou $C_3$ qui peuvent eux-mêmes être substitués par du chlore, du brome, de l'iode ou un radical phényle ou par des restes de formules $-OR^9$ ou $-NR^{10}R^{11}$ ou

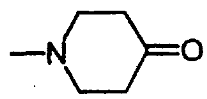

où

$R^9$ est l'hydrogène ou un groupe alkyle en $C_1$ à $C_3$,

et

$R^{10}$ et $R^{11}$ sont identiques ou différents et représentent l'hydrogène ou, un groupe phényle ou alkyle en $C_1$ à $C_3$, qui est éventuellement substitué par un radical phényle pouvant lui-même être substitué par du chlore, du brome, un radical hydroxy ou alkoxy en $C_1$ à $C_3$,

ou bien

$R^{10}$ et $R^{11}$ forment conjointement avec l'atome d'azote un reste de formule

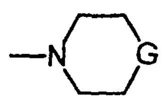

dans laquelle

G est un atome d'oxygène ou un reste de formule-$NR^{12}$,

dans laquelle

$R^{12}$ est l'hydrogène, un groupe phényle, benzyle, alkyle en $C_1$ à $C_3$, (alkoxy en $C_1$ à $C_3$)-carbonyle, pyridyle, pyrimidyle, pyridazinyle ou furyle, et/ou sont substitués par des groupes de formules $-CO_2-R^{13}$, $-NR^{14}R^{15}$, $-NR^{16}CO-R^{17}$, $-NR^{18}CO_2-R^{19}$ et $-CO-NR^{20}R^{21}$,

dans lesquelles,

$R^{13}$ est l'hydrogène, ou

représente un groupe alkyle en $C_1$ à $C_6$ ou un groupe allyle qui peuvent eux-mêmes être substitués par des restes de formules

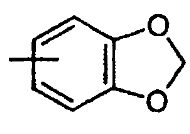 , 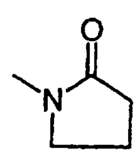 ,

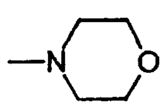 , 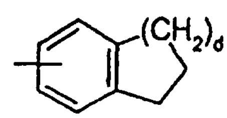

ou

par des restes phényle, naphtyle ou pyridyle,
formules dans lesquelles

d désigne le nombre 1 ou 2,

ou désigne
un groupe phényle qui est éventuellement substitué par un reste phényle qui peut lui-même porter un substituant cyano, chloro ou bromo,

$R^{14}$ et $R^{15}$ sont identiques ou différents et représentent l'hydrogène, un groupe cyclohexyle, phényle ou alkyle en $C_1$ à $C_4$ qui est éventuellement substitué par un radical cyclopropyle, cyclohexyle ou phényle pouvant lui-même être substitué une ou plusieurs fois identiques ou différentes par du chlore ou un radical alkoxy en $C_1$ à $C_3$,
$R^{16}$ est l'hydrogène, un groupe méthyle ou éthyle,
$R^{17}$ est l'hydrogène, un groupe adamantyle, cyclopentyle ou cyclohexyle, ou désigne un groupe alcényle en C2 ou $C_3$ ou un groupe alkyle en $C_1$ à $C_8$ qui est éventuellement substitué par un radical adamantyle, cyclopropyle, cyclopentyle, cyclohexyle, phényle, phénoxy, thiényle ou furyle, les noyaux pouvant eux-mêmes être substitués une ou plusieurs fois, identiques ou différentes, par les radicaux alkyle en $C_1$ à $C_3$, alkoxy en $C_1$ à $C_3$, hydroxy, nitro, fluoro, chloro ou bromo, et/ou alkyle éventuellement substitué par un reste de formule

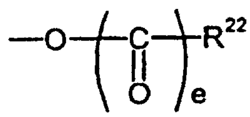

dans laquelle

e représente le nombre 0 ou 1 et
$R^{22}$ est un groupe alkyle en $C_1$ à $C_3$, un groupe phényle ou un groupe naphtyle, qui sont éventuellement substitués une ou plusieurs fois identiques ou différentes par du fluor, du chlore, du brome, des radicaux nitro, hydroxy ou alkoxy en $C_1$ à $C_3$,

ou désigne
des restes phényle, naphtyle, thiényle ou furyle qui peuvent eux-mêmes être substitués le cas échéant une ou plusieurs fois identiques ou différentes par des radicaux alkoxy en $C_1$ à $C_3$, alkyle en $C_1$ à $C_3$, nitro, fluoro, chloro ou bromo,
ou désigne
un reste de formule

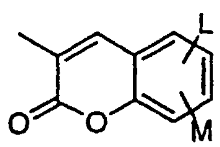

ou $-NR^{23}R^{24}$,
formule dans laquelle
L et M sont identiques ou différents et représentent l'hydrogène, le fluor ou le chlore, $R^{23}$ et $R^{24}$ ont les définitions indiquées ci-dessus pour $R^{10}$ et $R^{11}$ et y sont identiques ou en sont différents,

$R^{18}$ a la définition indiquée ci-dessus pour $R^{16}$ et y est identique ou en est différent,
$R^{19}$ désigne un reste alkyle en $C_1$ à $C_4$ ou un reste alcényle en $C_3$ à $C_5$ qui sont eux-mêmes éventuellement substitués par des substituants choisis dans le groupe chlore, phényle, hydroxy, morpholinyle, cyclopropyle, cyclohexyle, et par un groupe de formule $-SiR^{25}R^{26}R^{27}$,
où
$R^{25}$, $R^{26}$ et $R^{27}$ sont identiques ou différents et désignent des groupes méthyle,

$R^{20}$ et $R^{21}$ sont identiques ou différents et représentent l'hydrogène, des groupes adamantyle, cyclopropyle, cyclopentyle, cyclohexyle, phényle, phényle à substituant phénoxy, thiazolyle ou pyrryle, ou désignent

des restes alcényle en $C_2$ ou $C_3$, alkyle en $C_1$ à $C_7$ ou alcynyle en $C_3$ à $C_5$ qui sont éventuellement substitués par des radicaux hydroxy, cyclopropyle, cyclopentyle, cyclohexyle, alkoxy en $C_1$ à $C_3$, hydroxy, trifluorométhyle, phényle, pyridyle, furyle, thiényle ou pyrryle, les noyaux étant éventuellement substitués jusqu'à deux fois identiques ou différentes par des radicaux alkoxy en $C_1$ à $C_3$, (alkoxy en $C_1$ à $C_6$)-carbonyle, fluoro, chloro, bromo, phénoxy, hydroxy ou alkyle en $C_1$ à $C_3$,

et/ou le groupe alkyle indiqué pour $R^{20}/R^{21}$ est éventuellement substitué par des restes de formules $-SCF_3$,

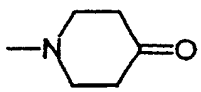
,
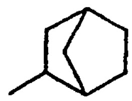
,

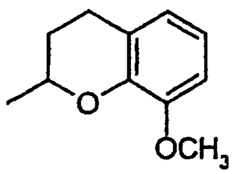

ou $-NR^{28}R^{29}$,

dans lesquelles

$R^{28}$ et $R^{29}$ sont identiques ou différents et représentent l'hydrogène ou des groupes alkyle en $C_1$ à $C_3$,

ou bien

$R^{20}$ ou $R^{21}$ représente un reste de formule

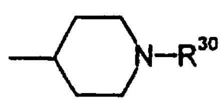

dans laquelle

$R^{30}$ a la définition indiquée ci-dessus pour $R^{12}$ et y est identique ou en est différent,

ou bien,

$R^{20}$ et $R^{21}$ forment conjointement avec l'atome d'azote un reste de formule

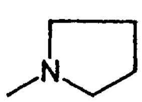
ou
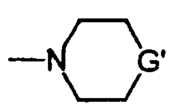

dans laquelle

G' a la définition indiquée ci-dessus pour G et y est identique ou en est différent,

$R^2$ et $R^3$ sont identiques ou différents et représentent l'hydrogène ou des groupes méthyle,

et

D et E forment ensemble des restes de formules

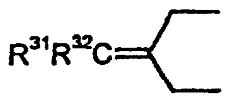

,

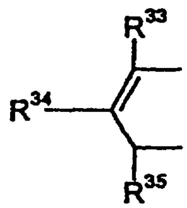

ou

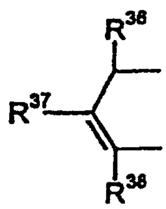

dans lesquelles
$R^{31}$, $R^{32}$, $R^{33}$, $R^{34}$, $R^{35}$, $R^{36}$, $R^{37}$ et $R^{38}$ sont identiques ou différents et représentent l'hydrogène ou des groupes méthyle,

et leurs sels acceptables du point de vue pharmaceutique, à l'exception de la (3aS*, 6aR*)-6a-méthyl-3,3a,6,6a-tétrahydro-lH-cyclopénta[c]furanne-1-one.

**4.** Composés de formule générale (I) suivant l'une quelconque des revendications 1 à 3, dans lesquels

A représente le groupe $-CH_2-$,

à l'exception de la (3aS*, 6aR*)-6a-méthyl-3,3a,6,6a-tétrahydro-1H-cyclopénta[c]furanne-1-one.

**5.** Composés de formule (I) suivant les revendications 1 à 4, formule dans laquelle

A est le groupe $-CH_2-$,
$R^1$ est un groupe phényle ou naphtyle,
tous les noyaux indiqués ci-dessus étant éventuellement substitués une ou plusieurs fois identiques ou différentes par des substituants choisis dans le groupe fluor, chlore, brome, iode, cyano, nitro, trifluorométhyle, ou alkoxy en $C_1$ à $C_4$, et/ou substitués par des groupes alkyle en $C_1$ à $C_4$,
et/ou sont substitués par des groupes de formules $-NR^{16}CO-R^{17}$, $-NR^{18}CO_2-R^{19}$ et $-CO-NR^{20}R^{21}$, dans lesquelles,
$R^{16}$ désigne l'hydrogène,
$R^{17}$ un groupe alkyle en $C_1$ à $C_8$ qui est éventuellement substitué par un radical cyclopropyle, cyclopentyle, cyclohexyle, phényle, thiényle ou furyle, les noyaux pouvant eux-mêmes être substitués une ou plusieurs fois, identiques ou différentes, par des radicaux alkyle en $C_1$ à $C_3$, alkoxy en $C_1$ à $C_3$, hydroxy, nitro, fluoro, chloro ou bromo,
$R^{18}$ a la définition indiquée ci-dessus pour $R^{16}$ et y est identique ou en est différent,
$R^{19}$ désigne un groupe alkyle en $C_1$ à $C_4$ ou un groupe alcényle en $C_3$ à $C_5$,
$R^{20}$ et $R^{21}$ sont identiques ou différents et représentent l'hydrogène, des groupes alcényle en $C_2$ ou $C_3$, alkyle en $C_1$ à $C_7$, alcynyle en $C_3$ à $C_5$, qui sont éventuellement substitués par des restes phényle, pyridyle, furyle, thiényle ou pyrryle, les noyaux étant éventuellement substitués jusqu'à deux fois identiques ou différentes par des radicaux alkoxy en $C_1$ à $C_3$, fluoro, chloro, bromo ou alkyle en $C_1$ à $C_3$,
$R^2$ et $R^3$ représentent l'hydrogène ou des groupes méthyle, et
D et E forment ensemble des restes de formules

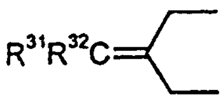 ou 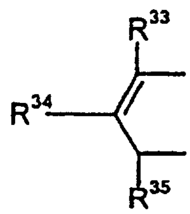

dans lesquelles
$R^{31}$, $R^{32}$, $R^{33}$, $R^{34}$, $R^{35}$ représentent l'hydrogène,

et leurs sels acceptables du point de vue pharmaceutique.

**6.** Composés suivant l'une quelconque des revendications 1 à 3, choisis dans le groupe comprenant :

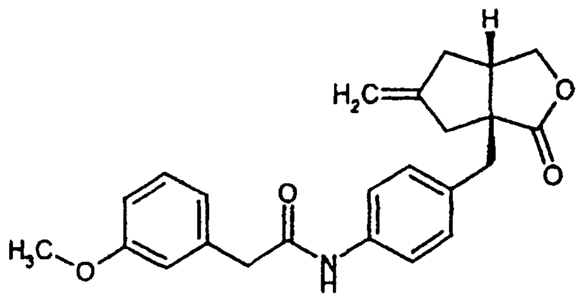

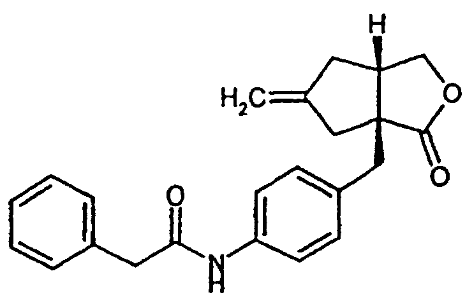

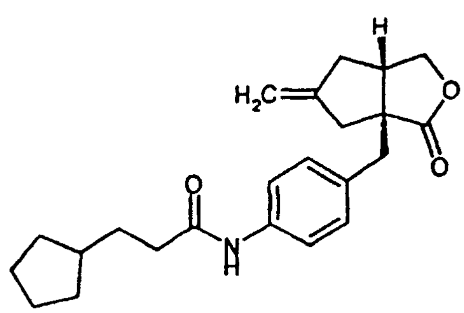

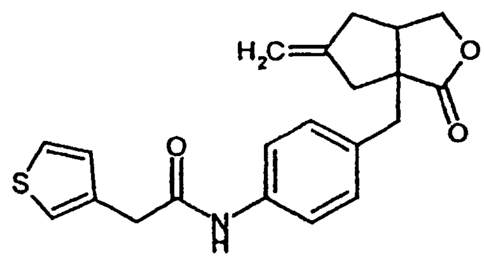

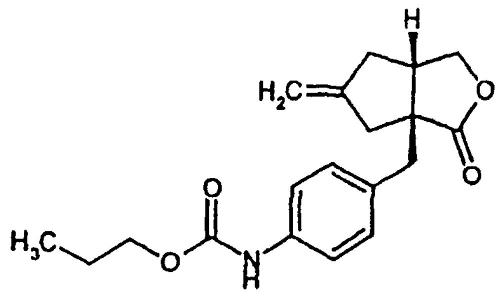

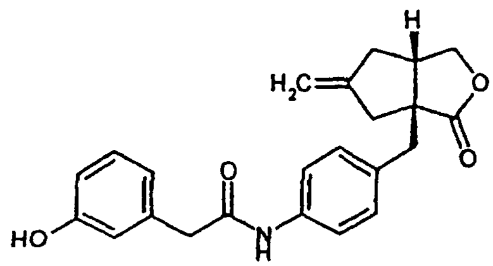

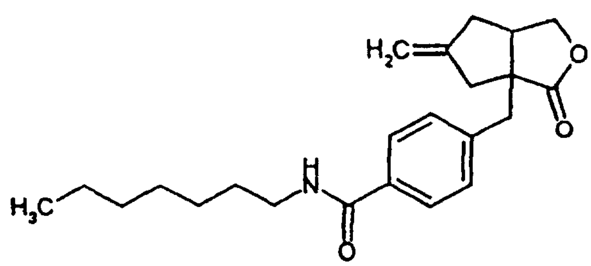

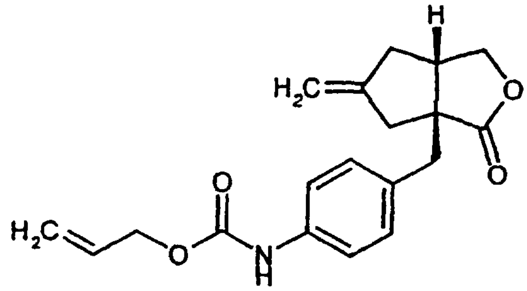

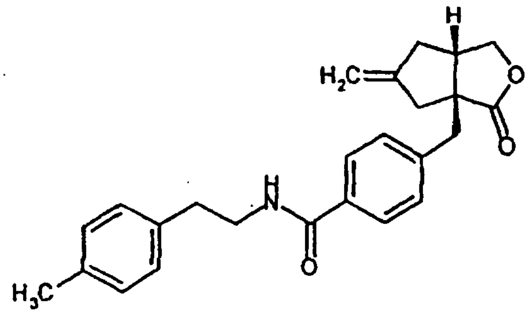

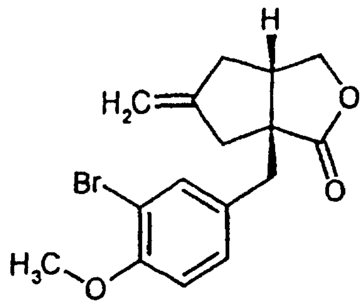

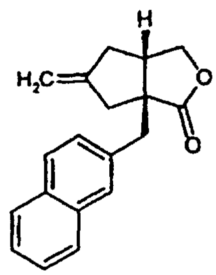

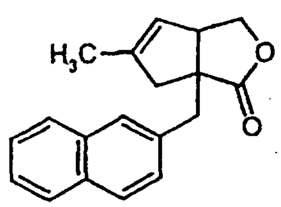

ces composés pouvant exister sous forme de racémate ou d'énantiomère pur.

**7.** Procédé de production de composés de formule (I) suivant l'une quelconque des revendications 1 à 6, dans lequel : on fait réagir des composés de formule générale (II)

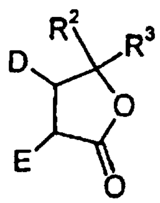

(II)

dans laquelle
D, E, $R^2$ et $R^3$ ont la définition indiquée ci-dessus,
avec des composés de formule générale (III),

$$T\text{-}A\text{-}R^1 \qquad (III)$$

dans laquelle

T représente un halogène, de préférence le brome,

et

A et $R^1$ ont la définition indiquée ci-dessus,

dans des solvants inertes et en présence d'une base,
et le cas échéant, on transforme en dérivé le substituant $R^1$ selon des modes opératoires classiques.

**8.** Procédé de production des composés de l'invention de formule générale (I') selon l'une des revendications 1 à 6

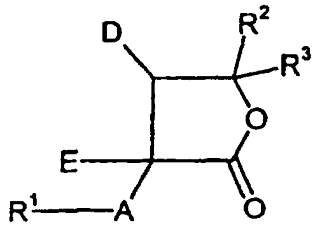

(I'),

dans laquelle
A, $R^1$, $R^2$ et $R^3$ ont la définition indiquée ci-dessus,
et
D et E forment conjointement des restes de formules

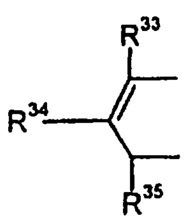

ou

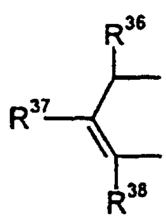

dans lesquelles

$R^{33}$, $R^{35}$, $R^{36}$ et $R^{38}$ représentent l'hydrogène, et
$R^{34}$ et $R^{37}$ ont la définition indiquée ci-dessus,
**caractérisé en ce qu'**on isomérise un composé de formule générale (I")

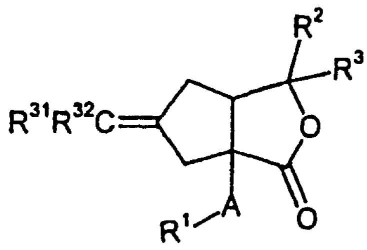

(I"),

dans laquelle
A, $R^1$, $R^2$, $R^3$, $R^{31}$ et $R^{32}$ ont la définition indiquée ci-dessus,
en présence d'un catalyseur et en présence éventuelle d'un solvant.

**9.** Composés de formule générale (II),

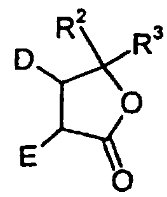

(II)

dans laquelle
$R^2$ et $R^3$ sont identiques ou différents et représentent l'hydrogène ou un groupe alkyle en $C_1$ à $C_6$,
et
D et E forment conjointement des restes de formules

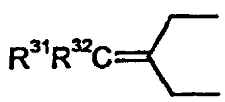

,

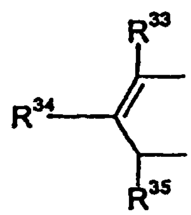

ou

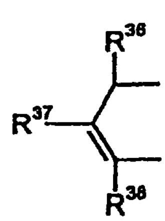

dans lesquelles
$R^{31}$, $R^{32}$, $R^{33}$, $R^{34}$, $R^{35}$, $R^{36}$, $R^{37}$ et $R^{38}$ sont identiques ou différents et représentent l'hydrogène, des groupes phényle ou des groupes alkyle en $C_1$ à $C_6$,

à l'exception des (3aS*, 6aR*)-, (3aS*, 6S*, 6aR*)-6-méthyl- et (3aS*, 6R*, 6aR*)-6-méthyl-3,3a,6,6a-tétrahydro-1H-cyclopénta[c]furanne-1-ones.

**10.** Composés de formule générale (II) suivant la revendication 9, dans laquelle

$R^2$ et $R^3$ représentent l'hydrogène ou un groupe méthyle,

et

D et E forment conjointement des restes de formules

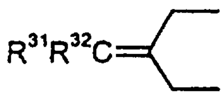

ou

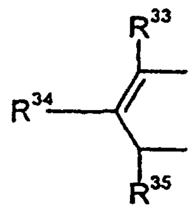

dans lesquelles

$R^{31}$, $R^{32}$, $R^{33}$, $R^{34}$ et $R^{35}$ représentent l'hydrogène,

à l'exception des (3aS*, 6aR*)-, (3aS*, 6S*, 6aR*)-6-méthyl- et (3aS*, 6R*, 6aR*)-6-méthyl-3,3a,6,6a-tétrahydro-1H-cyclopénta[c]furanne-1-ones.

**11.** Composés suivant les revendications 1 à 6 ou la (3aS*, 6aR*)-6a-méthyl-3,3a,6,6a-tétrahydro-1H-cyclopénta[c]furanne-1-one destinés à être utilisés comme médicament dans le traitement d'êtres humains et d'animaux.

**12.** Compositions pharmaceutiques qui comprennent comme composant actif au moins un composé suivant les revendications 1 à 6 ou la (3aS*, 6aR*)-6a-méthyl-3,3a,6,6a-tétrahydro-lH-cyclopénta[c]furanne-1-one en association avec au moins un support ou excipient principalement non toxique, acceptable du point de vue pharmaceutique.

**13.** Utilisation des composés suivant les revendications 1 à 6 ou de la (3aS*, 6aR*)-6a-méthyl-3,3a,6,6a-tétrahydro-1H-cyclopénta[c]furanne-1-one pour la préparation d'un médicament destiné au traitement préventif et/ou curatif de maladies qui sont déclenchées par une hyperfonction ou une hypofonction du système glutamatergène.

**14.** Utilisation des composés suivant les revendications 1 à 6 ou de la (3aS*, 6aR*)-6a-méthyl-3,3a,6,6a-tétrahydro-1H-cyclopénta[c]furanne-1-one pour la préparation d'un médicament destiné au traitement préventif et/ou curatif d'ischémies cérébrales, de traumatismes cranio-cérébraux, d'états douloureux ou de spasmes sous la médiation du système nerveux central.

**15.** Utilisation de composés suivant les revendications 1 à 6 pour la préparation d'un médicament destiné au traitement préventif et/ou curatif d'ischémies cérébrales, de traumatismes cranio-cérébraux, d'états douloureux ou de spasmes sous la médiation du système nerveux central.